(19) Europäisches Patentamt
European Patent Office
Office européen des brevets

(11) EP 2 546 336 A1

# (12) EUROPEAN PATENT APPLICATION

(43) Date of publication:
**16.01.2013 Bulletin 2013/03**

(21) Application number: **11173470.3**

(22) Date of filing: **11.07.2011**

(51) Int Cl.:
***C12N 9/02*** *(2006.01)*
***C12N 9/12*** *(2006.01)*
***C12N 9/90*** *(2006.01)*
***C12P 1/02*** *(2006.01)*
***C12P 7/06*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **DSM IP Assets B.V.**
**6411 TE Heerlen (NL)**

(72) Inventors:
- **De Bont, Johannes Adrianus Maria**
  **6707 AK Wageningen (NL)**
- **Teunissen, Aloysius Wilhelmus Rudolphus Hubertus**
  **3052 HP Rotterdam (NL)**

(74) Representative: **Swinkels, Bart Willem**
**Nederlandsch Octrooibureau**
**J. W. Frisolaan 13**
**2517 JS Den Haag (NL)**

## (54) Yeast strains that consume uronic acids and produce fermentation products such as ethanol

(57) The present invention relates to yeast strains that have been engineered to produce fermentation products from uronic acids such as galacturonic acid or glucuronic acid. For this purpose the yeast strain have been modified by introduction of at least genes coding for a uronokinase, a UTP-monosaccharide-1-phosphate uridylyltransferase and a UDP-glucose 6-dehydrogenase. The invention further relates to the use of such engineered yeast strains for the production of fermentation products and to process for the production of a fermentation product. The process comprises the steps of a) fermenting a medium with a yeast cell of the invention, whereby the medium contains or is fed with a source of a uronic acid and whereby the yeast cell ferments the uronic acid to the fermentation product; and optionally, b) recovery of the fermentation product. A preferred fermentation product is ethanol.



Printed by Jouve, 75001 PARIS (FR)

SEQUENCE LISTING

<110> C5 Yeast Company B.V.

<120> Yeast strains that ferment uronic acids via a UDP-glucose-6 dehydrogenase

<130> P6035530EP

<160> 60

<170> PatentIn version 3.3

<210> 1
<211> 424
<212> PRT
<213> Arabidopsis thaliana

<400> 1

```
Met Ser Trp Pro Thr Asp Ser Glu Leu Asn Ser Ile Lys Glu Ala Val
1               5                   10                  15

Ala Gln Met Ser Gly Arg Asp Lys Gly Glu Val Arg Val Val Val Ala
            20                  25                  30

Pro Tyr Arg Ile Cys Pro Leu Gly Ala His Ile Asp His Gln Gly Gly
        35                  40                  45

Thr Val Ser Ala Met Thr Ile Asn Lys Gly Ile Leu Leu Gly Phe Val
    50                  55                  60

Pro Ser Gly Asp Thr Gln Val Gln Leu Arg Ser Ala Gln Phe Glu Gly
65                  70                  75                  80

Glu Val Cys Phe Arg Val Asp Glu Ile Gln His Pro Ile Gly Leu Ala
                85                  90                  95

Asn Lys Asn Gly Ala Ser Thr Pro Ser Pro Ser Lys Glu Lys Ser Ile
            100                 105                 110

Trp Gly Thr Tyr Ala Arg Gly Ala Val Tyr Ala Leu Gln Ser Ser Lys
        115                 120                 125

Lys Asn Leu Lys Gln Gly Ile Ile Gly Tyr Leu Ser Gly Ser Asn Gly
    130                 135                 140

Leu Asp Ser Ser Gly Leu Ser Ser Ser Ala Ala Val Gly Val Ala Tyr
145                 150                 155                 160

Leu Leu Ala Leu Glu Asn Ala Asn Glu Leu Thr Val Ser Pro Thr Glu
                165                 170                 175

Asn Ile Glu Tyr Asp Arg Leu Ile Glu Asn Gly Tyr Leu Gly Leu Arg
```

180 185 190

Asn Gly Ile Leu Asp Gln Ser Ala Ile Leu Leu Ser Asn Tyr Gly Cys
195 200 205

Leu Thr Tyr Met Asp Cys Lys Thr Leu Asp His Glu Leu Val Gln Ala
210 215 220

Pro Glu Leu Glu Lys Pro Phe Arg Ile Leu Leu Ala Phe Ser Gly Leu
225 230 235 240

Arg Gln Ala Leu Thr Thr Asn Pro Gly Phe Asn Leu Arg Val Ser Glu
245 250 255

Cys Gln Glu Ala Ala Lys Val Leu Leu Thr Ala Ser Gly Asn Ser Glu
260 265 270

Leu Glu Pro Thr Leu Cys Asn Val Glu His Ala Val Tyr Glu Ala His
275 280 285

Lys His Glu Leu Lys Pro Val Leu Ala Lys Arg Ala Glu His Tyr Phe
290 295 300

Ser Glu Asn Met Arg Val Ile Lys Gly Arg Glu Ala Trp Ala Ser Gly
305 310 315 320

Asn Leu Glu Glu Phe Gly Lys Leu Ile Ser Ala Ser Gly Leu Ser Ser
325 330 335

Ile Glu Asn Tyr Glu Cys Gly Ala Glu Pro Leu Ile Gln Leu Tyr Lys
340 345 350

Ile Leu Leu Lys Ala Pro Gly Val Tyr Gly Ala Arg Phe Ser Gly Ala
355 360 365

Gly Phe Arg Gly Cys Cys Leu Ala Phe Val Asp Ala Glu Lys Ala Glu
370 375 380

Ala Ala Ala Ser Tyr Val Lys Asp Glu Tyr Glu Lys Ala Gln Pro Glu
385 390 395 400

Phe Ala Asn Asn Leu Asn Gly Gly Lys Pro Val Leu Ile Cys Glu Ala
405 410 415

Gly Asp Ala Ala Arg Val Leu Leu
420

<210> 2

<211> 362
<212> PRT
<213> Arabidopsis thaliana

<400> 2

Met Asp Pro Asn Ser Thr Val Ser Gly Asp Gly Gln Ala Thr Ala Ala
1 5 10 15

Ile Glu His Arg Ser Phe Ala Arg Ile Gly Phe Leu Gly Asn Pro Ser
20 25 30

Asp Val Tyr Phe Gly Arg Thr Ile Ser Leu Thr Ile Gly Asn Phe Trp
35 40 45

Ala Ser Val Lys Leu Glu Pro Ser Glu His Leu Val Ile Lys Pro His
50 55 60

Pro Phe His Asp Leu Val Gln Phe Thr Ser Leu Asp His Leu Leu Asn
65 70 75 80

Arg Leu Gln Asn Glu Gly Tyr Tyr Gly Gly Val Arg Leu Leu Met Ala
85 90 95

Ile Cys Lys Val Phe Arg Asn Tyr Cys Lys Glu Asn Asp Ile Gln Leu
100 105 110

His Gln Ala Asn Phe Ser Leu Ser Tyr Asp Thr Asn Ile Pro Arg Gln
115 120 125

Thr Gly Leu Ser Gly Ser Ser Ala Ile Val Ser Ala Ala Leu Asn Cys
130 135 140

Leu Leu Asp Phe Tyr Asn Val Arg His Leu Ile Lys Val Gln Val Arg
145 150 155 160

Pro Asn Ile Val Leu Ser Ala Glu Lys Glu Leu Gly Ile Val Ala Gly
165 170 175

Leu Gln Asp Arg Val Ala Gln Val Tyr Gly Gly Leu Val His Met Asp
180 185 190

Phe Ser Lys Glu His Met Asp Lys Leu Gly His Gly Ile Tyr Thr Pro
195 200 205

Met Asp Ile Ser Leu Leu Pro Pro Leu His Leu Ile Tyr Ala Glu Asn
210 215 220

Pro Ser Asp Ser Gly Lys Val His Ser Met Val Arg Gln Arg Trp Leu
225 230 235 240

Asp Gly Asp Glu Phe Ile Ile Ser Ser Met Lys Glu Val Gly Ser Leu
245 250 255

Ala Glu Glu Gly Arg Thr Ala Leu Leu Asn Lys Asp His Ser Lys Leu
260 265 270

Val Glu Leu Met Asn Leu Asn Phe Asp Ile Arg Arg Arg Met Phe Gly
275 280 285

Asp Glu Cys Leu Gly Ala Met Asn Ile Glu Met Val Glu Val Ala Arg
290 295 300

Arg Val Gly Ala Ala Ser Lys Phe Thr Gly Ser Gly Gly Ala Val Val
305 310 315 320

Val Phe Cys Pro Glu Gly Pro Ser Gln Val Lys Leu Leu Glu Glu Glu
325 330 335

Cys Arg Lys Ala Gly Phe Thr Leu Gln Pro Val Lys Ile Ala Pro Ser
340 345 350

Cys Leu Asn Asp Ser Asp Ile Gln Thr Leu
355 360

<210> 3
<211> 600
<212> PRT
<213> Pisum sativum

<400> 3

Met Ala Ser Ser Leu Gly Asp Asn Phe Asn Leu Leu Ser Pro Gln Gln
1 5 10 15

Arg Glu Leu Val Lys Met Leu Leu Asp Asn Gly Gln Asp His Leu Phe
20 25 30

Arg Asp Trp Pro Asn Pro Gly Val Asp Asp Asp Glu Lys Lys Ala Phe
35 40 45

Phe Asp Gln Leu Val Leu Leu Asp Ser Ser Tyr Pro Gly Gly Leu Val
50 55 60

Ala Tyr Ile Asn Asn Ala Lys Arg Leu Leu Ala Asp Ser Lys Ala Gly
65 70 75 80

Asn Asn Pro Phe Asp Gly Phe Thr Pro Ser Val Pro Thr Gly Glu Thr
85 90 95

Leu Lys Phe Gly Asp Glu Asn Phe Asn Lys Tyr Glu Glu Ala Gly Val

```
                100                 105                 110

Arg Glu Ala Arg Arg Ala Ala Phe Val Leu Val Ala Gly Gly Leu Gly
        115                 120                 125

Glu Arg Leu Gly Tyr Asn Gly Ile Lys Val Ala Leu Pro Ala Glu Thr
    130                 135                 140

Thr Thr Gly Thr Cys Phe Leu Gln His Tyr Ile Glu Ser Ile Leu Ala
145                 150                 155                 160

Leu Gln Glu Ala Ser Ser Glu Gly Glu Gly Gln Thr His Ile Pro Phe
                165                 170                 175

Val Ile Met Thr Ser Asp Asp Thr His Gly Arg Thr Leu Asp Leu Leu
                180                 185                 190

Glu Ser Asn Ser Tyr Phe Gly Met Gln Pro Thr Gln Val Thr Leu Leu
        195                 200                 205

Lys Gln Glu Lys Val Ala Cys Leu Glu Asp Asn Asp Ala Arg Leu Ala
    210                 215                 220

Leu Asp Pro Gln Asn Arg Tyr Arg Val Gln Thr Lys Pro His Gly His
225                 230                 235                 240

Gly Asp Val His Ser Leu Leu His Ser Ser Gly Ile Leu Lys Val Trp
                245                 250                 255

Tyr Asn Ala Gly Leu Lys Trp Val Leu Phe Phe Gln Asp Thr Asn Gly
                260                 265                 270

Leu Leu Phe Lys Ala Ile Pro Ser Ala Leu Gly Val Ser Ser Thr Lys
        275                 280                 285

Gln Tyr His Val Asn Ser Leu Ala Val Pro Arg Lys Ala Lys Glu Ala
    290                 295                 300

Ile Gly Gly Ile Thr Arg Leu Thr His Ser Asp Gly Arg Ser Met Val
305                 310                 315                 320

Ile Asn Val Glu Tyr Asn Gln Leu Asp Pro Leu Leu Arg Ala Ser Gly
                325                 330                 335

Tyr Pro Asp Gly Asp Val Asn Ser Glu Thr Gly Tyr Ser Pro Phe Pro
                340                 345                 350

Gly Asn Ile Asn Gln Leu Ile Leu Glu Leu Gly Pro Tyr Ile Glu Glu
```

355 360 365

Leu Ala Lys Thr Gly Gly Ala Ile Gln Glu Phe Val Asn Pro Lys Tyr
370 375 380

Lys Asp Ala Ser Lys Thr Ser Phe Lys Ser Ser Thr Arg Leu Glu Cys
385 390 395 400

Met Met Gln Asp Tyr Pro Lys Thr Leu Pro Pro Ser Ser Arg Val Gly
405 410 415

Phe Thr Val Met Glu Thr Trp Phe Ala Tyr Ala Pro Val Lys Asn Asn
420 425 430

Ala Glu Asp Ala Ala Lys Val Pro Lys Gly Asn Pro Tyr His Ser Ala
435 440 445

Thr Ser Gly Glu Met Ala Ile Tyr Arg Ala Asn Ser Leu Ile Leu Lys
450 455 460

Lys Ala Gly Phe Gln Val Ala Asp Pro Val Leu Gln Val Ile Asn Gly
465 470 475 480

Gln Glu Val Glu Val Trp Pro Arg Ile Thr Trp Lys Pro Lys Trp Gly
485 490 495

Leu Thr Phe Ser Leu Val Lys Ser Lys Val Ser Gly Asn Cys Ser Ile
500 505 510

Ser Gln Arg Ser Thr Leu Ala Ile Lys Gly Arg Lys Ile Phe Ile Glu
515 520 525

Asn Leu Ser Val Asp Gly Ala Leu Ile Val Asp Ala Val Asp Asp Ala
530 535 540

Glu Val Asn Val Ser Gly Ser Val Gln Asn Asn Gly Trp Ala Leu Glu
545 550 555 560

Pro Val Asp Tyr Lys Asp Ser Ser Glu Pro Glu Val Leu Arg Ile Arg
565 570 575

Gly Phe Lys Phe Asn Lys Val Glu Gln Val Glu Lys Lys Tyr Ser Glu
580 585 590

Pro Gly Lys Phe Asp Phe Lys Ala
595 600

<210> 4

<211> 614
<212> PRT
<213> Arabidopsis thaliana

<400> 4

Met Ala Ser Thr Val Asp Ser Asn Phe Phe Ser Ser Val Pro Ala Leu
1 5 10 15

His Ser Asn Leu Gly Leu Leu Ser Pro Asp Gln Ile Glu Leu Ala Lys
20 25 30

Ile Leu Leu Glu Asn Gly Gln Ser His Leu Phe Gln Gln Trp Pro Glu
35 40 45

Leu Gly Val Asp Asp Lys Glu Lys Leu Ala Phe Phe Asp Gln Ile Ala
50 55 60

Arg Leu Asn Ser Ser Tyr Pro Gly Gly Leu Ala Ala Tyr Ile Lys Thr
65 70 75 80

Ala Lys Glu Leu Leu Ala Asp Ser Lys Val Gly Lys Asn Pro Tyr Asp
85 90 95

Gly Phe Ser Pro Ser Val Pro Ser Gly Glu Asn Leu Thr Phe Gly Thr
100 105 110

Asp Asn Phe Ile Glu Met Glu Lys Arg Gly Val Val Glu Ala Arg Asn
115 120 125

Ala Ala Phe Val Leu Val Ala Gly Gly Leu Gly Glu Arg Leu Gly Tyr
130 135 140

Asn Gly Ile Lys Val Ala Leu Pro Arg Glu Thr Thr Thr Gly Thr Cys
145 150 155 160

Phe Leu Gln His Tyr Ile Glu Ser Ile Leu Ala Leu Gln Glu Ala Ser
165 170 175

Asn Lys Ile Asp Ser Asp Gly Ser Glu Arg Asp Ile Pro Phe Ile Ile
180 185 190

Met Thr Ser Asp Asp Thr His Ser Arg Thr Leu Asp Leu Leu Glu Leu
195 200 205

Asn Ser Tyr Phe Gly Met Lys Pro Thr Gln Val His Leu Leu Lys Gln
210 215 220

Glu Lys Val Ala Cys Leu Asp Asp Asn Asp Ala Arg Leu Ala Leu Asp
225 230 235 240

Pro His Asn Lys Tyr Ser Ile Gln Thr Lys Pro His Gly His Gly Asp
245 250 255

Val His Ser Leu Leu Tyr Ser Ser Gly Leu Leu His Lys Trp Leu Glu
260 265 270

Ala Gly Leu Lys Trp Val Leu Phe Phe Gln Asp Thr Asn Gly Leu Leu
275 280 285

Phe Asn Ala Ile Pro Ala Ser Leu Gly Val Ser Ala Thr Lys Gln Tyr
290 295 300

His Val Asn Ser Leu Ala Val Pro Arg Lys Ala Lys Glu Ala Ile Gly
305 310 315 320

Gly Ile Ser Lys Leu Thr His Val Asp Gly Arg Ser Met Val Ile Asn
325 330 335

Val Glu Tyr Asn Gln Leu Asp Pro Leu Leu Arg Ala Ser Gly Phe Pro
340 345 350

Asp Gly Asp Val Asn Cys Glu Thr Gly Phe Ser Pro Phe Pro Gly Asn
355 360 365

Ile Asn Gln Leu Ile Leu Glu Leu Gly Pro Tyr Lys Asp Glu Leu Gln
370 375 380

Lys Thr Gly Gly Ala Ile Lys Glu Phe Val Asn Pro Lys Tyr Lys Asp
385 390 395 400

Ser Thr Lys Thr Ala Phe Lys Ser Ser Thr Arg Leu Glu Cys Met Met
405 410 415

Gln Asp Tyr Pro Lys Thr Leu Pro Pro Thr Ala Arg Val Gly Phe Thr
420 425 430

Val Met Asp Ile Trp Leu Ala Tyr Ala Pro Val Lys Asn Asn Pro Glu
435 440 445

Asp Ala Ala Lys Val Pro Lys Gly Asn Pro Tyr His Ser Ala Thr Ser
450 455 460

Gly Glu Met Ala Ile Tyr Arg Ala Asn Ser Leu Ile Leu Gln Lys Ala
465 470 475 480

Gly Val Lys Val Glu Glu Pro Val Lys Gln Val Leu Asn Gly Gln Glu
485 490 495

Val Glu Val Trp Ser Arg Ile Thr Trp Lys Pro Lys Trp Gly Met Ile
500 505 510

Phe Ser Asp Ile Lys Lys Lys Val Ser Gly Asn Cys Glu Val Ser Gln
515 520 525

Arg Ser Thr Met Ala Ile Lys Gly Arg Asn Val Phe Ile Lys Asp Leu
530 535 540

Ser Leu Asp Gly Ala Leu Ile Val Asp Ser Ile Asp Asp Ala Glu Val
545 550 555 560

Lys Leu Gly Gly Leu Ile Lys Asn Asn Gly Trp Thr Met Glu Ser Val
565 570 575

Asp Tyr Lys Asp Thr Ser Val Pro Glu Glu Ile Arg Ile Arg Gly Phe
580 585 590

Arg Phe Asn Lys Val Glu Gln Leu Glu Lys Lys Leu Thr Gln Pro Gly
595 600 605

Lys Phe Ser Val Glu Asp
610

<210> 5
<211> 453
<212> PRT
<213> Pseudomonas aeruginosa

<400> 5

Met Arg Leu Cys Val Ile Gly Ala Gly Tyr Val Gly Leu Val Thr Ala
1 5 10 15

Ala Cys Phe Ala Glu Met Gly Asn Gln Val Arg Cys Val Glu Arg Asp
20 25 30

Arg Glu Arg Val Ala Arg Leu Arg Arg Gly Glu Met Pro Ile Tyr Glu
35 40 45

Pro Gly Leu Glu Ser Ile Leu Arg Asp Gln Leu Asp Ala Ala Arg Leu
50 55 60

Thr Phe Thr Ala Ser Leu Ala Glu Gly Leu Ala Asp Ala Glu Val Val
65 70 75 80

Phe Ile Ala Val Gly Thr Pro Cys Gly Glu Asp Gly Ser Ala Asp Leu
85 90 95

Ser His Val Leu Ala Val Ala Glu Gln Leu Gly Ala Gln Leu Arg Gln

100 105 110

Ala Cys Ile Val Val Asn Lys Ser Thr Val Pro Val Gly Thr Ala Glu
115 120 125

Arg Val Glu Glu Ile Ile Arg Leu Gly Leu Ala Arg Arg Arg Lys Arg
130 135 140

Phe Arg Val Ala Val Ala Ser Asn Pro Glu Phe Leu Lys Glu Gly Ser
145 150 155 160

Ala Val Asp Asp Phe Arg Arg Pro Asp Arg Val Ile Ile Gly Ser Ala
165 170 175

Glu Thr Gln Ala Gly Glu Thr Leu Arg Gln Leu Tyr Ala Pro Phe Leu
180 185 190

Arg Asn His Glu Arg Val Leu Leu Met Gly Arg Arg Glu Ala Glu Phe
195 200 205

Ser Lys Tyr Ala Ala Asn Ala Phe Leu Ala Thr Lys Ile Ser Phe Met
210 215 220

Asn Glu Met Ala Gly Leu Cys Ala Leu Thr Gly Val Asp Ile Glu Asp
225 230 235 240

Val Arg Arg Gly Met Gly Ser Asp Lys Arg Ile Gly Thr His Phe Ile
245 250 255

Tyr Ala Gly Cys Gly Tyr Gly Gly Ser Cys Phe Pro Lys Asp Val Arg
260 265 270

Ala Leu Ile Arg Ser Ala Glu Gln Gln Gly Tyr Asp Ser Gln Ile Leu
275 280 285

Arg Ala Val Glu Ala Arg Asn Ala Arg Gln Lys Glu Leu Leu Phe Glu
290 295 300

Thr Leu Gly Glu Leu Phe Gln Gly Arg Trp Gln Gly Arg Thr Val Ala
305 310 315 320

Leu Trp Gly Leu Ala Phe Lys Pro Gly Thr Asp Asp Leu Arg Glu Ala
325 330 335

Pro Ser Leu Val Leu Leu Glu Ala Leu Leu Arg His Gly Val Arg Val
340 345 350

Arg Ala His Asp Pro Val Ala Asn Ala Gly Val Ala Ala Arg Tyr Pro

355 360 365

Glu Ala Val Ala Cys Ala Arg Leu Thr Leu His Asp Ser Pro Tyr Ala
370 375 380

Ala Val Glu Gly Ala Asp Ala Leu Val Leu Val Thr Glu Trp Lys Gln
385 390 395 400

Phe Arg Gln Pro Asp Phe Gln Lys Ile Arg Gly Ser Met Arg Thr Pro
405 410 415

Leu Leu Val Asp Gly Arg Asn Leu Tyr Ala Pro Ala Arg Met Ala Glu
420 425 430

Leu Gly Phe Ile Tyr Gln Gly Ile Gly Arg Pro Arg Ala Gly His Cys
435 440 445

Lys Ala Ser Ala Ala
450

<210> 6
<211> 464
<212> PRT
<213> Pseudomonas aeruginosa

<400> 6

Met Lys Ile Ser Val Phe Gly Ser Gly Tyr Val Gly Leu Val Gln Ala
1 5 10 15

Ala Val Leu Ala Glu Val Gly His Asp Val Leu Cys Met Asp Ile Asp
20 25 30

Arg Asn Lys Val Glu Arg Leu Ala Gln Gly Leu Ala Ser Ile Tyr Glu
35 40 45

Pro Gly Leu Asp Ala Leu Leu Arg Glu Gly Leu Asp Ser Gly Arg Leu
50 55 60

Arg Phe Ser Ser Asp Ala Arg Leu Ala Val Glu His Gly Arg Val Gln
65 70 75 80

Phe Ile Ala Val Gly Thr Pro Pro Gly Glu Asp Gly Ala Ala Asp Leu
85 90 95

Gly Ala Val Phe Val Val Ala Asp Ala Ile Ala Glu His Arg Arg Lys
100 105 110

Pro Val Ile Val Val Glu Lys Ser Thr Val Pro Val Gly Thr Gly Asp
115 120 125

```
Arg Leu Arg Ala His Ile Glu Arg Arg Leu Glu Ala Asp Gly Arg Glu
    130                 135                 140

Leu Glu Phe Glu Ile Val Ser Asn Pro Glu Phe Leu Lys Glu Gly Ser
145                 150                 155                 160

Ala Val Ala Asp Cys Arg Arg Pro Asp Arg Ile Val Val Gly Cys Asp
                165                 170                 175

Asn Glu Glu Val Arg Gln Val Met Arg Glu Leu Tyr Glu Pro Phe Asn
            180                 185                 190

Arg Asn His Asp Arg Met Leu Phe Met Gly Leu Arg Ser Ala Glu Leu
        195                 200                 205

Thr Lys Tyr Ala Ala Asn Gly Met Leu Ala Thr Lys Ile Ser Phe Ile
    210                 215                 220

Asn Gln Ile Ala Glu Leu Ala Glu His Leu Gly Ala Asp Ile Glu Ala
225                 230                 235                 240

Val Arg Gln Gly Ile Gly Ala Asp Pro Arg Ile Gly Tyr His Phe Ile
                245                 250                 255

Tyr Pro Gly Cys Gly Tyr Gly Gly Ser Cys Phe Pro Lys Asp Met Arg
            260                 265                 270

Ala Leu Ile His Ser Ala Glu Gln Ala His Cys Ser Ser Asp Leu Leu
        275                 280                 285

Gln Ala Val Glu Ala Ile Asn Arg Arg Gln Lys His Lys Leu Phe Glu
    290                 295                 300

Arg Ile Arg Val Phe Tyr Asp Gly Asp Leu Arg Gly Lys Thr Phe Ala
305                 310                 315                 320

Leu Trp Gly Leu Ala Phe Lys Pro Asn Thr Asp Asp Met Arg Asp Ala
                325                 330                 335

Pro Ser Arg Glu Leu Met Glu Ala Leu Trp Arg His Gly Ala Gln Val
            340                 345                 350

Arg Ala Tyr Asp Pro Glu Ala Met Gln Glu Thr Gln Arg Leu Tyr Gly
        355                 360                 365

His Asp Glu Arg Leu Ser Leu Met Gly Thr Pro Glu Ala Thr Leu Gly
    370                 375                 380
```

Gly Ala Asp Ala Leu Val Ile Cys Thr Glu Trp Gln Gln Phe Lys Ala
385 390 395 400

Pro Asp Phe Glu Leu Leu Lys Glu Arg Leu Lys Ala Pro Val Ile Phe
405 410 415

Asp Gly Arg Asn Leu Tyr Asp Pro Glu Arg Met Ala Arg His Gly Phe
420 425 430

His Tyr Tyr Pro Met Gly Arg Gly Gln Ser Cys Ser Leu Pro Ile Asn
435 440 445

Glu Ala Ser Leu Ala Gln Glu Asp Gly Met Arg Leu Leu Arg Gln Ala
450 455 460

<210> 7
<211> 429
<212> PRT
<213> Arabidopsis thaliana

<400> 7

Met Pro Ser Ile Glu Asp Glu Leu Phe Pro Ser Thr Pro Gly Lys Phe
1 5 10 15

Lys Ile Asp Arg Ser Asn Arg Gln Leu His Arg Cys Phe Ala Ser Thr
20 25 30

Ser Thr Met Phe Leu Trp Ala Leu Phe Leu Ile Ala Leu Thr Ala Ser
35 40 45

Tyr Leu Ser Phe Gln Ser Phe Val Asp Ser Gly Ser Arg Tyr Leu Thr
50 55 60

Ala Ser Trp Gly Gly Ile Gln Trp Glu Lys Gln Val Arg Thr Ser Ala
65 70 75 80

Gln Ile His Arg Ser Gly Gly Ile Ser Val Leu Val Thr Gly Ala Thr
85 90 95

Gly Phe Val Gly Ser His Val Ser Leu Ala Leu Arg Lys Arg Gly Asp
100 105 110

Gly Val Val Gly Leu Asp Asn Phe Asn Asn Tyr Tyr Asp Pro Ser Leu
115 120 125

Lys Arg Ala Arg Arg Ser Leu Leu Ser Ser Arg Gly Ile Phe Val Val
130 135 140

Glu Gly Asp Leu Asn Asp Ala Lys Leu Leu Ala Lys Leu Phe Asp Val

145 150 155 160

Val Ala Phe Thr His Val Met His Leu Ala Ala Gln Ala Gly Val Arg
165 170 175

Tyr Ala Leu Glu Asn Pro Gln Ser Tyr Val His Ser Asn Ile Ala Gly
180 185 190

Leu Val Asn Leu Leu Glu Ile Cys Lys Ala Ala Asn Pro Gln Pro Ala
195 200 205

Ile Val Trp Ala Ser Ser Ser Ser Val Tyr Gly Leu Asn Glu Lys Val
210 215 220

Pro Phe Ser Glu Ser Asp Arg Thr Asp Gln Pro Ala Ser Leu Tyr Ala
225 230 235 240

Ala Thr Lys Lys Ala Gly Glu Glu Ile Thr His Thr Tyr Asn His Ile
245 250 255

Tyr Gly Leu Ala Ile Thr Gly Leu Arg Phe Phe Thr Val Tyr Gly Pro
260 265 270

Trp Gly Arg Pro Asp Met Ala Tyr Phe Ser Phe Thr Arg Asn Ile Leu
275 280 285

Gln Gly Lys Pro Ile Thr Ile Tyr Arg Gly Lys Asn Arg Val Asp Leu
290 295 300

Ala Arg Asp Phe Thr Tyr Ile Asp Asp Ile Val Lys Gly Cys Leu Gly
305 310 315 320

Ser Leu Asp Ser Ser Gly Lys Ser Thr Gly Ser Gly Gly Lys Lys Arg
325 330 335

Gly Ala Ala Pro Tyr Arg Ile Phe Asn Leu Gly Asn Thr Ser Pro Val
340 345 350

Thr Val Pro Ile Leu Val Asp Ile Leu Glu Lys His Leu Lys Val Lys
355 360 365

Ala Lys Arg Asn Phe Val Glu Met Pro Gly Asn Gly Asp Val Pro Phe
370 375 380

Thr His Ala Asn Ile Ser Ser Ala Arg Asn Glu Phe Gly Tyr Lys Pro
385 390 395 400

Thr Thr Asp Leu Glu Thr Gly Leu Lys Lys Phe Val Arg Trp Tyr Leu

```
            405                 410                 415

Ser Tyr Tyr Gly Tyr Asn Thr Lys Ala Lys Leu Val His
            420                 425


<210>  8
<211>  366
<212>  PRT
<213>  Saccharomyces cerevisiae

<400>  8

Met Thr Ala Glu Glu Phe Asp Phe Ser Ser His Ser His Arg Arg Tyr
1               5                   10                  15

Asn Pro Leu Thr Asp Ser Trp Ile Leu Val Ser Pro His Arg Ala Lys
            20                  25                  30

Arg Pro Trp Leu Gly Gln Gln Glu Ala Ala Tyr Lys Pro Thr Ala Pro
        35                  40                  45

Leu Tyr Asp Pro Lys Cys Tyr Leu Cys Pro Gly Asn Lys Arg Ala Thr
    50                  55                  60

Gly Asn Leu Asn Pro Arg Tyr Glu Ser Thr Tyr Ile Phe Pro Asn Asp
65                  70                  75                  80

Tyr Ala Ala Val Arg Leu Asp Gln Pro Ile Leu Pro Gln Asn Asp Ser
                85                  90                  95

Asn Glu Asp Asn Leu Lys Asn Arg Leu Leu Lys Val Gln Ser Val Arg
            100                 105                 110

Gly Asn Cys Phe Val Ile Cys Phe Ser Pro Asn His Asn Leu Thr Ile
        115                 120                 125

Pro Gln Met Lys Gln Ser Asp Leu Val His Ile Val Asn Ser Trp Gln
    130                 135                 140

Ala Leu Thr Asp Asp Leu Ser Arg Glu Ala Arg Glu Asn His Lys Pro
145                 150                 155                 160

Phe Lys Tyr Val Gln Ile Phe Glu Asn Lys Gly Thr Ala Met Gly Cys
                165                 170                 175

Ser Asn Leu His Pro His Gly Gln Ala Trp Cys Leu Glu Ser Ile Pro
            180                 185                 190

Ser Glu Val Ser Gln Glu Leu Lys Ser Phe Asp Lys Tyr Lys Arg Glu
        195                 200                 205
```

His Asn Thr Asp Leu Phe Ala Asp Tyr Val Lys Leu Glu Ser Arg Glu
    210                 215                 220

Lys Ser Arg Val Val Val Glu Asn Glu Ser Phe Ile Val Val Val Pro
225                 230                 235                 240

Tyr Trp Ala Ile Trp Pro Phe Glu Thr Leu Val Ile Ser Lys Lys Lys
                245                 250                 255

Leu Ala Ser Ile Ser Gln Phe Asn Gln Met Val Lys Glu Asp Leu Ala
            260                 265                 270

Ser Ile Leu Lys Gln Leu Thr Ile Lys Tyr Asp Asn Leu Phe Glu Thr
        275                 280                 285

Ser Phe Pro Tyr Ser Met Gly Ile His Gln Ala Pro Leu Asn Ala Thr
    290                 295                 300

Gly Asp Glu Leu Ser Asn Ser Trp Phe His Met His Phe Tyr Pro Pro
305                 310                 315                 320

Leu Leu Arg Ser Ala Thr Val Arg Lys Phe Leu Val Gly Phe Glu Leu
                325                 330                 335

Leu Gly Glu Pro Gln Arg Asp Leu Thr Ser Glu Gln Ala Ala Glu Lys
            340                 345                 350

Leu Arg Asn Leu Asp Gly Gln Ile His Tyr Leu Gln Arg Leu
        355                 360                 365

<210> 9
<211> 699
<212> PRT
<213> Saccharomyces cerevisiae

<400> 9

Met Thr Ala Gln Leu Gln Ser Glu Ser Thr Ser Lys Ile Val Leu Val
1               5                   10                  15

Thr Gly Gly Ala Gly Tyr Ile Gly Ser His Thr Val Val Glu Leu Ile
            20                  25                  30

Glu Asn Gly Tyr Asp Cys Val Val Ala Asp Asn Leu Ser Asn Ser Thr
        35                  40                  45

Tyr Asp Ser Val Ala Arg Leu Glu Val Leu Thr Lys His His Ile Pro
    50                  55                  60

Phe Tyr Glu Val Asp Leu Cys Asp Arg Lys Gly Leu Glu Lys Val Phe

```
65                  70                  75                  80

Lys Glu Tyr Lys Ile Asp Ser Val Ile His Phe Ala Gly Leu Lys Ala
                85                  90                  95

Val Gly Glu Ser Thr Gln Ile Pro Leu Arg Tyr Tyr His Asn Asn Ile
            100                 105                 110

Leu Gly Thr Val Val Leu Leu Glu Leu Met Gln Gln Tyr Asn Val Ser
        115                 120                 125

Lys Phe Val Phe Ser Ser Ser Ala Thr Val Tyr Gly Asp Ala Thr Arg
    130                 135                 140

Phe Pro Asn Met Ile Pro Ile Pro Glu Glu Cys Pro Leu Gly Pro Thr
145                 150                 155                 160

Asn Pro Tyr Gly His Thr Lys Tyr Ala Ile Glu Asn Ile Leu Asn Asp
                165                 170                 175

Leu Tyr Asn Ser Asp Lys Lys Ser Trp Lys Phe Ala Ile Leu Arg Tyr
            180                 185                 190

Phe Asn Pro Ile Gly Ala His Pro Ser Gly Leu Ile Gly Glu Asp Pro
        195                 200                 205

Leu Gly Ile Pro Asn Asn Leu Leu Pro Tyr Met Ala Gln Val Ala Val
    210                 215                 220

Gly Arg Arg Glu Lys Leu Tyr Ile Phe Gly Asp Asp Tyr Asp Ser Arg
225                 230                 235                 240

Asp Gly Thr Pro Ile Arg Asp Tyr Ile His Val Val Asp Leu Ala Lys
                245                 250                 255

Gly His Ile Ala Ala Leu Gln Tyr Leu Glu Ala Tyr Asn Glu Asn Glu
            260                 265                 270

Gly Leu Cys Arg Glu Trp Asn Leu Gly Ser Gly Lys Gly Ser Thr Val
        275                 280                 285

Phe Glu Val Tyr His Ala Phe Cys Lys Ala Ser Gly Ile Asp Leu Pro
    290                 295                 300

Tyr Lys Val Thr Gly Arg Arg Ala Gly Asp Val Leu Asn Leu Thr Ala
305                 310                 315                 320

Lys Pro Asp Arg Ala Lys Arg Glu Leu Lys Trp Gln Thr Glu Leu Gln
```

325 330 335

Val Glu Asp Ser Cys Lys Asp Leu Trp Lys Trp Thr Thr Glu Asn Pro
340 345 350

Phe Gly Tyr Gln Leu Arg Gly Val Glu Ala Arg Phe Ser Ala Glu Asp
355 360 365

Met Arg Tyr Asp Ala Arg Phe Val Thr Ile Gly Ala Gly Thr Arg Phe
370 375 380

Gln Ala Thr Phe Ala Asn Leu Gly Ala Ser Ile Val Asp Leu Lys Val
385 390 395 400

Asn Gly Gln Ser Val Val Leu Gly Tyr Glu Asn Glu Glu Gly Tyr Leu
405 410 415

Asn Pro Asp Ser Ala Tyr Ile Gly Ala Thr Ile Gly Arg Tyr Ala Asn
420 425 430

Arg Ile Ser Lys Gly Lys Phe Ser Leu Cys Asn Lys Asp Tyr Gln Leu
435 440 445

Thr Val Asn Asn Gly Val Asn Ala Asn His Ser Ser Ile Gly Ser Phe
450 455 460

His Arg Lys Arg Phe Leu Gly Pro Ile Ile Gln Asn Pro Ser Lys Asp
465 470 475 480

Val Phe Thr Ala Glu Tyr Met Leu Ile Asp Asn Glu Lys Asp Thr Glu
485 490 495

Phe Pro Gly Asp Leu Leu Val Thr Ile Gln Tyr Thr Val Asn Val Ala
500 505 510

Gln Lys Ser Leu Glu Met Val Tyr Lys Gly Lys Leu Thr Ala Gly Glu
515 520 525

Ala Thr Pro Ile Asn Leu Thr Asn His Ser Tyr Phe Asn Leu Asn Lys
530 535 540

Pro Tyr Gly Asp Thr Ile Glu Gly Thr Glu Ile Met Val Arg Ser Lys
545 550 555 560

Lys Ser Val Asp Val Asp Lys Asn Met Ile Pro Thr Gly Asn Ile Val
565 570 575

Asp Arg Glu Ile Ala Thr Phe Asn Ser Thr Lys Pro Thr Val Leu Gly

580 585 590

Pro Lys Asn Pro Gln Phe Asp Cys Cys Phe Val Val Asp Glu Asn Ala
595 600 605

Lys Pro Ser Gln Ile Asn Thr Leu Asn Asn Glu Leu Thr Leu Ile Val
610 615 620

Lys Ala Phe His Pro Asp Ser Asn Ile Thr Leu Glu Val Leu Ser Thr
625 630 635 640

Glu Pro Thr Tyr Gln Phe Tyr Thr Gly Asp Phe Leu Ser Ala Gly Tyr
645 650 655

Glu Ala Arg Gln Gly Phe Ala Ile Glu Pro Gly Arg Tyr Ile Asp Ala
660 665 670

Ile Asn Gln Glu Asn Trp Lys Asp Cys Val Thr Leu Lys Asn Gly Glu
675 680 685

Thr Tyr Gly Ser Lys Ile Val Tyr Arg Phe Ser
690 695

<210> 10
<211> 499
<212> PRT
<213> Saccharomyces cerevisiae

<400> 10

Met Ser Thr Lys Lys His Thr Lys Thr His Ser Thr Tyr Ala Phe Glu
1 5 10 15

Ser Asn Thr Asn Ser Val Ala Ala Ser Gln Met Arg Asn Ala Leu Asn
20 25 30

Lys Leu Ala Asp Ser Ser Lys Leu Asp Asp Ala Ala Arg Ala Lys Phe
35 40 45

Glu Asn Glu Leu Asp Ser Phe Phe Thr Leu Phe Arg Arg Tyr Leu Val
50 55 60

Glu Lys Ser Ser Arg Thr Thr Leu Glu Trp Asp Lys Ile Lys Ser Pro
65 70 75 80

Asn Pro Asp Glu Val Val Lys Tyr Glu Ile Ile Ser Gln Gln Pro Glu
85 90 95

Asn Val Ser Asn Leu Ser Lys Leu Ala Val Leu Lys Leu Asn Gly Gly
100 105 110

Leu Gly Thr Ser Met Gly Cys Val Gly Pro Lys Ser Val Ile Glu Val
115 120 125

Arg Glu Gly Asn Thr Phe Leu Asp Leu Ser Val Arg Gln Ile Glu Tyr
130 135 140

Leu Asn Arg Gln Tyr Asp Ser Asp Val Pro Leu Leu Leu Met Asn Ser
145 150 155 160

Phe Asn Thr Asp Lys Asp Thr Glu His Leu Ile Lys Lys Tyr Ser Ala
165 170 175

Asn Arg Ile Arg Ile Arg Ser Phe Asn Gln Ser Arg Phe Pro Arg Val
180 185 190

Tyr Lys Asp Ser Leu Leu Pro Val Pro Thr Glu Tyr Asp Ser Pro Leu
195 200 205

Asp Ala Trp Tyr Pro Pro Gly His Gly Asp Leu Phe Glu Ser Leu His
210 215 220

Val Ser Gly Glu Leu Asp Ala Leu Ile Ala Gln Gly Arg Glu Ile Leu
225 230 235 240

Phe Val Ser Asn Gly Asp Asn Leu Gly Ala Thr Val Asp Leu Lys Ile
245 250 255

Leu Asn His Met Ile Glu Thr Gly Ala Glu Tyr Ile Met Glu Leu Thr
260 265 270

Asp Lys Thr Arg Ala Asp Val Lys Gly Gly Thr Leu Ile Ser Tyr Asp
275 280 285

Gly Gln Val Arg Leu Leu Glu Val Ala Gln Val Pro Lys Glu His Ile
290 295 300

Asp Glu Phe Lys Asn Ile Arg Lys Phe Thr Asn Phe Asn Thr Asn Asn
305 310 315 320

Leu Trp Ile Asn Leu Lys Ala Val Lys Arg Leu Ile Glu Ser Ser Asn
325 330 335

Leu Glu Met Glu Ile Ile Pro Asn Gln Lys Thr Ile Thr Arg Asp Gly
340 345 350

His Glu Ile Asn Val Leu Gln Leu Glu Thr Ala Cys Gly Ala Ala Ile
355 360 365

Arg His Phe Asp Gly Ala His Gly Val Val Val Pro Arg Ser Arg Phe
    370                 375                 380

Leu Pro Val Lys Thr Cys Ser Asp Leu Leu Leu Val Lys Ser Asp Leu
385                 390                 395                 400

Phe Arg Leu Glu His Gly Ser Leu Lys Leu Asp Pro Ser Arg Phe Gly
                405                 410                 415

Pro Asn Pro Leu Ile Lys Leu Gly Ser His Phe Lys Lys Val Ser Gly
            420                 425                 430

Phe Asn Ala Arg Ile Pro His Ile Pro Lys Ile Val Glu Leu Asp His
        435                 440                 445

Leu Thr Ile Thr Gly Asn Val Phe Leu Gly Lys Asp Val Thr Leu Arg
    450                 455                 460

Gly Thr Val Ile Ile Val Cys Ser Asp Gly His Lys Ile Asp Ile Pro
465                 470                 475                 480

Asn Gly Ser Ile Leu Glu Asn Val Val Val Thr Gly Asn Leu Gln Ile
                485                 490                 495

Leu Glu His

<210>  11
<211>  312
<212>  PRT
<213>  Saccharomyces cerevisiae

<400>  11

Met Pro Ala Thr Leu His Asp Ser Thr Lys Ile Leu Ser Leu Asn Thr
1               5                   10                  15

Gly Ala Gln Ile Pro Gln Ile Gly Leu Gly Thr Trp Gln Ser Lys Glu
            20                  25                  30

Asn Asp Ala Tyr Lys Ala Val Leu Thr Ala Leu Lys Asp Gly Tyr Arg
        35                  40                  45

His Ile Asp Thr Ala Ala Ile Tyr Arg Asn Glu Asp Gln Val Gly Gln
    50                  55                  60

Ala Ile Lys Asp Ser Gly Val Pro Arg Glu Glu Ile Phe Val Thr Thr
65                  70                  75                  80

Lys Leu Trp Cys Thr Gln His His Glu Pro Glu Val Ala Leu Asp Gln

85 90 95

Ser Leu Lys Arg Leu Gly Leu Asp Tyr Val Asp Leu Tyr Leu Met His
100 105 110

Trp Pro Ala Arg Leu Asp Pro Ala Tyr Ile Lys Asn Glu Asp Ile Leu
115 120 125

Ser Val Pro Thr Lys Lys Asp Gly Ser Arg Ala Val Asp Ile Thr Asn
130 135 140

Trp Asn Phe Ile Lys Thr Trp Glu Leu Met Gln Glu Leu Pro Lys Thr
145 150 155 160

Gly Lys Thr Lys Ala Val Gly Val Ser Asn Phe Ser Ile Asn Asn Leu
165 170 175

Lys Asp Leu Leu Ala Ser Gln Gly Asn Lys Leu Thr Pro Ala Ala Asn
180 185 190

Gln Val Glu Ile His Pro Leu Leu Pro Gln Asp Glu Leu Ile Asn Phe
195 200 205

Cys Lys Ser Lys Gly Ile Val Val Glu Ala Tyr Ser Pro Leu Gly Ser
210 215 220

Thr Asp Ala Pro Leu Leu Lys Glu Pro Val Ile Leu Glu Ile Ala Lys
225 230 235 240

Lys Asn Asn Val Gln Pro Gly His Val Val Ile Ser Trp His Val Gln
245 250 255

Arg Gly Tyr Val Val Leu Pro Lys Ser Val Asn Pro Asp Arg Ile Lys
260 265 270

Thr Asn Arg Lys Ile Phe Thr Leu Ser Thr Glu Asp Phe Glu Ala Ile
275 280 285

Asn Asn Ile Ser Lys Glu Lys Gly Glu Lys Arg Val Val His Pro Asn
290 295 300

Trp Ser Pro Phe Glu Val Phe Lys
305 310

<210> 12
<211> 584
<212> PRT
<213> Saccharomyces cerevisiae

<400> 12

```
Met Ser Ala Lys Ser Phe Glu Val Thr Asp Pro Val Asn Ser Ser Leu
1               5                   10                  15

Lys Gly Phe Ala Leu Ala Asn Pro Ser Ile Thr Leu Val Pro Glu Glu
            20                  25                  30

Lys Ile Leu Phe Arg Lys Thr Asp Ser Asp Lys Ile Ala Leu Ile Ser
        35                  40                  45

Gly Gly Gly Ser Gly His Glu Pro Thr His Ala Gly Phe Ile Gly Lys
    50                  55                  60

Gly Met Leu Ser Gly Ala Val Val Gly Glu Ile Phe Ala Ser Pro Ser
65                  70                  75                  80

Thr Lys Gln Ile Leu Asn Ala Ile Arg Leu Val Asn Glu Asn Ala Ser
                85                  90                  95

Gly Val Leu Leu Ile Val Lys Asn Tyr Thr Gly Asp Val Leu His Phe
            100                 105                 110

Gly Leu Ser Ala Glu Arg Ala Arg Ala Leu Gly Ile Asn Cys Arg Val
        115                 120                 125

Ala Val Ile Gly Asp Asp Val Ala Val Gly Arg Glu Lys Gly Gly Met
    130                 135                 140

Val Gly Arg Arg Ala Leu Ala Gly Thr Val Leu Val His Lys Ile Val
145                 150                 155                 160

Gly Ala Phe Ala Glu Glu Tyr Ser Ser Lys Tyr Gly Leu Asp Gly Thr
                165                 170                 175

Ala Lys Val Ala Lys Ile Ile Asn Asp Asn Leu Val Thr Ile Gly Ser
            180                 185                 190

Ser Leu Asp His Cys Lys Val Pro Gly Arg Lys Phe Glu Ser Glu Leu
        195                 200                 205

Asn Glu Lys Gln Met Glu Leu Gly Met Gly Ile His Asn Glu Pro Gly
    210                 215                 220

Val Lys Val Leu Asp Pro Ile Pro Ser Thr Glu Asp Leu Ile Ser Lys
225                 230                 235                 240

Tyr Met Leu Pro Lys Leu Leu Asp Pro Asn Asp Lys Asp Arg Ala Phe
                245                 250                 255

Val Lys Phe Asp Glu Asp Asp Glu Val Val Leu Leu Val Asn Asn Leu
```

260 265 270

Gly Gly Val Ser Asn Phe Val Ile Ser Ser Ile Thr Ser Lys Thr Thr
275 280 285

Asp Phe Leu Lys Glu Asn Tyr Asn Ile Thr Pro Val Gln Thr Ile Ala
290 295 300

Gly Thr Leu Met Thr Ser Phe Asn Gly Asn Gly Phe Ser Ile Thr Leu
305 310 315 320

Leu Asn Ala Thr Lys Ala Thr Lys Ala Leu Gln Ser Asp Phe Glu Glu
325 330 335

Ile Lys Ser Val Leu Asp Leu Leu Asn Ala Phe Thr Asn Ala Pro Gly
340 345 350

Trp Pro Ile Ala Asp Phe Glu Lys Thr Ser Ala Pro Ser Val Asn Asp
355 360 365

Asp Leu Leu His Asn Glu Val Thr Ala Lys Ala Val Gly Thr Tyr Asp
370 375 380

Phe Asp Lys Phe Ala Glu Trp Met Lys Ser Gly Ala Glu Gln Val Ile
385 390 395 400

Lys Ser Glu Pro His Ile Thr Glu Leu Asp Asn Gln Val Gly Asp Gly
405 410 415

Asp Cys Gly Tyr Thr Leu Val Ala Gly Val Lys Gly Ile Thr Glu Asn
420 425 430

Leu Asp Lys Leu Ser Lys Asp Ser Leu Ser Gln Ala Val Ala Gln Ile
435 440 445

Ser Asp Phe Ile Glu Gly Ser Met Gly Gly Thr Ser Gly Gly Leu Tyr
450 455 460

Ser Ile Leu Leu Ser Gly Phe Ser His Gly Leu Ile Gln Val Cys Lys
465 470 475 480

Ser Lys Asp Glu Pro Val Thr Lys Glu Ile Val Ala Lys Ser Leu Gly
485 490 495

Ile Ala Leu Asp Thr Leu Tyr Lys Tyr Thr Lys Ala Arg Lys Gly Ser
500 505 510

Ser Thr Met Ile Asp Ala Leu Glu Pro Phe Val Lys Glu Phe Thr Ala

515 520 525

Ser Lys Asp Phe Asn Lys Ala Val Lys Ala Ala Glu Glu Gly Ala Lys
530 535 540

Ser Thr Ala Thr Phe Glu Ala Lys Phe Gly Arg Ala Ser Tyr Val Gly
545 550 555 560

Asp Ser Ser Gln Val Glu Asp Pro Gly Ala Val Gly Leu Cys Glu Phe
565 570 575

Leu Lys Gly Val Gln Ser Ala Leu
580

<210> 13
<211> 591
<212> PRT
<213> Saccharomyces cerevisiae

<400> 13

Met Ser His Lys Gln Phe Lys Ser Asp Gly Asn Ile Val Thr Pro Tyr
1 5 10 15

Leu Leu Gly Leu Ala Arg Ser Asn Pro Gly Leu Thr Val Ile Lys His
20 25 30

Asp Arg Val Val Phe Arg Thr Ala Ser Ala Pro Asn Ser Gly Asn Pro
35 40 45

Pro Lys Val Ser Leu Val Ser Gly Gly Gly Ser Gly His Glu Pro Thr
50 55 60

His Ala Gly Phe Val Gly Glu Gly Ala Leu Asp Ala Ile Ala Ala Gly
65 70 75 80

Ala Ile Phe Ala Ser Pro Ser Thr Lys Gln Ile Tyr Ser Ala Ile Lys
85 90 95

Ala Val Glu Ser Pro Lys Gly Thr Leu Ile Ile Val Lys Asn Tyr Thr
100 105 110

Gly Asp Ile Ile His Phe Gly Leu Ala Ala Glu Arg Ala Lys Ala Ala
115 120 125

Gly Met Lys Val Glu Leu Val Ala Val Gly Asp Asp Val Ser Val Gly
130 135 140

Lys Lys Lys Gly Ser Leu Val Gly Arg Arg Gly Leu Gly Ala Thr Val
145 150 155 160

Leu Val His Lys Ile Ala Gly Ala Ala Ala Ser His Gly Leu Glu Leu
165 170 175

Ala Glu Val Ala Glu Val Ala Gln Ser Val Val Asp Asn Ser Val Thr
180 185 190

Ile Ala Ala Ser Leu Asp His Cys Thr Val Pro Gly His Lys Pro Glu
195 200 205

Ala Ile Leu Gly Glu Asn Glu Tyr Glu Ile Gly Met Gly Ile His Asn
210 215 220

Glu Ser Gly Thr Tyr Lys Ser Ser Pro Leu Pro Ser Ile Ser Glu Leu
225 230 235 240

Val Ser Gln Met Leu Pro Leu Leu Leu Asp Glu Asp Glu Asp Arg Ser
245 250 255

Tyr Val Lys Phe Glu Pro Lys Glu Asp Val Val Leu Met Val Asn Asn
260 265 270

Met Gly Gly Met Ser Asn Leu Glu Leu Gly Tyr Ala Ala Glu Val Ile
275 280 285

Ser Glu Gln Leu Ile Asp Lys Tyr Gln Ile Val Pro Lys Arg Thr Ile
290 295 300

Thr Gly Ala Phe Ile Thr Ala Leu Asn Gly Pro Gly Phe Gly Ile Thr
305 310 315 320

Leu Met Asn Ala Ser Lys Ala Gly Gly Asp Ile Leu Lys Tyr Phe Asp
325 330 335

Tyr Pro Thr Thr Ala Ser Gly Trp Asn Gln Met Tyr His Ser Ala Lys
340 345 350

Asp Trp Glu Val Leu Ala Lys Gly Gln Val Pro Thr Ala Pro Ser Leu
355 360 365

Lys Thr Leu Arg Asn Glu Lys Gly Ser Gly Val Lys Ala Asp Tyr Asp
370 375 380

Thr Phe Ala Lys Ile Leu Leu Ala Gly Ile Ala Lys Ile Asn Glu Val
385 390 395 400

Glu Pro Lys Val Thr Trp Tyr Asp Thr Ile Ala Gly Asp Gly Asp Cys
405 410 415

Gly Thr Thr Leu Val Ser Gly Gly Glu Ala Leu Glu Glu Ala Ile Lys
420 425 430

Asn His Thr Leu Arg Leu Glu Asp Ala Ala Leu Gly Ile Glu Asp Ile
435 440 445

Ala Tyr Met Val Glu Asp Ser Met Gly Gly Thr Ser Gly Gly Leu Tyr
450 455 460

Ser Ile Tyr Leu Ser Ala Leu Ala Gln Gly Val Arg Asp Ser Gly Asp
465 470 475 480

Lys Glu Leu Thr Ala Glu Thr Phe Lys Lys Ala Ser Asn Val Ala Leu
485 490 495

Asp Ala Leu Tyr Lys Tyr Thr Arg Ala Arg Pro Gly Tyr Arg Thr Leu
500 505 510

Ile Asp Ala Leu Gln Pro Phe Val Glu Ala Leu Lys Ala Gly Lys Gly
515 520 525

Pro Arg Ala Ala Ala Gln Ala Ala Tyr Asp Gly Ala Glu Lys Thr Arg
530 535 540

Lys Met Asp Ala Leu Val Gly Arg Ala Ser Tyr Val Ala Lys Glu Glu
545 550 555 560

Leu Arg Lys Leu Asp Ser Glu Gly Gly Leu Pro Asp Pro Gly Ala Val
565 570 575

Gly Leu Ala Ala Leu Leu Asp Gly Phe Val Thr Ala Ala Gly Tyr
580 585 590

<210> 14
<211> 560
<212> PRT
<213> Saccharomyces cerevisiae

<400> 14

Met Ser Leu Ile Ser Ile Leu Ser Pro Leu Ile Thr Ser Glu Gly Leu
1 5 10 15

Asp Ser Arg Ile Lys Pro Ser Pro Lys Lys Asp Ala Ser Thr Thr Thr
20 25 30

Lys Pro Ser Leu Trp Lys Thr Thr Glu Phe Lys Phe Tyr Tyr Ile Ala
35 40 45

Phe Leu Val Val Val Pro Leu Met Phe Tyr Ala Gly Leu Gln Ala Ser

```
    50                  55                  60

Ser Pro Glu Asn Pro Asn Tyr Ala Arg Tyr Glu Arg Leu Leu Ser Gln
65                  70                  75                  80

Gly Trp Leu Phe Gly Arg Lys Val Asp Asn Ser Asp Ser Gln Tyr Arg
                85                  90                  95

Phe Phe Arg Asp Asn Phe Ala Leu Leu Ser Val Leu Met Leu Val His
            100                 105                 110

Thr Ser Ile Lys Arg Ile Val Leu Tyr Ser Thr Asn Ile Thr Lys Leu
        115                 120                 125

Arg Phe Asp Leu Ile Phe Gly Leu Ile Phe Leu Val Ala Ala His Gly
    130                 135                 140

Val Asn Ser Ile Arg Ile Leu Ala His Met Leu Ile Leu Tyr Ala Ile
145                 150                 155                 160

Ala His Val Leu Lys Asn Phe Arg Arg Ile Ala Thr Ile Ser Ile Trp
                165                 170                 175

Ile Tyr Gly Ile Ser Thr Leu Phe Ile Asn Asp Asn Phe Arg Ala Tyr
            180                 185                 190

Pro Phe Gly Asn Ile Cys Ser Phe Leu Ser Pro Leu Asp His Trp Tyr
        195                 200                 205

Arg Gly Ile Ile Pro Arg Trp Asp Val Phe Phe Asn Phe Thr Leu Leu
    210                 215                 220

Arg Val Leu Ser Tyr Asn Leu Asp Phe Leu Glu Arg Trp Glu Asn Leu
225                 230                 235                 240

Gln Lys Lys Lys Ser Pro Ser Tyr Glu Ser Lys Glu Ala Lys Ser Ala
                245                 250                 255

Ile Leu Leu Asn Glu Arg Ala Arg Leu Thr Ala Ala His Pro Ile Gln
            260                 265                 270

Asp Tyr Ser Leu Met Asn Tyr Ile Ala Tyr Val Thr Tyr Thr Pro Leu
        275                 280                 285

Phe Ile Ala Gly Pro Ile Ile Thr Phe Asn Asp Tyr Val Tyr Gln Ser
    290                 295                 300

Lys His Thr Leu Pro Ser Ile Asn Phe Lys Phe Ile Phe Tyr Tyr Ala
```

305 310 315 320

Val Arg Phe Val Ile Ala Leu Leu Ser Met Glu Phe Ile Leu His Phe
325 330 335

Leu His Val Val Ala Ile Ser Lys Thr Lys Ala Trp Glu Asn Asp Thr
340 345 350

Pro Phe Gln Ile Ser Met Ile Gly Leu Phe Asn Leu Asn Ile Ile Trp
355 360 365

Leu Lys Leu Leu Ile Pro Trp Arg Leu Phe Arg Leu Trp Ala Leu Leu
370 375 380

Asp Gly Ile Asp Thr Pro Glu Asn Met Ile Arg Cys Val Asp Asn Asn
385 390 395 400

Tyr Ser Ser Leu Ala Phe Trp Arg Ala Trp His Arg Ser Tyr Asn Lys
405 410 415

Trp Val Val Arg Tyr Ile Tyr Ile Pro Leu Gly Gly Ser Lys Asn Arg
420 425 430

Val Leu Thr Ser Leu Ala Val Phe Ser Phe Val Ala Ile Trp His Asp
435 440 445

Ile Glu Leu Lys Leu Leu Leu Trp Gly Trp Leu Ile Val Leu Phe Leu
450 455 460

Leu Pro Glu Ile Phe Ala Thr Gln Ile Phe Ser His Tyr Thr Asp Ala
465 470 475 480

Val Trp Tyr Arg His Val Cys Ala Val Gly Ala Val Phe Asn Ile Trp
485 490 495

Val Met Met Ile Ala Asn Leu Phe Gly Phe Cys Leu Gly Ser Asp Gly
500 505 510

Thr Lys Lys Leu Leu Ser Asp Met Phe Cys Thr Val Ser Gly Phe Lys
515 520 525

Phe Val Ile Leu Ala Ser Val Ser Leu Phe Ile Ala Val Gln Ile Met
530 535 540

Phe Glu Ile Arg Glu Glu Glu Lys Arg His Gly Ile Tyr Leu Lys Cys
545 550 555 560

<210> 15

<211> 609
<212> PRT
<213> Saccharomyces cerevisiae

<400> 15

```
Met Ser Met Leu Arg Ile Trp Ser Cys Ile Val His Phe Phe Ser Val
1               5                   10                  15

Gln Ala Leu Asp Ser Arg Ile Lys Pro Asp Ile Glu Phe Lys Arg Arg
            20                  25                  30

Gln Arg Ile Phe Ile Asn Ser Ser Lys Glu Glu Asn Gly Ser Ser Ser
        35                  40                  45

Ser Ala Val Thr Val Thr Arg Asn Pro Val Leu Ser Ser Asn Ser Pro
    50                  55                  60

Ser Pro Pro Leu Trp Asn Thr Trp Glu Phe Arg Leu Tyr Tyr Leu Ala
65                  70                  75                  80

Phe Thr Val Val Val Pro Phe Met Ile Lys Ala Ala Leu Ala Thr Ser
                85                  90                  95

Ser Glu Ser Asn Pro Asn Tyr Tyr Lys Phe Ser Gly Leu Leu Ala His
            100                 105                 110

Gly Trp Ile Leu Gly Arg Lys Val Asp Asn Ser Asp Pro Gln Tyr Arg
        115                 120                 125

Phe Phe Arg Ser Asn Phe Phe Leu Leu Ala Ile Leu Ile Leu Leu Gln
    130                 135                 140

Ile Ile Leu Lys Lys Val Phe Val Lys Phe Ser Lys Ile Pro Lys Thr
145                 150                 155                 160

Lys Phe Asp Phe Ala Cys Gly Leu Val Phe Val Cys Phe Met Tyr Gly
                165                 170                 175

Ile Asn Ser Val Lys Leu Phe Thr His Ala Phe Ile Phe Phe Thr Leu
            180                 185                 190

Ala His Ser Leu Lys Arg Lys Arg Leu Ile Ala Ala Phe Ala Ile Trp
        195                 200                 205

Ser Tyr Gly Ile Phe Thr Leu Phe Ile Asn Gln Lys Met Lys Asn Leu
    210                 215                 220

Pro Phe Asn Asn Ile Ala Ile Ile Leu Ser Pro Met Asp Gln Trp Tyr
225                 230                 235                 240
```

Lys Gly Ile Val Pro Arg Trp Asp Phe Phe Phe Asn Phe Thr Leu Leu
245 250 255

Arg Leu Leu Ser Tyr Ser Met Asp Phe Leu Glu Arg Trp His Glu Gln
260 265 270

Leu Ser Arg Gln Pro Ser Ile Asp Tyr Asp Asp Arg Arg Pro Glu Phe
275 280 285

Arg Lys Ser Leu Ser Gly Ser Thr Leu Gln Thr Ile Tyr Glu Ser Gly
290 295 300

Lys Asn Val Leu Glu Glu Lys Glu Arg Leu Val Ala Glu His His Ile
305 310 315 320

Gln Asp Tyr Asn Phe Ile Asn Phe Ile Ala Tyr Ile Thr Tyr Ala Pro
325 330 335

Leu Phe Leu Val Gly Pro Ile Ile Thr Phe Asn Asp Tyr Leu Tyr Gln
340 345 350

Ser Glu Asn Lys Leu Pro Ser Leu Thr Lys Lys Asn Ile Gly Phe Tyr
355 360 365

Ala Leu Lys Val Phe Ser Ser Leu Leu Leu Met Glu Ile Ile Leu His
370 375 380

Tyr Ile Tyr Val Gly Ala Ile Ala Arg Thr Lys Ala Trp Asn Asn Asp
385 390 395 400

Thr Pro Leu Gln Gln Ala Met Ile Ala Leu Phe Asn Leu Asn Ile Met
405 410 415

Tyr Leu Lys Leu Leu Ile Pro Trp Arg Leu Phe Arg Leu Trp Ala Met
420 425 430

Val Asp Gly Ile Asp Ala Pro Glu Asn Met Leu Arg Cys Val Asp Asn
435 440 445

Asn Tyr Ser Thr Val Gly Phe Trp Arg Ala Trp His Thr Ser Phe Asn
450 455 460

Lys Trp Val Ile Arg Tyr Ile Tyr Val Pro Phe Gly Gly Ser Asn Asn
465 470 475 480

Lys Ile Leu Thr Ser Phe Ala Val Phe Ser Phe Val Ala Ile Trp His
485 490 495

Asp Ile Gln Leu Arg Val Leu Phe Trp Gly Trp Leu Thr Val Leu Leu
500 505 510

Leu Leu Gly Glu Thr Tyr Ile Thr Asn Cys Phe Ser Arg Tyr Arg Phe
515 520 525

Arg Ser Trp Tyr Arg Phe Val Cys Gly Ile Gly Ala Ala Ile Asn Ile
530 535 540

Cys Met Met Met Ile Ile Asn Val Tyr Gly Phe Cys Leu Gly Ala Glu
545 550 555 560

Gly Thr Lys Leu Leu Leu Lys Gly Ile Phe Asn Asn Ser His Ser Pro
565 570 575

Glu Phe Leu Thr Ala Val Met Val Ser Leu Phe Ile Ala Val Gln Val
580 585 590

Met Phe Glu Ile Arg Glu Glu Glu Lys Arg His Gly Ile Asn Leu Lys
595 600 605

Cys

<210> 16
<211> 669
<212> PRT
<213> Saccharomyces cerevisiae

<400> 16

Met Ser Asn Pro Gln Lys Ala Leu Asn Asp Phe Leu Ser Ser Glu Ser
1 5 10 15

Val His Thr His Asp Ser Ser Arg Lys Gln Ser Asn Lys Gln Ser Ser
20 25 30

Asp Glu Gly Arg Ser Ser Ser Gln Pro Ser His His His Ser Gly Gly
35 40 45

Thr Asn Asn Asn Asn Asn Asn Asn Asn Asn Asn Asn Asn Ser Asn Asn
50 55 60

Asn Asn Asn Gly Asn Asp Gly Gly Asn Asp Asp Asp Tyr Asp Tyr Glu
65 70 75 80

Met Gln Asp Tyr Arg Pro Ser Pro Gln Ser Ala Arg Pro Thr Pro Thr
85 90 95

Tyr Val Pro Gln Tyr Ser Val Glu Ser Gly Thr Ala Phe Pro Ile Gln

```
            100                 105                 110

Glu Val Ile Pro Ser Ala Tyr Ile Asn Thr Gln Asp Ile Asn His Lys
        115                 120                 125

Asp Asn Gly Pro Pro Ser Ala Ser Ser Asn Arg Ala Phe Arg Pro Arg
    130                 135                 140

Gly Gln Thr Thr Val Ser Ala Asn Val Leu Asn Ile Glu Asp Phe Tyr
145                 150                 155                 160

Lys Asn Ala Asp Asp Ala His Thr Ile Pro Glu Ser His Leu Ser Arg
            165                 170                 175

Arg Arg Ser Arg Ser Arg Ala Thr Ser Asn Ala Gly His Ser Ala Asn
            180                 185                 190

Thr Gly Ala Thr Asn Gly Arg Thr Thr Gly Ala Gln Thr Asn Met Glu
        195                 200                 205

Ser Asn Glu Ser Pro Arg Asn Val Pro Ile Met Val Lys Pro Lys Thr
    210                 215                 220

Leu Tyr Gln Asn Pro Gln Thr Pro Thr Val Leu Pro Ser Thr Tyr His
225                 230                 235                 240

Pro Ile Asn Lys Trp Ser Ser Val Lys Asn Thr Tyr Leu Lys Glu Phe
            245                 250                 255

Leu Ala Glu Phe Met Gly Thr Met Val Met Ile Ile Phe Gly Ser Ala
            260                 265                 270

Val Val Cys Gln Val Asn Val Ala Gly Lys Ile Gln Gln Asp Asn Phe
        275                 280                 285

Asn Val Ala Leu Asp Asn Leu Asn Val Thr Gly Ser Ser Ala Glu Thr
    290                 295                 300

Ile Asp Ala Met Lys Ser Leu Thr Ser Leu Val Ser Ser Val Ala Gly
305                 310                 315                 320

Gly Thr Phe Asp Asp Val Ala Leu Gly Trp Ala Ala Ala Val Val Met
            325                 330                 335

Gly Tyr Phe Cys Ala Gly Gly Ser Ala Ile Ser Gly Ala His Leu Asn
            340                 345                 350

Pro Ser Ile Thr Leu Ala Asn Leu Val Tyr Arg Gly Phe Pro Leu Lys
```

355 360 365

Lys Val Pro Tyr Tyr Phe Ala Gly Gln Leu Ile Gly Ala Phe Thr Gly
370 375 380

Ala Leu Ile Leu Phe Ile Trp Tyr Lys Arg Val Leu Gln Glu Ala Tyr
385 390 395 400

Ser Asp Trp Trp Met Asn Glu Ser Val Ala Gly Met Phe Cys Val Phe
405 410 415

Pro Lys Pro Tyr Leu Ser Ser Gly Arg Gln Phe Phe Ser Glu Phe Leu
420 425 430

Cys Gly Ala Met Leu Gln Ala Gly Thr Phe Ala Leu Thr Asp Pro Tyr
435 440 445

Thr Cys Leu Ser Ser Asp Val Phe Pro Leu Met Met Phe Ile Leu Ile
450 455 460

Phe Ile Ile Asn Ala Ser Met Ala Tyr Gln Thr Gly Thr Ala Met Asn
465 470 475 480

Leu Ala Arg Asp Leu Gly Pro Arg Leu Ala Leu Tyr Ala Val Gly Phe
485 490 495

Asp His Lys Met Leu Trp Val His His His His Phe Phe Trp Val Pro
500 505 510

Met Val Gly Pro Phe Ile Gly Ala Leu Met Gly Gly Leu Val Tyr Asp
515 520 525

Val Cys Ile Tyr Gln Gly His Glu Ser Pro Val Asn Trp Ser Leu Pro
530 535 540

Val Tyr Lys Glu Met Ile Met Arg Ala Trp Phe Arg Arg Pro Gly Trp
545 550 555 560

Lys Lys Arg Asn Arg Ala Arg Arg Thr Ser Asp Leu Ser Asp Phe Ser
565 570 575

Tyr Asn Asn Asp Asp Asp Glu Glu Phe Gly Glu Arg Met Ala Leu Gln
580 585 590

Lys Thr Lys Thr Lys Ser Ser Ile Ser Asp Asn Glu Asn Glu Ala Gly
595 600 605

Glu Lys Lys Val Gln Phe Lys Ser Val Gln Arg Gly Lys Arg Thr Phe

```
    610                 615                 620

Gly Gly Ile Pro Thr Ile Leu Glu Glu Glu Asp Ser Ile Glu Thr Ala
625                 630                 635                 640

Ser Leu Gly Ala Thr Thr Thr Asp Ser Ile Gly Leu Ser Asp Thr Ser
                645                 650                 655

Ser Glu Asp Ser His Tyr Gly Asn Ala Lys Lys Val Thr
            660                 665


<210>  17
<211>  440
<212>  PRT
<213>  Saccharomyces cerevisiae

<400>  17

Met Leu Ala Val Arg Arg Leu Thr Arg Tyr Thr Phe Leu Lys Arg Thr
1               5                   10                  15

His Pro Val Leu Tyr Thr Arg Arg Ala Tyr Lys Ile Leu Pro Ser Arg
            20                  25                  30

Ser Thr Phe Leu Arg Arg Ser Leu Leu Gln Thr Gln Leu His Ser Lys
        35                  40                  45

Met Thr Ala His Thr Asn Ile Lys Gln His Lys His Cys His Glu Asp
    50                  55                  60

His Pro Ile Arg Arg Ser Asp Ser Ala Val Ser Ile Val His Leu Lys
65                  70                  75                  80

Arg Ala Pro Phe Lys Val Thr Val Ile Gly Ser Gly Asn Trp Gly Thr
                85                  90                  95

Thr Ile Ala Lys Val Ile Ala Glu Asn Thr Glu Leu His Ser His Ile
            100                 105                 110

Phe Glu Pro Glu Val Arg Met Trp Val Phe Asp Glu Lys Ile Gly Asp
        115                 120                 125

Glu Asn Leu Thr Asp Ile Ile Asn Thr Arg His Gln Asn Val Lys Tyr
    130                 135                 140

Leu Pro Asn Ile Asp Leu Pro His Asn Leu Val Ala Asp Pro Asp Leu
145                 150                 155                 160

Leu His Ser Ile Lys Gly Ala Asp Ile Leu Val Phe Asn Ile Pro His
                165                 170                 175
```

```
Gln Phe Leu Pro Asn Ile Val Lys Gln Leu Gln Gly His Val Ala Pro
        180                 185                 190

His Val Arg Ala Ile Ser Cys Leu Lys Gly Phe Glu Leu Gly Ser Lys
    195                 200                 205

Gly Val Gln Leu Leu Ser Ser Tyr Val Thr Asp Glu Leu Gly Ile Gln
210                 215                 220

Cys Gly Ala Leu Ser Gly Ala Asn Leu Ala Pro Glu Val Ala Lys Glu
225                 230                 235                 240

His Trp Ser Glu Thr Thr Val Ala Tyr Gln Leu Pro Lys Asp Tyr Gln
                245                 250                 255

Gly Asp Gly Lys Asp Val Asp His Lys Ile Leu Lys Leu Leu Phe His
            260                 265                 270

Arg Pro Tyr Phe His Val Asn Val Ile Asp Asp Val Ala Gly Ile Ser
        275                 280                 285

Ile Ala Gly Ala Leu Lys Asn Val Val Ala Leu Ala Cys Gly Phe Val
    290                 295                 300

Glu Gly Met Gly Trp Gly Asn Asn Ala Ser Ala Ala Ile Gln Arg Leu
305                 310                 315                 320

Gly Leu Gly Glu Ile Ile Lys Phe Gly Arg Met Phe Phe Pro Glu Ser
                325                 330                 335

Lys Val Glu Thr Tyr Tyr Gln Glu Ser Ala Gly Val Ala Asp Leu Ile
            340                 345                 350

Thr Thr Cys Ser Gly Gly Arg Asn Val Lys Val Ala Thr Tyr Met Ala
        355                 360                 365

Lys Thr Gly Lys Ser Ala Leu Glu Ala Glu Lys Glu Leu Leu Asn Gly
    370                 375                 380

Gln Ser Ala Gln Gly Ile Ile Thr Cys Arg Glu Val His Glu Trp Leu
385                 390                 395                 400

Gln Thr Cys Glu Leu Thr Gln Glu Phe Pro Leu Phe Glu Ala Val Tyr
                405                 410                 415

Gln Ile Val Tyr Asn Asn Val Arg Met Glu Asp Leu Pro Glu Met Ile
            420                 425                 430
```

Glu Glu Leu Asp Ile Asp Asp Glu
435 440

<210> 18
<211> 391
<212> PRT
<213> Saccharomyces cerevisiae

<400> 18

Met Ser Ala Ala Ala Asp Arg Leu Asn Leu Thr Ser Gly His Leu Asn
1 5 10 15

Ala Gly Arg Lys Arg Ser Ser Ser Ser Val Ser Leu Lys Ala Ala Glu
20 25 30

Lys Pro Phe Lys Val Thr Val Ile Gly Ser Gly Asn Trp Gly Thr Thr
35 40 45

Ile Ala Lys Val Val Ala Glu Asn Cys Lys Gly Tyr Pro Glu Val Phe
50 55 60

Ala Pro Ile Val Gln Met Trp Val Phe Glu Glu Glu Ile Asn Gly Glu
65 70 75 80

Lys Leu Thr Glu Ile Ile Asn Thr Arg His Gln Asn Val Lys Tyr Leu
85 90 95

Pro Gly Ile Thr Leu Pro Asp Asn Leu Val Ala Asn Pro Asp Leu Ile
100 105 110

Asp Ser Val Lys Asp Val Asp Ile Ile Val Phe Asn Ile Pro His Gln
115 120 125

Phe Leu Pro Arg Ile Cys Ser Gln Leu Lys Gly His Val Asp Ser His
130 135 140

Val Arg Ala Ile Ser Cys Leu Lys Gly Phe Glu Val Gly Ala Lys Gly
145 150 155 160

Val Gln Leu Leu Ser Ser Tyr Ile Thr Glu Glu Leu Gly Ile Gln Cys
165 170 175

Gly Ala Leu Ser Gly Ala Asn Ile Ala Thr Glu Val Ala Gln Glu His
180 185 190

Trp Ser Glu Thr Thr Val Ala Tyr His Ile Pro Lys Asp Phe Arg Gly
195 200 205

Glu Gly Lys Asp Val Asp His Lys Val Leu Lys Ala Leu Phe His Arg

210 215 220

Pro Tyr Phe His Val Ser Val Ile Glu Asp Val Ala Gly Ile Ser Ile
225 230 235 240

Cys Gly Ala Leu Lys Asn Val Val Ala Leu Gly Cys Gly Phe Val Glu
245 250 255

Gly Leu Gly Trp Gly Asn Asn Ala Ser Ala Ala Ile Gln Arg Val Gly
260 265 270

Leu Gly Glu Ile Ile Arg Phe Gly Gln Met Phe Phe Pro Glu Ser Arg
275 280 285

Glu Glu Thr Tyr Tyr Gln Glu Ser Ala Gly Val Ala Asp Leu Ile Thr
290 295 300

Thr Cys Ala Gly Gly Arg Asn Val Lys Val Ala Arg Leu Met Ala Thr
305 310 315 320

Ser Gly Lys Asp Ala Trp Glu Cys Glu Lys Glu Leu Leu Asn Gly Gln
325 330 335

Ser Ala Gln Gly Leu Ile Thr Cys Lys Glu Val His Glu Trp Leu Glu
340 345 350

Thr Cys Gly Ser Val Glu Asp Phe Pro Leu Phe Glu Ala Val Tyr Gln
355 360 365

Ile Val Tyr Asn Asn Tyr Pro Met Lys Asn Leu Pro Asp Met Ile Glu
370 375 380

Glu Leu Asp Leu His Glu Asp
385 390

<210> 19
<211> 1290
<212> DNA
<213> Artificial

<220>
<223> Codon optimized synthetic ORF 2.7.1.44 Arabidopsis thaliana

<400> 19
gaattcaaaa tgtcttggcc aaccgactct gaattgaact ctatcaagga agctgttgct 60

caaatgtctg gtagagacaa gggtgaagtt agagttgttg ttgctcccata cagaatctgt 120

ccattgggtg ctcacatcga ccaccaaggt ggtactgttt ctgctatgac catcaacaag 180

ggtatcttgt tgggtttcgt tccatctggt gacacccaag ttcaattgag atctgctcaa 240

ttcgaaggtg aagtttgttt cagagttgac gaaatccaac acccaatcgg tttggctaac 300

```
aagaacggtg cttctacccc atctccatct aaggaaaagt ctatctgggg tacttacgct     360

agaggtgctg tttacgcttt gcaatcttct aagaagaact tgaagcaagg tatcatcggt     420

tacttgtctg gttctaacgg tttggactct tcaggtttat cttcttctgc tgctgttggt     480

gttgcttact tgttagcttt ggaaaacgct aacgaattga ccgtttctcc aaccgaaaac     540

atcgaatacg acagattgat cgaaaacggt tacttgggtt tgagaaacgg tatcttggac     600

caatctgcta tcttgttgtc taactacggt tgtttgacct acatggactg taagaccttg     660

gaccacgaat tggttcaagc tccagaattg gaaaagccat tcagaatctt gttggctttc     720

tctggtttga gacaagcttt gaccaccaac ccaggtttca acttgagagt ttctgaatgt     780

caagaagctg ctaaggtttt gttgaccgct tcaggtaact ctgaattgga accaaccttg     840

tgtaacgttg aacacgctgt ttacgaagct cacaagcacg aattgaagcc tgttttggct     900

aagagagctg aacactactt ctctgaaaac atgagagtta tcaagggtag agaagcttgg     960

gcttctggta acttggaaga atttggtaag ttgatctctg cttctggttt gtcttctatc    1020

gaaaactacg aatgtggtgc tgaaccattg atccaattgt acaagatttt gttgaaggct    1080

ccaggtgttt acggtgctag attctctggt gctggtttca gaggttgttg tttggctttc    1140

gttgacgctg aaaaggctga agctgctgct tcttacgtta aggacgaata cgaaaaggct    1200

caaccagaat ttgctaacaa cttgaacggt ggtaagccag ttttgatctg tgaagctggt    1260

gacgctgcta gagttttgtt gtaaggatcc                                    1290


<210>  20
<211>  1104
<212>  DNA
<213>  Artificial

<220>
<223>  Codon optimized synthetic ORF 2.7.1.43 Arabidopsis thaliana

<400>  20
gaattcaaaa tggacccaaa ctctaccgtt tctggtgacg gtcaagctac cgctgctatc      60

gaacacagat ctttcgctag aatcggtttc ttgggtaacc cttctgacgt ttacttcggt     120

agaaccatct ctttgaccat cggtaacttc tgggcttctg ttaagttgga accatctgaa     180

cacttggtta tcaagccaca cccattccac gacttggttc aattcacctc tttggaccac     240

ttgttgaaca gattgcaaaa cgaaggttac tacggtggtg ttagattgtt gatggctatc     300

tgtaaggttt tcagaaacta ctgtaaggaa aacgacatcc aattgcacca agctaacttc     360

tcttttgtctt acgacaccaa catcccaaga caaactggtt tgtctggttc ttctgctatc     420

gtttctgctg ctttgaactg tttgttggac ttctacaacg ttagacactt gatcaaggtt     480

caagttagac caaacatcgt tttgtctgct gaaaaggaat tgggtatcgt tgctggtttg     540

caagacagag ttgctcaagt ttacggtggt ttggttcaca tggacttctc taaggaacac     600
```

```
atggacaagt tgggtcacgg tatctacacc ccaatggaca tctctttgtt gccaccattg     660

cacttgatct acgctgaaaa cccatctgac tctggtaagg ttcactctat ggttagacaa     720

agatggttgg acggtgacga atttatcatc tcttctatga aggaagttgg ttctttggct     780

gaagaaggta gaaccgcttt gttgaacaag gaccactcta agttggttga attgatgaac     840

ttgaacttcg acatcagaag gagaatgttc ggtgacgaat gtttgggtgc tatgaacatc     900

gaaatggttg aagttgctag aagagttggt gctgcttcta agttcactgg ttctggtggt     960

gctgttgttg ttttctgtcc agaaggtcca tctcaagtta agttgttgga agaagaatgt    1020

agaaaggctg gtttcacctt gcaaccagtt aagatcgctc catcttgttt gaacgactct    1080

gacatccaaa ccttgtaagg atcc                                            1104
```

<210>  21
<211>  1818
<212>  DNA
<213>  Artificial

<220>
<223>  Codon optimized synthetic ORF 2.7.7.64 Pisum sativum

<400>  21

```
aagcttaaaa tggcttcttc tttgggtgac aacttcaact tgttgtctcc acaacaaaga      60

gaattggtta agatgttgtt ggacaacgga caagaccact tgttcagaga ctggccaaac     120

ccaggtgttg acgacgacga aaagaaggct ttcttcgacc aattggtttt gttggactct     180

tcttacccag gtggtttggt tgcttacatc aacaacgcta agagattgtt ggctgactct     240

aaggctggta acaacccatt cgacggtttc accccatctg ttccaactgg tgaaaccttg     300

aagttcggtg acgaaaactt caacaagtac gaagaagctg gtgttagaga agctagaaga     360

gctgctttcg ttttggttgc tggtggtttg ggtgaaagat tgggttacaa cggtatcaag     420

gttgctttgc cagctgaaac caccactggt acctgtttct tgcaacacta catcgaatct     480

atcttggctt tgcaagaagc aagttctgaa ggtgaaggac aaacccacat cccattcgtt     540

atcatgacct ctgacgacac ccacggtaga accttggact tgttggaatc taactcttac     600

ttcggtatgc aaccaaccca agttaccttg ttgaagcaag aaaaggttgc ttgtttggaa     660

gacaacgacg ctagattggc tttggaccca caaaacagat acagagttca aaccaagcca     720

cacggtcacg gtgacgttca ctctttgttg cactcttctg gtatcttgaa ggtttggtac     780

aacgctggtt tgaagtgggt tttgttcttc caagacacca acggtttgtt gttcaaggct     840

atcccatctg ctttgggtgt ttcttctacc aagcaatacc acgttaactc tttggctgtt     900

ccaagaaagg ctaaggaagc tatcggtggt atcaccagat tgacccactc tgacggtaga     960

tctatggtta tcaacgttga atacaaccaa ttggacccat tgttgagagc ttctggttac    1020

ccagacggtg acgttaactc tgaaactggt tactctccat tcccaggtaa catcaaccaa    1080
```

```
ttgatcttgg aattgggtcc atacatcgaa gaattggcta agactggtgg tgctatccaa   1140

gaatttgtta acccaaagta caaggacgct tctaagacct ctttcaagtc ttctaccaga   1200

ttggaatgta tgatgcaaga ctacccaaag accttgccac catcttcaag agttggtttc   1260

accgttatgg aaacctggtt cgcttacgct ccagttaaga acaacgctga agacgctgct   1320

aaggttccaa agggtaaccc ataccactct gctacctctg gtgaaatggc tatctacaga   1380

gctaactctt tgatcttgaa gaaggctggt ttccaagttg ctgacccagt tttgcaagtt   1440

atcaacggtc aagaagttga agtttggcca agaatcacct ggaagccaaa gtggggtttg   1500

accttctctt tggttaagtc taaggtttct ggtaactgtt ctatctctca aagatctacc   1560

ttggctatca agggtagaaa gatcttcatc gaaaacttgt ctgttgacgg tgctttgatc   1620

gttgacgctg ttgacgacgc tgaagttaac gtttctggtt ctgttcaaaa caacggttgg   1680

gctttggaac cagttgacta caaggactct tctgaaccag aagtttttgag aatcagaggt   1740

ttcaagttca acaaggttga acaagttgaa aagaagtact ctgaaccagg taagttcgac   1800

ttcaaggctt aagcgcgc                                                 1818
```

```
<210>  22
<211>  1860
<212>  DNA
<213>  Artificial

<220>
<223>  Codon optimized synthetic ORF 2.7.7.64 Arabidopsis thaliana

<400>  22
gctagcaaaa tggcttctac cgttgactct aacttcttct cttctgttcc agctttgcac     60

tctaacttgg gtttgttgtc tccagaccaa atcgaattgg ctaagatctt gttggaaaac    120

ggtcaatctc acttgttcca acaatggcca gaattgggtg ttgacgacaa ggaaaagttg    180

gctttcttcg accaaatcgc tagattgaac tcttcttacc caggtggttt ggctgcttac    240

atcaagaccg ctaaggaatt gttggctgac tctaaggttg gtaagaaccc atacgacggt    300

ttttctccat ctgttccatc tggtgaaaac ttgaccttcg gtaccgacaa cttcatcgaa    360

atggaaaaga gaggtgttgt tgaagctaga aacgctgctt tcgttttggt tgctggtggt    420

ttgggtgaaa gattgggtta caacggtatc aaggttgctt tgccaagaga aaccaccact    480

ggtacctgtt tcttgcaaca ctacatcgaa tctatcttgg ctttgcaaga agcttctaac    540

aagatcgact ctgacggttc tgaaagagac atcccattca tcatcatgac ctctgacgac    600

acccactcta gaaccttgga cttgttggaa ttgaactctt acttcggtat gaagccaacc    660

caagttcact tgttgaagca agaaaaggtt gcttgtttgg acgacaacga cgctagattg    720

gctttggacc cacacaacaa gtactctatc caaaccaagc cacacggtca cggtgacgtt    780

cactctttgt tgtactcttc tggtttgttg cacaagtggt tggaagctgg tttgaagtgg    840
```

gttttgttct tccaagacac caacggtttg ttgttcaacg ctatcccagc ttctttgggt 900

gtttctgcta ccaagcaata ccacgttaac tctttggctg ttccaagaaa ggctaaggaa 960

gctatcggtg gtatctctaa gttgacccac gttgacggta gatctatggt tatcaacgtt 1020

gaatacaacc aattggaccc attgttgaga gcttctggtt tcccagacgg tgacgttaac 1080

tgtgaaactg gtttctctcc attcccaggt aacatcaacc aattgatctt ggaattgggt 1140

ccatacaagg acgaattgca aaagactggt ggtgctatca aggaattcgt taacccaaag 1200

tacaaggact ctaccaagac cgctttcaag tcttctacca gattggaatg tatgatgcaa 1260

gactacccaa agaccttgcc accaaccgct agagttggtt tcaccgttat ggacatctgg 1320

ttggcttacg ctccagttaa gaacaaccca gaagacgctg ctaaggttcc aaagggtaac 1380

ccataccact ctgctacctc tggtgaaatg gctatctaca gagctaactc tttgatcttg 1440

caaaaggctg gtgttaaggt tgaagaacca gttaagcaag ttttgaacgg tcaagaagtt 1500

gaagtttggt ctagaatcac ctggaagcca aagtggggta tgatcttctc tgacatcaag 1560

aagaaggttt ctggtaactg tgaagtttct caaagatcta ccatggctat caagggtaga 1620

aacgttttca tcaaggactt gtctttggac ggtgctttga tcgttgactc tatcgacgac 1680

gctgaagtta agttgggtgg tttgatcaag aacaacggtt ggaccatgga atctgttgac 1740

tacaaggaca cctctgttcc agaagaaatc agaatcagag gtttcagatt caacaaggtt 1800

gaacaattgg aaaagaagtt gacccaacca ggtaagttct ctgttgaaga ctaagcgcgc 1860

<210> 23
<211> 1377
<212> DNA
<213> Artificial

<220>
<223> Codon optimized synthetic ORF 1.1.1.22 PA2022 P. aeruginosa

<400> 23
ctgcagaaaa tgagattgtg tgttatcggt gctggttacg ttggtttggt taccgctgct 60

tgtttcgctg aaatgggtaa ccaagttaga tgtgttgaaa gagacagaga aagagttgct 120

agattgagaa gaggtgaaat gccaatctac gaaccaggtt tggaatctat cttgagagac 180

caattggacg ctgctagatt aaccttcacc gcttctttgg ctgaaggttt ggctgacgct 240

gaagttgttt tcatcgctgt tggtacccca tgtggtgaag acggttcagc tgacttgtct 300

cacgttttgg ctgttgctga acaattgggt gctcaattga gacaagcttg tatcgttgtt 360

aacaagtcta ccgttccagt tggtaccgct gaaagagttg aagaaatcat cagattgggt 420

ttggctagaa gaagaaagag attcagagtt gctgttgctt ctaacccaga attcttgaag 480

gaaggttctg ctgttgacga cttcagaaga ccagacagag ttatcatcgg ttctgctgaa 540

acccaagctg gtgaaacctt gagacaattg tatgctccat tcttgagaaa ccacgaaaga 600

```
gttttgttga tgggtagaag agaagctgaa ttctctaagt acgctgctaa cgctttcttg    660

gctaccaaga tctctttcat gaacgaaatg gctggtttgt gtgctttgac cggtgttgac    720

atcgaagacg ttagaagagg tatgggttct gacaagagaa tcggtaccca ctttatctac    780

gctggttgtg gttacggtgg ttcttgtttc ccaaaggacg ttagagcttt gatcagatct    840

gctgaacaac aaggttacga ctctcaaatc ttgagagctg ttgaagctag aaacgctaga    900

caaaaggaat tgttgttcga aaccttgggt gaattgttcc aaggtagatg gcaaggtaga    960

accgttgctt tgtggggttt ggctttcaag ccaggtaccg acgacttgag agaagctcca   1020

tctttggttt tgttggaagc tttgttgaga cacggtgtta gagttagagc acacgaccca   1080

gttgctaacg ctggtgttgc tgctagatac ccagaagctg ttgcttgtgc tagattgacc   1140

ttgcacgact ctccatacgc tgctgttgaa ggtgctgacg ctttggtttt ggttaccgaa   1200

tggaagcaat tcagacaacc agacttccaa aagatcagag gttctatgag aaccccattg   1260

ttggttgacg gtagaaactt gtacgctcca gctagaatgg ctgaattggg ttttatctac   1320

caaggtatcg gtagaccaag agctggtcac tgtaaggctt ctgctgctta aggatcc      1377
```

```
<210>  24
<211>  1410
<212>  DNA
<213>  Artificial

<220>
<223>  Codon optimized synthetic ORF 1.1.1.22 PA 3559 P.aeruginosa

<400>  24
ctgcagaaaa tgaagatctc tgttttcggt tctggttacg ttggtttggt tcaagctgct     60

gttttggctg aagttggtca cgacgttttg tgtatggaca tcgacagaaa caaggttgaa    120

agattggctc aaggtttggc ttctatctac gaaccaggtt tggacgcttt gttgagagaa    180

ggtttggact ctggtagatt gagattctct tctgacgcta gattggctgt tgaacacggt    240

agagttcaat tcatcgctgt tggtacccca ccaggtgaag acggtgctgc tgacttgggt    300

gctgttttcg ttgttgctga cgctatcgct gaacacagaa gaaagccagt tatcgttgtt    360

gaaaagtcta ccgttccagt tggtacaggt gacagattga gagcacacat cgaaagaaga    420

ttggaagctg acggtagaga attggaattt gaaatcgttt ctaacccaga atttttgaag    480

gaaggttctg ctgttgctga ctgtagaaga ccagacagaa tcgttgttgg ttgtgacaac    540

gaagaagtta gacaagttat gagagaactt tacgaaccat tcaacagaaa ccacgacaga    600

atgttgttca tgggtttgag atctgctgaa ttgaccaagt acgctgctaa cggtatgttg    660

gctaccaaga tttctttcat caaccaaatc gctgaattgg ctgaacactt aggtgctgac    720

atcgaagctg ttagacaagg tatcggtgct gacccaagaa tcggttacca cttcatctac    780

ccaggttgtg gttacggtgg ttcttgtttc ccaaaggaca tgagagcttt gatccactct    840
```

gctgaacaag ctcactgttc ttctgacttg ttgcaagctg ttgaagctat caacagaaga 900

caaaagcaca agttgttcga aagaatcaga gttttctacg acggtgactt gagaggtaag 960

accttcgctt tgtggggttt ggctttcaag ccaaacaccg acgacatgag agacgctcca 1020

tcaagagaat tgatggaagc tttgtggaga cacggtgctc aagttagagc ttacgatcca 1080

gaagctatgc aagaaaccca aagattgtac ggtcacgacg aaagattgtc tttgatgggt 1140

accccagaag ctaccttggg tggtgctgac gctttggtta tctgtaccga atggcaacaa 1200

ttcaaggctc cagacttcga attgttgaag gaaagattga aggctccagt tatcttcgac 1260

ggtagaaact tgtacgaccc agaaagaatg gctagacacg gtttccacta ctacccaatg 1320

ggtagaggtc aatcttgttc tttgccaatc aacgaggctt ctttggctca agaagacggt 1380

atgagattgt tgagacaagc ttagggatcc 1410

<210> 25
<211> 1306
<212> DNA
<213> Artificial

<220>
<223> Codon optimized synthetic ORF 5.1.3.6 Arabidopsis thaliana

<400> 25
ctgcagaaaa atgccatcta tcgaagacga attgttccca tctaccccag gtaagttcaa 60

gatcgacaga tctaacagac aattgcacag atgtttcgct tctacctcta ccatgttctt 120

gtgggctttg ttcttgatcg ctttgactgc ttcttacttg tctttccaat ctttcgttga 180

ctctggttct agatacttga ccgcttcttg ggtggtatc caatgggaaa agcaagttag 240

aacctctgct caaatccaca gatctggtgg tatctctgtt ttggttaccg gtgctaccgg 300

tttcgttggt tctcacgttt ctttggcttt gagaaagaga ggtgacggtg ttgttggttt 360

ggacaacttc aacaactact acgacccatc tttgaagaga gctagaagat ctttgttgtc 420

ttctagaggt atcttcgttg ttgaaggtga cttgaacgac gctaagttgt tggctaagtt 480

gttcgacgtt gttgctttca cccacgttat gcacttggct gctcaagctg gtgttagata 540

cgctttggaa aacccacaat cttacgttca ctctaacatc gctggtttgg ttaacttgtt 600

ggaaatctgt aaggctgcta acccacaacc agctatcgtt tgggcttctt cttcttctgt 660

ttacggtttg aacgaaaagg ttccattctc tgaatctgac agaaccgacc aaccagcttc 720

tttgtacgct gctaccaaga aggctggtga agaaatcacc cacacctaca accacatcta 780

cggtttggct atcaccggtt tgagattctt caccgtttac ggtccatggg gtagaccaga 840

catggcttac ttctctttca ccagaaacat cttgcaaggt aagccaatca ccatctacag 900

aggtaagaac agagttgact tggctagaga cttcacctac atcgacgaca tcgttaaggg 960

ttgtttgggt tctttggact cttctggtaa gtctaccggt tctggtggta agaagagagg 1020

tgctgctcca tacagaatct tcaacttggg taacacctct ccagttaccg ttccaatctt 1080

ggttgacatc ttggaaaagc acttgaaggt taaggctaag agaaacttcg ttgaaatgcc 1140

aggtaacggt gacgttccat tcacccacgc taacatctct tctgctagaa acgaattcgg 1200

ttacaagcca accaccgact tggaaaccgg tttgaagaag ttcgttagat ggtacttgtc 1260

ttactacggt tacaacacca aggctaagtt ggttcactaa gcgcgc 1306

<210> 26
<211> 5949
<212> DNA
<213> Artificial

<220>
<223> pRN599 shuttle vector

<400> 26
ggaatacagg taagcaaatt gatactaatg gctcaacgtg ataaggaaaa agaattgcac 60

tttaacatta atattgacaa ggaggagggc accacacaaa aagttaggtg taacagaaaa 120

tcatgaaact acgattccta atttgatatt ggaggatttt ctctaaaaaa aaaaaaatac 180

aacaaataaa aaacactcaa tgacctgacc atttgatgga gtttaagtca ataccttctt 240

gaaccatttc ccataatggt gaaagttccc tcaagaattt tactctgtca gaaacggcct 300

tacgacgtag tcgatatggt gcactctcag tacaatctgc tctgatgccg catagttaag 360

ccagccccga cacccgccaa cacccgctga cgcgccctga cgggcttgtc tgctcccggc 420

atccgcttac agacaagctg tgaccgtctc cgggagctgc atgtgtcaga ggttttcacc 480

gtcatcaccg aaacgcgcga gacgaaaggg cctcgtgata cgcctatttt tataggttaa 540

tgtcatgata ataatggttt cttagacgtc aggtggcact tttcggggaa atgtgcgcgg 600

aacccctatt tgtttatttt tctaaataca ttcaaatatg tatccgctca tgagacaata 660

accctgataa atgcttcaat aatattgaaa aaggaagagt atgagtattc aacatttccg 720

tgtcgccctt attccctttt ttgcggcatt ttgccttcct gtttttgctc acccagaaac 780

gctggtgaaa gtaaaagatg ctgaagatca gttgggtgca cgagtgggtt acatcgaact 840

ggatctcaac agcggtaaga tccttgagag ttttcgcccc gaagaacgtt ttccaatgat 900

gagcactttt aaagttctgc tatgtggcgc ggtattatcc cgtattgacg ccgggcaaga 960

gcaactcggt cgccgcatac actattctca gaatgacttg gttgagtact caccagtcac 1020

agaaaagcat cttacggatg gcatgacagt aagagaatta tgcagtgctg ccataaccat 1080

gagtgataac actgcggcca acttacttct gacaacgatc ggaggaccga aggagctaac 1140

cgcttttttt cacaacatgg gggatcatgt aactcgcctt gatcgttggg aaccggagct 1200

gaatgaagcc ataccaaacg acgagcgtga caccacgatg cctgtagcaa tggcaacaac 1260

gttgcgcaaa ctattaactg gcgaactact tactctagct tcccggcaac aattaataga 1320

```
ctggatggag gcggataaag ttgcaggacc acttctgcgc tcggcccttc cggctggctg   1380
gtttattgct gataaatctg gagccggtga gcgtgggtct cgcggtatca ttgcagcact   1440
ggggccagat ggtaagccct cccgtatcgt agttatctac acgacgggca gtcaggcaac   1500
tatggatgaa cgaaatagac agatcgctga gataggtgcc tcactgatta agcattggta   1560
actgtcagac caagtttact catatatact ttagattgat ttaaaacttc atttttaatt   1620
taaaaggatc taggtgaaga tcctttttga taatctcatg accaaaatcc cttaacgtga   1680
gttttcgttc cactgagcgt cagaccccgt agaaaagatc aaaggatctt cttgagatcc   1740
tttttttctg cgcgtaatct gctgcttgca aacaaaaaaa ccaccgctac cagcggtggt   1800
ttgtttgccg gatcaagagc taccaactct ttttccgaag gtaactggct tcagcagagc   1860
gcagatacca aatactgtcc ttctagtgta gccgtagtta ggccaccact tcaagaactc   1920
tgtagcaccg cctacatacc tcgctctgct aatcctgtta ccagtggctg ctgccagtgg   1980
cgataagtcg tgtcttaccg ggttggactc aagacgatag ttaccggata aggcgcagcg   2040
gtcgggctga acggggggtt cgtgcacaca gcccagcttg gagcgaacga cctacaccga   2100
actgagatac ctacagcgtg agcattgaga aagcgccacg cttcccgaag ggagaaaggc   2160
ggacaggtat ccggtaagcg gcagggtcgg aacaggagag cgcacgaggg agcttccagg   2220
ggggaacgcc tggtatcttt atagtcctgt cgggtttcgc cacctctgac ttgagcgtcg   2280
atttttgtga tgctcgtcag gggggcgag cctatggaaa aacgccagca acgcggcctt   2340
tttacggttc ctggcctttt gctggccttt tgctcacatg ttctttcctg cgttatcccc   2400
tgattctgtg gataaccgta ttaccgcctt tgagtgagct gataccgctc gccgcagccg   2460
aacgaccgag cgcagcgagt cagtgagcga ggaagcggaa gagcgcccaa tacgcaaacc   2520
gcctctcccc gcgcgttggc cgattcatta atgcagctgg cacgacaggt ttcccgactg   2580
gaaagcgggc agtgagcgca acgcaattaa tgtgagttac ctcactcatt aggcacccca   2640
ggctttacac tttatgcttc cggctcctat gttgtgtgga attgtgagcg gataacaatt   2700
tcacacagga aacagctatg accatgatta cgccaagctc ggaattaacc ctcactaaag   2760
ggaacaaaag ctgggtaccg ggccccccct cgaggtcgac ggtatcgata agcttgatat   2820
cgaattcctg cagcccgggg gatccactag ttctagagcg gccgcatgct agctccggat   2880
tatcgatgat aagctgtcaa agatgagaat taattccacg gactatagac tatactagat   2940
actccgtcta ctgtacgata cacttccgct caggtccttg tcctttaacg aggccttacc   3000
actcttttgt tactctattg atccagctca gcaaaggcag tgtgatctaa gattctatct   3060
tcgcgatgta gtaaaactag ctagaccgag aaagagacta gaaatgcaaa aggcacttct   3120
acaatggctg ccatcattat tatccgatgt gacgctgcag cttctcaatg atattcgaat   3180
acgctttgag gagatacagc ctaatatccg acaaactgtt ttacagattt acgatcgtac   3240
```

ttgttaccca tcattgaatt ttgaacatcc gaacctggga gttttccctg aaacagatag 3300

tatatttgaa cctgtataat aatatatagt ctagcgcttt acggaagaca atgtatgtat 3360

ttcggttcct ggagaaacta ttgcatctat tgcataggta atcttgcacg tcgcatcccc 3420

ggttcatttt ctgcgtttcc atcttgcact tcaatagcat atctttgtta acgaagcatc 3480

tgtgcttcat tttgtagaac aaaaatgcaa cgcgagagcg ctaatttttc aaacaaagaa 3540

tctgagctgc atttttacag aacagaaatg caacgcgaaa gcgctatttt accaacgaag 3600

aatctgtgct tcatttttgt aaaacaaaaa tgcaacgcga cgagagcgct aatttttcaa 3660

acaaagaatc tgagctgcat ttttacagaa cagaaatgca acgcgagagc gctattttac 3720

caacaaagaa tctatacttc ttttttgttc tacaaaaatg catcccgaga gcgctatttt 3780

tctaacaaag catcttagat tacttttttt ctcctttgtg cgctctataa tgcagtctct 3840

tgataacttt ttgcactgta ggtccgttaa ggttagaaga aggctacttt ggtgtctatt 3900

ttctcttcca taaaaaaagc ctgactccac ttcccgcgtt tactgattac tagcgaagct 3960

gcgggtgcat tttttcaaga taaaggcatc cccgattata ttctataccg atgtggattg 4020

cgcatacttt gtgaacagaa agtgatagcg ttgatgattc ttcattggtc agaaaattat 4080

gaacggtttc ttctattttg tctctatata ctacgtatag gaaatgttta cattttcgta 4140

ttgttttcga ttcactctat gaatagttct tactacaatt tttttgtcta aagagtaata 4200

ctagagataa acataaaaaa tgtagaggtc gagtttagat gcaagttcaa ggagcgaaag 4260

gtggatgggt aggttatata gggatatagc acagagatat atagcaaaga gatacttttg 4320

agcaatgttt gtggaagcgg tattcgcaat gccggagctc gtacgttcga acttaaggcc 4380

tcgtccccgc cgggtcaccc ggccagcgac atggaggccc agaataccct ccttgacagt 4440

cttgacgtgc gcagctcagg ggcatgatgt gactgtcgcc cgtacattta gcccatacat 4500

ccccatgtat aatcatttgc atccatacat tttgatggcc gcacggcgcg aagcaaaaat 4560

tacggctcct cgctgcagac ctgcgagcag ggaaacgctc ccctcacaga cgcgttgaat 4620

tgtccccacg ccgcgcccct gtagagaaat ataaaaggtt aggatttgcc actgaggttc 4680

ttctttcata tacttccttt taaaatcttg ctaggataca gttctcacat cacatccgaa 4740

cataaacaac catgggtaag gaaaagactc acgtttcgag gccgcgatta aattccaaca 4800

tggatgctga tttatatggg tataaatggg ctcgcgataa tgtcgggcaa tcaggtgcga 4860

caatctatcg attgtatggg aagcccgatg cgccagagtt gtttctgaaa catggcaaag 4920

gtagcgttgc caatgatgtt acagatgaga tggtcagact aaactggctg acggaattta 4980

tgcctcttcc gaccatcaag cattttatcc gtactcctga tgatgcatgg ttactcacca 5040

ctgcgatccc cggcaaaaca gcattccagg tattagaaga atatcctgat tcaggtgaaa 5100

atattgttga tgcgctggca gtgttcctgc gccggttgca ttcgattcct gtttgtaatt 5160

```
gtccttttaa cagcgatcgc gtatttcgtc tcgctcaggc gcaatcacga atgaataacg   5220

gtttggttga tgcgagtgat tttgatgacg agcgtaatgg ctggcctgtt gaacaagtct   5280

ggaaagaaat gcataagctt ttgccattct caccggattc agtcgtcact catggtgatt   5340

tctcacttga taaccttatt tttgacgagg ggaaattaat aggttgtatt gatgttggac   5400

gagtcggaat cgcagaccga taccaggatc ttgccatcct atggaactgc ctcggtgagt   5460

tttctccttc attacagaaa cggctttttc aaaaatatgg tattgataat cctgatatga   5520

ataaattgca gtttcatttg atgctcgatg agtttttcta atcagtactg acaataaaaa   5580

gattcttgtt ttcaagaact tgtcatttgt atagtttttt tatattgtag ttgttctatt   5640

ttaatcaaat gttagcgtga tttatatttt ttttcgcctc gacatcatct gcccagatgc   5700

gaagttaagt gcgcagaaag taatatcatg cgtcaatcgt atgtgaatgc tggtcgctat   5760

actgctgtcg attcgatact aacgccgcca tccagtgtcg acggatccta ggtgtacata   5820

aactttataa atgaaattca taatagaaac gacacgaaat tacaaaatgg aatatgttca   5880

tagggtagac gaaactatat acgcaatcta catacattta tcaagaagga gaaaaaggag   5940

gatagtaaa                                                          5949
```

```
<210>  27
<211>  2577
<212>  DNA
<213>  Artificial

<220>
<223>  Expression cassette for EC 2.7.7.64 from Pisum sativum

<400>  27
gtcgaccggt cccacacacc atagcttcaa aatgtttcta ctcctttttt actcttccag     60

attttctcgg actccgcgca tcgccgtacc acttcaaaac acccaagcac agcatactaa    120

atttcccctc tttcttcctc tagggtgtcg ttaattaccc gtactaaagg tttggaaaag    180

aaaaaagaga ccgcctcgtt tctttttctt cgtcgaaaaa ggcaataaaa atttttatca    240

cgtttctttt tcttgaaaat tttttttttt gatttttttc tctttcgatg acctcccatt    300

gatatttaag ttaataaacg gtcttcaatt tctcaagttt cagtttcatt tttcttgttc    360

tattacaact ttttttactt cttgctcatt agaaagaaag catagcaatc taatctaagc    420

tgcagaagct taaaatggct tcttctttgg gtgacaactt caacttgttg tctccacaac    480

aaagagaatt ggttaagatg ttgttggaca acggacaaga ccacttgttc agagactggc    540

caaacccagg tgttgacgac gacgaaaaga aggctttctt cgaccaattg gttttgttgg    600

actcttctta cccaggtggt ttggttgctt acatcaacaa cgctaagaga ttgttggctg    660

actctaaggc tggtaacaac ccattcgacg gtttcacccc atctgttcca actggtgaaa    720

ccttgaagtt cggtgacgaa aacttcaaca agtacgaaga agctggtgtt agagaagcta    780
```

gaagagctgc tttcgttttg gttgctggtg gtttgggtga aagattgggt tacaacggta 840
tcaaggttgc tttgccagct gaaaccacca ctggtacctg tttcttgcaa cactacatcg 900
aatctatctt ggctttgcaa gaagcaagtt ctgaaggtga aggacaaacc cacatcccat 960
tcgttatcat gacctctgac gacacccacg gtagaacctt ggacttgttg gaatctaact 1020
cttacttcgg tatgcaacca acccaagtta ccttgttgaa gcaagaaaag gttgcttgtt 1080
tggaagacaa cgacgctaga ttggctttgg acccacaaaa cagatacaga gttcaaacca 1140
agccacacgg tcacggtgac gttcactctt tgttgcactc ttctggtatc ttgaaggttt 1200
ggtacaacgc tggtttgaag tgggttttgt tcttccaaga caccaacggt ttgttgttca 1260
aggctatccc atctgctttg ggtgtttctt ctaccaagca ataccacgtt aactctttgg 1320
ctgttccaag aaaggctaag gaagctatcg gtggtatcac cagattgacc cactctgacg 1380
gtagatctat ggttatcaac gttgaataca accaattgga cccattgttg agagcttctg 1440
gttacccaga cggtgacgtt aactctgaaa ctggttactc tccattccca ggtaacatca 1500
accaattgat cttggaattg ggtccataca tcgaagaatt ggctaagact ggtggtgcta 1560
tccaagaatt tgttaaccca aagtacaagg acgcttctaa gacctctttc aagtcttcta 1620
ccagattgga atgtatgatg caagactacc caaagacctt gccaccatct tcaagagttg 1680
gtttcaccgt tatggaaacc tggttcgctt acgctccagt taagaacaac gctgaagacg 1740
ctgctaaggt tccaaagggt aacccatacc actctgctac ctctggtgaa atggctatct 1800
acagagctaa ctctttgatc ttgaagaagg ctggtttcca agttgctgac ccagttttgc 1860
aagttatcaa cggtcaagaa gttgaagttt ggccaagaat cacctggaag ccaaagtggg 1920
gtttgacctt ctctttggtt aagtctaagg tttctggtaa ctgttctatc tctcaaagat 1980
ctaccttggc tatcaagggt agaaagatct tcatcgaaaa cttgtctgtt gacggtgctt 2040
tgatcgttga cgctgttgac gacgctgaag ttaacgtttc tggttctgtt caaaacaacg 2100
gttgggcttt ggaaccagtt gactacaagg actcttctga accagaagtt ttgagaatca 2160
gaggtttcaa gttcaacaag gttgaacaag ttgaaaagaa gtactctgaa ccaggtaagt 2220
tcgacttcaa ggcttaagcg cgcgaatttc ttatgattta tgatttttat tattaaataa 2280
gttataaaaa aaataagtgt atacaaattt taaagtgact cttaggtttt aaaacgaaaa 2340
ttcttattct tgagtaactc tttcctgtag gtcaggttgc tttctcaggt atagcatgag 2400
gtcgctctta ttgaccacac ctctaccggc atgccgagca aatgcctgca aatcgctccc 2460
catttcaccc aattgtagat atgctaactc cagcaatgag ttgatgaatc tcggtgtgta 2520
ttttatgtcc tcagaggaca acacctgttg taatcgttct tccacacgta cgtcgac 2577

<210> 28
<211> 2463

<212> DNA
<213> Artificial

<220>
<223> Expression cassette for EC 1.1.1.22 PA2022 from P.aeruginosa

<400> 28

```
gagctcgtac gcatatgatc atgtgtcgtc gcacacatat atatatgcct gtatgtgtca      60
gcactaaagt tgcctggcca tccacgctat atatacacgc ctggcggatc tgctcgagga     120
ttgcctacgc gtgggcttga tccaccaacc aacgctcgcc aaatgaactg gcgctttggt     180
cttctgccat cgtccgtaaa ccccggccaa agagaccgga aagatcggtg aaaacatctt     240
gatcttgctc ccgggaattt tagattcagg taggaaattg attacatcaa tactgttacc     300
ctgaatcata ttcgacgatg tcgtctcaca cggaaatata attcatttct tggttttcca     360
aaaaaatttt catttttttt cacttttttg tttcgtcctc cttttttttt tttttttttt     420
attttttttc ctgtgttcac cttttttttt ttcagttgac atctttctgc attctttctt     480
gtgttttttt tttttttttt cgtttttcca ttgttcgttc gttgcctgtt ttttcgccct     540
attgttctcg agcctaaaaa tttttttcctt tcctgctttc ctttcttcgt tcaaagtttc    600
ctattccatt gttctctttg gtaaactcat tgttgtcgga actcagatat attcaggtca     660
atttactgta cttcaattga ctttttttctt gaaatttcaa cttgcctttt caacttgttc    720
ttcttttttta atcttattct acactttagt tcccttacct tgttcctaat tattgtctag    780
caaaaagaaa acatacacct atttcattca cacactgcag aaaatgagat tgtgtgttat     840
cggtgctggt tacgttggtt tggttaccgc tgcttgtttc gctgaaatgg gtaaccaagt     900
tagatgtgtt gaaagagaca gagaaagagt tgctagattg agaagaggtg aaatgccaat     960
ctacgaacca ggtttggaat ctatcttgag agaccaattg gacgctgcta gattaacctt    1020
caccgcttct ttggctgaag gtttggctga cgctgaagtt gttttcatcg ctgttggtac    1080
cccatgtggt gaagacggtt cagctgactt gtctcacgtt ttggctgttg ctgaacaatt    1140
gggtgctcaa ttgagacaag cttgtatcgt tgttaacaag tctaccgttc cagttggtac    1200
cgctgaaaga gttgaagaaa tcatcagatt gggtttggct agaagaagaa agagattcag    1260
agttgctgtt gcttctaacc cagaattctt gaaggaaggt tctgctgttg acgacttcag    1320
aagaccagac agagttatca tcggttctgc tgaaacccaa gctggtgaaa ccttgagaca    1380
attgtatgct ccattcttga gaaaccacga aagagttttg ttgatgggta gaagagaagc    1440
tgaattctct aagtacgctg ctaacgcttt cttggctacc aagatctctt tcatgaacga    1500
aatggctggt ttgtgtgctt tgactggtgt tgacatcgaa gacgttagaa gaggtatggg    1560
ttctgacaag agaatcggta cccactttat ctacgctggt tgtggttacg gtggttcttg    1620
tttcccaaag gacgttagag ctttgatcag atctgctgaa caacaaggtt acgactctca    1680
aatcttgaga gctgttgaag ctagaaacgc tagacaaaag gaattgttgt tcgaaacctt    1740
```

gggtgaattg ttccaaggta gatggcaagg tagaaccgtt gctttgtggg gtttggcttt 1800

caagccaggt accgacgact tgagagaagc tccatctttg gttttgttgg aagctttgtt 1860

gagacacggt gttagagtta gagcacacga cccagttgct aacgctggtg ttgctgctag 1920

atacccagaa gctgttgctt gtgctagatt gaccttgcac gactctccat acgctgctgt 1980

tgaaggtgct gacgctttgg ttttggttac cgaatggaag caattcagac aaccagactt 2040

ccaaaagatc agaggttcta tgagaacccc attgttggtt gacggtagaa acttgtacgc 2100

tccagctaga atggctgaat tgggttttat ctaccaaggt atcggtagac caagagctgg 2160

tcactgtaag gcttctgctg cttaaggatc ccctttttcct ttgtcgatat catgtaatta 2220

gttatgtcac gcttacattc acgccctcct cccacatccg ctctaaccga aaaggaagga 2280

gttagacaac ctgaagtcta ggtccctatt tatttttttt aatagttatg ttagtattaa 2340

gaacgttatt tatatttcaa atttttcttt tttttctgta caaacgcgtg tacgcatgta 2400

acattatact gaaaaccttg cttgagaagg ttttgggacg ctcgaaggct tcctagggtc 2460

gac 2463

<210> 29
<211> 2386
<212> DNA
<213> Artificial

<220>
<223> ADH 1 expression cassette for EC 1.1.1.22 PA2022 from P.aeruginosa

<400> 29
aagagctccg gactagtcgt acgaattcta tccttttgtt gtttccgggt gtacaatatg 60

gacttcctct tttctggcaa ccaaacccat acatcgggat tcctataata ccttcgttgg 120

tctccctaac atgtaggtgg cggaggggag atatacaata gaacagatac cagacaagac 180

ataatgggct aaacaagact acaccaatta cactgcctca ttgatggtgg tacataacga 240

actaatactg tagccctaga cttgatagcc atcatcatat cgaagtttca ctaccctttt 300

tccatttgcc atctattgaa gtaataatag gcgcatgcaa cttcttttct tttttttttct 360

tttctctctc ccccgttgtt gtctcaccat atccgcaatg acaaaaaaat gatggaagac 420

actaaaggaa aaaattaacg acaaagacag caccaacaga tgtcgttgtt ccagagctga 480

tgaggggtat ctcgaagcac acgaaacttt ttccttcctt cattcacgca cactactctc 540

taatgagcaa cggtatacgg ccttccttcc agttacttga atttgaaata aaaaaagttt 600

gctgtcttgc tatcaagtat aaatagacct gcaattatta atcttttgtt tcctcgtcat 660

tgttctcgtt ccctttcttc cttgtttctt tttctgcaca atatttcaag ctataccaag 720

catacaatca actccagctg cagaaaatga gattgtgtgt tatcggtgct ggttacgttg 780

gtttggttac cgctgcttgt ttcgctgaaa tgggtaacca agttagatgt gttgaaagag 840

acagagaaag agttgctaga ttgagaagag gtgaaatgcc aatctacgaa ccaggtttgg 900

aatctatctt gagagaccaa ttggacgctg ctagattaac cttcaccgct tctttggctg 960

aaggtttggc tgacgctgaa gttgttttca tcgctgttgg taccccatgt ggtgaagacg 1020

gttcagctga cttgtctcac gttttggctg ttgctgaaca attgggtgct caattgagac 1080

aagcttgtat cgttgttaac aagtctaccg ttccagttgg taccgctgaa agagttgaag 1140

aaatcatcag attgggtttg gctagaagaa gaaagagatt cagagttgct gttgcttcta 1200

acccagaatt cttgaaggaa ggttctgctg ttgacgactt cagaagacca gacagagtta 1260

tcatcggttc tgctgaaacc caagctggtg aaaccttgag acaattgtat gctccattct 1320

tgagaaacca cgaaagagtt ttgttgatgg gtagaagaga agctgaattc tctaagtacg 1380

ctgctaacgc tttcttggct accaagatct ctttcatgaa cgaaatggct ggtttgtgtg 1440

ctttgactgg tgttgacatc gaagacgtta gaagaggtat gggttctgac aagagaatcg 1500

gtacccactt tatctacgct ggttgtggtt acggtggttc ttgtttccca aaggacgtta 1560

gagctttgat cagatctgct gaacaacaag gttacgactc tcaaatcttg agagctgttg 1620

aagctagaaa cgctagacaa aaggaattgt tgttcgaaac cttgggtgaa ttgttccaag 1680

gtagatggca aggtagaacc gttgctttgt ggggtttggc tttcaagcca ggtaccgacg 1740

acttgagaga agctccatct ttggttttgt tggaagcttt gttgagacac ggtgttagag 1800

ttagagcaca cgacccagtt gctaacgctg gtgttgctgc tagataccca gaagctgttg 1860

cttgtgctag attgaccttg cacgactctc catacgctgc tgttgaaggt gctgacgctt 1920

tggttttggt taccgaatgg aagcaattca gacaaccaga cttccaaaag atcagaggtt 1980

ctatgagaac cccattgttg gttgacggta gaaacttgta cgctccagct agaatggctg 2040

aattgggttt tatctaccaa ggtatcggta gaccaagagc tggtcactgt aaggcttctg 2100

ctgcttaagg atcccctttt cctttgtcga tatcatgtaa ttagttatgt cacgcttaca 2160

ttcacgccct cctcccacat ccgctctaac cgaaaaggaa ggagttagac aacctgaagt 2220

ctaggtccct atttattttt tttaatagtt atgttagtat taagaacgtt atttatattt 2280

caaatttttc ttttttttct gtacaaacgc gtgtacgcat gtaacattat actgaaaacc 2340

ttgcttgaga aggttttggg acgctcgaag gcttcctagg gtcgac 2386

<210> 30
<211> 10868
<212> DNA
<213> Artificial

<220>
<223> pRN830

<400> 30
ggaatacagg taagcaaatt gatactaatg gctcaacgtg ataaggaaaa agaattgcac 60

```
tttaacatta atattgacaa ggaggagggc accacacaaa aagttaggtg taacagaaaa     120
tcatgaaact acgattccta atttgatatt ggaggatttt ctctaaaaaa aaaaaaaatac   180
aacaaataaa aaacactcaa tgacctgacc atttgatgga gtttaagtca ataccttctt   240
gaaccatttc ccataatggt gaaagttccc tcaagaattt tactctgtca gaaacggcct   300
tacgacgtag tcgatatggt gcactctcag tacaatctgc tctgatgccg catagttaag   360
ccagccccga cacccgccaa cacccgctga cgcgccctga cgggcttgtc tgctcccggc   420
atccgcttac agacaagctg tgaccgtctc cgggagctgc atgtgtcaga ggttttcacc   480
gtcatcaccg aaacgcgcga gacgaaaggg cctcgtgata cgcctatttt tataggttaa   540
tgtcatgata ataatggttt cttagacgtc aggtggcact tttcggggaa atgtgcgcgg   600
aacccctatt tgtttatttt tctaaataca ttcaaatatg tatccgctca tgagacaata   660
accctgataa atgcttcaat aatattgaaa aaggaagagt atgagtattc aacatttccg   720
tgtcgccctt attccctttt ttgcggcatt ttgccttcct gtttttgctc acccagaaac   780
gctggtgaaa gtaaaagatg ctgaagatca gttgggtgca cgagtgggtt acatcgaact   840
ggatctcaac agcggtaaga tccttgagag ttttcgcccc gaagaacgtt ttccaatgat   900
gagcactttt aaagttctgc tatgtggcgc ggtattatcc cgtattgacg ccgggcaaga   960
gcaactcggt cgccgcatac actattctca gaatgacttg gttgagtact caccagtcac  1020
agaaaagcat cttacggatg gcatgacagt aagagaatta tgcagtgctg ccataaccat  1080
gagtgataac actgcggcca acttacttct gacaacgatc ggaggaccga aggagctaac  1140
cgcttttttt cacaacatgg gggatcatgt aactcgcctt gatcgttggg aaccggagct  1200
gaatgaagcc ataccaaacg acgagcgtga caccacgatg cctgtagcaa tggcaacaac  1260
gttgcgcaaa ctattaactg gcgaactact tactctagct tcccggcaac aattaataga  1320
ctggatggag gcggataaag ttgcaggacc acttctgcgc tcggcccttc cggctggctg  1380
gtttattgct gataaatctg gagccggtga gcgtgggtct cgcggtatca ttgcagcact  1440
ggggccagat ggtaagccct cccgtatcgt agttatctac acgacgggca gtcaggcaac  1500
tatggatgaa cgaaatagac agatcgctga gataggtgcc tcactgatta agcattggta  1560
actgtcagac caagtttact catatatact ttagattgat ttaaaacttc atttttaatt  1620
taaaaggatc taggtgaaga tcctttttga taatctcatg accaaaatcc cttaacgtga  1680
gttttcgttc cactgagcgt cagaccccgt agaaaagatc aaaggatctt cttgagatcc  1740
tttttttctg cgcgtaatct gctgcttgca aacaaaaaaa ccaccgctac cagcggtggt  1800
ttgtttgccg gatcaagagc taccaactct ttttccgaag gtaactggct tcagcagagc  1860
gcagatacca aatactgtcc ttctagtgta gccgtagtta ggccaccact tcaagaactc  1920
tgtagcaccg cctacatacc tcgctctgct aatcctgtta ccagtggctg ctgccagtgg  1980
```

cgataagtcg tgtcttaccg ggttggactc aagacgatag ttaccggata aggcgcagcg 2040

gtcgggctga acggggggtt cgtgcacaca gcccagcttg gagcgaacga cctacaccga 2100

actgagatac ctacagcgtg agcattgaga aagcgccacg cttcccgaag ggagaaaggc 2160

ggacaggtat ccggtaagcg gcagggtcgg aacaggagag cgcacgaggg agcttccagg 2220

ggggaacgcc tggtatcttt atagtcctgt cgggtttcgc cacctctgac ttgagcgtcg 2280

atttttgtga tgctcgtcag gggggccgag cctatggaaa aacgccagca acgcggcctt 2340

tttacggttc ctggcctttt gctggccttt tgctcacatg ttctttcctg cgttatcccc 2400

tgattctgtg gataaccgta ttaccgcctt tgagtgagct gataccgctc gccgcagccg 2460

aacgaccgag cgcagcgagt cagtgagcga ggaagcggaa gagcgcccaa tacgcaaacc 2520

gcctctcccc gcgcgttggc cgattcatta atgcagctgg cacgacaggt ttcccgactg 2580

gaaagcgggc agtgagcgca acgcaattaa tgtgagttac ctcactcatt aggcacccca 2640

ggctttacac tttatgcttc cggctcctat gttgtgtgga attgtgagcg gataacaatt 2700

tcacacagga aacagctatg accatgatta cgccaagctc ggaattaacc ctcactaaag 2760

ggaacaaaag ctgggtacgc atatgatcat gtgtcgtcgc acacatatat atatgcctgt 2820

atgtgtcagc actaaagttg cctggccatc cacgctatat atacacgcct ggcggatctg 2880

ctcgaggatt gcctacgcgt gggcttgatc caccaaccaa cgctcgccaa atgaactggc 2940

gctttggtct tctgccatcg tccgtaaacc ccggccaaag agaccggaaa gatcggtgaa 3000

aacatcttga tcttgctccc gggaatttta gattcaggta ggaaattgat tacatcaata 3060

ctgttaccct gaatcatatt cgacgatgtc gtctcacacg gaaatataat tcatttcttg 3120

gttttccaaa aaaattttca ttttttttca ctttttttgtt tcgtcctcct ttttttttt 3180

ttttttttat tttttttcct gtgttcacct tttttttttt cagttgacat ctttctgcat 3240

tcttttctgt gttttttttt tttttttcg tttttccatt gttcgttcgt tgcctgtttt 3300

ttcgccctat tgttctcgag cctaaaaatt ttttcctttc ctgctttcct ttctttcgttc 3360

aaagtttcct attccattgt tctctttggt aaactcattg ttgtcggaac tcagatatat 3420

tcaggtcaat ttactgtact tcaattgact tttttcttga aatttcaact tgccttttca 3480

acttgttctt cttttttaat cttattctac actttagttc ccttaccttg ttcctaatta 3540

ttgtctagca aaaagaaaac atacacctat ttcattcaca cactgcagaa aatgagattg 3600

tgtgttatcg gtgctggtta cgttggtttg gttaccgctg cttgtttcgc tgaaatgggt 3660

aaccaagtta gatgtgttga aagagacaga gaaagagttg ctagattgag aagaggtgaa 3720

atgccaatct acgaaccagg tttggaatct atcttgagag accaattgga cgctgctaga 3780

ttaaccttca ccgcttcttt ggctgaaggt ttggctgacg ctgaagttgt tttcatcgct 3840

gttggtaccc catgtggtga agacggttca gctgacttgt ctcacgtttt ggctgttgct 3900

gaacaattgg gtgctcaatt gagacaagct tgtatcgttg ttaacaagtc taccgttcca 3960

gttggtaccg ctgaaagagt tgaagaaatc atcagattgg gtttggctag aagaagaaag 4020

agattcagag ttgctgttgc ttctaaccca gaattcttga aggaaggttc tgctgttgac 4080

gacttcagaa gaccagacag agttatcatc ggttctgctg aaacccaagc tggtgaaacc 4140

ttgagacaat tgtatgctcc attcttgaga aaccacgaaa gagttttgtt gatgggtaga 4200

agagaagctg aattctctaa gtacgctgct aacgctttct tggctaccaa gatctctttc 4260

atgaacgaaa tggctggttt gtgtgctttg actggtgttg acatcgaaga cgttagaaga 4320

ggtatgggtt ctgacaagag aatcggtacc cactttatct acgctggttg tggttacggt 4380

ggttcttgtt tcccaaagga cgttagagct ttgatcagat ctgctgaaca acaaggttac 4440

gactctcaaa tcttgagagc tgttgaagct agaaacgcta gacaaaagga attgttgttc 4500

gaaaccttgg gtgaattgtt ccaaggtaga tggcaaggta gaaccgttgc tttgtggggt 4560

ttggctttca agccaggtac cgacgacttg agagaagctc catctttggt tttgttggaa 4620

gctttgttga gacacggtgt tagagttaga gcacacgacc cagttgctaa cgctggtgtt 4680

gctgctagat acccagaagc tgttgcttgt gctagattga ccttgcacga ctctccatac 4740

gctgctgttg aaggtgctga cgctttggtt ttggttaccg aatggaagca attcagacaa 4800

ccagacttcc aaaagatcag aggttctatg agaaccccat tgttggttga cggtagaaac 4860

ttgtacgctc cagctagaat ggctgaattg ggtttttatct accaaggtat cggtagacca 4920

agagctggtc actgtaaggc ttctgctgct taaggatccc cttttccttt gtcgatatca 4980

tgtaattagt tatgtcacgc ttacattcac gccctcctcc cacatccgct ctaaccgaaa 5040

aggaaggagt tagacaacct gaagtctagg tccctattta tttttttttaa tagttatgtt 5100

agtattaaga acgttattta tatttcaaat ttttcttttt tttctgtaca aacgcgtgta 5160

cgcatgtaac attatactga aaaccttgct tgagaaggtt ttgggacgct cgaaggcttc 5220

ctagggtcga cgtacgtgtg gaagaacgat tacaacaggt gttgtcctct gaggacataa 5280

aatacacacc gagattcatc aactcattgc tggagttagc atatctacaa ttgggtgaaa 5340

tggggagcga tttgcaggca tttgctcggc atgccggtag aggtgtggtc aataagagcg 5400

acctcatgct atacctgaga aagcaacctg acctacagga aagagttact caagaataag 5460

aattttcgtt ttaaaaccta agagtcactt taaaatttgt atacacttat tttttttata 5520

acttatttaa taataaaaat cataaatcat aagaaattcg cgcgcttaag ccttgaagtc 5580

gaacttacct ggttcagagt acttcttttc aacttgttca accttgttga acttgaaacc 5640

tctgattctc aaaacttctg gttcagaaga gtccttgtag tcaactggtt ccaaagccca 5700

accgttgttt tgaacagaac cagaaacgtt aacttcagcg tcgtcaacag cgtcaacgat 5760

caaagcaccg tcaacagaca agttttcgat gaagatcttt ctacccttga tagccaaggt 5820

agatctttga gagatagaac agttaccaga aaccttagac ttaaccaaag agaaggtcaa 5880
accccacttt ggcttccagg tgattcttgg ccaaacttca acttcttgac cgttgataac 5940
ttgcaaaact gggtcagcaa cttggaaacc agccttcttc aagatcaaag agttagctct 6000
gtagatagcc atttcaccag aggtagcaga gtggtatggg ttaccctttg gaaccttagc 6060
agcgtcttca gcgttgttct taactggagc gtaagcgaac caggtttcca taacggtgaa 6120
accaactctt gaagatggtg gcaaggtctt tgggtagtct tgcatcatac attccaatct 6180
ggtagaagac ttgaaagagg tcttagaagc gtccttgtac tttgggttaa caaattcttg 6240
gatagcacca ccagtcttag ccaattcttc gatgtatgga cccaattcca agatcaattg 6300
gttgatgtta cctgggaatg gagagtaacc agtttcagag ttaacgtcac cgtctgggta 6360
accagaagct ctcaacaatg ggtccaattg gttgtattca acgttgataa ccatagatct 6420
accgtcagag tgggtcaatc tggtgatacc accgatagct tccttagcct ttcttggaac 6480
agccaaagag ttaacgtggt attgcttggt agaagaaaca cccaaagcag atgggatagc 6540
cttgaacaac aaaccgttgg tgtcttggaa gaacaaaacc cacttcaaac cagcgttgta 6600
ccaaaccttc aagataccag aagagtgcaa caaagagtga acgtcaccgt gaccgtgtgg 6660
cttggtttga actctgtatc tgttttgtgg gtccaaagcc aatctagcgt cgttgtcttc 6720
caaacaagca accttttctt gcttcaacaa ggtaacttgg gttggttgca taccgaagta 6780
agagttagat tccaacaagt ccaaggttct accgtgggtg tcgtcagagg tcatgataac 6840
gaatgggatg tgggtttgtc cttcaccttc agaacttgct tcttgcaaag ccaagataga 6900
ttcgatgtag tgttgcaaga aacaggtacc agtggtggtt tcagctggca aagcaacctt 6960
gataccgttg taacccaatc tttcacccaa accaccagca accaaaacga aagcagctct 7020
tctagcttct ctaacaccag cttcttcgta cttgttgaag ttttcgtcac cgaacttcaa 7080
ggtttcacca gttggaacag atggggtgaa accgtcgaat gggttgttac cagccttaga 7140
gtcagccaac aatctcttag cgttgttgat gtaagcaacc aaaccacctg ggtaagaaga 7200
gtccaacaaa accaattggt cgaagaaagc cttcttttcg tcgtcgtcaa cacctgggtt 7260
tggccagtct ctgaacaagt ggtcttgtcc gttgtccaac aacatcttaa ccaattctct 7320
ttgttgtgga gacaacaagt tgaagttgtc acccaaagaa gaagccattt taagcttctg 7380
cagcttagat tagattgcta tgctttcttt ctaatgagca agaagtaaaa aaagttgtaa 7440
tagaacaaga aaaatgaaac tgaaacttga gaaattgaag accgtttatt aacttaaata 7500
tcaatgggag gtcatcgaaa gagaaaaaaa tcaaaaaaaa aaattttcaa gaaaaagaaa 7560
cgtgataaaa atttttattg cctttttcga cgaagaaaaa gaaacgaggc ggtctctttt 7620
ttcttttcca aacctttagt acgggtaatt aacgacaccc tagaggaaga aagaggggaa 7680
atttagtatg ctgtgcttgg gtgttttgaa gtggtacggc gatgcgcgga gtccgagaaa 7740

atctggaaga gtaaaaaagg agtagaaaca ttttgaagct atggtgtgtg ggaccggatt 7800
atcgatgata agctgtcaaa gatgagaatt aattccacgg actatagact atactagata 7860
ctccgtctac tgtacgatac acttccgctc aggtccttgt cctttaacga ggccttacca 7920
ctcttttgtt actctattga tccagctcag caaaggcagt gtgatctaag attctatctt 7980
cgcgatgtag taaaactagc tagaccgaga aagagactag aaatgcaaaa ggcacttcta 8040
caatggctgc catcattatt atccgatgtg acgctgcagc ttctcaatga tattcgaata 8100
cgctttgagg agatacagcc taatatccga caaactgttt tacagattta cgatcgtact 8160
tgttacccat cattgaattt tgaacatccg aacctgggag ttttccctga aacagatagt 8220
atatttgaac ctgtataata atatatagtc tagcgcttta cggaagacaa tgtatgtatt 8280
tcggttcctg gagaaactat tgcatctatt gcataggtaa tcttgcacgt cgcatccccg 8340
gttcattttc tgcgtttcca tcttgcactt caatagcata tctttgttaa cgaagcatct 8400
gtgcttcatt ttgtagaaca aaaatgcaac gcgagagcgc taatttttca aacaaagaat 8460
ctgagctgca tttttacaga acagaaatgc aacgcgaaag cgctatttta ccaacgaaga 8520
atctgtgctt catttttgta aaacaaaaat gcaacgcgac gagagcgcta atttttcaaa 8580
caaagaatct gagctgcatt tttacagaac agaaatgcaa cgcgagagcg ctattttacc 8640
aacaaagaat ctatacttct tttttgttct acaaaaatgc atcccgagag cgctattttt 8700
ctaacaaagc atcttagatt actttttttc tcctttgtgc gctctataat gcagtctctt 8760
gataactttt tgcactgtag gtccgttaag gttagaagaa ggctactttg gtgtctattt 8820
tctcttccat aaaaaaagcc tgactccact tcccgcgttt actgattact agcgaagctg 8880
cgggtgcatt ttttcaagat aaaggcatcc ccgattatat tctataccga tgtggattgc 8940
gcatactttg tgaacagaaa gtgatagcgt tgatgattct tcattggtca gaaaattatg 9000
aacggtttct tctattttgt ctctatatac tacgtatagg aaatgtttac attttcgtat 9060
tgttttcgat tcactctatg aatagttctt actacaattt ttttgtctaa agagtaatac 9120
tagagataaa cataaaaaat gtagaggtcg agtttagatg caagttcaag gagcgaaagg 9180
tggatgggta ggttatatag ggatatagca cagagatata tagcaaagag atacttttga 9240
gcaatgtttg tggaagcggt attcgcaatg ccggagctcg tacgttcgaa cttaaggcct 9300
cgtccccgcc gggtcacccg gccagcgaca tggaggccca gaataccctc cttgacagtc 9360
ttgacgtgcg cagctcaggg gcatgatgtg actgtcgccc gtacatttag cccatacatc 9420
cccatgtata atcatttgca tccatacatt ttgatggccg cacggcgcga agcaaaaatt 9480
acggctcctc gctgcagacc tgcgagcagg gaaacgctcc cctcacagac gcgttgaatt 9540
gtccccacgc cgcgcccctg tagagaaata taaaaggtta ggatttgcca ctgaggttct 9600
tctttcatat acttcctttt aaaatcttgc taggatacag ttctcacatc acatccgaac 9660

```
ataaacaacc atgggtaagg aaaagactca cgtttcgagg ccgcgattaa attccaacat    9720

ggatgctgat ttatatgggt ataaatgggc tcgcgataat gtcgggcaat caggtgcgac    9780

aatctatcga ttgtatggga agcccgatgc gccagagttg tttctgaaac atggcaaagg    9840

tagcgttgcc aatgatgtta cagatgagat ggtcagacta aactggctga cggaatttat    9900

gcctcttccg accatcaagc attttatccg tactcctgat gatgcatggt tactcaccac    9960

tgcgatcccc ggcaaaacag cattccaggt attagaagaa tatcctgatt caggtgaaaa   10020

tattgttgat gcgctggcag tgttcctgcg ccggttgcat tcgattcctg tttgtaattg   10080

tccttttaac agcgatcgcg tatttcgtct cgctcaggcg caatcacgaa tgaataacgg   10140

tttggttgat gcgagtgatt ttgatgacga gcgtaatggc tggcctgttg aacaagtctg   10200

gaaagaaatg cataagcttt tgccattctc accggattca gtcgtcactc atggtgattt   10260

ctcacttgat aaccttattt ttgacgaggg gaaattaata ggttgtattg atgttggacg   10320

agtcggaatc gcagaccgat accaggatct tgccatccta tggaactgcc tcggtgagtt   10380

ttctccttca ttacagaaac ggctttttca aaaatatggt attgataatc ctgatatgaa   10440

taaattgcag tttcatttga tgctcgatga gtttttctaa tcagtactga caataaaaag   10500

attcttgttt tcaagaactt gtcatttgta tagttttttt atattgtagt tgttctattt   10560

taatcaaatg ttagcgtgat ttatattttt tttcgcctcg acatcatctg cccagatgcg   10620

aagttaagtg cgcagaaagt aatatcatgc gtcaatcgta tgtgaatgct ggtcgctata   10680

ctgctgtcga ttcgatacta acgccgccat ccagtgtcga cggatcctag gtgtacataa   10740

actttataaa tgaaattcat aatagaaacg acacgaaatt acaaaatgga atatgttcat   10800

agggtagacg aaactatata cgcaatctac atacatttat caagaaggag aaaaaggagg   10860

atagtaaa                                                            10868
```

<210> 31
<211> 10857
<212> DNA
<213> Artificial

<220>
<223> pRN831

<400> 31

```
ggaatacagg taagcaaatt gatactaatg gctcaacgtg ataaggaaaa agaattgcac      60

tttaacatta atattgacaa ggaggagggc accacacaaa aagttaggtg taacagaaaa     120

tcatgaaact acgattccta atttgatatt ggaggatttt ctctaaaaaa aaaaaaatac     180

aacaaataaa aaacactcaa tgacctgacc atttgatgga gtttaagtca ataccttctt     240

gaaccatttc ccataatggt gaaagttccc tcaagaattt tactctgtca gaaacggcct     300

tacgacgtag tcgatatggt gcactctcag tacaatctgc tctgatgccg catagttaag     360
```

ccagccccga cacccgccaa cacccgctga cgcgccctga cgggcttgtc tgctcccggc 420
atccgcttac agacaagctg tgaccgtctc cgggagctgc atgtgtcaga ggttttcacc 480
gtcatcaccg aaacgcgcga gacgaaaggg cctcgtgata cgcctatttt tataggttaa 540
tgtcatgata ataatggttt cttagacgtc aggtggcact tttcggggaa atgtgcgcgg 600
aacccctatt tgtttatttt tctaaataca ttcaaatatg tatccgctca tgagacaata 660
accctgataa atgcttcaat aatattgaaa aaggaagagt atgagtattc aacatttccg 720
tgtcgccctt attccctttt ttgcggcatt ttgccttcct gtttttgctc acccagaaac 780
gctggtgaaa gtaaaagatg ctgaagatca gttgggtgca cgagtgggtt acatcgaact 840
ggatctcaac agcggtaaga tccttgagag ttttcgcccc gaagaacgtt ttccaatgat 900
gagcactttt aaagttctgc tatgtggcgc ggtattatcc cgtattgacg ccgggcaaga 960
gcaactcggt cgccgcatac actattctca gaatgacttg gttgagtact caccagtcac 1020
agaaaagcat cttacggatg gcatgacagt aagagaatta tgcagtgctg ccataaccat 1080
gagtgataac actgcggcca acttacttct gacaacgatc ggaggaccga aggagctaac 1140
cgcttttttt cacaacatgg gggatcatgt aactcgcctt gatcgttggg aaccggagct 1200
gaatgaagcc ataccaaacg acgagcgtga caccacgatg cctgtagcaa tggcaacaac 1260
gttgcgcaaa ctattaactg gcgaactact tactctagct tcccggcaac aattaataga 1320
ctggatggag gcggataaag ttgcaggacc acttctgcgc tcggcccttc cggctggctg 1380
gtttattgct gataaatctg gagccggtga gcgtgggtct cgcggtatca ttgcagcact 1440
ggggccagat ggtaagccct cccgtatcgt agttatctac acgacgggca gtcaggcaac 1500
tatggatgaa cgaaatagac agatcgctga gataggtgcc tcactgatta agcattggta 1560
actgtcagac caagtttact catatatact ttagattgat ttaaaacttc atttttaatt 1620
taaaaggatc taggtgaaga tcctttttga taatctcatg accaaaatcc cttaacgtga 1680
gttttcgttc cactgagcgt cagaccccgt agaaaagatc aaaggatctt cttgagatcc 1740
tttttttctg cgcgtaatct gctgcttgca aacaaaaaaa ccaccgctac cagcggtggt 1800
ttgtttgccg gatcaagagc taccaactct ttttccgaag gtaactggct tcagcagagc 1860
gcagatacca aatactgtcc ttctagtgta gccgtagtta ggccaccact tcaagaactc 1920
tgtagcaccg cctacatacc tcgctctgct aatcctgtta ccagtggctg ctgccagtgg 1980
cgataagtcg tgtcttaccg ggttggactc aagacgatag ttaccggata aggcgcagcg 2040
gtcgggctga acggggggtt cgtgcacaca gcccagcttg gagcgaacga cctacaccga 2100
actgagatac ctacagcgtg agcattgaga aagcgccacg cttcccgaag ggagaaaggc 2160
ggacaggtat ccggtaagcg gcagggtcgg aacaggagag cgcacgaggg agcttccagg 2220
ggggaacgcc tggtatcttt atagtcctgt cgggtttcgc cacctctgac ttgagcgtcg 2280

```
atttttgtga tgctcgtcag gggggccgag cctatggaaa aacgccagca acgcggcctt   2340
tttacggttc ctggcctttt gctggccttt tgctcacatg ttctttcctg cgttatcccc   2400
tgattctgtg gataaccgta ttaccgcctt tgagtgagct gataccgctc gccgcagccg   2460
aacgaccgag cgcagcgagt cagtgagcga ggaagcggaa gagcgcccaa tacgcaaacc   2520
gcctctcccc gcgcgttggc cgattcatta atgcagctgg cacgacaggt ttcccgactg   2580
gaaagcgggc agtgagcgca acgcaattaa tgtgagttac ctcactcatt aggcacccca   2640
ggctttacac tttatgcttc cggctcctat gttgtgtgga attgtgagcg gataacaatt   2700
tcacacagga aacagctatg accatgatta cgccaagctc ggaattaacc ctcactaaag   2760
ggaacaaaag ctgggtaccg ggccccccct cgaggtcgac ggtatcgata agcttgatat   2820
cgaattcctg cagcccgggg gatccactag tcgtacgaat tctatccttt tgttgtttcc   2880
gggtgtacaa tatggacttc ctcttttctg gcaaccaaac ccatacatcg ggattcctat   2940
aataccttcg ttggtctccc taacatgtag gtggcggagg ggagatatac aatagaacag   3000
ataccagaca agacataatg ggctaaacaa gactacacca attacactgc ctcattgatg   3060
gtggtacata acgaactaat actgtagccc tagacttgat agccatcatc atatcgaagt   3120
ttcactaccc tttttccatt tgccatctat tgaagtaata ataggcgcat gcaacttctt   3180
ttcttttttt ttcttttctc tctcccccgt tgttgtctca ccatatccgc aatgacaaaa   3240
aaatgatgga agacactaaa ggaaaaaatt aacgacaaag acagcaccaa cagatgtcgt   3300
tgttccagag ctgatgaggg gtatctcgaa gcacacgaaa ctttttcctt ccttcattca   3360
cgcacactac tctctaatga gcaacggtat acggccttcc ttccagttac ttgaatttga   3420
aataaaaaaa gtttgctgtc ttgctatcaa gtataaatag acctgcaatt attaatcttt   3480
tgtttcctcg tcattgttct cgttcccttt cttccttgtt tctttttctg cacaatattt   3540
caagctatac caagcataca atcaactcca gctgcagaaa atgagattgt gtgttatcgg   3600
tgctggttac gttggtttgg ttaccgctgc ttgtttcgct gaaatgggta accaagttag   3660
atgtgttgaa agagacagag aaagagttgc tagattgaga agaggtgaaa tgccaatcta   3720
cgaaccaggt ttggaatcta tcttgagaga ccaattggac gctgctagat taaccttcac   3780
cgcttctttg gctgaaggtt tggctgacgc tgaagttgtt ttcatcgctg ttggtacccc   3840
atgtggtgaa gacggttcag ctgacttgtc tcacgttttg gctgttgctg aacaattggg   3900
tgctcaattg agacaagctt gtatcgttgt taacaagtct accgttccag ttggtaccgc   3960
tgaaagagtt gaagaaatca tcagattggg tttggctaga agaagaaaga gattcagagt   4020
tgctgttgct tctaacccag aattcttgaa ggaaggttct gctgttgacg acttcagaag   4080
accagacaga gttatcatcg gttctgctga aacccaagct ggtgaaacct tgagacaatt   4140
gtatgctcca ttcttgagaa accacgaaag agttttgttg atgggtagaa gagaagctga   4200
```

```
attctctaag tacgctgcta acgctttctt ggctaccaag atctctttca tgaacgaaat   4260
ggctggtttg tgtgctttga ctggtgttga catcgaagac gttagaagag gtatgggttc   4320
tgacaagaga atcggtaccc actttatcta cgctggttgt ggttacggtg gttcttgttt   4380
cccaaaggac gttagagctt tgatcagatc tgctgaacaa caaggttacg actctcaaat   4440
cttgagagct gttgaagcta gaaacgctag acaaaaggaa ttgttgttcg aaaccttggg   4500
tgaattgttc caaggtagat ggcaaggtag aaccgttgct ttgtggggtt tggctttcaa   4560
gccaggtacc gacgacttga gagaagctcc atctttggtt ttgttggaag ctttgttgag   4620
acacggtgtt agagttagag cacacgaccc agttgctaac gctggtgttg ctgctagata   4680
cccagaagct gttgcttgtg ctagattgac cttgcacgac tctccatacg ctgctgttga   4740
aggtgctgac gctttggttt tggttaccga atggaagcaa ttcagacaac cagacttcca   4800
aaagatcaga ggttctatga gaaccccatt gttggttgac ggtagaaact tgtacgctcc   4860
agctagaatg gctgaattgg gttttatcta ccaaggtatc ggtagaccaa gagctggtca   4920
ctgtaaggct tctgctgctt aaggatcccc ttttcctttg tcgatatcat gtaattagtt   4980
atgtcacgct tacattcacg ccctcctccc acatccgctc taaccgaaaa ggaaggagtt   5040
agacaacctg aagtctaggt ccctatttat ttttttaat agttatgtta gtattaagaa   5100
cgttatttat atttcaaatt tttctttttt ttctgtacaa acgcgtgtac gcatgtaaca   5160
ttatactgaa aaccttgctt gagaaggttt tgggacgctc gaaggcttcc tagggtcgac   5220
gtacgtgtgg aagaacgatt acaacaggtg ttgtcctctg aggacataaa atacacaccg   5280
agattcatca actcattgct ggagttagca tatctacaat tgggtgaaat ggggagcgat   5340
ttgcaggcat ttgctcggca tgccggtaga ggtgtggtca ataagagcga cctcatgcta   5400
tacctgagaa agcaacctga cctacaggaa agagttactc aagaataaga attttcgttt   5460
taaaacctaa gagtcacttt aaaatttgta tacacttatt ttttttataa cttatttaat   5520
aataaaaatc ataaatcata agaaattcgc gcgcttaagc cttgaagtcg aacttacctg   5580
gttcagagta cttcttttca acttgttcaa ccttgttgaa cttgaaacct ctgattctca   5640
aaacttctgg ttcagaagag tccttgtagt caactggttc caaagcccaa ccgttgtttt   5700
gaacagaacc agaaacgtta acttcagcgt cgtcaacagc gtcaacgatc aaagcaccgt   5760
caacagacaa gttttcgatg aagatctttc tacccttgat agccaaggta gatctttgag   5820
agatagaaca gttaccagaa accttagact taaccaaaga gaaggtcaaa ccccactttg   5880
gcttccaggt gattcttggc caaacttcaa cttcttgacc gttgataact tgcaaaactg   5940
ggtcagcaac ttggaaacca gccttcttca agatcaaaga gttagctctg tagatagcca   6000
tttcaccaga ggtagcagag tggtatgggt taccctttgg aaccttagca gcgtcttcag   6060
cgttgttctt aactggagcg taagcgaacc aggtttccat aacggtgaaa ccaactcttg   6120
```

aagatggtgg caaggtcttt gggtagtctt gcatcataca ttccaatctg gtagaagact 6180
tgaaagaggt cttagaagcg tccttgtact ttgggttaac aaattcttgg atagcaccac 6240
cagtcttagc caattcttcg atgtatggac ccaattccaa gatcaattgg ttgatgttac 6300
ctgggaatgg agagtaacca gtttcagagt taacgtcacc gtctgggtaa ccagaagctc 6360
tcaacaatgg gtccaattgg ttgtattcaa cgttgataac catagatcta ccgtcagagt 6420
gggtcaatct ggtgatacca ccgatagctt ccttagcctt tcttggaaca gccaaagagt 6480
taacgtggta ttgcttggta gaagaaacac ccaaagcaga tgggatagcc ttgaacaaca 6540
aaccgttggt gtcttggaag aacaaaaccc acttcaaacc agcgttgtac caaaccttca 6600
agataccaga agagtgcaac aaagagtgaa cgtcaccgtg accgtgtggc ttggtttgaa 6660
ctctgtatct gttttgtggg tccaaagcca atctagcgtc gttgtcttcc aaacaagcaa 6720
ccttttcttg cttcaacaag gtaacttggg ttggttgcat accgaagtaa gagttagatt 6780
ccaacaagtc caaggttcta ccgtgggtgt cgtcagaggt catgataacg aatgggatgt 6840
gggtttgtcc ttcaccttca gaacttgctt cttgcaaagc caagatagat tcgatgtagt 6900
gttgcaagaa acaggtacca gtggtggttt cagctggcaa agcaaccttg ataccgttgt 6960
aacccaatct ttcacccaaa ccaccagcaa ccaaaacgaa agcagctctt ctagcttctc 7020
taacaccagc ttcttcgtac ttgttgaagt tttcgtcacc gaacttcaag gtttcaccag 7080
ttggaacaga tggggtgaaa ccgtcgaatg ggttgttacc agccttagag tcagccaaca 7140
atctcttagc gttgttgatg taagcaacca aaccacctgg gtaagaagag tccaacaaaa 7200
ccaattggtc gaagaaagcc ttcttttcgt cgtcgtcaac acctgggttt ggccagtctc 7260
tgaacaagtg gtcttgtccg ttgtccaaca acatcttaac caattctctt tgttgtggag 7320
acaacaagtt gaagttgtca cccaaagaag aagccatttt aagcttctgc agcttagatt 7380
agattgctat gctttctttc taatgagcaa gaagtaaaaa aagttgtaat agaacaagaa 7440
aaatgaaact gaaacttgag aaattgaaga ccgtttatta acttaaatat caatgggagg 7500
tcatcgaaag agaaaaaaat caaaaaaaaa aattttcaag aaaaagaaac gtgataaaaa 7560
tttttattgc ctttttcgac gaagaaaaag aaacgaggcg gtctcttttt tcttttccaa 7620
acctttagta cgggtaatta acgacaccct agaggaagaa agaggggaaa tttagtatgc 7680
tgtgcttggg tgttttgaag tggtacggcg atgcgcggag tccgagaaaa tctggaagag 7740
taaaaaagga gtagaaacat tttgaagcta tggtgtgtgg gaccggatta tcgatgataa 7800
gctgtcaaag atgagaatta attccacgga ctatagacta tactagatac tccgtctact 7860
gtacgataca cttccgctca ggtccttgtc ctttaacgag gccttaccac tcttttgtta 7920
ctctattgat ccagctcagc aaaggcagtg tgatctaaga ttctatcttc gcgatgtagt 7980
aaaactagct agaccgagaa agagactaga aatgcaaaag gcacttctac aatggctgcc 8040

```
atcattatta tccgatgtga cgctgcagct tctcaatgat attcgaatac gctttgagga   8100
gatacagcct aatatccgac aaactgtttt acagatttac gatcgtactt gttacccatc   8160
attgaatttt gaacatccga acctgggagt ttccctgaa acagatagta tatttgaacc   8220
tgtataataa tatatagtct agcgctttac ggaagacaat gtatgtattt cggttcctgg   8280
agaaactatt gcatctattg cataggtaat cttgcacgtc gcatccccgg ttcattttct   8340
gcgtttccat cttgcacttc aatagcatat ctttgttaac gaagcatctg tgcttcattt   8400
tgtagaacaa aaatgcaacg cgagagcgct aatttttcaa acaaagaatc tgagctgcat   8460
ttttacagaa cagaaatgca acgcgaaagc gctattttac caacgaagaa tctgtgcttc   8520
atttttgtaa aacaaaaatg caacgcgacg agagcgctaa tttttcaaac aaagaatctg   8580
agctgcattt ttacagaaca gaaatgcaac gcgagagcgc tattttacca acaaagaatc   8640
tatacttctt ttttgttcta caaaaatgca tcccgagagc gctatttttc taacaaagca   8700
tcttagatta cttttttct cctttgtgcg ctctataatg cagtctcttg ataacttttt   8760
gcactgtagg tccgttaagg ttagaagaag gctactttgg tgtctatttt ctcttccata   8820
aaaaaagcct gactccactt cccgcgttta ctgattacta gcgaagctgc gggtgcattt   8880
tttcaagata aaggcatccc cgattatatt ctataccgat gtggattgcg catactttgt   8940
gaacagaaag tgatagcgtt gatgattctt cattggtcag aaaattatga acggtttctt   9000
ctattttgtc tctatatact acgtatagga aatgtttaca ttttcgtatt gttttcgatt   9060
cactctatga atagttctta ctacaatttt tttgtctaaa gagtaatact agagataaac   9120
ataaaaaatg tagaggtcga gtttagatgc aagttcaagg agcgaaaggt ggatgggtag   9180
gttatatagg gatatagcac agagatatat agcaaagaga tacttttgag caatgtttgt   9240
ggaagcggta ttcgcaatgc cggagctcgt acgttcgaac ttaaggcctc gtccccgccg   9300
ggtcacccgg ccagcgacat ggaggcccag aataccctcc ttgacagtct tgacgtgcgc   9360
agctcagggg catgatgtga ctgtcgcccg tacatttagc ccatacatcc ccatgtataa   9420
tcatttgcat ccatacattt tgatggccgc acggcgcgaa gcaaaaatta cggctcctcg   9480
ctgcagacct gcgagcaggg aaacgctccc ctcacagacg cgttgaattg tccccacgcc   9540
gcgcccctgt agagaaatat aaaaggttag gatttgccac tgaggttctt ctttcatata   9600
cttcctttta aaatcttgct aggatacagt tctcacatca catccgaaca taaacaacca   9660
tgggtaagga aaagactcac gtttcgaggc cgcgattaaa ttccaacatg gatgctgatt   9720
tatatgggta taaatgggct cgcgataatg tcgggcaatc aggtgcgaca atctatcgat   9780
tgtatgggaa gcccgatgcg ccagagttgt ttctgaaaca tggcaaaggt agcgttgcca   9840
atgatgttac agatgagatg gtcagactaa actggctgac ggaatttatg cctcttccga   9900
ccatcaagca ttttatccgt actcctgatg atgcatggtt actcaccact gcgatccccg   9960
```

gcaaaacagc attccaggta ttagaagaat atcctgattc aggtgaaaat attgttgatg 10020

cgctggcagt gttcctgcgc cggttgcatt cgattcctgt ttgtaattgt ccttttaaca 10080

gcgatcgcgt atttcgtctc gctcaggcgc aatcacgaat gaataacggt ttggttgatg 10140

cgagtgattt tgatgacgag cgtaatggct ggcctgttga acaagtctgg aaagaaatgc 10200

ataagctttt gccattctca ccggattcag tcgtcactca tggtgatttc tcacttgata 10260

accttatttt tgacgagggg aaattaatag gttgtattga tgttggacga gtcggaatcg 10320

cagaccgata ccaggatctt gccatcctat ggaactgcct cggtgagttt tctccttcat 10380

tacagaaacg gctttttcaa aaatatggta ttgataatcc tgatatgaat aaattgcagt 10440

ttcatttgat gctcgatgag tttttctaat cagtactgac aataaaaaga ttcttgtttt 10500

caagaacttg tcatttgtat agtttttta tattgtagtt gttctatttt aatcaaatgt 10560

tagcgtgatt tatatttttt ttcgcctcga catcatctgc ccagatgcga agttaagtgc 10620

gcagaaagta atatcatgcg tcaatcgtat gtgaatgctg gtcgctatac tgctgtcgat 10680

tcgatactaa cgccgccatc cagtgtcgac ggatcctagg tgtacataaa ctttataaat 10740

gaaattcata atagaaacga cacgaaatta caaaatggaa tatgttcata gggtagacga 10800

aactatatac gcaatctaca tacatttatc aagaaggaga aaaaggagga tagtaaa 10857

<210> 32
<211> 5202
<212> DNA
<213> Artificial

<220>
<223> pRN600 shuttle vector

<400> 32
tctcagaatg acttggttga gtactcacca gtcacagaaa agcatcttac ggatggcatg 60

acagtaagag aattatgcag tgctgccata accatgagtg ataacactgc ggccaactta 120

cttctgacaa cgatcggagg accgaaggag ctaaccgctt ttttcacaa catgggggat 180

catgtaactc gccttgatcg ttgggaaccg gagctgaatg aagccatacc aaacgacgag 240

cgtgacacca cgatgcctgt agcaatggca acaacgttgc gcaaactatt aactggcgaa 300

ctacttactc tagcttcccg gcaacaatta atagactgga tggaggcgga taaagttgca 360

ggaccacttc tgcgctcggc ccttccggct ggctggttta ttgctgataa atctggagcc 420

ggtgagcgtg ggtctcgcgg tatcattgca gcactggggc cagatggtaa gccctcccgt 480

atcgtagtta tctacacgac gggcagtcag gcaactatgg atgaacgaaa tagacagatc 540

gctgagatag gtgcctcact gattaagcat tggtaactgt cagaccaagt ttactcatat 600

atactttaga ttgatttaaa acttcatttt taatttaaaa ggatctaggt gaagatcctt 660

tttgataatc tcatgaccaa aatcccttaa cgtgagtttt cgttccactg agcgtcagac 720

cccgtagaaa agatcaaagg atcttcttga gatccttttt ttctgcgcgt aatctgctgc 780

ttgcaaacaa aaaaaccacc gctaccagcg gtggtttgtt tgccggatca agagctacca 840

actctttttc cgaaggtaac tggcttcagc agagcgcaga taccaaatac tgtccttcta 900

gtgtagccgt agttaggcca ccacttcaag aactctgtag caccgcctac atacctcgct 960

ctgctaatcc tgttaccagt ggctgctgcc agtggcgata agtcgtgtct taccgggttg 1020

gactcaagac gatagttacc ggataaggcg cagcggtcgg gctgaacggg gggttcgtgc 1080

acacagccca gcttggagcg aacgacctac accgaactga gatacctaca gcgtgagcat 1140

tgagaaagcg ccacgcttcc cgaagggaga aaggcggaca ggtatccggt aagcggcagg 1200

gtcggaacag gagagcgcac gagggagctt ccagggggga acgcctggta tctttatagt 1260

cctgtcgggt ttcgccacct ctgacttgag cgtcgatttt tgtgatgctc gtcagggggg 1320

ccgagcctat ggaaaaacgc cagcaacgcg gcctttttac ggttcctggc cttttgctgg 1380

ccttttgctc acatgttctt tcctgcgtta tcccctgatt ctgtggataa ccgtattacc 1440

gcctttgagt gagctgatac cgctcgccgc agccgaacga ccgagcgcag cgagtcagtg 1500

agcgaggaag cggaagagcg cccaatacgc aaaccgcctc tccccgcgcg ttggccgatt 1560

cattaatgca gctggcacga caggtttccc gactggaaag cgggcagtga gcgcaacgca 1620

attaatgtga gttacctcac tcattaggca ccccaggctt tacactttat gcttccggct 1680

cctatgttgt gtggaattgt gagcggataa caatttcaca caggaaacag ctatgaccat 1740

gattacgcca agctcggaat taaccctcac taaagggaac aaaagctggg taccgggccc 1800

cccctcgagg tcgacggtat cgataagctt gatatcgaat tcctgcagcc cgggggatcc 1860

actagttcta gagcggccgc caccgcggtg gagctcgtac gttcgaactt aaggcctcgt 1920

ccccgccggg tcacccggcc agcgacatgg aggcccagaa taccctcctt gacagtcttg 1980

acgtgcgcag ctcaggggca tgatgtgact gtcgcccgta catttagccc atacatcccc 2040

atgtataatc atttgcatcc atacattttg atggccgcac ggcgcgaagc aaaaattacg 2100

gctcctcgct gcagacctgc gagcagggaa acgctcccct cacagacgcg tgaattgtcc 2160

ccacgccgcg cccctgtaga gaaatataaa aggttaggat ttgccactga ggttcttctt 2220

tcatatactt ccttttaaaa tcttgctagg atacagttct cacatcacat ccgaacataa 2280

acaaccatgg gtaaaaagcc tgaactcacc gcgacgtctg tcgagaagtt tctgatcgaa 2340

aagttcgaca gcgtctccga cctgatgcag ctctcggagg gcgaagaatc tcgtgctttc 2400

agcttcgatg taggagggcg tggatatgtc ctgcgggtaa atagctgcgc cgatggtttc 2460

tacaaagatc gttatgttta tcggcacttt gcatcggccg cgctcccgat tccggaagtg 2520

cttgacattg gggaattcag cgagagcctg acctattgca tctcccgccg tgcacagggt 2580

gtcacgttgc aagacctgcc tgaaaccgaa ctgcccgctg ttctgcagcc ggtcgcggag 2640

gccatggatg cgatcgctgc ggccgatctt agccagacga gcgggttcgg cccattcgga 2700
ccgcaaggaa tcggtcaata cactacatgg cgtgatttca tatgcgcgat tgctgatccc 2760
catgtgtatc actggcaaac tgtgatggac gacaccgtca gtgcgtccgt cgcgcaggct 2820
ctcgatgagc tgatgctttg ggccgaggac tgccccgaag tccggcacct cgtgcacgcg 2880
gatttcggct ccaacaatgt cctgacggac aatggccgca taacagcggt cattgactgg 2940
agcgaggcga tgttcgggga ttcccaatac gaggtcgcca acatcttctt ctggaggccg 3000
tggttggctt gtatggagca gcagacgcgc tacttcgagc ggaggcatcc ggagcttgca 3060
ggatcgccgc ggctccgggc gtatatgctc cgcattggtc ttgaccaact ctatcagagc 3120
ttggttgacg gcaatttcga tgatgcagct tgggcgcagg gtcgatgcga cgcaatcgtc 3180
cgatccggag ccgggactgt cgggcgtaca caaatcgccc gcagaagcgc ggccgtctgg 3240
accgatggct gtgtagaagt actcgccgat agtggaaacc gacgccccag cactcgtccg 3300
agggcaaagg aataatcagt actgacaata aaaagattct tgttttcaag aacttgtcat 3360
ttgtatagtt tttttatatt gtagttgttc tattttaatc aaatgttagc gtgatttata 3420
ttttttttcg cctcgacatc atctgcccag atgcgaagtt aagtgcgcag aaagtaatat 3480
catgcgtcaa tcgtatgtga atgctggtcg ctatactgct gtcgattcga tactaacgcc 3540
gccatccagt gtcgacggat cctaggtgta cataaacttt ataaatgaaa ttcataatag 3600
aaacgacacg aaattacaaa atggaatatg ttcatagggt agacgaaact atatacgcaa 3660
tctacataca tttatcaaga aggagaaaaa ggaggatagt aaaggaatac aggtaagcaa 3720
attgatacta atggctcaac gtgataagga aaaagaattg cactttaaca ttaatattga 3780
caaggaggag ggcaccacac aaaaagttag gtgtaacaga aaatcatgaa actacgattc 3840
ctaatttgat attggaggat tttctctaaa aaaaaaaaaa tacaacaaat aaaaaacact 3900
caatgacctg accatttgat ggagtttaag tcaatacctt cttgaaccat ttcccataat 3960
ggtgaaagtt ccctcaagaa ttttactctg tcagaaacgg ccttacgacg tagtcgatat 4020
ggtgcactct cagtacaatc tgctctgatg ccgcatagtt aagccagccc cgacacccgc 4080
caacacccgc tgacgcgccc tgacgggctt gtctgctccc ggcatccgct tacagacaag 4140
ctgtgaccgt ctccgggagc tgcatgtgtc agaggttttc accgtcatca ccgaaacgcg 4200
cgagacgaaa gggcctcgtg atacgcctat ttttataggt taatgtcatg ataataatgg 4260
tttcttagga cggatcgctt gcctgtaact tacacgcgcc tcgtatcttt taatgatgga 4320
ataatttggg aatttactct gtgtttattt atttttatgt tttgtatttg gattttagaa 4380
agtaaataaa gaaggtagaa gagttacgga atgaagaaaa aaaaataaac aaaggtttaa 4440
aaaatttcaa caaaaagcgt actttacata tatatttatt agacaagaaa agcagattaa 4500
atagatatac attcgattaa cgataagtaa aatgtaaaat cacaggattt tcgtgtgtgg 4560

tcttctacac agacaagatg aaacaattcg gcattaatac ctgagagcag gaagagcaag 4620

ataaaaggta gtatttgttg gcgatccccc tagagtcttt tacatcttcg gaaaacaaaa 4680

actatttttt ctttaatttc tttttttact ttctattttt aatttatata tttatattaa 4740

aaaatttaaa ttataattat ttttatagca cgtgatgaaa aggacccagg tggcactttt 4800

cggggaaatg tgcgcggaac ccctatttgt ttatttttct aaatacattc aaatatgtat 4860

ccgctcatga gacaataacc ctgataaatg cttcaataat attgaaaaag gaagagtatg 4920

agtattcaac atttccgtgt cgcccttatt cccttttttg cggcattttg ccttcctgtt 4980

tttgctcacc cagaaacgct ggtgaaagta aaagatgctg aagatcagtt gggtgcacga 5040

gtgggttaca tcgaactgga tctcaacagc ggtaagatcc ttgagagttt tcgccccgaa 5100

gaacgttttc caatgatgag cacttttaaa gttctgctat gtggcgcggt attatcccgt 5160

attgacgccg ggcaagagca actcggtcgc cgcatacact at 5202

<210> 33
<211> 2242
<212> DNA
<213> Artificial

<220>
<223> Expression cassette for EC 2.7.1.44 GalAK Y250F

<400> 33
ggtacctagg accggtttat cattatcaat actgccattt caaagaatac gtaaataatt 60

aatagtagtg attttcctaa ctttatttag tcaaaaaatt agccttttaa ttctgctgta 120

acccgtacat gcccaaaata gggggcgggt tacacagaat atataacatc gtaggtgtct 180

gggtgaacag tttattcctg gcatccacta aatataatgg agcccgcttt ttaagctggc 240

atccagaaaa aaaaagaatc ccagcaccaa aatattgttt tcttcaccaa ccatcagttc 300

ataggtccat tctcttagcg caactacaga gaacaggggc acaaacaggc aaaaaacggg 360

cacaacctca atggagtgat gcaacctgcc tggagtaaat gatgacacaa ggcaattgac 420

ccacgcatgt atctatctca ttttcttaca ccttctatta ccttctgctc tctctgattt 480

ggaaaaagct gaaaaaaaag gttgaaacca gttccctgaa attattcccc tacttgacta 540

ataagtatat aaagacggta ggtattgatt gtaattctgt aaatctattt cttaaacttc 600

ttaaattcta cttttatagt tagtcttttt tttagtttta aaacaccaag aacttagttt 660

cgaataaaca cacataaaga attcaaaatg tcttggccaa ccgactctga attgaactct 720

atcaaggaag ctgttgctca aatgtctggt agagacaagg gtgaagttag agttgttgtt 780

gctccataca gaatctgtcc attgggtgct cacatcgacc accaaggtgg tactgtttct 840

gctatgacca tcaacaaggg tatcttgttg ggtttcgttc catctggtga cacccaagtt 900

caattgagat ctgctcaatt cgaaggtgaa gtttgtttca gagttgacga aatccaacac 960

ccaatcggtt tggctaacaa gaacggtgct tctaccccat ctccatctaa ggaaaagtct 1020

atctggggta cttacgctag aggtgctgtt tacgctttgc aatcttctaa gaagaacttg 1080

aagcaaggta tcatcggtta cttgtctggt tctaacggtt tggactcttc aggtttatct 1140

tcttctgctg ctgttggtgt tgcttacttg ttagctttgg aaaacgctaa cgaattgacc 1200

gtttctccaa ccgaaaacat cgaatacgac agattgatcg aaaacggtta cttgggtttg 1260

agaaacggta tcttggacca atctgctatc ttgttgtcta actacggttg tttgacctac 1320

atggactgta agaccttgga ccacgaattg gttcaagctc cagaattgga aaagccattc 1380

agaatcttgt tggctttctc tggtttgaga caagctttga ccaccaaccc aggtttcaac 1440

ttgagagttt ctgaatgtca agaagctgct aaggttttgt tgaccgcttc aggtaactct 1500

gaattggaac caaccttgtg taacgttgaa cacgctgttt acgaagctca caagcacgaa 1560

ttgaagcctg ttttggctaa gagagctgaa cactacttct ctgaaaacat gagagttatc 1620

aagggtagag aagcttgggc ttctggtaac ttggaagaat ttggtaagtt gatctctgct 1680

tctggtttgt cttctatcga aaactacgaa tgtggtgctg aaccattgat ccaattgtac 1740

aagattttgt tgaaggctcc aggtgtttac ggtgctagat tctctggtgc tggtttcaga 1800

ggttgttgtt tggctttcgt tgacgctgaa aaggctgaag ctgctgcttc ttacgttaag 1860

gacgaatacg aaaaggctca accagaattt gctaacaact tgaacggtgg taagccagtt 1920

ttgatctgtg aagctggtga cgctgctaga gttttgttgt aaggatcccc ttttcctttg 1980

tcgatatcat gtaattagtt atgtcacgct tacattcacg ccctcctccc acatccgctc 2040

taaccgaaaa ggaaggagtt agacaacctg aagtctaggt ccctatttat ttttttaat 2100

agttatgtta gtattaagaa cgttatttat atttcaaatt tttctttttt ttctgtacaa 2160

acgcgtgtac gcatgtaaca ttatactgaa aaccttgctt gagaaggttt tgggacgctc 2220

gaaggcttcc taggctcgag tt 2242

<210> 34
<211> 2189
<212> DNA
<213> Artificial

<220>
<223> Expression cassette for EC 5.1.3.6 At GAE1

<400> 34
actagtaaat gtgtggggaa gcgggtaagc tgccacagca attaatgcac aacatttaac 60

ctacattctt ccttatcgga tcctcaaaac ccttaaaaac atatgcctca ccctaacata 120

ttttccaatt aaccctcaat atttctctgt cacccggcct ctattttcca ttttcttctt 180

tacccgccac gcgttttttt ctttcaaatt tttttcttcc ttcttctttt tcttccacgt 240

cctcttgcat aaataaataa accgttttga aaccaaactc gcctctctct ctccttttttg 300

```
aaatattttt gggtttgttt gatcctttcc ttcccaatct ctcttgttta atatatattc     360
atttatatca cgctctcttt ttatcttcct ttttttcctc tctcttgtat tcttccttcc     420
cctttctact caaaccaaga agaaaaagaa aaggtcaatc tttgttaaag aataggatct     480
tctactacat cagcttttag atttttcacg cttactgctt ttttcttccc aagatcgaaa     540
atttactgaa ttaactgcag aaaaatgcca tctatcgaag acgaattgtt cccatctacc     600
ccaggtaagt tcaagatcga cagatctaac agacaattgc acagatgttt cgcttctacc     660
tctaccatgt tcttgtgggc tttgttcttg atcgctttga ctgcttctta cttgtctttc     720
caatctttcg ttgactctgg ttctagatac ttgaccgctt cttggggtgg tatccaatgg     780
gaaaagcaag ttagaacctc tgctcaaatc cacagatctg gtggtatctc tgttttggtt     840
accggtgcta ccggtttcgt tggttctcac gtttctttgg ctttgagaaa gagaggtgac     900
ggtgttgttg gtttggacaa cttcaacaac tactacgacc catctttgaa gagagctaga     960
agatctttgt tgtcttctag aggtatcttc gttgttgaag gtgacttgaa cgacgctaag    1020
ttgttggcta agttgttcga cgttgttgct ttcacccacg ttatgcactt ggctgctcaa    1080
gctggtgtta gatacgcttt ggaaaaccca caatcttacg ttcactctaa catcgctggt    1140
ttggttaact tgttggaaat ctgtaaggct gctaacccac aaccagctat cgtttgggct    1200
tcttcttctt ctgtttacgg tttgaacgaa aaggttccat tctctgaatc tgacagaacc    1260
gaccaaccag cttctttgta cgctgctacc aagaaggctg gtgaagaaat cacccacacc    1320
tacaaccaca tctacggttt ggctatcacc ggtttgagat tcttcaccgt ttacggtcca    1380
tggggtagac cagacatggc ttacttctct ttcaccagaa acatcttgca aggtaagcca    1440
atcaccatct acagaggtaa gaacagagtt gacttggcta gagacttcac ctacatcgac    1500
gacatcgtta agggttgttt gggttctttg gactcttctg gtaagtctac cggttctggt    1560
ggtaagaaga gaggtgctgc tccatacaga atcttcaact tgggtaacac ctctccagtt    1620
accgttccaa tcttggttga catcttggaa aagcacttga aggttaaggc taagagaaac    1680
ttcgttgaaa tgccaggtaa cggtgacgtt ccattcaccc acgctaacat ctcttctgct    1740
agaaacgaat tcggttacaa gccaaccacc gacttggaaa ccggtttgaa gaagttcgtt    1800
agatggtact tgtcttacta cggttacaac accaaggcta agttggttca ctaagcgcgc    1860
gaatttctta tgatttatga tttttattat taaataagtt ataaaaaaaa taagtgtata    1920
caaattttaa agtgactctt aggttttaaa acgaaaattc ttattcttga gtaactcttt    1980
cctgtaggtc aggttgcttt ctcaggtata gcatgaggtc gctcttattg accacacctc    2040
taccggcatg ccgagcaaat gcctgcaaat cgctccccat ttcacccaat tgtagatatg    2100
ctaactccag caatgagttg atgaatctcg gtgtgtattt tatgtcctca gaggacaaca    2160
cctgttgtaa tcgttcttcc acacgtacg                                      2189
```

<210> 35
<211> 9935
<212> DNA
<213> Artificial

<220>
<223> pRN761

<400> 35
gtgtcggggc tggcttaact atgcggcatc agagcagatt gtactgagag tgcaccatat 60

cgactacgtc gtaaggccgt ttctgacaga gtaaaattct tgagggaact ttcaccatta 120

tgggaaatgg ttcaagaagg tattgactta aactccatca aatggtcagg tcattgagtg 180

tttttatttt gttgtatttt tttttttta gagaaaatcc tccaatatca aattaggaat 240

cgtagtttca tgattttctg ttacacctaa ctttttgtgt ggtgccctcc tccttgtcaa 300

tattaatgtt aaagtgcaat tctttttcct tatcacgttg agccattagt atcaatttgc 360

ttacctgtat tcctttacta tcctcctttt tctccttctt gataaatgta tgtagattgc 420

gtatatagtt tcgtctaccc tatgaacata ttccattttg taatttcgtg tcgtttctat 480

tatgaatttc atttataaag tttatgtaca cctaggatcc gtcgacactg gatggcggcg 540

ttagtatcga atcgacagca gtatagcgac cagcattcac atacgattga cgcatgatat 600

tactttctgc gcacttaact tcgcatctgg gcagatgatg tcgaggcgaa aaaaaatata 660

aatcacgcta acatttgatt aaaatagaac aactacaata taaaaaaact atacaaatga 720

caagttcttg aaaacaagaa tctttttatt gtcagtactg attattcctt tgccctcgga 780

cgagtgctgg ggcgtcggtt tccactatcg gcgagtactt ctacacagcc atcggtccag 840

acggccgcgc ttctgcgggc gatttgtgta cgcccgacag tcccggctcc ggatcggacg 900

attgcgtcgc atcgaccctg cgcccaagct gcatcatcga aattgccgtc aaccaagctc 960

tgatagagtt ggtcaagacc aatgcggagc atatacgccc ggagccgcgg cgatcctgca 1020

agctccggat gcctccgctc gaagtagcgc gtctgctgct ccatacaagc caaccacggc 1080

ctccagaaga agatgttggc gacctcgtat tgggaatccc cgaacatcgc ctcgctccag 1140

tcaatgaccg ctgttatgcg gccattgtcc gtcaggacat tgttggagcc gaaatccgcg 1200

tgcacgaggt gccggacttc ggggcagtcc tcggcccaaa gcatcagctc atcgagagcc 1260

tgcgcgacgg acgcactgac ggtgtcgtcc atcacagttt gccagtgata cacatgggga 1320

tcagcaatcg cgcatatgaa atcacgccat gtagtgtatt gaccgattcc ttgcggtccg 1380

aatgggccga acccgctcgt ctggctaaga tcggccgcag cgatcgcatc catggcctcc 1440

gcgaccggct gcagaacagc gggcagttcg gtttcaggca ggtcttgcaa cgtgacaccc 1500

tgtgcacggc gggagatgca ataggtcagg ctctcgctga attccccaat gtcaagcact 1560

tccggaatcg ggagcgcggc cgatgcaaag tgccgataaa cataacgatc tttgtagaaa 1620

ccatcggcgc agctatttac ccgcaggaca tatccacgcc ctcctacatc gaagctgaaa 1680
gcacgagatt cttcgccctc cgagagctgc atcaggtcgg agacgctgtc gaacttttcg 1740
atcagaaact tctcgacaga cgtcgcggtg agttcaggct ttttacccat ggttgtttat 1800
gttcggatgt gatgtgagaa ctgtatccta gcaagatttt aaaaggaagt atatgaaaga 1860
agaacctcag tggcaaatcc taacctttta tatttctcta caggggcgcg gcgtggggac 1920
aattcacgcg tctgtgaggg gagcgtttcc ctgctcgcag gtctgcagcg aggagccgta 1980
atttttgctt cgcgccgtgc ggccatcaaa atgtatggat gcaaatgatt atacatgggg 2040
atgtatgggc taaatgtacg ggcgacagtc acatcatgcc cctgagctgc gcacgtcaag 2100
actgtcaagg agggtattct gggcctccat gtcgctggcc gggtgacccg gcggggacga 2160
ggccttaagt tggcgtacaa ttgaagttct ttacggattt ttagtaaacc ttgttcaggt 2220
ctaacactac cggcgaacgt ggcgagaaag gaagggaaga aagcgaaagg agcgggcgct 2280
agggcgctgg caagtgtagc ggtcacgctg cgcgtaacca ccacacccgc cgcgcttaat 2340
gcgccgctac agggcgcgtc gcgccattcg ccattcaggc tgcgcaactg ttgggaaggg 2400
cgatcggtgc gggcctcttc gctattacgc cagctggcga aggggggatg tgctgcaagg 2460
cgattaagtt gggtaacgcc agggttttcc cagtcacgac gttgtaaaac gacggccagt 2520
gaattgtaat acgactcact atagggcgaa ttggagctcc accgcggtgg cggccgctct 2580
agaactagta aatgtgtggg gaagcgggta agctgccaca gcaattaatg cacaacattt 2640
aacctacatt cttccttatc ggatcctcaa aacccttaaa aacatatgcc tcaccctaac 2700
atattttcca attaaccctc aatatttctc tgtcacccgg cctctatttt ccattttctt 2760
ctttacccgc cacgcgtttt tttctttcaa attttttttct tccttcttct ttttcttcca 2820
cgtcctcttg cataaataaa taaaccgttt tgaaaccaaa ctcgcctctc tctctccttt 2880
ttgaaatatt tttgggtttg tttgatcctt tccttcccaa tctctcttgt ttaatatata 2940
ttcatttata tcacgctctc ttttttatctt ccttttttttc ctctctcttg tatttcttcct 3000
tccccttttct actcaaacca agaagaaaaa gaaaaggtca atctttgtta aagaatagga 3060
tcttctacta catcagcttt tagatttttc acgcttactg cttttttctt cccaagatcg 3120
aaaatttact gaattaactg cagaaaaatg ccatctatcg aagacgaatt gttcccatct 3180
accccaggta agttcaagat cgacagatct aacagacaat tgcacagatg tttcgcttct 3240
acctctacca tgttcttgtg ggctttgttc ttgatcgctt tgactgcttc ttacttgtct 3300
ttccaatctt tcgttgactc tggttctaga tacttgaccg cttcttgggg tggtatccaa 3360
tgggaaaagc aagttagaac ctctgctcaa atccacagat ctggtggtat ctctgttttg 3420
gttaccggtg ctaccggttt cgttggttct cacgtttctt tggctttgag aaagagaggt 3480
gacggtgttg ttggtttgga caacttcaac aactactacg acccatcttt gaagagagct 3540

agaagatctt tgttgtcttc tagaggtatc ttcgttgttg aaggtgactt gaacgacgct 3600
aagttgttgg ctaagttgtt cgacgttgtt gctttcaccc acgttatgca cttggctgct 3660
caagctggtg ttagatacgc tttggaaaac ccacaatctt acgttcactc taacatcgct 3720
ggtttggtta acttgttgga aatctgtaag gctgctaacc cacaaccagc tatcgtttgg 3780
gcttcttctt cttctgttta cggtttgaac gaaaaggttc cattctctga atctgacaga 3840
accgaccaac cagcttcttt gtacgctgct accaagaagg ctggtgaaga aatcacccac 3900
acctacaacc acatctacgg tttggctatc accggtttga gattcttcac cgtttacggt 3960
ccatggggta gaccagacat ggcttacttc tctttcacca gaaacatctt gcaaggtaag 4020
ccaatcacca tctacagagg taagaacaga gttgacttgg ctagagactt cacctacatc 4080
gacgacatcg ttaagggttg tttgggttct ttggactctt ctggtaagtc taccggttct 4140
ggtggtaaga agagaggtgc tgctccatac agaatcttca acttgggtaa cacctctcca 4200
gttaccgttc caatcttggt tgacatcttg gaaaagcact tgaaggttaa ggctaagaga 4260
aacttcgttg aaatgccagg taacggtgac gttccattca cccacgctaa catctcttct 4320
gctagaaacg aattcggtta caagccaacc accgacttgg aaaccggttt gaagaagttc 4380
gttagatggt acttgtctta ctacggttac aacaccaagg ctaagttggt tcactaagcg 4440
cgcgaatttc ttatgattta tgatttttat tattaaataa gttataaaaa aaataagtgt 4500
atacaaattt taaagtgact cttaggtttt aaaacgaaaa ttcttattct tgagtaactc 4560
tttcctgtag gtcaggttgc tttctcaggt atagcatgag gtcgctctta ttgaccacac 4620
ctctaccggc atgccgagca aatgcctgca aatcgctccc catttcaccc aattgtagat 4680
atgctaactc cagcaatgag ttgatgaatc tcggtgtgta ttttatgtcc tcagaggaca 4740
acacctgttg taatcgttct tccacacgta cctaggaccg gtttatcatt atcaatactg 4800
ccatttcaaa gaatacgtaa ataattaata gtagtgattt tcctaacttt atttagtcaa 4860
aaaattagcc ttttaattct gctgtaaccc gtacatgccc aaaatagggg gcgggttaca 4920
cagaatatat aacatcgtag gtgtctgggt gaacagttta ttcctggcat ccactaaata 4980
taatggagcc cgctttttaa gctggcatcc agaaaaaaaa agaatcccag caccaaaata 5040
ttgttttctt caccaaccat cagttcatag gtccattctc ttagcgcaac tacagagaac 5100
aggggcacaa acaggcaaaa aacgggcaca acctcaatgg agtgatgcaa cctgcctgga 5160
gtaaatgatg acacaaggca attgacccac gcatgtatct atctcatttt cttacacctt 5220
ctattacctt ctgctctctc tgatttggaa aaagctgaaa aaaaaggttg aaaccagttc 5280
cctgaaatta ttcccctact tgactaataa gtatataaag acggtaggta ttgattgtaa 5340
ttctgtaaat ctatttctta aacttcttaa attctacttt tatagttagt cttttttttta 5400
gttttaaaac accaagaact tagtttcgaa taaacacaca taaagaattc aaaatgtctt 5460

ggccaaccga ctctgaattg aactctatca aggaagctgt tgctcaaatg tctggtagag 5520
acaagggtga agttagagtt gttgttgctc catacagaat ctgtccattg ggtgctcaca 5580
tcgaccacca aggtggtact gtttctgcta tgaccatcaa caagggtatc ttgttgggtt 5640
tcgttccatc tggtgacacc caagttcaat tgagatctgc tcaattcgaa ggtgaagttt 5700
gtttcagagt tgacgaaatc caacacccaa tcggtttggc taacaagaac ggtgcttcta 5760
ccccatctcc atctaaggaa aagtctatct ggggtactta cgctagaggt gctgtttacg 5820
ctttgcaatc ttctaagaag aacttgaagc aaggtatcat cggttacttg tctggttcta 5880
acggtttgga ctcttcaggt ttatcttctt ctgctgctgt tggtgttgct tacttgttag 5940
ctttggaaaa cgctaacgaa ttgaccgttt ctccaaccga aaacatcgaa tacgacagat 6000
tgatcgaaaa cggttacttg ggtttgagaa acggtatctt ggaccaatct gctatcttgt 6060
tgtctaacta cggttgtttg acctacatgg actgtaagac cttggaccac gaattggttc 6120
aagctccaga attggaaaag ccattcagaa tcttgttggc tttctctggt ttgagacaag 6180
ctttgaccac caacccaggt ttcaacttga gagtttctga atgtcaagaa gctgctaagg 6240
ttttgttgac cgcttcaggt aactctgaat tggaaccaac cttgtgtaac gttgaacacg 6300
ctgtttacga agctcacaag cacgaattga agcctgtttt ggctaagaga gctgaacact 6360
acttctctga aaacatgaga gttatcaagg gtagagaagc ttgggcttct ggtaacttgg 6420
aagaatttgg taagttgatc tctgcttctg gtttgtcttc tatcgaaaac tacgaatgtg 6480
gtgctgaacc attgatccaa ttgtacaaga ttttgttgaa ggctccaggt gtttacggtg 6540
ctagattctc tggtgctggt ttcagaggtt gttgtttggc tttcgttgac gctgaaaagg 6600
ctgaagctgc tgcttcttac gttaaggacg aatacgaaaa ggctcaacca gaatttgcta 6660
acaacttgaa cggtggtaag ccagttttga tctgtgaagc tggtgacgct gctagagttt 6720
tgttgtaagg atcccctttt cctttgtcga tatcatgtaa ttagttatgt cacgcttaca 6780
ttcacgccct cctcccacat ccgctctaac cgaaaaggaa ggagttagac aacctgaagt 6840
ctaggtccct atttattttt tttaatagtt atgttagtat taagaacgtt atttatattt 6900
caaatttttc ttttttttct gtacaaacgc gtgtacgcat gtaacattat actgaaaacc 6960
ttgcttgaga aggttttggg acgctcgaag gcttcctagg ctcgaggggg ggcccggtac 7020
ccagcttttg ttccctttag tgagggttaa ttccgagctt ggcgtaatca tggtcatagc 7080
tgtttcctgt gtgaaattgt tatccgctca caattccaca caacatagga gccggaagca 7140
taaagtgtaa agcctggggt gcctaatgag tgaggtaact cacattaatt gcgttgcgct 7200
cactgcccgc tttccagtcg ggaaacctgt cgtgccagct gcattaatga atcggccaac 7260
gcgcggggag aggcggtttg cgtattgggc gctcttccgc ttcctcgctc actgactcgc 7320
tgcgctcggt cgttcggctg cggcgagcgg tatcagctca ctcaaaggcg gtaatacggt 7380

tatccacaga atcaggggat aacgcaggaa agaacatgtg agcaaaaggc cagcaaaagg 7440

ccaggaaccg taaaaaggcc gcgttgctgg cgtttttcca taggctcggc cccctgacg 7500

agcatcacaa aaatcgacgc tcaagtcaga ggtggcgaaa cccgacagga ctataaagat 7560

accaggcgtt cccccctgga agctccctcg tgcgctctcc tgttccgacc ctgccgctta 7620

ccggatacct gtccgccttt ctcccttcgg gaagcgtggc gctttctcaa tgctcacgct 7680

gtaggtatct cagttcggtg taggtcgttc gctccaagct gggctgtgtg cacgaacccc 7740

ccgttcagcc cgaccgctgc gccttatccg gtaactatcg tcttgagtcc aacccggtaa 7800

gacacgactt atcgccactg gcagcagcca ctggtaacag gattagcaga gcgaggtatg 7860

taggcggtgc tacagagttc ttgaagtggt ggcctaacta cggctacact agaaggacag 7920

tatttggtat ctgcgctctg ctgaagccag ttaccttcgg aaaaagagtt ggtagctctt 7980

gatccggcaa acaaaccacc gctggtagcg gtggtttttt tgtttgcaag cagcagatta 8040

cgcgcagaaa aaaaggatct caagaagatc ctttgatctt ttctacgggg tctgacgctc 8100

agtggaacga aaactcacgt taagggattt tggtcatgag attatcaaaa aggatcttca 8160

cctagatcct tttaaattaa aaatgaagtt ttaaatcaat ctaaagtata tatgagtaaa 8220

cttggtctga cagttaccaa tgcttaatca gtgaggcacc tatctcagcg atctgtctat 8280

ttcgttcatc catagttgcc tgactgcccg tcgtgtagat aactacgata cgggagggct 8340

taccatctgg ccccagtgct gcaatgatac cgcgagaccc acgctcaccg gctccagatt 8400

tatcagcaat aaaccagcca gccggaaggg ccgagcgcag aagtggtcct gcaactttat 8460

ccgcctccat ccagtctatt aattgttgcc gggaagctag agtaagtagt tcgccagtta 8520

atagtttgcg caacgttgtt gccattgcta caggcatcgt ggtgtcacgc tcgtcgtttg 8580

gtatggcttc attcagctcc ggttcccaac gatcaaggcg agttacatga tcccccatgt 8640

tgtgaaaaaa agcggttagc tccttcggtc ctccgatcgt tgtcagaagt aagttggccg 8700

cagtgttatc actcatggtt atggcagcac tgcataattc tcttactgtc atgccatccg 8760

taagatgctt ttctgtgact ggtgagtact caaccaagtc attctgagaa tagtgtatgc 8820

ggcgaccgag ttgctcttgc ccggcgtcaa tacgggataa taccgcgcca catagcagaa 8880

ctttaaaagt gctcatcatt ggaaaacgtt cttcggggcg aaaactctca aggatcttac 8940

cgctgttgag atccagttcg atgtaaccca ctcgtgcacc caactgatct tcagcatctt 9000

ttactttcac cagcgtttct gggtgagcaa aaacaggaag gcaaaatgcc gcaaaaaagg 9060

gaataagggc gacacggaaa tgttgaatac tcatactctt cctttttcaa tattattgaa 9120

gcatttatca gggttattgt ctcatgagcg gatacatatt tgaatgtatt tagaaaaata 9180

aacaaatagg ggttccgcgc acatttcccc gaaaagtgcc acctgggtcc ttttcatcac 9240

gtgctataaa aataattata atttaaattt tttaatataa atatataaat taaaaataga 9300

aagtaaaaaa agaaattaaa gaaaaaatag tttttgtttt ccgaagatgt aaaagactct 9360

aggggggatcg ccaacaaata ctacctttta tcttgctctt cctgctctca ggtattaatg 9420

ccgaattgtt tcatcttgtc tgtgtagaag accacacacg aaaatcctgt gattttacat 9480

tttacttatc gttaatcgaa tgtatatcta tttaatctgc ttttcttgtc taataaatat 9540

atatgtaaag tacgcttttt gttgaaattt tttaaacctt tgtttatttt tttttcttca 9600

ttccgtaact cttctacctt ctttatttac tttctaaaat ccaaatacaa aacataaaaa 9660

taaataaaca cagagtaaat tcccaaatta ttccatcatt aaaagatacg aggcgcgtgt 9720

aagttacagg caagcgatcc gtcctaagaa accattatta tcatgacatt aacctataaa 9780

aataggcgta tcacgaggcc ctttcgtctc gcgcgtttcg gtgatgacgg tgaaaacctc 9840

tgacacatgc agctcccgga gacggtcaca gcttgtctgt aagcggatgc cgggagcaga 9900

caagcccgtc agggcgcgtc agcgggtgtt ggcgg 9935

<210> 36
<211> 6341
<212> DNA
<213> Artificial

<220>
<223> pRN615 shuttle vector

<400> 36
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatcga ctacgtcgta aggccgtttc tgacagagta aaattcttga gggaactttc 240

accattatgg gaaatggttc aagaaggtat tgacttaaac tccatcaaat ggtcaggtca 300

ttgagtgttt tttatttgtt gtattttttt ttttttagag aaaatcctcc aatatcaaat 360

taggaatcgt agtttcatga ttttctgtta cacctaactt tttgtgtggt gcccctcctc 420

ttgtcaatat taatgttaaa gtgcaattct ttttccttat cacgttgagc cattagtatc 480

aatttgctta cctgtattcc tttactatcc tcctttttct ccttcttgat aaatgtatgt 540

agattgcgta tatagtttcg tctaccctat gaacatattc cattttgtaa tttcgtgtcg 600

tttctattat gaatttcatt tataaagttt atgtacacct aggatccgtc gacactggat 660

ggcggcgtta gtatcgaatc gacagcagta tagcgaccag cattcacata cgattgacgc 720

atgatattac ttttctgcgca cttaacttcg catctgggca gatgatgtcg aggcgaaaaa 780

aaatataaat cacgctaaca tttgattaaa atagaacaac tacaatataa aaaaactata 840

caaatgacaa gttcttgaaa acaagaatct ttttattgtc agtgtgtcag tcctgctcct 900

cggccacgaa gtgcacgcag ttgccggccg ggtcgcgcag ggcgaactcc cgcccccacg 960

gctgctcgcc gatctcggtc atggccggcc cggaggcgtc ccggaagttc gtggacacga 1020
cctccgacca ctcggcgtac agctcgtcca ggccgcgcac ccacacccag gccagggtgt 1080
tgtccggcac cacctggtcc tggaccgcgc tgatgaacag ggtcacgtcg tcccggacca 1140
caccggcgaa gtcgtcctcc acgaagtccc gggagaaccc gagccggtcg gtccagaact 1200
cgaccgctcc ggcgacgtcg cgcgcggtga gcaccggaac ggcactggtc aacttggcca 1260
tggttgttta tgttcggatg tgatgtgaga actgtatcct agcaagattt taaaaggaag 1320
tatatgaaag aagaacctca gtggcaaatc ctaacctttt atatttctct acaggggcgc 1380
ggcgtgggga caattcaacg cgtctgtgag gggagcgttt ccctgctcgc aggtctgcag 1440
cgaggagccg taatttttgc ttcgcgccgt gcggccatca aaatgtatgg atgcaaatga 1500
ttatacatgg ggatgtatgg gctaaatgta cgggcgacag tcacatcatg cccctgagct 1560
gcgcacgtca agactgtcaa ggagggtatt ctgggcctcc atgtcgctgg ccgggtgacc 1620
cggcggggac gaggccttaa gcggccgcat gctagctccg gattatcgat gataagctgt 1680
caaacatgag aattaattcc acggactata gactatacct agtatactcc gtctactgta 1740
cgatacactt ccgctcaggt ccttgtcctt taacgaggcc ttaccactct tttgttactc 1800
tattgatcca gctcagcaaa ggcagtgtga tctaagattc tatcttcgcg atgtagtaaa 1860
actagctaga ccgagaaaga gactagaaat gcaaaaggca cttctacaat ggctgccatc 1920
attattatcc gatgtgacgc tgcagcttct caatgatatt cgaatacgct ttgaggagat 1980
acagcctaat atccgacaaa ctgttttaca gatttacgat cgtacttgtt acccatcatt 2040
gaattttgaa catccgaacc tgggagtttt ccctgaaaca gatagtatat ttgaacctgt 2100
ataataatat atagtctagc gctttacgga agacaatgta tgtatttcgg ttcctggaga 2160
aactattgca tctattgcat aggtaatctt gcacgtcgca tccccggttc attttctgcg 2220
tttccatctt gcacttcaat agcatatctt tgttaacgaa gcatctgtgc ttcattttgt 2280
agaacaaaaa tgcaacgcga gagcgctaat ttttcaaaca aagaatctga gctgcatttt 2340
tacagaacag aaatgcaacg cgaaagcgct attttaccaa cgaagaatct gtgcttcatt 2400
tttgtaaaac aaaaatgcaa cgcgagagcg ctaatttttc aaacaaagaa tctgagctgc 2460
atttttacag aacagaaatg caacgcgaga gcgctatttt accaacaaag aatctatact 2520
tcttttttgt tctacaaaaa tgcatcccga gagcgctatt tttctaacaa agcatcttag 2580
attacttttt ttctcctttg tgcgctctat aatgcagtct cttgataact ttttgcactg 2640
taggtccgtt aaggttagaa gaaggctact ttggtgtcta ttttctcttc cataaaaaaa 2700
gcctgactcc acttcccgcg tttactgatt actagcgaag ctgcgggtgc atttttcaa 2760
gataaaggca tccccgatta tattctatac cgatgtggat tgcgcatact ttgtgaacag 2820
aaagtgatag cgttgatgat tcttcattgg tcagaaaatt atgaacggtt tcttctattt 2880

tgtctctata tactacgtat aggaaatgtt tacattttcg tattgttttc gattcactct 2940
atgaatagtt cttactacaa tttttttgtc taaagagtaa tactagagat aaacataaaa 3000
aatgtagagg tcgagtttag atgcaagttc aaggagcgaa aggtggatgg gtaggttata 3060
tagggatata gcacagagat atatagcaaa gagatacttt tgagcaatgt ttgtggaaag 3120
cggtattcgc aatgccggca aaagggcgaa ttgattttga agagaatgtg gattttgatg 3180
taattgttgg gattccattt ttaataaggc aataatatta ggtatgtgga tatactagaa 3240
gttctcctcg accgtcgata tgcggtgtga aataccgcac agatgcgtaa ggagaaaata 3300
ccgcatcagg aaattgtaaa cgttaatatt ttgttaaaat tcgcgttaaa tttttgttaa 3360
atcagctcat tttttaacca ataggccgaa atcggcaaaa tcccttataa atcaaaagaa 3420
tagaccgaga tagggttgag tgttgttcca gtttggaaca agagtccact attaaagaac 3480
gtggactcca acgtcaaagg gcgaaaaacc gtctatcagg gcgatggccc actacgtgaa 3540
ccatcaccct aatcaagttt tttggggtcg aggtgccgta aagcactaaa tcggaaccct 3600
aaagggagcc cccgatttag agcttgacgg ggaaagccgg cgaacgtggc gagaaaggaa 3660
gggaagaaag cgaaaggagc gggcgctagg gcgctggcaa gtgtagcggt cacgctgcgc 3720
gtaaccacca cacccgccgc gcttaatgcg ccgctacagg gcgcgtcgcg ccattcgcca 3780
ttcaggctgc gcaactgttg ggaagggcga tcggtgcggg cctcttcgct attacgccag 3840
ctggcgaagg ggggatgtgc tgcaaggcga ttaagttggg taacgccagg gttttcccag 3900
tcacgacgtt gtaaaacgac ggccagtgaa ttgtaatacg actcactata gggcgaattg 3960
gagctccacc gcggtggcgg ccgctctaga actagtggat cccccgggct gcaggaattc 4020
gatatcaagc ttatcgatac cgtcgacctc gagggggggc ccggtaccca gcttttgttc 4080
cctttagtga gggttaattc cgagcttggc gtaatcatgg tcatagctgt ttcctgtgtg 4140
aaattgttat ccgctcacaa ttccacacaa cataggagcc ggaagcataa agtgtaaagc 4200
ctggggtgcc taatgagtga ggtaactcac attaattgcg ttgcgctcac tgcccgcttt 4260
ccagtcggga aacctgtcgt gccagctgca ttaatgaatc ggccaacgcg cggggagagg 4320
cggtttgcgt attgggcgct cttccgcttc ctcgctcact gactcgctgc gctcggtcgt 4380
tcggctgcgg cgagcggtat cagctcactc aaaggcggta atacggttat ccacagaatc 4440
aggggataac gcaggaaaga acatgtgagc aaaaggccag caaaaggcca ggaaccgtaa 4500
aaaggccgcg ttgctggcgt ttttccatag gctcggcccc cctgacgagc atcacaaaaa 4560
tcgacgctca agtcagaggt ggcgaaaccc gacaggacta taaagatacc aggcgtttcc 4620
ccctggaagc tccctcgtgc gctctcctgt tccgaccctg ccgcttaccg gatacctgtc 4680
cgcctttctc ccttcgggaa gcgtggcgct ttctcaatgc tcacgctgta ggtatctcag 4740
ttcggtgtag gtcgttcgct ccaagctggg ctgtgtgcac gaaccccccg ttcagcccga 4800

ccgctgcgcc ttatccggta actatcgtct tgagtccaac ccggtaagac acgacttatc 4860
gccactggca gcagccactg gtaacaggat tagcagagcg aggtatgtag gcggtgctac 4920
agagttcttg aagtggtggc ctaactacgg ctacactaga aggacagtat ttggtatctg 4980
cgctctgctg aagccagtta ccttcggaaa aagagttggt agctcttgat ccggcaaaca 5040
aaccaccgct ggtagcggtg gtttttttgt ttgcaagcag cagattacgc gcagaaaaaa 5100
aggatctcaa gaagatcctt tgatcttttc tacggggtct gacgctcagt ggaacgaaaa 5160
ctcacgttaa gggattttgg tcatgagatt atcaaaaagg atcttcacct agatcctttt 5220
aaattaaaaa tgaagtttta aatcaatcta aagtatatat gagtaaactt ggtctgacag 5280
ttaccaatgc ttaatcagtg aggcacctat ctcagcgatc tgtctatttc gttcatccat 5340
agttgcctga ctgccccgtcg tgtagataac tacgatacgg gagggcttac catctggccc 5400
cagtgctgca atgataccgc gagacccacg ctcaccggct ccagatttat cagcaataaa 5460
ccagccagcc ggaagggccg agcgcagaag tggtcctgca actttatccg cctccatcca 5520
gtctattaat tgttgccggg aagctagagt aagtagttcg ccagttaata gtttgcgcaa 5580
cgttgttgcc attgctacag gcatcgtggt gtcacgctcg tcgtttggta tggcttcatt 5640
cagctccggt tcccaacgat caaggcgagt tacatgatcc cccatgttgt gaaaaaagc 5700
ggttagctcc ttcggtcctc cgatcgttgt cagaagtaag ttggccgcag tgttatcact 5760
catggttatg gcagcactgc ataattctct tactgtcatg ccatccgtaa gatgcttttc 5820
tgtgactggt gagtactcaa ccaagtcatt ctgagaatag tgtatgcggc gaccgagttg 5880
ctcttgcccg gcgtcaatac gggataatac cgcgccacat agcagaactt taaaagtgct 5940
catcattgga aaacgttctt cggggcgaaa actctcaagg atcttaccgc tgttgagatc 6000
cagttcgatg taacccactc gtgcacccaa ctgatcttca gcatctttta ctttcaccag 6060
cgtttctggg tgagcaaaaa caggaaggca aaatgccgca aaaaagggaa taagggcgac 6120
acggaaatgt tgaatactca tactcttcct ttttcaatat tattgaagca tttatcaggg 6180
ttattgtctc atgagcggat acatatttga atgtatttag aaaaataaac aaataggggt 6240
tccgcgcaca tttccccgaa aagtgccacc tgacgtctaa gaaaccatta ttatcatgac 6300
attaacctat aaaaataggc gtatcacgag gccctttcgt c 6341

<210> 37
<211> 2056
<212> DNA
<213> Artificial

<220>
<223> Expression cassette for EC 2.7.1.43 At GlcAK

<400> 37
ggtacctagg accggtttat cattatcaat actgccattt caaagaatac gtaaataatt 60

aatagtagtg attttcctaa ctttatttag tcaaaaaatt agccttttaa ttctgctgta 120

acccgtacat gcccaaaata gggggcgggt tacacagaat atataacatc gtaggtgtct 180

gggtgaacag tttattcctg gcatccacta aatataatgg agcccgcttt ttaagctggc 240

atccagaaaa aaaaagaatc ccagcaccaa aatattgttt tcttcaccaa ccatcagttc 300

ataggtccat tctcttagcg caactacaga gaacaggggc acaaacaggc aaaaaacggg 360

cacaacctca atggagtgat gcaacctgcc tggagtaaat gatgacacaa ggcaattgac 420

ccacgcatgt atctatctca ttttcttaca ccttctatta ccttctgctc tctctgattt 480

ggaaaaagct gaaaaaaaag gttgaaacca gttccctgaa attattcccc tacttgacta 540

ataagtatat aaagacggta ggtattgatt gtaattctgt aaatctattt cttaaacttc 600

ttaaattcta cttttatagt tagtcttttt tttagtttta aaacaccaag aacttagttt 660

cgaataaaca cacataaaga attcaaaatg gacccaaact ctaccgtttc tggtgacggt 720

caagctaccg ctgctatcga acacagatct ttcgctagaa tcggtttctt gggtaaccct 780

tctgacgttt acttcggtag aaccatctct ttgaccatcg gtaacttctg ggcttctgtt 840

aagttggaac catctgaaca cttggttatc aagccacacc cattccacga cttggttcaa 900

ttcacctctt tggaccactt gttgaacaga ttgcaaaacg aaggttacta cggtggtgtt 960

agattgttga tggctatctg taaggttttc agaaactact gtaaggaaaa cgacatccaa 1020

ttgcaccaag ctaacttctc tttgtcttac gacaccaaca tcccaagaca aactggtttg 1080

tctggttctt ctgctatcgt ttctgctgct ttgaactgtt tgttggactt ctacaacgtt 1140

agacacttga tcaaggttca agttagacca aacatcgttt tgtctgctga aaaggaattg 1200

ggtatcgttg ctggtttgca agacagagtt gctcaagttt acggtggttt ggttcacatg 1260

gacttctcta aggaacacat ggacaagttg ggtcacggta tctacacccc aatggacatc 1320

tctttgttgc caccattgca cttgatctac gctgaaaacc catctgactc tggtaaggtt 1380

cactctatgg ttagacaaag atggttggac ggtgacgaat ttatcatctc ttctatgaag 1440

gaagttggtt ctttggctga agaaggtaga accgctttgt tgaacaagga ccactctaag 1500

ttggttgaat tgatgaactt gaacttcgac atcagaagga gaatgttcgg tgacgaatgt 1560

ttgggtgcta tgaacatcga aatggttgaa gttgctagaa gagttggtgc tgcttctaag 1620

ttcactggtt ctggtggtgc tgttgttgtt ttctgtccag aaggtccatc tcaagttaag 1680

ttgttggaag aagaatgtag aaaggctggt ttcaccttgc aaccagttaa gatcgctcca 1740

tcttgtttga acgactctga catccaaacc ttgtaaggat ccccttttcc tttgtcgata 1800

tcatgtaatt agttatgtca cgcttacatt cacgccctcc tcccacatcc gctctaaccg 1860

aaaaggaagg agttagacaa cctgaagtct aggtccctat ttattttttt taatagttat 1920

gttagtatta agaacgttat ttatatttca aatttttctt ttttttctgt acaaacgcgt 1980

gtacgcatgt aacattatac tgaaaacctt gcttgagaag gttttgggac gctcgaaggc 2040

ttcctaggct cgagtt 2056

<210> 38
<211> 8374
<212> DNA
<213> Artificial

<220>
<223> pRN762

<400> 38
gacgaaaggg cctcgtgata cgcctatttt tataggttaa tgtcatgata ataatggttt 60

cttagacgtc aggtggcact tttcggggaa atgtgcgcgg aacccctatt tgtttatttt 120

tctaaataca ttcaaatatg tatccgctca tgagacaata accctgataa atgcttcaat 180

aatattgaaa aaggaagagt atgagtattc aacatttccg tgtcgccctt attccctttt 240

ttgcggcatt ttgccttcct gtttttgctc acccagaaac gctggtgaaa gtaaaagatg 300

ctgaagatca gttgggtgca cgagtgggtt acatcgaact ggatctcaac agcggtaaga 360

tccttgagag ttttcgcccc gaagaacgtt ttccaatgat gagcactttt aaagttctgc 420

tatgtggcgc ggtattatcc cgtattgacg ccgggcaaga gcaactcggt cgccgcatac 480

actattctca gaatgacttg gttgagtact caccagtcac agaaaagcat cttacggatg 540

gcatgacagt aagagaatta tgcagtgctg ccataaccat gagtgataac actgcggcca 600

acttacttct gacaacgatc ggaggaccga aggagctaac cgcttttttt cacaacatgg 660

gggatcatgt aactcgcctt gatcgttggg aaccggagct gaatgaagcc ataccaaacg 720

acgagcgtga caccacgatg cctgtagcaa tggcaacaac gttgcgcaaa ctattaactg 780

gcgaactact tactctagct tcccggcaac aattaataga ctggatggag gcggataaag 840

ttgcaggacc acttctgcgc tcggcccttc cggctggctg gtttattgct gataaatctg 900

gagccggtga gcgtgggtct cgcggtatca ttgcagcact ggggccagat ggtaagccct 960

cccgtatcgt agttatctac acgacgggca gtcaggcaac tatggatgaa cgaaatagac 1020

agatcgctga gataggtgcc tcactgatta agcattggta actgtcagac caagtttact 1080

catatatact ttagattgat ttaaaacttc atttttaatt taaaaggatc taggtgaaga 1140

tcctttttga taatctcatg accaaaatcc cttaacgtga gttttcgttc cactgagcgt 1200

cagaccccgt agaaaagatc aaaggatctt cttgagatcc tttttttctg cgcgtaatct 1260

gctgcttgca aacaaaaaaa ccaccgctac cagcggtggt ttgtttgccg gatcaagagc 1320

taccaactct ttttccgaag gtaactggct tcagcagagc gcagatacca aatactgtcc 1380

ttctagtgta gccgtagtta ggccaccact tcaagaactc tgtagcaccg cctacatacc 1440

tcgctctgct aatcctgtta ccagtggctg ctgccagtgg cgataagtcg tgtcttaccg 1500

ggttggactc aagacgatag ttaccggata aggcgcagcg gtcgggctga acggggggtt 1560

cgtgcacaca gcccagcttg gagcgaacga cctacaccga actgagatac ctacagcgtg 1620

agcattgaga aagcgccacg cttcccgaag ggagaaaggc ggacaggtat ccggtaagcg 1680

gcagggtcgg aacaggagag cgcacgaggg agcttccagg ggggaacgcc tggtatcttt 1740

atagtcctgt cgggtttcgc cacctctgac ttgagcgtcg atttttgtga tgctcgtcag 1800

gggggccgag cctatggaaa aacgccagca acgcggcctt tttacggttc ctggcctttt 1860

gctggccttt tgctcacatg ttctttcctg cgttatcccc tgattctgtg gataaccgta 1920

ttaccgcctt tgagtgagct gataccgctc gccgcagccg aacgaccgag cgcagcgagt 1980

cagtgagcga ggaagcggaa gagcgcccaa tacgcaaacc gcctctcccc gcgcgttggc 2040

cgattcatta atgcagctgg cacgacaggt ttcccgactg gaaagcgggc agtgagcgca 2100

acgcaattaa tgtgagttac ctcactcatt aggcacccca ggctttacac tttatgcttc 2160

cggctcctat gttgtgtgga attgtgagcg gataacaatt tcacacagga aacagctatg 2220

accatgatta cgccaagctc ggaattaacc ctcactaaag ggaacaaaag ctgggtacct 2280

aggaccggtt tatcattatc aatactgcca tttcaaagaa tacgtaaata attaatagta 2340

gtgattttcc taactttatt tagtcaaaaa attagccttt taattctgct gtaacccgta 2400

catgcccaaa ataggggggcg ggttacacag aatatataac atcgtaggtg tctgggtgaa 2460

cagtttattc ctggcatcca ctaaatataa tggagcccgc tttttaagct ggcatccaga 2520

aaaaaaaaga atcccagcac caaaatattg ttttcttcac caaccatcag ttcataggtc 2580

cattctctta gcgcaactac agagaacagg ggcacaaaca ggcaaaaaac gggcacaacc 2640

tcaatggagt gatgcaacct gcctggagta aatgatgaca caaggcaatt gacccacgca 2700

tgtatctatc tcattttctt acaccttcta ttaccttctg ctctctctga tttggaaaaa 2760

gctgaaaaaa aaggttgaaa ccagttccct gaaattattc ccctacttga ctaataagta 2820

tataaagacg gtaggtattg attgtaattc tgtaaatcta tttcttaaac ttcttaaatt 2880

ctacttttat agttagtctt ttttttagtt ttaaaacacc aagaacttag tttcgaataa 2940

acacacataa agaattcaaa atggacccaa actctaccgt ttctggtgac ggtcaagcta 3000

ccgctgctat cgaacacaga tctttcgcta gaatcggttt cttgggtaac ccttctgacg 3060

tttacttcgg tagaaccatc tctttgacca tcggtaactt ctgggcttct gttaagttgg 3120

aaccatctga acacttggtt atcaagccac acccattcca cgacttggtt caattcacct 3180

ctttggacca cttgttgaac agattgcaaa acgaaggtta ctacggtggt gttagattgt 3240

tgatggctat ctgtaaggtt ttcagaaact actgtaagga aaacgacatc caattgcacc 3300

aagctaactt ctctttgtct tacgacacca acatcccaag acaaactggt ttgtctggtt 3360

cttctgctat cgtttctgct gctttgaact gtttgttgga cttctacaac gttagacact 3420

```
tgatcaaggt tcaagttaga ccaaacatcg ttttgtctgc tgaaaaggaa ttgggtatcg   3480
ttgctggttt gcaagacaga gttgctcaag tttacggtgg tttggttcac atggacttct   3540
ctaaggaaca catggacaag ttgggtcacg gtatctacac cccaatggac atctctttgt   3600
tgccaccatt gcacttgatc tacgctgaaa acccatctga ctctggtaag gttcactcta   3660
tggttagaca aagatggttg gacggtgacg aatttatcat ctcttctatg aaggaagttg   3720
gttctttggc tgaagaaggt agaaccgctt tgttgaacaa ggaccactct aagttggttg   3780
aattgatgaa cttgaacttc gacatcagaa ggagaatgtt cggtgacgaa tgtttgggtg   3840
ctatgaacat cgaaatggtt gaagttgcta gaagagttgg tgctgcttct aagttcactg   3900
gttctggtgg tgctgttgtt gttttctgtc cagaaggtcc atctcaagtt aagttgttgg   3960
aagaagaatg tagaaaggct ggtttcacct tgcaaccagt taagatcgct ccatcttgtt   4020
tgaacgactc tgacatccaa accttgtaag gatccccttt tcctttgtcg atatcatgta   4080
attagttatg tcacgcttac attcacgccc tcctcccaca tccgctctaa ccgaaaagga   4140
aggagttaga caacctgaag tctaggtccc tatttatttt ttttaatagt tatgttagta   4200
ttaagaacgt tatttatatt tcaaattttt cttttttttc tgtacaaacg cgtgtacgca   4260
tgtaacatta tactgaaaac cttgcttgag aaggttttgg gacgctcgaa ggctttaatt   4320
gctcgaggtc gacggtatcg ataagcttga tatcgaattc ctgcagcccg ggggatccac   4380
tagttctaga gcggccgcca ccgcggtgga gctccaattc gccctatagt gagtcgtatt   4440
acaattcact ggccgtcgtt ttacaacgtc gtgactggga aaaccctggc gttacccaac   4500
ttaatcgcct tgcagcacat ccccctttcg ccagctggcg taatagcgaa gaggcccgca   4560
ccgatcgccc ttcccaacag ttgcgcagcc tgaatggcga atggcgcgac gcgccctgta   4620
gcggcgcatt aagcgcggcg ggtgtggtgg ttacgcgcag cgtgaccgct acacttgcca   4680
gcgccctagc gcccgctcct ttcgctttct tcccttcctt tctcgccacg ttcgccggct   4740
ttccccgtca agctctaaat cgggggctcc ctttagggtt ccgatttagt gctttacggc   4800
acctcgaccc caaaaaactt gattagggtg atggttcacg tagtgggcca tcgccctgat   4860
agacggtttt tcgccctttg acgttggagt ccacgttctt taatagtgga ctcttgttcc   4920
aaactggaac aacactcaac cctatctcgg tctattcttt tgatttataa gggattttgc   4980
cgatttcggc ctattggtta aaaaatgagc tgatttaaca aaaatttaac gcgaatttta   5040
acaaaatatt aacgtttaca atttcctgat gcggtatttt ctccttacgc atctgtgcgg   5100
tatttcacac cgcatatcga cggtcgagga gaacttctag tatatccaca tacctaatat   5160
tattgcctta ttaaaaatgg aatcccaaca attacatcaa aatccacatt ctcttcaaaa   5220
tcaattcgcc cttttgccgg cattgcgaat accgctttcc acaaacattg ctcaaaagta   5280
tctctttgct atatatctct gtgctatatc cctatataac ctacccatcc acctttcgct   5340
```

ccttgaactt gcatctaaac tcgacctcta catttttat gtttatctct agtattactc 5400

tttagacaaa aaaattgtag taagaactat tcatagagtg aatcgaaaac aatacgaaaa 5460

tgtaaacatt tcctatacgt agtatataga gacaaaatag aagaaaccgt tcataatttt 5520

ctgaccaatg aagaatcatc aacgctatca ctttctgttc acaaagtatg cgcaatccac 5580

atcggtatag aatataatcg gggatgcctt tatcttgaaa aaatgcaccc gcagcttcgc 5640

tagtaatcag taaacgcggg aagtggagtc aggcttttttt tatggaagag aaaatagaca 5700

ccaaagtagc cttcttctaa ccttaacgga cctacagtgc aaaaagttat caagagactg 5760

cattatagag cgcacaaagg agaaaaaaag taatctaaga tgctttgtta gaaaaatagc 5820

gctctcggga tgcatttttg tagaacaaaa aagaagtata gattctttgt tggtaaaata 5880

gcgctctcgc gttgcatttc tgttctgtaa aaatgcagct cagattcttt gtttgaaaaa 5940

ttagcgctct cgcgttgcat ttttgtttta caaaaatgaa gcacagattc ttcgttggta 6000

aaatagcgct ttcgcgttgc atttctgttc tgtaaaaatg cagctcagat tctttgtttg 6060

aaaaattagc gctctcgcgt tgcatttttg ttctacaaaa tgaagcacag atgcttcgtt 6120

aacaaagata tgctattgaa gtgcaagatg gaaacgcaga aaatgaaccg gggatgcgac 6180

gtgcaagatt acctatgcaa tagatgcaat agtttctcca ggaaccgaaa tacatacatt 6240

gtcttccgta aagcgctaga ctatatatta ttatacaggt tcaaatatac tatctgtttc 6300

agggaaaact cccaggttcg gatgttcaaa attcaatgat gggtaacaag tacgatcgta 6360

aatctgtaaa acagtttgtc ggatattagg ctgtatctcc tcaaagcgta ttcgaatatc 6420

attgagaagc tgcagcgtca catcggataa taatgatggc agccattgta gaagtgcctt 6480

ttgcatttct agtctctttc tcggtctagc tagttttact acatcgcgaa gatagaatct 6540

tagatcacac tgcctttgct gagctggatc aatagagtaa caaaagagtg gtaaggcctc 6600

gttaaaggac aaggacctga gcggaagtgt atcgtacagt agacggagta tactaggtat 6660

agtctatagt ccgtggaatt aattctcatg tttgacagct tatcatcgat aatccggagc 6720

tagcatgcgg ccgcttaagg cctcgtcccc gccgggtcac ccggccagcg acatggaggc 6780

ccagaatacc ctccttgaca gtcttgacgt gcgcagctca ggggcatgat gtgactgtcg 6840

cccgtacatt tagcccatac atccccatgt ataatcattt gcatccatac attttgatgg 6900

ccgcacggcg cgaagcaaaa attacggctc ctcgctgcag acctgcgagc agggaaacgc 6960

tcccctcaca gacgcgttga attgtcccca cgccgcgccc ctgtagagaa atataaaagg 7020

ttaggatttg ccactgaggt tcttctttca tatacttcct tttaaaatct tgctaggata 7080

cagttctcac atcacatccg aacataaaca accatggcca agttgaccag tgccgttccg 7140

gtgctcaccg cgcgcgacgt cgccggagcg gtcgagttct ggaccgaccg gctcgggttc 7200

tcccgggact tcgtggagga cgacttcgcc ggtgtggtcc gggacgacgt gaccctgttc 7260

```
atcagcgcgg tccaggacca ggtggtgccg gacaacaccc tggcctgggt gtgggtgcgc    7320
ggcctggacg agctgtacgc cgagtggtcg gaggtcgtgt ccacgaactt ccgggacgcc    7380
tccgggccgg ccatgaccga gatcggcgag cagccgtggg ggcgggagtt cgccctgcgc    7440
gacccggccg gcaactgcgt gcacttcgtg gccgaggagc aggactgaca cactgacaat    7500
aaaaagattc ttgttttcaa gaacttgtca tttgtatagt ttttttatat tgtagttgtt    7560
ctattttaat caaatgttag cgtgatttat attttttttc gcctcgacat catctgccca    7620
gatgcgaagt taagtgcgca gaaagtaata tcatgcgtca atcgtatgtg aatgctggtc    7680
gctatactgc tgtcgattcg atactaacgc cgccatccag tgtcgacgga tcctaggtgt    7740
acataaactt tataaatgaa attcataata gaaacgacac gaaattacaa aatggaatat    7800
gttcataggg tagacgaaac tatatacgca atctacatac atttatcaag aaggagaaaa    7860
aggaggatag taaaggaata caggtaagca aattgatact aatggctcaa cgtgataagg    7920
aaaaagaatt gcactttaac attaatattg acaaggagga gggcaccaca caaaaagtta    7980
ggtgtaacag aaaatcatga aactacgatt cctaatttga tattggagga ttttctctaa    8040
aaaaaaaaaa atacaacaaa taaaaaacac tcaatgacct gaccatttga tggagtttaa    8100
gtcaatacct tcttgaacca tttcccataa tggtgaaagt tccctcaaga attttactct    8160
gtcagaaacg gccttacgac gtagtcgata tggtgcactc tcagtacaat ctgctctgat    8220
gccgcatagt taagccagcc ccgacacccg ccaacacccg ctgacgcgcc ctgacgggct    8280
tgtctgctcc cggcatccgc ttacagacaa gctgtgaccg tctccgggag ctgcatgtgt    8340
cagaggtttt caccgtcatc accgaaacgc gcga                                8374
```

<210> 39
<211> 569
<212> PRT
<213> Saccharomyces cerevisiae

<400> 39

```
Met Ser Phe Gln Ile Glu Thr Val Pro Thr Lys Pro Tyr Glu Asp Gln
1               5                   10                  15

Lys Pro Gly Thr Ser Gly Leu Arg Lys Lys Thr Lys Val Phe Lys Asp
            20                  25                  30

Glu Pro Asn Tyr Thr Glu Asn Phe Ile Gln Ser Ile Met Glu Ala Ile
        35                  40                  45

Pro Glu Gly Ser Lys Gly Ala Thr Leu Val Val Gly Gly Asp Gly Arg
    50                  55                  60

Tyr Tyr Asn Asp Val Ile Leu His Lys Ile Ala Ala Ile Gly Ala Ala
```

```
65                  70                  75                  80

Asn Gly Ile Lys Lys Leu Val Ile Gly Gln His Gly Leu Leu Ser Thr
                85                  90                  95

Pro Ala Ala Ser His Ile Met Arg Thr Tyr Glu Glu Lys Cys Thr Gly
            100                 105                 110

Gly Ile Ile Leu Thr Ala Ser His Asn Pro Gly Gly Pro Glu Asn Asp
        115                 120                 125

Met Gly Ile Lys Tyr Asn Leu Ser Asn Gly Gly Pro Ala Pro Glu Ser
    130                 135                 140

Val Thr Asn Ala Ile Trp Glu Ile Ser Lys Lys Leu Thr Ser Tyr Lys
145                 150                 155                 160

Ile Ile Lys Asp Phe Pro Glu Leu Asp Leu Gly Thr Ile Gly Lys Asn
                165                 170                 175

Lys Lys Tyr Gly Pro Leu Leu Val Asp Ile Ile Asp Ile Thr Lys Asp
            180                 185                 190

Tyr Val Asn Phe Leu Lys Glu Ile Phe Asp Phe Asp Leu Ile Lys Lys
        195                 200                 205

Phe Ile Asp Asn Gln Arg Ser Thr Lys Asn Trp Lys Leu Leu Phe Asp
    210                 215                 220

Ser Met Asn Gly Val Thr Gly Pro Tyr Gly Lys Ala Ile Phe Val Asp
225                 230                 235                 240

Glu Phe Gly Leu Pro Ala Asp Glu Val Leu Gln Asn Trp His Pro Ser
                245                 250                 255

Pro Asp Phe Gly Gly Met His Pro Asp Pro Asn Leu Thr Tyr Ala Ser
            260                 265                 270

Ser Leu Val Lys Arg Val Asp Arg Glu Lys Ile Glu Phe Gly Ala Ala
        275                 280                 285

Ser Asp Gly Asp Gly Asp Arg Asn Met Ile Tyr Gly Tyr Gly Pro Ser
    290                 295                 300

Phe Val Ser Pro Gly Asp Ser Val Ala Ile Ile Ala Glu Tyr Ala Ala
305                 310                 315                 320

Glu Ile Pro Tyr Phe Ala Lys Gln Gly Ile Tyr Gly Leu Ala Arg Ser
```

325 330 335

Phe Pro Thr Ser Gly Ala Ile Asp Arg Val Ala Lys Ala His Gly Leu
340 345 350

Asn Cys Tyr Glu Val Pro Thr Gly Trp Lys Phe Phe Cys Ala Leu Phe
355 360 365

Asp Ala Lys Lys Leu Ser Ile Cys Gly Glu Glu Ser Phe Gly Thr Gly
370 375 380

Ser Asn His Val Arg Glu Lys Asp Gly Val Trp Ala Ile Met Ala Trp
385 390 395 400

Leu Asn Ile Leu Ala Ile Tyr Asn Lys His His Pro Glu Asn Glu Ala
405 410 415

Ser Ile Lys Thr Ile Gln Asn Glu Phe Trp Ala Lys Tyr Gly Arg Thr
420 425 430

Phe Phe Thr Arg Tyr Asp Phe Glu Lys Val Glu Thr Glu Lys Ala Asn
435 440 445

Lys Ile Val Asp Gln Leu Arg Ala Tyr Val Thr Lys Ser Gly Val Val
450 455 460

Asn Ser Ala Phe Pro Ala Asp Glu Ser Leu Lys Val Thr Asp Cys Gly
465 470 475 480

Asp Phe Ser Tyr Thr Asp Leu Asp Gly Ser Val Ser Asp His Gln Gly
485 490 495

Leu Tyr Val Lys Leu Ser Asn Gly Ala Arg Phe Val Leu Arg Leu Ser
500 505 510

Gly Thr Gly Ser Ser Gly Ala Thr Ile Arg Leu Tyr Ile Glu Lys Tyr
515 520 525

Cys Asp Asp Lys Ser Gln Tyr Gln Lys Thr Ala Glu Glu Tyr Leu Lys
530 535 540

Pro Ile Ile Asn Ser Val Ile Lys Phe Leu Asn Phe Lys Gln Val Leu
545 550 555 560

Gly Thr Glu Glu Pro Thr Val Arg Thr
565

<210> 40

<211> 2629
<212> DNA
<213> Artificial

<220>
<223> Expression cassette for UGP1

<400> 40
actagtctcg agctcttcaa ctcaagacgc acagatatta taacatctgc ataataggca 60

tttgcaagaa ttactcgtga gtaaggaaag agtgaggaac tatcgcatac ctgcatttaa 120

agatgccgat ttgggcgcga atcctttatt ttggcttcac cctcatacta ttatcagggc 180

cagaaaaagg aagtgtttcc ctccttcttg aattgatgtt accctcataa agcacgtggc 240

ctcttatcga gaaagaaatt accgtcgctc gtgatttgtt tgcaaaaaga acaaaactga 300

aaaaacccag acacgctcga cttcctgtct tcctattgat tgcagcttcc aatttcgtca 360

cacaacaagg tcctagcgac ggctcacagg ttttgtaaca agcaatcgaa ggttctggaa 420

tggcgggaaa gggtttagta ccacatgcta tgatgcccac tgtgatctcc agagcaaagt 480

tcgttcgatc gtactgttac tctctctctt tcaaacagaa ttgtccgaat cgtgtgacaa 540

caacagcctg ttctcacaca ctcttttctt ctaaccaagg gggtggttta gtttagtaga 600

acctcgtgaa acttacattt acatatatat aaacttgcat aaattggtca atgcaagaaa 660

tacatatttg gtcttttcta attcgtagtt tttcaagttc ttagatgctt tctttttctc 720

ttttttacag atcatcaagg aagtaattat ctacttttta caacaaatat atctagactg 780

cagaaaatgt ccactaagaa gcacaccaaa acacattcca cttatgcatt cgagagcaac 840

acaaacagcg ttgctgcctc acaaatgaga aacgccttaa acaagttggc ggactctagt 900

aaacttgacg atgctgctcg cgctaagttt gagaacgaac tggattcgtt tttcacgctt 960

ttcaggagat atttggtaga gaagtcttct agaaccacct tggaatggga caagatcaag 1020

tctcccaacc cggatgaagt ggttaagtat gaaattattt ctcagcagcc cgagaatgtc 1080

tcaaaccttt ccaaattggc tgttttgaag ttgaacggtg ggctgggtac ctccatgggc 1140

tgcgttggcc ctaaatctgt tattgaagtg agagagggaa acaccttttt ggatttgtct 1200

gttcgtcaaa ttgaatactt gaacagacag tacgatagcg acgtgccatt gttattgatg 1260

aattctttca acactgacaa ggatacggaa cacttgatta agaagtattc cgctaacaga 1320

atcagaatca gatctttcaa tcaatccagg ttcccaagag tctacaagga ttctttattg 1380

cctgtcccca ccgaatacga ttctccactg gatgcttggt atccaccagg tcacggtgat 1440

ttgtttgaat ctttacacgt atctggtgaa ctggatgcct taattgccca aggaagagaa 1500

atattatttg tttctaacgg tgacaacttg ggtgctaccg tcgacttaaa aattttaaac 1560

cacatgatcg agactggtgc cgaatatata atggaattga ctgataagac cagagccgat 1620

gttaaaggtg gtactttgat ttcttacgat ggtcaagtcc gtttattgga agtcgcccaa 1680

gttccaaaag aacacattga cgaattcaaa aatatcagaa agtttaccaa cttcaacacg 1740

aataacttat ggatcaatct gaaagcagta aagaggttga tcgaatcgag caatttggag 1800

atggaaatca ttccaaacca aaaaactata acaagagacg gtcatgaaat taatgtctta 1860

caattagaaa ccgcttgtgg tgctgctatc aggcattttg atggtgctca cggtgttgtc 1920

gttccaagat caagattctt gcctgtcaag acctgttccg atttgttgct ggttaaatca 1980

gatctattcc gtctggaaca cggttctttg aagttagacc catcccgttt tggtccaaac 2040

ccattaatca agttgggctc gcatttcaaa aaggtttctg gttttaacgc aagaatccct 2100

cacatcccaa aaatcgtcga gctagatcat ttgaccatca ctggtaacgt ctttttaggt 2160

aaagatgtca ctttgagggg tactgtcatc atcgtttgct ccgacggtca taaaatcgat 2220

attccaaacg gctccatatt ggaaaatgtt gtcgttactg gtaatttgca aatcttggaa 2280

cattgagcgc gcgaatttct tatgatttat gatttttatt attaaataag ttataaaaaa 2340

aataagtgta tacaaatttt aaagtgactc ttaggtttta aaacgaaaat tcttattctt 2400

gagtaactct ttcctgtagg tcaggttgct ttctcaggta tagcatgagg tcgctcttat 2460

tgaccacacc tctaccggca tgccgagcaa atgcctgcaa atcgctcccc atttcaccca 2520

attgtagata tgctaactcc agcaatgagt tgatgaatct cggtgtgtat tttatgtcct 2580

cagaggacaa cacctgttgt aatcgttctt ccacacgcct aggctcgag 2629

<210> 41
<211> 7421
<212> DNA
<213> Artificial

<220>
<223> pRN832

<400> 41
tctcagaatg acttggttga gtactcacca gtcacagaaa agcatcttac ggatggcatg 60

acagtaagag aattatgcag tgctgccata accatgagtg ataacactgc ggccaactta 120

cttctgacaa cgatcggagg accgaaggag ctaaccgctt ttttttcacaa catgggggat 180

catgtaactc gccttgatcg ttgggaaccg gagctgaatg aagccatacc aaacgacgag 240

cgtgacacca cgatgcctgt agcaatggca acaacgttgc gcaaactatt aactggcgaa 300

ctacttactc tagcttcccg gcaacaatta atagactgga tggaggcgga taaagttgca 360

ggaccacttc tgcgctcggc ccttccggct ggctggttta ttgctgataa atctggagcc 420

ggtgagcgtg ggtctcgcgg tatcattgca gcactggggc cagatggtaa gccctcccgt 480

atcgtagtta tctacacgac gggcagtcag gcaactatgg atgaacgaaa tagacagatc 540

gctgagatag gtgcctcact gattaagcat tggtaactgt cagaccaagt ttactcatat 600

atactttaga ttgatttaaa acttcatttt taatttaaaa ggatctaggt gaagatcctt 660

tttgataatc tcatgaccaa aatcccttaa cgtgagtttt cgttccactg agcgtcagac 720

```
cccgtagaaa agatcaaagg atcttcttga gatccttttt ttctgcgcgt aatctgctgc     780
ttgcaaacaa aaaaaccacc gctaccagcg gtggtttgtt tgccggatca agagctacca     840
actctttttc cgaaggtaac tggcttcagc agagcgcaga taccaaatac tgtccttcta     900
gtgtagccgt agttaggcca ccacttcaag aactctgtag caccgcctac atacctcgct     960
ctgctaatcc tgttaccagt ggctgctgcc agtggcgata agtcgtgtct taccgggttg    1020
gactcaagac gatagttacc ggataaggcg cagcggtcgg gctgaacggg gggttcgtgc    1080
acacagccca gcttggagcg aacgacctac accgaactga gatacctaca gcgtgagcat    1140
tgagaaagcg ccacgcttcc cgaagggaga aaggcggaca ggtatccggt aagcggcagg    1200
gtcggaacag gagagcgcac gagggagctt ccagggggga acgcctggta tctttatagt    1260
cctgtcgggt ttcgccacct ctgacttgag cgtcgatttt tgtgatgctc gtcagggggg    1320
ccgagcctat ggaaaaacgc cagcaacgcg gcctttttac ggttcctggc cttttgctgg    1380
ccttttgctc acatgttctt tcctgcgtta tcccctgatt ctgtggataa ccgtattacc    1440
gcctttgagt gagctgatac cgctcgccgc agccgaacga ccgagcgcag cgagtcagtg    1500
agcgaggaag cggaagagcg cccaatacgc aaaccgcctc tccccgcgcg ttggccgatt    1560
cattaatgca gctggcacga caggtttccc gactggaaag cgggcagtga gcgcaacgca    1620
attaatgtga gttacctcac tcattaggca ccccaggctt tacactttat gcttccggct    1680
cctatgttgt gtggaattgt gagcggataa caatttcaca caggaaacag ctatgaccat    1740
gattacgcca agctcggaat taaccctcac taaagggaac aaaagctggg tacctaggac    1800
cggtttatca ttatcaatac tgccatttca aagaatacgt aaataattaa tagtagtgat    1860
tttcctaact ttatttagtc aaaaaattag ccttttaatt ctgctgtaac ccgtacatgc    1920
ccaaaatagg gggcgggtta cacagaatat ataacatcgt aggtgtctgg gtgaacagtt    1980
tattcctggc atccactaaa tataatggag cccgcttttt aagctggcat ccagaaaaaa    2040
aaagaatccc agcaccaaaa tattgttttc ttcaccaacc atcagttcat aggtccattc    2100
tcttagcgca actacagaga acaggggcac aaacaggcaa aaaacgggca caacctcaat    2160
ggagtgatgc aacctgcctg gagtaaatga tgacacaagg caattgaccc acgcatgtat    2220
ctatctcatt ttcttacacc ttctattacc ttctgctctc tctgatttgg aaaaagctga    2280
aaaaaaaggt tgaaaccagt tccctgaaat tattccccta cttgactaat aagtatataa    2340
agacggtagg tattgattgt aattctgtaa atctatttct taaacttctt aaattctact    2400
tttatagtta gtcttttttt tagttttaaa acaccaagaa cttagtttcg aataaacaca    2460
cataaagaat tcaaaatgtc ttggccaacc gactctgaat tgaactctat caaggaagct    2520
gttgctcaaa tgtctggtag agacaagggt gaagttagag ttgttgttgc tccatacaga    2580
atctgtccat tgggtgctca catcgaccac caaggtggta ctgtttctgc tatgaccatc    2640
```

aacaagggta tcttgttggg tttcgttcca tctggtgaca cccaagttca attgagatct 2700

gctcaattcg aaggtgaagt ttgtttcaga gttgacgaaa tccaacaccc aatcggtttg 2760

gctaacaaga acggtgcttc taccccatct ccatctaagg aaaagtctat ctggggtact 2820

tacgctagag gtgctgttta cgctttgcaa tcttctaaga agaacttgaa gcaaggtatc 2880

atcggttact tgtctggttc taacggtttg gactcttcag gtttatcttc ttctgctgct 2940

gttggtgttg cttacttgtt agctttggaa aacgctaacg aattgaccgt ttctccaacc 3000

gaaaacatcg aatacgacag attgatcgaa aacggttact tgggtttgag aaacggtatc 3060

ttggaccaat ctgctatctt gttgtctaac tacggttgtt tgacctacat ggactgtaag 3120

accttggacc acgaattggt tcaagctcca gaattggaaa agccattcag aatcttgttg 3180

gctttctctg gtttgagaca agctttgacc accaacccag gtttcaactt gagagtttct 3240

gaatgtcaag aagctgctaa ggtttttgttg accgcttcag gtaactctga attggaacca 3300

accttgtgta acgttgaaca cgctgtttac gaagctcaca agcacgaatt gaagcctgtt 3360

ttggctaaga gagctgaaca ctacttctct gaaaacatga gagttatcaa gggtagagaa 3420

gcttgggctt ctggtaactt ggaagaattt ggtaagttga tctctgcttc tggtttgtct 3480

tctatcgaaa actacgaatg tggtgctgaa ccattgatcc aattgtacaa gattttgttg 3540

aaggctccag gtgtttacgg tgctagattc tctggtgctg gtttcagagg ttgttgtttg 3600

gctttcgttg acgctgaaaa ggctgaagct gctgcttctt acgttaagga cgaatacgaa 3660

aaggctcaac cagaatttgc taacaacttg aacggtggta agccagtttt gatctgtgaa 3720

gctggtgacg ctgctagagt tttgttgtaa ggatcccctt ttcctttgtc gatatcatgt 3780

aattagttat gtcacgctta cattcacgcc ctcctcccac atccgctcta accgaaaagg 3840

aaggagttag acaacctgaa gtctaggtcc ctatttattt tttttaatag ttatgttagt 3900

attaagaacg ttatttatat ttcaaatttt tctttttttt ctgtacaaac gcgtgtacgc 3960

atgtaacatt atactgaaaa ccttgcttga gaaggttttg ggacgctcga aggctttcta 4020

ggctcgaggt cgacggtatc gataagcttg atatcgaatt cctgcagccc gggggatcca 4080

ctagttctag agcggccgcc accgcggtgg agctcgtacg ttcgaactta aggcctcgtc 4140

cccgccgggt cacccggcca gcgacatgga ggcccagaat accctccttg acagtcttga 4200

cgtgcgcagc tcaggggcat gatgtgactg tcgcccgtac atttagccca tacatcccca 4260

tgtataatca tttgcatcca tacattttga tggccgcacg gcgcgaagca aaaattacgg 4320

ctcctcgctg cagacctgcg agcagggaaa cgctcccctc acagacgcgt gaattgtccc 4380

cacgccgcgc ccctgtagag aaatataaaa ggttaggatt tgccactgag gttcttcttt 4440

catatacttc cttttaaaat cttgctagga tacagttctc acatcacatc cgaacataaa 4500

caaccatggg taaaaagcct gaactcaccg cgacgtctgt cgagaagttt ctgatcgaaa 4560

agttcgacag cgtctccgac ctgatgcagc tctcggaggg cgaagaatct cgtgctttca 4620

gcttcgatgt aggagggcgt ggatatgtcc tgcgggtaaa tagctgcgcc gatggtttct 4680

acaaagatcg ttatgtttat cggcactttg catcggccgc gctcccgatt ccggaagtgc 4740

ttgacattgg ggaattcagc gagagcctga cctattgcat ctcccgccgt gcacagggtg 4800

tcacgttgca agacctgcct gaaaccgaac tgcccgctgt tctgcagccg gtcgcggagg 4860

ccatggatgc gatcgctgcg gccgatctta gccagacgag cgggttcggc ccattcggac 4920

cgcaaggaat cggtcaatac actacatggc gtgatttcat atgcgcgatt gctgatcccc 4980

atgtgtatca ctggcaaact gtgatggacg acaccgtcag tgcgtccgtc gcgcaggctc 5040

tcgatgagct gatgctttgg gccgaggact gccccgaagt ccggcacctc gtgcacgcgg 5100

atttcggctc caacaatgtc ctgacggaca atggccgcat aacagcggtc attgactgga 5160

gcgaggcgat gttcggggat tcccaatacg aggtcgccaa catcttcttc tggaggccgt 5220

ggttggcttg tatggagcag cagacgcgct acttcgagcg gaggcatccg gagcttgcag 5280

gatcgccgcg gctccgggcg tatatgctcc gcattggtct tgaccaactc tatcagagct 5340

tggttgacgg caatttcgat gatgcagctt gggcgcaggg tcgatgcgac gcaatcgtcc 5400

gatccggagc cgggactgtc gggcgtacac aaatcgcccg cagaagcgcg gccgtctgga 5460

ccgatggctg tgtagaagta ctcgccgata gtggaaaccg acgccccagc actcgtccga 5520

gggcaaagga ataatcagta ctgacaataa aaagattctt gttttcaaga acttgtcatt 5580

tgtatagttt ttttatattg tagttgttct attttaatca aatgttagcg tgatttatat 5640

ttttttttcgc ctcgacatca tctgcccaga tgcgaagtta agtgcgcaga aagtaatatc 5700

atgcgtcaat cgtatgtgaa tgctggtcgc tatactgctg tcgattcgat actaacgccg 5760

ccatccagtg tcgacggatc ctaggtgtac ataaacttta taaatgaaat tcataataga 5820

aacgacacga aattacaaaa tggaatatgt tcatagggta gacgaaacta tatacgcaat 5880

ctacatacat ttatcaagaa ggagaaaaag gaggatagta aaggaataca ggtaagcaaa 5940

ttgatactaa tggctcaacg tgataaggaa aaagaattgc actttaacat taatattgac 6000

aaggaggagg gcaccacaca aaaagttagg tgtaacagaa aatcatgaaa ctacgattcc 6060

taatttgata ttggaggatt ttctctaaaa aaaaaaaaat acaacaaata aaaaacactc 6120

aatgacctga ccatttgatg gagtttaagt caataccttc ttgaaccatt tcccataatg 6180

gtgaaagttc cctcaagaat tttactctgt cagaaacggc cttacgacgt agtcgatatg 6240

gtgcactctc agtacaatct gctctgatgc cgcatagtta agccagcccc gacacccgcc 6300

aacacccgct gacgcgccct gacgggcttg tctgctcccg gcatccgctt acagacaagc 6360

tgtgaccgtc tccgggagct gcatgtgtca gaggttttca ccgtcatcac cgaaacgcgc 6420

gagacgaaag ggcctcgtga tacgcctatt tttataggtt aatgtcatga taataatggt 6480

ttcttaggac ggatcgcttg cctgtaactt acacgcgcct cgtatctttt aatgatggaa 6540

taatttggga atttactctg tgtttattta tttttatgtt ttgtatttgg attttagaaa 6600

gtaaataaag aaggtagaag agttacggaa tgaagaaaaa aaaataaaca aaggtttaaa 6660

aaatttcaac aaaaagcgta ctttacatat atatttatta gacaagaaaa gcagattaaa 6720

tagatataca ttcgattaac gataagtaaa atgtaaaatc acaggatttt cgtgtgtggt 6780

cttctacaca gacaagatga aacaattcgg cattaatacc tgagagcagg aagagcaaga 6840

taaaaggtag tatttgttgg cgatccccct agagtctttt acatcttcgg aaaacaaaaa 6900

ctatttttc tttaatttct ttttttactt tctattttta atttatatat ttatattaaa 6960

aaatttaaat tataattatt tttatagcac gtgatgaaaa ggacccaggt ggcacttttc 7020

ggggaaatgt gcgcggaacc cctatttgtt tatttttcta aatacattca aatatgtatc 7080

cgctcatgag acaataaccc tgataaatgc ttcaataata ttgaaaaagg aagagtatga 7140

gtattcaaca tttccgtgtc gcccttattc cctttttgc ggcattttgc cttcctgttt 7200

ttgctcaccc agaaacgctg gtgaaagtaa aagatgctga agatcagttg ggtgcacgag 7260

tgggttacat cgaactggat ctcaacagcg gtaagatcct tgagagtttt cgccccgaag 7320

aacgttttcc aatgatgagc acttttaaag ttctgctatg tggcgcggta ttatcccgta 7380

ttgacgccgg gcaagagcaa ctcggtcgcc gcatacacta t 7421

<210> 42
<211> 7235
<212> DNA
<213> Artificial

<220>
<223> pRN833

<400> 42
tctcagaatg acttggttga gtactcacca gtcacagaaa agcatcttac ggatggcatg 60

acagtaagag aattatgcag tgctgccata accatgagtg ataacactgc ggccaactta 120

cttctgacaa cgatcggagg accgaaggag ctaaccgctt ttttcacaa catgggggat 180

catgtaactc gccttgatcg ttgggaaccg gagctgaatg aagccatacc aaacgacgag 240

cgtgacacca cgatgcctgt agcaatggca acaacgttgc gcaaactatt aactggcgaa 300

ctacttactc tagcttcccg gcaacaatta atagactgga tggaggcgga taaagttgca 360

ggaccacttc tgcgctcggc ccttccggct ggctggttta ttgctgataa atctggagcc 420

ggtgagcgtg ggtctcgcgg tatcattgca gcactggggc cagatggtaa gccctcccgt 480

atcgtagtta tctacacgac ggggcagtcag gcaactatgg atgaacgaaa tagacagatc 540

gctgagatag gtgcctcact gattaagcat tggtaactgt cagaccaagt ttactcatat 600

atactttaga ttgatttaaa acttcatttt taatttaaaa ggatctaggt gaagatcctt 660

```
tttgataatc tcatgaccaa aatcccttaa cgtgagtttt cgttccactg agcgtcagac     720
cccgtagaaa agatcaaagg atcttcttga gatccttttt ttctgcgcgt aatctgctgc     780
ttgcaaacaa aaaaaccacc gctaccagcg gtggtttgtt tgccggatca agagctacca     840
actctttttc cgaaggtaac tggcttcagc agagcgcaga taccaaatac tgtccttcta     900
gtgtagccgt agttaggcca ccacttcaag aactctgtag caccgcctac atacctcgct     960
ctgctaatcc tgttaccagt ggctgctgcc agtggcgata agtcgtgtct taccgggttg    1020
gactcaagac gatagttacc ggataaggcg cagcggtcgg gctgaacggg gggttcgtgc    1080
acacagccca gcttggagcg aacgacctac accgaactga gatacctaca gcgtgagcat    1140
tgagaaagcg ccacgcttcc cgaagggaga aaggcggaca ggtatccggt aagcggcagg    1200
gtcggaacag gagagcgcac gagggagctt ccagggggaa acgcctggta tctttatagt    1260
cctgtcgggt ttcgccacct ctgacttgag cgtcgatttt tgtgatgctc gtcagggggg    1320
ccgagcctat ggaaaaacgc cagcaacgcg gcctttttac ggttcctggc cttttgctgg    1380
ccttttgctc acatgttctt tcctgcgtta tcccctgatt ctgtggataa ccgtattacc    1440
gcctttgagt gagctgatac cgctcgccgc agccgaacga ccgagcgcag cgagtcagtg    1500
agcgaggaag cggaagagcg cccaatacgc aaaccgcctc tccccgcgcg ttggccgatt    1560
cattaatgca gctggcacga caggtttccc gactggaaag cgggcagtga gcgcaacgca    1620
attaatgtga gttacctcac tcattaggca ccccaggctt tacactttat gcttccggct    1680
cctatgttgt gtggaattgt gagcggataa caatttcaca caggaaacag ctatgaccat    1740
gattacgcca agctcggaat taaccctcac taaagggaac aaaagctggg tacctaggac    1800
cggtttatca ttatcaatac tgccatttca aagaatacgt aaataattaa tagtagtgat    1860
tttcctaact ttatttagtc aaaaaattag ccttttaatt ctgctgtaac ccgtacatgc    1920
ccaaaatagg gggcgggtta cacagaatat ataacatcgt aggtgtctgg gtgaacagtt    1980
tattcctggc atccactaaa tataatggag cccgcttttt aagctggcat ccagaaaaaa    2040
aaagaatccc agcaccaaaa tattgttttc ttcaccaacc atcagttcat aggtccattc    2100
tcttagcgca actacagaga acaggggcac aaacaggcaa aaaacgggca caacctcaat    2160
ggagtgatgc aacctgcctg gagtaaatga tgacacaagg caattgaccc acgcatgtat    2220
ctatctcatt ttcttacacc ttctattacc ttctgctctc tctgatttgg aaaaagctga    2280
aaaaaaaggt tgaaaccagt tccctgaaat tattccccta cttgactaat aagtatataa    2340
agacggtagg tattgattgt aattctgtaa atctatttct taaacttctt aaattctact    2400
tttatagtta gtcttttttt tagttttaaa acaccaagaa cttagtttcg aataaacaca    2460
cataaagaat tcaaaatgga cccaaactct accgtttctg gtgacggtca agctaccgct    2520
gctatcgaac acagatcttt cgctagaatc ggtttcttgg gtaacccttc tgacgtttac    2580
```

ttcggtagaa ccatctcttt gaccatcggt aacttctggg cttctgttaa gttggaacca 2640
tctgaacact tggttatcaa gccacaccca ttccacgact tggttcaatt cacctctttg 2700
gaccacttgt tgaacagatt gcaaaacgaa ggttactacg gtggtgttag attgttgatg 2760
gctatctgta aggttttcag aaactactgt aaggaaaacg acatccaatt gcaccaagct 2820
aacttctctt tgtcttacga caccaacatc ccaagacaaa ctggtttgtc tggttcttct 2880
gctatcgttt ctgctgcttt gaactgtttg ttggacttct acaacgttag acacttgatc 2940
aaggttcaag ttagaccaaa catcgttttg tctgctgaaa aggaattggg tatcgttgct 3000
ggtttgcaag acagagttgc tcaagtttac ggtggtttgg ttcacatgga cttctctaag 3060
gaacacatgg acaagttggg tcacggtatc tacaccccaa tggacatctc tttgttgcca 3120
ccattgcact tgatctacgc tgaaaaccca tctgactctg gtaaggttca ctctatggtt 3180
agacaaagat ggttggacgg tgacgaattt atcatctctt ctatgaagga agttggttct 3240
ttggctgaag aaggtagaac cgctttgttg aacaaggacc actctaagtt ggttgaattg 3300
atgaacttga acttcgacat cagaaggaga atgttcggtg acgaatgttt gggtgctatg 3360
aacatcgaaa tggttgaagt tgctagaaga gttggtgctg cttctaagtt cactggttct 3420
ggtggtgctg ttgttgtttt ctgtccagaa ggtccatctc aagttaagtt gttggaagaa 3480
gaatgtagaa aggctggttt caccttgcaa ccagttaaga tcgctccatc ttgtttgaac 3540
gactctgaca tccaaacctt gtaaggatcc ccttttcctt tgtcgatatc atgtaattag 3600
ttatgtcacg cttacattca cgccctcctc ccacatccgc tctaaccgaa aaggaaggag 3660
ttagacaacc tgaagtctag gtccctattt atttttttta atagttatgt tagtattaag 3720
aacgttattt atatttcaaa tttttctttt ttttctgtac aaacgcgtgt acgcatgtaa 3780
cattatactg aaaaccttgc ttgagaaggt tttgggacgc tcgaaggctt cctaggctcg 3840
aggtcgacgg tatcgataag cttgatatcg aattcctgca gcccggggga tccactagtt 3900
ctagagcggc cgccaccgcg gtggagctcg tacgttcgaa cttaaggcct cgtccccgcc 3960
gggtcacccg gccagcgaca tggaggccca gaataccctc cttgacagtc ttgacgtgcg 4020
cagctcaggg gcatgatgtg actgtcgccc gtacatttag cccatacatc cccatgtata 4080
atcatttgca tccatacatt ttgatggccg cacggcgcga agcaaaaatt acggctcctc 4140
gctgcagacc tgcgagcagg gaaacgctcc cctcacagac gcgtgaattg tccccacgcc 4200
gcgcccctgt agagaaatat aaaaggttag gatttgccac tgaggttctt ctttcatata 4260
cttcctttta aaatcttgct aggatacagt tctcacatca catccgaaca taaacaacca 4320
tgggtaaaaa gcctgaactc accgcgacgt ctgtcgagaa gtttctgatc gaaaagttcg 4380
acagcgtctc cgacctgatg cagctctcgg agggcgaaga atctcgtgct ttcagcttcg 4440
atgtaggagg gcgtggatat gtcctgcggg taaatagctg cgccgatggt ttctacaaag 4500

atcgttatgt ttatcggcac tttgcatcgg ccgcgctccc gattccggaa gtgcttgaca 4560
ttggggaatt cagcgagagc ctgacctatt gcatctcccg ccgtgcacag ggtgtcacgt 4620
tgcaagacct gcctgaaacc gaactgcccg ctgttctgca gccggtcgcg gaggccatgg 4680
atgcgatcgc tgcggccgat cttagccaga cgagcgggtt cggcccattc ggaccgcaag 4740
gaatcggtca atacactaca tggcgtgatt tcatatgcgc gattgctgat ccccatgtgt 4800
atcactggca aactgtgatg gacgacaccg tcagtgcgtc cgtcgcgcag gctctcgatg 4860
agctgatgct ttgggccgag gactgccccg aagtccggca cctcgtgcac gcggatttcg 4920
gctccaacaa tgtcctgacg gacaatggcc gcataacagc ggtcattgac tggagcgagg 4980
cgatgttcgg ggattcccaa tacgaggtcg ccaacatctt cttctggagg ccgtggttgg 5040
cttgtatgga gcagcagacg cgctacttcg agcggaggca tccggagctt gcaggatcgc 5100
cgcggctccg ggcgtatatg ctccgcattg gtcttgacca actctatcag agcttggttg 5160
acggcaattt cgatgatgca gcttgggcgc agggtcgatg cgacgcaatc gtccgatccg 5220
gagccgggac tgtcgggcgt acacaaatcg cccgcagaag cgcggccgtc tggaccgatg 5280
gctgtgtaga agtactcgcc gatagtggaa accgacgccc cagcactcgt ccgagggcaa 5340
aggaataatc agtactgaca ataaaaagat tcttgttttc aagaacttgt catttgtata 5400
gtttttttat attgtagttg ttctatttta atcaaatgtt agcgtgattt atattttttt 5460
tcgcctcgac atcatctgcc cagatgcgaa gttaagtgcg cagaaagtaa tatcatgcgt 5520
caatcgtatg tgaatgctgg tcgctatact gctgtcgatt cgatactaac gccgccatcc 5580
agtgtcgacg gatcctaggt gtacataaac tttataaatg aaattcataa tagaaacgac 5640
acgaaattac aaaatggaat atgttcatag ggtagacgaa actatatacg caatctacat 5700
acatttatca agaaggagaa aaaggaggat agtaaaggaa tacaggtaag caaattgata 5760
ctaatggctc aacgtgataa ggaaaaagaa ttgcacttta acattaatat tgacaaggag 5820
gagggcacca cacaaaagtt taggtgtaac agaaaatcat gaaactacga ttcctaattt 5880
gatattggag gattttctct aaaaaaaaaa aaatacaaca aataaaaaac actcaatgac 5940
ctgaccattt gatggagttt aagtcaatac cttcttgaac catttcccat aatggtgaaa 6000
gttccctcaa gaattttact ctgtcagaaa cggccttacg acgtagtcga tatggtgcac 6060
tctcagtaca atctgctctg atgccgcata gttaagccag ccccgacacc cgccaacacc 6120
cgctgacgcg ccctgacggg cttgtctgct cccggcatcc gcttacagac aagctgtgac 6180
cgtctccggg agctgcatgt gtcagaggtt ttcaccgtca tcaccgaaac gcgcgagacg 6240
aaagggcctc gtgatacgcc tatttttata ggttaatgtc atgataataa tggtttctta 6300
ggacggatcg cttgcctgta acttacacgc gcctcgtatc ttttaatgat ggaataattt 6360
gggaatttac tctgtgttta tttattttta tgttttgtat ttggatttta gaaagtaaat 6420

aaagaaggta gaagagttac ggaatgaaga aaaaaaaata aacaaaggtt taaaaaattt 6480

caacaaaaag cgtactttac atatatattt attagacaag aaaagcagat taaatagata 6540

tacattcgat taacgataag taaaatgtaa aatcacagga ttttcgtgtg tggtcttcta 6600

cacagacaag atgaaacaat tcggcattaa tacctgagag caggaagagc aagataaaag 6660

gtagtatttg ttggcgatcc ccctagagtc ttttacatct tcggaaaaca aaaactattt 6720

tttctttaat ttcttttttt actttctatt tttaatttat atatttatat taaaaaattt 6780

aaattataat tatttttata gcacgtgatg aaaaggaccc aggtggcact tttcggggaa 6840

atgtgcgcgg aacccctatt tgtttatttt tctaaataca ttcaaatatg tatccgctca 6900

tgagacaata accctgataa atgcttcaat aatattgaaa aaggaagagt atgagtattc 6960

aacatttccg tgtcgccctt attccctttt ttgcggcatt ttgccttcct gtttttgctc 7020

acccagaaac gctggtgaaa gtaaaagatg ctgaagatca gttgggtgca cgagtgggtt 7080

acatcgaact ggatctcaac agcggtaaga tccttgagag ttttcgcccc gaagaacgtt 7140

ttccaatgat gagcactttt aaagttctgc tatgtggcgc ggtattatcc cgtattgacg 7200

ccgggcaaga gcaactcggt cgccgcatac actat 7235

<210> 43
<211> 9762
<212> DNA
<213> Artificial

<220>
<223> pRN834

<400> 43
tctcagaatg acttggttga gtactcacca gtcacagaaa agcatcttac ggatggcatg 60

acagtaagag aattatgcag tgctgccata accatgagtg ataacactgc ggccaactta 120

cttctgacaa cgatcggagg accgaaggag ctaaccgctt ttttcacaa catgggggat 180

catgtaactc gccttgatcg ttgggaaccg gagctgaatg aagccatacc aaacgacgag 240

cgtgacacca cgatgcctgt agcaatggca acaacgttgc gcaaactatt aactggcgaa 300

ctacttactc tagcttcccg gcaacaatta atagactgga tggaggcgga taaagttgca 360

ggaccacttc tgcgctcggc ccttccggct ggctggttta ttgctgataa atctggagcc 420

ggtgagcgtg ggtctcgcgg tatcattgca gcactggggc cagatggtaa gccctcccgt 480

atcgtagtta tctacacgac gggcagtcag gcaactatgg atgaacgaaa tagacagatc 540

gctgagatag gtgcctcact gattaagcat tggtaactgt cagaccaagt ttactcatat 600

atactttaga ttgatttaaa acttcatttt taatttaaaa ggatctaggt gaagatcctt 660

tttgataatc tcatgaccaa aatcccttaa cgtgagtttt cgttccactg agcgtcagac 720

cccgtagaaa agatcaaagg atcttcttga gatccttttt ttctgcgcgt aatctgctgc 780

ttgcaaacaa aaaaaccacc gctaccagcg gtggtttgtt tgccggatca agagctacca 840

actctttttc cgaaggtaac tggcttcagc agagcgcaga taccaaatac tgtccttcta 900

gtgtagccgt agttaggcca ccacttcaag aactctgtag caccgcctac atacctcgct 960

ctgctaatcc tgttaccagt ggctgctgcc agtggcgata agtcgtgtct taccgggttg 1020

gactcaagac gatagttacc ggataaggcg cagcggtcgg gctgaacggg gggttcgtgc 1080

acacagccca gcttggagcg aacgacctac accgaactga gatacctaca gcgtgagcat 1140

tgagaaagcg ccacgcttcc cgaagggaga aaggcggaca ggtatccggt aagcggcagg 1200

gtcggaacag gagagcgcac gagggagctt ccagggggga acgcctggta tctttatagt 1260

cctgtcgggt ttcgccacct ctgacttgag cgtcgatttt tgtgatgctc gtcagggggg 1320

ccgagcctat ggaaaaacgc cagcaacgcg gcctttttac ggttcctggc cttttgctgg 1380

ccttttgctc acatgttctt tcctgcgtta tcccctgatt ctgtggataa ccgtattacc 1440

gcctttgagt gagctgatac cgctcgccgc agccgaacga ccgagcgcag cgagtcagtg 1500

agcgaggaag cggaagagcg cccaatacgc aaaccgcctc tccccgcgcg ttggccgatt 1560

cattaatgca gctggcacga caggtttccc gactggaaag cgggcagtga gcgcaacgca 1620

attaatgtga gttacctcac tcattaggca ccccaggctt tacactttat gcttccggct 1680

cctatgttgt gtggaattgt gagcggataa caatttcaca caggaaacag ctatgaccat 1740

gattacgcca agctcggaat taaccctcac taaagggaac aaaagctggg tacctaggac 1800

cggtttatca ttatcaatac tgccatttca aagaatacgt aaataattaa tagtagtgat 1860

tttcctaact ttatttagtc aaaaaattag ccttttaatt ctgctgtaac ccgtacatgc 1920

ccaaaatagg gggcgggtta cacagaatat ataacatcgt aggtgtctgg gtgaacagtt 1980

tattcctggc atccactaaa tataatggag cccgcttttt aagctggcat ccagaaaaaa 2040

aaagaatccc agcaccaaaa tattgttttc ttcaccaacc atcagttcat aggtccattc 2100

tcttagcgca actacagaga acaggggcac aaacaggcaa aaaacgggca caacctcaat 2160

ggagtgatgc aacctgcctg gagtaaatga tgacacaagg caattgaccc acgcatgtat 2220

ctatctcatt ttcttacacc ttctattacc ttctgctctc tctgatttgg aaaaagctga 2280

aaaaaaaggt tgaaaccagt tccctgaaat tattccccta cttgactaat aagtatataa 2340

agacggtagg tattgattgt aattctgtaa atctatttct taaacttctt aaattctact 2400

tttatagtta gtcttttttt tagttttaaa acaccaagaa cttagtttcg aataaacaca 2460

cataaagaat tcaaaatgga cccaaactct accgtttctg gtgacggtca agctaccgct 2520

gctatcgaac acagatcttt cgctagaatc ggtttcttgg gtaacccttc tgacgtttac 2580

ttcggtagaa ccatctcttt gaccatcggt aacttctggg cttctgttaa gttggaacca 2640

tctgaacact tggttatcaa gccacaccca ttccacgact tggttcaatt cacctctttg 2700

gaccacttgt tgaacagatt gcaaaacgaa ggttactacg gtggtgttag attgttgatg 2760
gctatctgta aggttttcag aaactactgt aaggaaaacg acatccaatt gcaccaagct 2820
aacttctctt tgtcttacga caccaacatc ccaagacaaa ctggtttgtc tggttcttct 2880
gctatcgttt ctgctgcttt gaactgtttg ttggacttct acaacgttag acacttgatc 2940
aaggttcaag ttagaccaaa catcgttttg tctgctgaaa aggaattggg tatcgttgct 3000
ggtttgcaag acagagttgc tcaagtttac ggtggtttgg ttcacatgga cttctctaag 3060
gaacacatgg acaagttggg tcacggtatc tacaccccaa tggacatctc tttgttgcca 3120
ccattgcact tgatctacgc tgaaaaccca tctgactctg gtaaggttca ctctatggtt 3180
agacaaagat ggttggacgg tgacgaattt atcatctctt ctatgaagga agttggttct 3240
ttggctgaag aaggtagaac cgctttgttg aacaaggacc actctaagtt ggttgaattg 3300
atgaacttga acttcgacat cagaaggaga atgttcggtg acgaatgttt gggtgctatg 3360
aacatcgaaa tggttgaagt tgctagaaga gttggtgctg cttctaagtt cactggttct 3420
ggtggtgctg ttgttgtttt ctgtccagaa ggtccatctc aagttaagtt gttggaagaa 3480
gaatgtagaa aggctggttt caccttgcaa ccagttaaga tcgctccatc ttgtttgaac 3540
gactctgaca tccaaacctt gtaaggatcc ccttttcctt tgtcgatatc atgtaattag 3600
ttatgtcacg cttacattca cgccctcctc ccacatccgc tctaaccgaa aaggaaggag 3660
ttagacaacc tgaagtctag gtccctattt atttttttta atagttatgt tagtattaag 3720
aacgttattt atatttcaaa tttttctttt ttttctgtac aaacgcgtgt acgcatgtaa 3780
cattatactg aaaaccttgc ttgagaaggt tttgggacgc tcgaaggctt cctaggctcg 3840
agcctaggcg tgtggaagaa cgattacaac aggtgttgtc ctctgaggac ataaaataca 3900
caccgagatt catcaactca ttgctggagt tagcatatct acaattgggt gaaatgggga 3960
gcgatttgca ggcatttgct cggcatgccg gtagaggtgt ggtcaataag agcgacctca 4020
tgctatacct gagaaagcaa cctgacctac aggaaagagt tactcaagaa taagaatttt 4080
cgttttaaaa cctaagagtc actttaaaat ttgtatacac ttattttttt tataacttat 4140
ttaataataa aaatcataaa tcataagaaa ttcgcgcgct caatgttcca agatttgcaa 4200
attaccagta acgacaacat tttccaatat ggagccgttt ggaatatcga ttttatgacc 4260
gtcggagcaa acgatgatga cagtacccct caaagtgaca tctttaccta aaaagacgtt 4320
accagtgatg gtcaaatgat ctagctcgac gatttttggg atgtgaggga ttcttgcgtt 4380
aaaaccagaa acctttttga aatgcgagcc caacttgatt aatgggtttg gaccaaaacg 4440
ggatgggtct aacttcaaag aaccgtgttc cagacggaat agatctgatt taaccagcaa 4500
caaatcggaa caggtcttga caggcaagaa tcttgatctt ggaacgacaa caccgtgagc 4560
accatcaaaa tgcctgatag cagcaccaca agcggtttct aattgtaaga cattaatttc 4620

atgaccgtct cttgttatag ttttttggtt tggaatgatt tccatctcca aattgctcga 4680

ttcgatcaac ctctttactg ctttcagatt gatccataag ttattcgtgt tgaagttggt 4740

aaactttctg atatttttga attcgtcaat gtgttctttt ggaacttggg cgacttccaa 4800

taaacggact tgaccatcgt aagaaatcaa agtaccacct ttaacatcgg ctctggtctt 4860

atcagtcaat tccattatat attcggcacc agtctcgatc atgtggttta aaatttttaa 4920

gtcgacggta gcacccaagt tgtcaccgtt agaaacaaat aatatttctc ttccttgggc 4980

aattaaggca tccagttcac cagatacgtg taaagattca aacaaatcac cgtgacctgg 5040

tggataccaa gcatccagtg gagaatcgta ttcggtgggg acaggcaata aagaatcctt 5100

gtagactctt gggaacctgg attgattgaa agatctgatt ctgattctgt tagcggaata 5160

cttcttaatc aagtgttccg tatccttgtc agtgttgaaa gaattcatca ataacaatgg 5220

cacgtcgcta tcgtactgtc tgttcaagta ttcaatttga cgaacagaca aatccaaaaa 5280

ggtgtttccc tctctcactt caataacaga tttagggcca acgcagccca tggaggtacc 5340

cagcccaccg ttcaacttca aaacagccaa tttggaaagg tttgagacat tctcgggctg 5400

ctgagaaata atttcatact taaccacttc atccgggttg ggagacttga tcttgtccca 5460

ttccaaggtg gttctagaag acttctctac caaatatctc ctgaaaagcg tgaaaaacga 5520

atccagttcg ttctcaaact tagcgcgagc agcatcgtca agtttactag agtccgccaa 5580

cttgtttaag gcgtttctca tttgtgaggc agcaacgctg tttgtgttgc tctcgaatgc 5640

ataagtggaa tgtgttttgg tgtgcttctt agtggacatt ttctgcagtc tagatatatt 5700

tgttgtaaaa agtagataat tacttccttg atgatctgta aaaaagagaa aaagaaagca 5760

tctaagaact tgaaaaacta cgaattagaa aagaccaaat atgtatttct tgcattgacc 5820

aatttatgca agtttatata tatgtaaatg taagtttcac gaggttctac taaactaaac 5880

cacccccttg gttagaagaa aagagtgtgt gagaacaggc tgttgttgtc acacgattcg 5940

gacaattctg tttgaaagag agagagtaac agtacgatcg aacgaacttt gctctggaga 6000

tcacagtggg catcatagca tgtggtacta aaccctttcc cgccattcca gaaccttcga 6060

ttgcttgtta caaaacctgt gagccgtcgc taggaccttg ttgtgtgacg aaattggaag 6120

ctgcaatcaa taggaagaca ggaagtcgag cgtgtctggg ttttttcagt tttgttcttt 6180

ttgcaaacaa atcacgagcg acggtaattt ctttctcgat aagaggccac gtgctttatg 6240

agggtaacat caattcaaga aggagggaaa cacttccttt ttctggccct gataatagta 6300

tgagggtgaa gccaaaataa aggattcgcg cccaaatcgg catctttaaa tgcaggtatg 6360

cgatagttcc tcactctttc cttactcacg agtaattctt gcaaatgcct attatgcaga 6420

tgttataata tctgtgcgtc ttgagttgaa gagctcgtac gttcgaactt aaggcctcgt 6480

ccccgccggg tcacccggcc agcgacatgg aggcccagaa taccctcctt gacagtcttg 6540

acgtgcgcag ctcaggggca tgatgtgact gtcgcccgta catttagccc atacatcccc 6600
atgtataatc atttgcatcc atacattttg atggccgcac ggcgcgaagc aaaaattacg 6660
gctcctcgct gcagacctgc gagcagggaa acgctcccct cacagacgcg tgaattgtcc 6720
ccacgccgcg cccctgtaga gaaatataaa aggttaggat ttgccactga ggttcttctt 6780
tcatatactt ccttttaaaa tcttgctagg atacagttct cacatcacat ccgaacataa 6840
acaaccatgg gtaaaaagcc tgaactcacc gcgacgtctg tcgagaagtt tctgatcgaa 6900
aagttcgaca gcgtctccga cctgatgcag ctctcggagg gcgaagaatc tcgtgctttc 6960
agcttcgatg taggagggcg tggatatgtc ctgcgggtaa atagctgcgc cgatggtttc 7020
tacaaagatc gttatgttta tcggcacttt gcatcggccg cgctcccgat tccggaagtg 7080
cttgacattg gggaattcag cgagagcctg acctattgca tctcccgccg tgcacagggt 7140
gtcacgttgc aagacctgcc tgaaaccgaa ctgcccgctg ttctgcagcc ggtcgcggag 7200
gccatggatg cgatcgctgc ggccgatctt agccagacga gcgggttcgg cccattcgga 7260
ccgcaaggaa tcggtcaata cactacatgg cgtgatttca tatgcgcgat tgctgatccc 7320
catgtgtatc actggcaaac tgtgatggac gacaccgtca gtgcgtccgt cgcgcaggct 7380
ctcgatgagc tgatgctttg ggccgaggac tgccccgaag tccggcacct cgtgcacgcg 7440
gatttcggct ccaacaatgt cctgacggac aatggccgca taacagcggt cattgactgg 7500
agcgaggcga tgttcgggga ttcccaatac gaggtcgcca acatcttctt ctggaggccg 7560
tggttggctt gtatggagca gcagacgcgc tacttcgagc ggaggcatcc ggagcttgca 7620
ggatcgccgc ggctccgggc gtatatgctc cgcattggtc ttgaccaact ctatcagagc 7680
ttggttgacg gcaatttcga tgatgcagct tgggcgcagg gtcgatgcga cgcaatcgtc 7740
cgatccggag ccgggactgt cgggcgtaca caaatcgccc gcagaagcgc ggccgtctgg 7800
accgatggct gtgtagaagt actcgccgat agtggaaacc gacgccccag cactcgtccg 7860
agggcaaagg aataatcagt actgacaata aaaagattct tgttttcaag aacttgtcat 7920
ttgtatagtt tttttatatt gtagttgttc tattttaatc aaatgttagc gtgatttata 7980
tttttttttcg cctcgacatc atctgcccag atgcgaagtt aagtgcgcag aaagtaatat 8040
catgcgtcaa tcgtatgtga atgctggtcg ctatactgct gtcgattcga tactaacgcc 8100
gccatccagt gtcgacggat cctaggtgta cataaacttt ataaatgaaa ttcataatag 8160
aaacgacacg aaattacaaa atggaatatg ttcatagggt agacgaaact atatacgcaa 8220
tctacataca tttatcaaga aggagaaaaa ggaggatagt aaaggaatac aggtaagcaa 8280
attgatacta atggctcaac gtgataagga aaaagaattg cactttaaca ttaatattga 8340
caaggaggag ggcaccacac aaaaagttag gtgtaacaga aaatcatgaa actacgattc 8400
ctaatttgat attggaggat tttctctaaa aaaaaaaaaa tacaacaaat aaaaaacact 8460

caatgacctg accatttgat ggagtttaag tcaatacctt cttgaaccat ttcccataat 8520

ggtgaaagtt ccctcaagaa ttttactctg tcagaaacgg ccttacgacg tagtcgatat 8580

ggtgcactct cagtacaatc tgctctgatg ccgcatagtt aagccagccc cgacacccgc 8640

caacacccgc tgacgcgccc tgacgggctt gtctgctccc ggcatccgct tacagacaag 8700

ctgtgaccgt ctccgggagc tgcatgtgtc agaggttttc accgtcatca ccgaaacgcg 8760

cgagacgaaa gggcctcgtg atacgcctat ttttataggt taatgtcatg ataataatgg 8820

tttcttagga cggatcgctt gcctgtaact tacacgcgcc tcgtatcttt taatgatgga 8880

ataatttggg aatttactct gtgtttattt atttttatgt tttgtatttg gattttagaa 8940

agtaaataaa gaaggtagaa gagttacgga atgaagaaaa aaaaataaac aaaggtttaa 9000

aaaatttcaa caaaaagcgt actttacata tatatttatt agacaagaaa agcagattaa 9060

atagatatac attcgattaa cgataagtaa aatgtaaaat cacaggattt tcgtgtgtgg 9120

tcttctacac agacaagatg aaacaattcg gcattaatac ctgagagcag gaagagcaag 9180

ataaaaggta gtatttgttg gcgatccccc tagagtcttt tacatcttcg gaaaacaaaa 9240

actatttttt ctttaatttc tttttttact ttctattttt aatttatata tttatattaa 9300

aaaatttaaa ttataattat ttttatagca cgtgatgaaa aggacccagg tggcactttt 9360

cggggaaatg tgcgcggaac ccctatttgt ttatttttct aaatacattc aaatatgtat 9420

ccgctcatga gacaataacc ctgataaatg cttcaataat attgaaaaag gaagagtatg 9480

agtattcaac atttccgtgt cgcccttatt cccttttttg cggcattttg ccttcctgtt 9540

tttgctcacc cagaaacgct ggtgaaagta aaagatgctg aagatcagtt gggtgcacga 9600

gtgggttaca tcgaactgga tctcaacagc ggtaagatcc ttgagagttt tcgccccgaa 9660

gaacgttttc caatgatgag cacttttaaa gttctgctat gtggcgcggt attatcccgt 9720

attgacgccg ggcaagagca actcggtcgc cgcatacact at 9762

<210> 44
<211> 4654
<212> DNA
<213> Artificial

<220>
<223> pRN835

<400> 44
tacattatac gaagttattc tagtaacggc cgccatggaa ttcaaccggt cccacacacc 60

atagcttcaa aatgtttcta ctcctttttt actcttccag atttttctcgg actccgcgca 120

tcgccgtacc acttcaaaac acccaagcac agcatactaa atttcccctc tttcttcctc 180

tagggtgtcg ttaattaccc gtactaaagg tttggaaaag aaaaaagaga ccgcctcgtt 240

tctttttctt cgtcgaaaaa ggcaataaaa atttttatca cgtttctttt tcttgaaaat 300

tttttttttt gatttttttc tctttcgatg acctcccatt gatatttaag ttaataaacg 360
gtcttcaatt tctcaagttt cagtttcatt tttcttgttc tattacaact ttttttactt 420
cttgctcatt agaaagaaag catagcaatc taatctaagt ctagaaaaat gactgctgaa 480
gaatttgatt tttctagcca ttcccataga cgttacaatc cactaaccga ttcatggatc 540
ttagtttctc cacacagagc taaaagacct tggttaggtc aacaggaggc tgcttacaag 600
cccacagctc cattgtatga tccaaaatgc tatctatgtc ctggtaacaa aagagctact 660
ggtaacctaa acccaagata tgaatcaacg tatattttcc ccaatgatta tgctgccgtt 720
aggctcgatc aacctatttt accacagaat gattccaatg aggataatct taaaaatagg 780
ctgcttaaag tgcaatctgt gagaggcaat tgtttcgtca tatgttttag ccccaatcat 840
aatctaacca ttccacaaat gaaacaatca gatctggttc atattgttaa ttcttggcaa 900
gcattgactg acgatctctc cagagaagca agagaaaatc ataagccttt caaatatgtc 960
caaatatttg aaaacaaagg tacagccatg ggttgttcca acttacatcc acatggccaa 1020
gcttggtgct tagaatccgg acgtacgtat aacttcgtat agcatacatt atacgaagtt 1080
attctagtaa cggccgccag tgtgctggaa ttcgccctta aggcctcgtc cccgccgggt 1140
cacccggcca gcgacatgga ggcccagaat accctccttg acagtcttga cgtgcgcagc 1200
tcaggggcat gatgtgactg tcgcccgtac atttagccca tacatcccca tgtataatca 1260
tttgcatcca tacattttga tggccgcacg gcgcgaagca aaaattacgg ctcctcgctg 1320
cagacctgcg agcagggaaa cgctcccctc acagacgcgt tgaattgtcc ccacgccgcg 1380
cccctgtaga gaaatataaa aggttaggat ttgccactga ggttcttctt tcatatactt 1440
ccttttaaaa tcttgctagg atacagttct cacatcacat ccgaacataa acaaccatgt 1500
aaaatgacca ctcttgacga cacggcttac cggtaccgca ccagtgtccc gggggacgcc 1560
gaggccatcg aggcactgga tgggtccttc accaccgaca ccgtcttccg cgtcaccgcc 1620
accggggacg gcttcaccct gcgggaggtg ccggtggacc cgcccctgac caaggtgttc 1680
cccgacgacg aatcggacga cgaatcggac gccggggagg acggcgaccc ggactcccgg 1740
acgttcgtcg cgtacgggga cgacggcgac ctggcgggct tcgtggtcgt ctcgtactcc 1800
ggctggaacc gccggctgac cgtcgaggac atcgaggtcg ccccggagca ccgggggcac 1860
ggggtcgggc gcgcgttgat ggggctcgcg acggagttcg cccgcgagcg gggcgccggg 1920
cacctctggc tggaggtcac caacgtcaac gcaccggcga tccacgcgta ccggcggatg 1980
gggttcaccc tctgcggcct ggacaccgcc ctgtacgacg gcaccgcctc ggacggcgag 2040
caggcgctct acatgagcat gccctgcccc tagtactgac aataaaaaga ttcttgtttt 2100
caagaacttg tcatttgtat agttttttta tattgtagtt gttctatttt aatcaaatgt 2160
tagcgtgatt tatatttttt ttcgcctcga catcatctgc ccagatgcga agttaagtgc 2220

gcagaaagta atatcatgcg tcaatcgtat gtgaatgctg gtcgctatac tgctgtcgat 2280

tcgatactaa cgccgccatc cagtgtcgac aagggcgaat tccagcacac tggcggccgt 2340

tactagtgga tccgagctcg gtaccaagct tgatgcatag cttgagtatt ctatagtgtc 2400

acctaaatag cttggcgtaa tcatggtcat agctgtttcc tgtgtgaaat tgttatccgc 2460

tcacaattcc acacaacata cgagccggaa gcataaagtg taaagcctgg ggtgcctaat 2520

gagtgagcta actcacatta attgcgttgc gctcactgcc cgctttccag tcgggaaacc 2580

tgtcgtgcca gctgcattaa tgaatcggcc aacgcgcggg gagaggcggt ttgcgtattg 2640

ggcgctcttc cgcttcctcg ctcactgact cgctgcgctc ggtcgttcgg ctgcggcgag 2700

cggtatcagc tcactcaaag gcggtaatac ggttatccac agaatcaggg gataacgcag 2760

gaaagaacat gtgagcaaaa ggccagcaaa aggccaggaa ccgtaaaaag gccgcgttgc 2820

tggcgttttt ccataggctc cgcccccctg acgagcatca caaaaatcga cgctcaagtc 2880

agaggtggcg aaacccgaca ggactataaa gataccaggc gtttccccct ggaagctccc 2940

tcgtgcgctc tcctgttccg accctgccgc ttaccggata cctgtccgcc tttctccctt 3000

cgggaagcgt ggcgctttct catagctcac gctgtaggta tctcagttcg gtgtaggtcg 3060

ttcgctccaa gctgggctgt gtgcacgaac cccccgttca gcccgaccgc tgcgccttat 3120

ccggtaacta tcgtcttgag tccaacccgg taagacacga cttatcgcca ctggcagcag 3180

ccactggtaa caggattagc agagcgaggt atgtaggcgg tgctacagag ttcttgaagt 3240

ggtggcctaa ctacggctac actagaagaa cagtatttgg tatctgcgct ctgctgaagc 3300

cagttacctt cggaaaaaga gttggtagct cttgatccgg caaacaaacc accgctggta 3360

gcggtggttt ttttgtttgc aagcagcaga ttacgcgcag aaaaaaagga tctcaagaag 3420

atcctttgat cttttctacg gggtctgacg ctcagtggaa cgaaaactca cgttaaggga 3480

ttttggtcat gagattatca aaaaggatct tcacctagat ccttttaaat taaaaatgaa 3540

gttttaaatc aatctaaagt atatatgagt aaacttggtc tgacagttac caatgcttaa 3600

tcagtgaggc acctatctca gcgatctgtc tatttcgttc atccatagtt gcctgactcc 3660

ccgtcgtgta gataactacg atacgggagg gcttaccatc tggccccagt gctgcaatga 3720

taccgcgaga cccacgctca ccggctccag atttatcagc aataaaccag ccagccggaa 3780

gggccgagcg cagaagtggt cctgcaactt tatccgcctc catccagtct attaattgtt 3840

gccgggaagc tagagtaagt agttcgccag ttaatagttt gcgcaacgtt gttgccattg 3900

ctacaggcat cgtggtgtca cgctcgtcgt ttggtatggc ttcattcagc tccggttccc 3960

aacgatcaag gcgagttaca tgatccccca tgttgtgcaa aaaagcggtt agctccttcg 4020

gtcctccgat cgttgtcaga agtaagttgg ccgcagtgtt atcactcatg gttatggcag 4080

cactgcataa ttctcttact gtcatgccat ccgtaagatg cttttctgtg actggtgagt 4140

actcaaccaa gtcattctga gaatagtgta tgcggcgacc gagttgctct tgcccggcgt 4200

caatacggga taataccgcg ccacatagca gaactttaaa agtgctcatc attggaaaac 4260

gttcttcggg gcgaaaactc tcaaggatct taccgctgtt gagatccagt tcgatgtaac 4320

ccactcgtgc acccaactga tcttcagcat cttttacttt caccagcgtt tctgggtgag 4380

caaaaacagg aaggcaaaat gccgcaaaaa agggaataag ggcgacacgg aaatgttgaa 4440

tactcatact cttccttttt caattcagaa gaactcgtca agaaggcgat agaaggcgat 4500

gcgctgcgaa tcgggagcgg cgataccgta aagcacgagg aagcggtcag cccattcgcc 4560

gccaagctct tcagcaatat cacgggtagc caacgctatg tcctgatagc ggtccgccac 4620

acccagccgg acgtacgtat aacttcgtat agca 4654

<210> 45
<211> 5755
<212> DNA
<213> Artificial

<220>
<223> Plasmid pRN792

<400> 45
aagcaagaaa agaatacaaa caaaaaattg aaaaagattg atttagaatt aaaaagaaaa 60

atatttacgt aagaagggaa aatagtaaat gttgcaagtt cactaaactc ctaaattatg 120

ctgcccttta tattccctgt tacagcagcc gagccaaagg tatataggct cctttgcatt 180

agcatgcgta acaaaccacc tgtcagtttc aaccgaggtg gtatccgaga gaattgtgtg 240

attgctttaa ttaatttcgg agaatctcac atgccactga agattaaaaa ctggatgcca 300

gaaaaggggt gtccaggtgt aacatcaata gaggaagctg aaaagtctta gaacgggtaa 360

tcttccacca acctgatggg ttcctagata taatctcgaa gggaataagt agggtgatac 420

cgcagaagtg tctgaatgta ttaaggtcct cacagtttaa atcccgctca cactaacgta 480

ggattattat aactcaaaaa aatggcatta ttctaagtaa gttaaatatc cgtaatcttt 540

aaacactatg tagttaggtc tccctcacaa tcagtccatt tgggtagcac ggtcctcagg 600

acgtatctat tgatggattc gtccagttcc atcaccatta cgctcccgtt aggaacattg 660

gtaaacgatt caaactcttc gtatgtccat ctaaaccatt tcatcaggaa tactctggaa 720

taaataccat gtgtaactag gacaacaaca tctctgggtc ttctctcttg cctatcatgg 780

aagtgcctga ataaagtctc ttggaaactg gcgactctgt catatacatc tgccgcactt 840

tctccatgag ggaatctgaa gaagaaaccg gttgaattcc atggcggccg ttactagaat 900

aacttcgtat aatgtatgct atacgaagtt atacgtacgt ccggctgggt gtggcggacc 960

gctatcagga catagcgttg gctacccgtg atattgctga agagcttggc ggcgaatggg 1020

ctgaccgctt cctcgtgctt tacggtatcg ccgctcccga ttcgcagcgc atcgccttct 1080

atcgccttct tgacgagttc ttctgaattg aaaaaggaag agtatgagta ttcaacattt 1140
ccgtgtcgcc cttattccct tttttgcggc attttgcctt cctgtttttg ctcacccaga 1200
aacgctggtg aaagtaaaag atgctgaaga tcagttgggt gcacgagtgg gttacatcga 1260
actggatctc aacagcggta agatccttga gagttttcgc cccgaagaac gttttccaat 1320
gatgagcact tttaaagttc tgctatgtgg cgcggtatta tcccgtattg acgccgggca 1380
agagcaactc ggtcgccgca tacactattc tcagaatgac ttggttgagt actcaccagt 1440
cacagaaaag catcttacgg atggcatgac agtaagagaa ttatgcagtg ctgccataac 1500
catgagtgat aacactgcgg ccaacttact tctgacaacg atcggaggac cgaaggagct 1560
aaccgctttt ttgcacaaca tgggggatca tgtaactcgc cttgatcgtt gggaaccgga 1620
gctgaatgaa gccataccaa acgacgagcg tgacaccacg atgcctgtag caatggcaac 1680
aacgttgcgc aaactattaa ctggcgaact acttactcta gcttcccggc aacaattaat 1740
agactggatg gaggcggata aagttgcagg accacttctg cgctcggccc ttccggctgg 1800
ctggtttatt gctgataaat ctggagccgg tgagcgtggg tctcgcggta tcattgcagc 1860
actggggcca gatggtaagc cctcccgtat cgtagttatc tacacgacgg ggagtcaggc 1920
aactatggat gaacgaaata gacagatcgc tgagataggt gcctcactga ttaagcattg 1980
gtaactgtca gaccaagttt actcatatat actttagatt gatttaaaac ttcattttta 2040
atttaaaagg atctaggtga agatcctttt tgataatctc atgaccaaaa tcccttaacg 2100
tgagttttcg ttccactgag cgtcagaccc cgtagaaaag atcaaaggat cttcttgaga 2160
tcctttttttt ctgcgcgtaa tctgctgctt gcaaacaaaa aaaccaccgc taccagcggt 2220
ggtttgtttg ccggatcaag agctaccaac tctttttccg aaggtaactg gcttcagcag 2280
agcgcagata ccaaatactg ttcttctagt gtagccgtag ttaggccacc acttcaagaa 2340
ctctgtagca ccgcctacat acctcgctct gctaatcctg ttaccagtgg ctgctgccag 2400
tggcgataag tcgtgtctta ccgggttgga ctcaagacga tagttaccgg ataaggcgca 2460
gcggtcgggc tgaacggggg gttcgtgcac acagcccagc ttggagcgaa cgacctacac 2520
cgaactgaga tacctacagc gtgagctatg agaaagcgcc acgcttcccg aagggagaaa 2580
ggcggacagg tatccggtaa gcggcagggt cggaacagga gagcgcacga gggagcttcc 2640
agggggaaac gcctggtatc tttatagtcc tgtcgggttt cgccacctct gacttgagcg 2700
tcgatttttg tgatgctcgt caggggggcg gagcctatgg aaaaacgcca gcaacgcggc 2760
ctttttacgg ttcctggcct tttgctggcc ttttgctcac atgttctttc ctgcgttatc 2820
ccctgattct gtggataacc gtattaccgc ctttgagtga gctgataccg ctcgccgcag 2880
ccgaacgacc gagcgcagcg agtcagtgag cgaggaagcg gaagagcgcc caatacgcaa 2940
accgcctctc cccgcgcgtt ggccgattca ttaatgcagc tggcacgaca ggtttcccga 3000

ctggaaagcg ggcagtgagc gcaacgcaat taatgtgagt tagctcactc attaggcacc 3060
ccaggcttta cactttatgc ttccggctcg tatgttgtgt ggaattgtga gcggataaca 3120
atttcacaca ggaaacagct atgaccatga ttacgccaag ctatttaggt gacactatag 3180
aatactcaag ctatgcatca agcttggtac cgagctcgga tccactagta acggccgcca 3240
gtgtgctgga attcgccctt gtcgacactg gatggcggcg ttagtatcga atcgacagca 3300
gtatagcgac cagcattcac atacgattga cgcatgatat tactttctgc gcacttaact 3360
tcgcatctgg gcagatgatg tcgaggcgaa aaaaaatata aatcacgcta acatttgatt 3420
aaaatagaac aactacaata taaaaaaact atacaaatga caagttcttg aaaacaagaa 3480
tctttttatt gtcagtactg attagaaaaa ctcatcgagc atcaaatgaa actgcaattt 3540
attcatatca ggattatcaa taccatattt ttgaaaaagc cgtttctgta atgaaggaga 3600
aaactcaccg aggcagttcc ataggatggc aagatcctgg tatcggtctg cgattccgac 3660
tcgtccaaca tcaatacaac ctattaattt cccctcgtca aaaataaggt tatcaagtga 3720
gaaatcacca tgagtgacga ctgaatccgg tgagaatggc aaaagcttat gcatttcttt 3780
ccagacttgt tcaacaggcc agccattacg ctcgtcatca aaatcactcg catcaaccaa 3840
accgttattc attcgtgatt gcgcctgagc gagacgaaat acgcgatcgc tgttaaaagg 3900
acaattacaa acaggaatcg aatgcaaccg gcgcaggaac actgccagcg catcaacaat 3960
attttcacct gaatcaggat attcttctaa tacctggaat gctgttttgc cggggatcgc 4020
agtggtgagt aaccatgcat catcaggagt acggataaaa tgcttgatgg tcggaagagg 4080
cataaattcc gtcagccagt ttagtctgac catctcatct gtaacatcat tggcaacgct 4140
acctttgcca tgtttcagaa acaactctgg cgcatcgggc ttcccataca atcgatagat 4200
tgtcgcacct gattgcccga cattatcgcg agcccattta tacccatata aatcagcatc 4260
catgttggaa tttaatcgcg gcctcgaaac gtgagtcttt tccttaccca tggttgttta 4320
tgttcggatg tgatgtgaga actgtatcct agcaagattt taaaaggaag tatatgaaag 4380
aagaacctca gtggcaaatc ctaacctttt atatttctct acaggggcgc ggcgtgggga 4440
caattcaacg cgtctgtgag gggagcgttt ccctgctcgc aggtctgcag cgaggagccg 4500
taatttttgc ttcgcgccgt gcggccatca aaatgtatgg atgcaaatga ttatacatgg 4560
ggatgtatgg gctaaatgta cgggcgacag tcacatcatg cccctgagct gcgcacgtca 4620
agactgtcaa ggagggtatt ctgggcctcc atgtcgctgg ccgggtgacc cggcggggac 4680
gaggcaagct taagggcgaa ttccagcaca ctggcggccg ttactagaat aacttcgtat 4740
aatgtatgct atacgaagtt atacgtacgt ccggataagg gcgaattcca gcacactggc 4800
ggccgttact agtggatcta actctgcctg gacggcagga tcctctggtg acaccttgtt 4860
agacttagca atggaacgct tggcgtcttc taccttattc acctcacata aataacgtgg 4920

ggattcagga actaacgtca aagcgccaat cataaataat gaccaagcga aacatagccc 4980

taatggaact ctccattgaa ctgagttcga atagctcttt gtaccgtaat tagtacagta 5040

gcccaaaaag atacctgcag taatcatcag ctgataacaa gaaactagtg tgcctctcaa 5100

gtgctttgga gcaatttcag agatcaacat aggacataag acggcgatgc cgccgacacc 5160

caaaccagat atgattctac caatgaaata ttggtaccac ttgttgatag aggcaatttg 5220

aatgataata ccaactatat aaaccgagac gacaatcgaa agaccctttt tacggccata 5280

catatctcca cctttggaaa gtataatacc accaaaggca cagccaatat tgaaaatggc 5340

gacgattaaa cctgttctga cgtttgacaa atagtgggta ccatccttat gtttcatacc 5400

aaaccttctc aaaaagtctg tttggacaac aaacccagaa atagtaccgg tatcccagcc 5460

aaacatgaag ccgccgaagg caacacacaa acaaagcaag gaaacggtaa catattcaga 5520

catgggcttc ttgggtatct ctataggaac actttgggag ccttcagacc ctgactcacc 5580

ggctttcaat tcatcattag aatacttttg agattgtgcg cttaaatggg aatctttact 5640

gagtgaagag atcacgtctt caccagcttg ggttgctgt gaaacaacag gcatattgtt 5700

ctcctcaact gccattttct agatttatgt atgtgttttt tgtagttata gattt 5755

<210> 46
<211> 10485
<212> DNA
<213> Artificial

<220>
<223> BamHI released insert of pRN693

<400> 46
ggatcctgag aaatgggtga atgttgagat aattgttggg attccattgt tgataaaggc 60

tataatatta ggtatacaga atatactaga agttctcctc gaggatatag gaatcctcaa 120

aatggaatct atatttctac atactaatat tacgattatt cctcattccg ttttatatgt 180

ttatattcat tgatcctatt acattatcaa tccttgcgtt tcagcttcca ctaatttaga 240

tgactatttc tcatcatttg cgtcatcttc taacaccgta tatgataata tactagtaat 300

gtaaatacta gttagtagat gatagttgat ttttattcgt acatccggag ctagcatgcg 360

gccgccagtg tgatggatat ctcagaattc gcccttttaa gcttcgtacg ttttaaacag 420

ttgatgagaa cctttttcgc aagttcaagg tgctctaatt tttaaaattt ttacttttcg 480

cgacacaata aagtcttcac gacgctaaac tattagtgca cataatgtag ttacttggac 540

gctgttcaat aatgtataaa atttatttcc tttgcattac gtacattata taaccaaatc 600

ttaaaaatat agaaatatga tatgtgtata ataatataag caaaatttac gtatctttgc 660

ttataatata gctttaatgt tctttaggta tatatttaag agcgatttgt ctcgagctaa 720

tctagcaaat ctctagaacg caattccttc gagacttctt ctttcatgaa ggagataaca 780

tcgtgcgggt cagctgcagt gaaaacactg gtaccagcga caataacgtt ggcaccggct 840

ttggcggctt tcgggatggt ctccttgccc aaaccaccat cgacttggat attcaaatgg 900

gggaacttgg ctctcaaagt ttccactttt ggcatcatgt cttccatgaa tttttggcct 960

ccaaacccag gttccacagt cataacaaga gccatatcca aatgaggagc tagttcaaat 1020

aaaacgtcaa cagaagtacc aggtttgatg gcgcatgcag ctttgatgcc cttagactta 1080

atcaacttaa ctaaatgcaa agggtcttgt gtggcctcgt agtggaacgt aaattggtca 1140

gcaccacatt tagcaaaatc gtcgacccat ttttcaggat tttcaaccat catgtgacaa 1200

tcgaagaacg cagtgggctt cttttctgtg ttgctagcat cgccagggcg tggcacagaa 1260

cgacgtaggg aggtaacaat tggttggccc agagtaatgt ttggaacaaa atggccgtcc 1320

atgacatcga tatgtaacca atctgcgccg gcgttgatga ccttatgaca ttcgcaaccc 1380

aagttggcga agtcagaagc aaggatactg ggagctataa ttggtttgac cattttaatg 1440

cagaattcgc ccttgaattc tttatgtgtg tttattcgaa actaagttct tggtgtttta 1500

aaactaaaaa aaagactaac tataaaagta gaatttaaga agtttaagaa atagatttac 1560

agaattacaa tcaataccta ccgtctttat atacttatta gtcaagtagg ggaataattt 1620

cagggaactg gtttcaacct tttttttcag ctttttccaa atcagagaga gcagaaggta 1680

atagaaggtg taagaaaatg agatagatac atgcgtgggt caattgcctt gtgtcatcat 1740

ttactccagg caggttgcat cactccattg aggttgtgcc cgttttttgc ctgtttgtgc 1800

ccctgttctc tgtagttgcg ctaagagaat ggacctatga actgatggtt ggtgaagaaa 1860

acaatatttt ggtgctggga ttcttttttt ttctggatgc cagcttaaaa agcgggctcc 1920

attatattta gtggatgcca ggaataaact gttcacccag acacctacga tgttatatat 1980

tctgtgtaac ccgcccccta ttttgggcat gtacgggtta cagcagaatt aaaaggctaa 2040

tttttttgact aaataaagtt aggaaaatca ctactattaa ttatttacgt attctttgaa 2100

atggcagtat tgataatgat aaaccggtcc taggtaccaa gggcgaattc cagcacactg 2160

gcggccgtta ctagtaacgg ccgccagtgt gctggaattc gcccgtcgac cggtcccaca 2220

caccatagct tcaaaatgtt tctactcctt ttttactctt ccagattttc tcggactccg 2280

cgcatcgccg taccacttca aaacacccaa gcacagcata ctaaatttcc cctctttctt 2340

cctctagggt gtcgttaatt acccgtacta aaggtttgga aaagaaaaaa gagaccgcct 2400

cgtttctttt tcttcgtcga aaaaggcaat aaaaattttt atcacgtttc tttttcttga 2460

aaattttttt ttttgatttt tttctctttc gatgacctcc cattgatatt taagttaata 2520

aacggtcttc aatttctcaa gtttcagttt catttttctt gttctattac aacttttttt 2580

acttcttgct cattagaaag aaagcatagc aatctaatct aagctgcaga agcttaaaat 2640

gactcaattc actgacattg ataagctagc cgtctccacc ataagaattt tggctgtgga 2700

caccgtatcc aaggccaact caggtcaccc aggtgctcca ttgggtatgg caccagctgc 2760
acacgttcta tggagtcaaa tgcgcatgaa cccaaccaac ccagactgga tcaacagaga 2820
tagatttgtc ttgtctaacg gtcacgcggt cgctttgttg tattctatgc tacatttgac 2880
tggttacgat ctgtctattg aagacttgaa acagttcaga cagttgggtt ccagaacacc 2940
aggtcatcct gaatttgagt tgccaggtgt tgaagttact accggtccat taggtcaagg 3000
tatctccaac gctgttggta tggccatggc tcaagctaac cttgctgcca cttacaacaa 3060
gccaggcttt accttgtctg acaactacac ctatgttttc ttgggtgacg gttgtttgca 3120
agaaggtatt tcttcagaag cttcctcctt ggctggtcat ttgaaattgg gtaacttgat 3180
tgccatctac gatgacaaca agatcactat cgatggtgct accagtatct cattcgatga 3240
agatgttgct aagagatacg aagcctacgg ttgggaagtt ttgtacgtag aaaatggtaa 3300
cgaagatcta gccggtattg ccaaggctat tgctcaagct aagttatcca aggacaaacc 3360
aactttgatc aaaatgacca caaccattgg ttacggttcc ttgcatgccg gctctcactc 3420
tgtgcacggt gccccattga aagcagatga tgttaaacaa ctaaagagca aattcggttt 3480
caaccaagac aagtcctttg ttgttccaca agaagtttac gaccactacc aaaagacaat 3540
tttaaagcca ggtgtcgaag ccaacaacaa gtggaacaag ttgttcagcg aataccaaaa 3600
gaaattccca gaattaggtg ctgaattggc tagaagattg agcggccaac tacccgcaaa 3660
ttgggaatct aagttgccaa cttacaccgc caaggactct gccgtggcca ctagaaaatt 3720
atcagaaact gttcttgagg atgtttacaa tcaattgcca gagttgattg gtggttctgc 3780
cgatttaaca ccttctaact tgaccagatg gaaggaagcc cttgacttcc aacctccttc 3840
ttccggttca ggtaactact ctggtagata catcagatac ggtattagag aacacgctat 3900
gggtgccatc atgaacggta tttcagcttt cggtgccaac tacaaaccat acggtggtac 3960
tttcttgaac ttcgtttctt atgctgctgg tgccgttaga ttgtccgctt tgtctggcca 4020
cccagttatt tgggttgcta cacatgactc tatcggtgtc ggtgaagatg gtccaacaca 4080
tcaacctatt gaaactttag cacacttcag atccctacca aacattcaag tttggagacc 4140
agctgatggt aacgaagttt ctgcagccta caagaactct ttagaatcca agcatactcc 4200
aagtatcatt gctttgtcca gacaaaactt gccacaattg gaaggtagct ctattgaaag 4260
cgcttctaaa ggtggttacg tactacaaga tgttgctaac ccagatatta ttttagtggc 4320
tactggttct gaagtatctt tgagtgttga agctgctaag actttggccg caaagaacat 4380
caaggctcgt gttgtttctc taccagattt cttcactttt gacaaacaac ccctagaata 4440
cagactatca gtcttaccag acaacgttcc aatcatgtct gttgaagttt tggctaccac 4500
atgttggggc aaatacgctc atcaatcctt cggtattgac agatttggtg cctccggtaa 4560
ggcaccagaa gtcttcaagt tcttcggttt caccccagaa ggtgttgctg aaagagctca 4620

aaagaccatt gcattctata agggtgacaa gctaatttct cctttgaaaa aagctttcta 4680

gtcgagacaa atcgctctta aatatatacc taaagaacat taaagctata ttataagcaa 4740

agatacgtaa attttgctta tattattata cacatatcat atttctatat ttttaagatt 4800

tggttatata atgtacgtaa tgcaaaggaa ataaatttta tacattattg aacagcgtcc 4860

aagtaactac attatgtgca ctaatagttt agcgtcgtga agactttatt gtgtcgcgaa 4920

aagtaaaaat tttaaaaatt agagcacctt gaacttgcga aaaaggttct catcaactgt 4980

ttaaaacgta cgtgtggaag aacgattaca acaggtgttg tcctctgagg acataaaata 5040

cacaccgaga ttcatcaact cattgctggg gttagcatat ctacaattgg gtgaaatggg 5100

gagcgatttg caggcatttg ctcggcatgc cggtagaggt gtggtcaata agagcgacct 5160

catgctatac ctgagaaagc aacctgaccc acaggaaaga gttactcaag aataagaatt 5220

ttcgttttaa aacctaagag tcactttaaa atttgtatac acttattttt tttataactt 5280

atttaataat aaaaatcata aatcataaga aattcgcgcg ttaagcggta actttctttt 5340

caatcaagtc gaatagagta acaatatcgg cagagaattt tctgatacct tcggacaatt 5400

tttcagtggc catagcgtct tcattcaagt cgaatctgaa tttagattcg tcgctgatgt 5460

aagaaatctt gtcgccggct tccttcttag cggagacagg gtccaaaact cttgggaaag 5520

gttcagtact gttcatcaac ttgtccaata aagctggaga aattgttaga tagtcaacac 5580

cagccaagtt tttgatttcg tcagtgcttc tgaaagaagc acccataaca atagtcttgt 5640

aaccgtactt cttgtagtag ttgtagattt tcttgacgga aataacacct gggtcggctt 5700

cacccttgta atctttacca gtgctggatt tgtaccagtc tagaattcta ccaacaaatg 5760

gggaaatcaa agtaacttgg gcctcggcac aggcaactgc ttgaacgaag gagaataata 5820

gagtcaaatt acagtggata ccgtcctttt cttccaattc tttggcagct tgaatacctt 5880

cccaagtgga agcaatttta ataaggactc tttccttgga gacaccttct tgttcaaaca 5940

atttaatgat atgtctagcc ttttcaatgg tagcttgagt gtcaaaagac aatctagcat 6000

caacttcggt ggagactctg cctggaacaa tctttaagat ctccttaccg aattcgacta 6060

acaatctgtc cacagcattt tcgacttggt cttcggtggt cttaccatgc ttcttaccgt 6120

attccacggc aacatcgatc aacttggcgt aagttggttg cttggcagca gccaagatca 6180

atgatgggtt agttgtggag tcttgaggtt gaaacttggc aatagagccg aaatcaccag 6240

tgtcggcaac aacgacagtg ccggaggctt tcaattgttc tagagagttg ttagcaacct 6300

tttgtttctt ttgagctggt tcagacattt tactagattt atgtatgtgt tttttgtagt 6360

tatagattta agcaagaaaa gaatacaaac aaaaaattga aaaagattga tttagaatta 6420

aaaagaaaaa tatttacgta agaagggaaa atagtaaatg ttgcaagttc actaaactcc 6480

taaattatgc tgccctttat attccctgtt acagcagccg agccaaaggt atataggctc 6540

```
ctttgcatta gcatgcgtaa caaaccacct gtcagtttca accgaggtgg tatccgagag   6600
aattgtgtga ttgctttaat taatttcgga gaatctcaca tgccactgaa gattaaaaac   6660
tggatgccag aaaaggggtg tccaggtgta acatcaatag aggaagctga aaagtcttag   6720
aacgggtaat cttccaccaa cctgatgggt tcctagatat aatctcgaag ggaataagta   6780
gggtgatacc gcagaagtgt ctgaatgtat taaggtcctc acagtttaaa tcccgctcac   6840
actaacgtag gattattata actcaaaaaa atggcattat tctaagtaag ttaaatatcc   6900
gtaatcttta aacactatgt agttaggtct ccctcacaat cagtccattt gggtagcacg   6960
gtcctcagga cgtatctatt gatggattcg tccagttcca tcaccattac gctcccgtta   7020
ggaacattgg taaacgattc aaactcttcg tatgtccatc taaaccattt catcaggaat   7080
actctggaat aaataccatg tgtaactagg acaacaacat ctctgggtct tctctcttgc   7140
ctatcatgga agtgcctgaa taaagtctct tggaaactgg cgactctgtc atatacatct   7200
gccgcacttt ctccatgagg gaatctgaag aagaaaccgg tttatcatta tcaatactgc   7260
catttcaaag aatacgtaaa taattaatag tagtgatttt cctaacttta tttagtcaaa   7320
aaattagcct tttaattctg ctgtaacccg tacatgccca aaataggggg cgggttacac   7380
agaatatata acatcgtagg tgtctgggtg aacagtttat tcctggcatc cactaaatat   7440
aatggagccc gctttttaag ctggcatcca gaaaaaaaaa gaatcccagc accaaaatat   7500
tgttttcttc accaaccatc agttcatagg tccattctct tagcgcaact acagagaaca   7560
ggggcacaaa caggcaaaaa acgggcacaa cctcaatgga gtgatgcaac ctgcctggag   7620
taaatgatga cacaaggcaa ttgacccacg catgtatcta tctcattttc ttacaccttc   7680
tattaccttc tgctctctct gatttggaaa aagctgaaaa aaaaggttga aaccagttcc   7740
ctgaaattat tcccctactt gactaataag tatataaaga cggtaggtat tgattgtaat   7800
tctgtaaatc tatttcttaa acttcttaaa ttctactttt atagttagtc tttttttag   7860
ttttaaaaca ccaagaactt agtttcgaat aaacacacat aaagcttaaa atggctgccg   7920
gtgtcccaaa aattgatgcg ttagaatctt tgggcaatcc tttggaggat gccaagagag   7980
ctgcagcata cagagcagtt gatgaaaatt taaaatttga tgatcacaaa attattggaa   8040
ttggtagtgg tagcacagtg gtttatgttg ccgaaagaat tggacaatat ttgcatgacc   8100
ctaaatttta tgaagtagcg tctaaattca tttgcattcc aacaggattc caatcaagaa   8160
acttgatttt ggataacaag ttgcaattag gctccattga acagtatcct cgcattgata   8220
tagcgtttga cggtgctgat gaagtggatg agaatttaca attgattaaa ggtggtggtg   8280
cttgtctatt tcaagaaaaa ttggttagta ctagtgctaa aaccttcatt gtcgttgctg   8340
attcaagaaa aaagtcacca aaacatttag gtaagaactg gaggcaaggt gttcccattg   8400
aaattgtacc ttcctcatac gtgagggtca agaatgatct attagaacaa ttgcatgctg   8460
```

```
aaaaagttga catcagacaa ggaggttctg ctaaagcagg tcctgttgta actgacaata   8520
ataacttcat tgtcgatgcg gatttcggtg aaatttccga tccaagaaaa ttgcatagag   8580
aaatcaaact gttagtgggc gtggtggaaa caggtttatt catcgacaac gcttcaaaag   8640
cctacttcgg taattctgac ggtagtgttg aagttaccga aaagtagatc ccttttcct    8700
ttgtcgatat catgtaatta gttatgtcac gcttacattc acgccctcct cccacatccg   8760
ctctaaccga aaaggaagga gttagacaac ctgaagtcta ggtccctatt tattttttt    8820
aatagttatg ttagtattaa gaacgttatt tatatttcaa atttttcttt tttttctgta   8880
caaacgcgtg tacgcatgta acattatact gaaaaccttg cttgagaagg ttttgggacg   8940
ctcgaaggct tcctagattg gtgagcgcta ggagtcactg ccaggtatcg tttgaacacg   9000
gcattagtca gggaagtcat aacacagtcc tttcccgcaa ttttcttttt ctattactct   9060
tggcctcctc tagtacactc tatatttttt tatgcctcgg taatgatttt catttttttt   9120
tttccaccta gcggatgact cttttttttt cttagcgatt ggcattatca cataatgaat   9180
tatacattat ataaagtaat gtgatttctt cgaagaatat actaaaaaat gagcaggcaa   9240
gataaacgaa ggcaaagatg acagagcaga aagccctagt aaagcgtatt acaaatgaaa   9300
ccaagattca gattgcgatc tctttaaagg gtggtcccct agcgatagag cactcgatct   9360
tcccagaaaa agaggcagaa gcagtagcag aacaggccac acaatcgcaa gtgattaacg   9420
tccacacagg tatagggttt ctggaccata tgatacatgc tctggccaag cattccggct   9480
ggtcgctaat cgttgagtgc attggtgact tacacataga cgaccatcac accactgaag   9540
actgcgggat tgctctcggt caagctttta aagaggccct aggggccgtg cgtggagtaa   9600
aaaggtttgg atcaggattt gcgcctttgg atgaggcact ttccagagcg gtggtagatc   9660
tttcgaacag gccgtacgca gttgtcgaac ttggtttgca aagggagaaa gtaggagatc   9720
tctcttgcga gatgatcccg cattttcttg aaagctttgc agaggctagc agaattaccc   9780
tccacgttga ttgtctgcga ggcaagaatg atcatcaccg tagtgagagt gcgttcaagg   9840
ctcttgcggt tgccataaga gaagccacct cgcccaatgg taccaacgat gttccctcca   9900
ccaaaggtgt tcttatgtag tgacaccgat tatttaaagc tgcagcatac gatatatata   9960
catgtgtata tatgtatacc tatgaatgtc agtaagtatg tatacgaaca gtatgatact  10020
gaagatgaca aggtaatgca tcattctata cgtgtcattc tgaacgaggc gcgctttcct  10080
tttttctttt tgctttttct tttttttct cttgaactcg acggatcata tgcggtgtga   10140
aatactcgac tgagaaatgg gtgaatgttg agataattgt tgggattcca ttgttgataa  10200
aggctataat attaggtata cagaatatac tagaagttct cctcgaggat ttaggaatcc  10260
ataaaaggga atctgcaatt ctacacaatt ctataaatat tattatcatc gttttatatg  10320
ttaatattca ttgatcctat tacattatca atccttgcgt ttcagcttcc actaatttag  10380
```

atgactattt ctcatcattt gcgtcatctt ctaacaccgt atatgataat atactagtaa 10440

cgtaaatact agttagtaga tgatagttga tttttattcg gatcc 10485

<210> 47
<211> 12863
<212> DNA
<213> Artificial

<220>
<223> NsiI released insert of pRN755

<400> 47
caagctatgc atgagaaatg ggtgaatgtt gagataattg ttgggattcc attgttgata 60

aaggctataa tattaggtat acagaatata ctagaagttc tcctcgagga tataggaatc 120

ctcaaaatgg aatctatatt tctacatact aatattacga ttattcctca ttccgtttta 180

tatgtttata ttcattgatc ctattacatt atcaatcctt gcgtttcagc ttccactaat 240

ttagatgact atttctcatc atttgcgtca tcttctaaca ccgtatatga taatatacta 300

gtaatgtaaa tactagttag tagatgatag ttgattttta ttaagcttga tatcatccat 360

atggagctcc gcggctactt caagctgggg taagtatgct catcaatcgt tcggactcga 420

cgaatttggt cgttcaggca aggggcctga aatttacaaa ttgttcgatt tcacagcgga 480

cggtgttgcg tcaagggctg aaaagacaat caattactac aaaggaaagc agttgctttc 540

tcctatggga agagcttctt aagtctgaag aagtaaacag ttctttgcta tttcacactt 600

cctggttgat ggtcacttgc tgcctgaaat atatatatat gtatgtcata tgtacttgtt 660

ttcttttttg tgcctttgtt acgtctatat tctttgaaac tgattattcg attttcttct 720

tgctgaccgc ttctagaggc atcgcacagt tttagcgagg aaaactcttc aatagttttg 780

ccagcggaat tccacttgca attacataaa aaattccggc ggtttttcgc gtgtgagtca 840

atgtcgaaat acctgcctaa taaacatgaa catcgcccaa atgtatttga agacccgctg 900

ggagaagttc aagatatata agcaacaagc agccaatagt ataaaaaaaa atctgagttt 960

attacctttc ctggaatttc agtgaaaaac tgctaattat agagagatat cacagagtta 1020

ctcactaatg actaacgaaa aggtctggat agagaagttg gataatccaa ctctttcagt 1080

gttaccacat gactttttac gcccacaaca agaaccttat acgaaacaag ctacatattc 1140

gttacagcta cctcagctcg atgtgcctca tgatagtttt tctaacaaat acgctgtcgc 1200

tttgagtgta tgggctgcat tgatatatag agtaaccggt gacgatgata ttgttcttta 1260

tattgcgaat aacaaaatct taagattcaa tattcaacca acgtggtcat ttaatgagct 1320

gtattctaca attaacaatg agttgaacaa gctcaattct attgaggcca atttttcctt 1380

tgacgagcta gctgaaaaaa ttcaaagttg ccaagatctg gaaaggaccc ctcagttgtt 1440

ccgtttggcc tttttggaaa accaagattt caaattagac gagttcaagc atcatttagt 1500

ggactttgct ttgaatttgg ataccagtaa taatgcgcat gttttgaact taatttataa 1560

cagcttactg tattcgaatg aaagagtaac cattgttgcg gaccaattta ctcaatattt 1620

gactgctgcg ctaagcgatc catccaattg cataactaaa atctctctga tcaccgcatc 1680

atccaaggat agtttacctg atccaactaa gaacttgggc tggtgcgatt tcgtggggtg 1740

tattcacgac attttccagg acaatgctga agccttccca gagagaacct gtgttgtgga 1800

gactccaaca ctaaattccg acaagtcccg ttctttcact tatcgcgaca tcaaccgcac 1860

ttctaacata gttgcccatt atttgattaa aacaggtatc aaaagaggtg atgtagtgat 1920

gatctattct tctaggggtg tggatttgat ggtatgtgtg atgggtgtct tgaaagccgg 1980

cgcaaccttt tcagttatcg accctgcata tcccccagcc agacaaacca tttacttagg 2040

tgttgctaaa ccacgtgggt tgattgttat tagagctgct ggacaattgg atcaactagt 2100

agaagattac atcaatgatg aattggagat tgtttcaaga atcaattcca tcgctattca 2160

agaaaatggt accattgaag gtggcaaatt ggacaatggc gaggatgttt tggctccata 2220

tgatcactac aaagacacca gaacaggtgt tgtagttgga ccagattcca acccaaccct 2280

atctttcaca tctggttccg aaggtattcc taagggtgtt cttggtagac atttttcctt 2340

ggcttattat ttcaattgga tgtccaaaag gttcaactta acagaaaatg ataaattcac 2400

aatgctgagc ggtattgcac atgatccaat tcaaagagat atgtttacac cattattttt 2460

aggtgcccaa ttgtatgtcc ctactcaaga tgatattggt acaccgggcc gtttagcgga 2520

atggatgagt aagtatggtt gcacagttac ccatttaaca cctgccatgg gtcaattact 2580

tactgcccaa gctactacac cattccctaa gttacatcat gcgttctttg tgggtgacat 2640

tttaacaaaa cgtgattgtc tgaggttaca aaccttggca gaaaattgcc gtattgttaa 2700

tatgtacggt accactgaaa cacagcgtgc agtttcttat ttcgaagtta aatcaaaaaa 2760

tgacgatcca aactttttga aaaaattgaa agatgtcatg cctgctggta aaggtatgtt 2820

gaacgttcag ctactagttg ttaacaggaa cgatcgtact caaatatgtg gtattggcga 2880

aataggtgag atttatgttc gtgcaggtgg tttggccgaa ggttatagag gattaccaga 2940

attgaataaa gaaaaatttg tgaacaactg gtttgttgaa aaagatcact ggaattattt 3000

ggataaggat aatggtgaac cttggagaca attctggtta ggtccaagag atagattgta 3060

cagaacgggt gatttaggtc gttatctacc aaacggtgac tgtgaatgtt gcggtagggc 3120

tgatgatcaa gttaaaattc gtgggttcag aatcgaatta ggagaaatag atacgcacat 3180

ttcccaacat ccattggtaa gagaaaacat tactttagtt cgcaaaaatg ccgacaatga 3240

gccaacattg atcacattta tggtcccaag atttgacaag ccagatgact tgtctaagtt 3300

ccaaagtgat gttccaaagg aggttgaaac tgaccctata gttaagggct taatcggtta 3360

ccatctttta tccaaggaca tcaggacttt cttaaagaaa agattggcta gctatgctat 3420

```
gccttccttg attgtggtta tggataaact accattgaat ccaaatggta aagttgataa   3480
gcctaaactt caattcccaa ctcccaagca attaaatttg gtagctgaaa atacagtttc   3540
tgaaactgac gactctcagt ttaccaatgt tgagcgcgag gttagagact tatggttaag   3600
tatattacct accaagccag catctgtatc accagatgat tcgtttttcg atttaggtgg   3660
tcattctatc ttggctacca aaatgatttt taccttaaag aaaaagctgc aagttgattt   3720
accattgggc acaattttca agtatccaac gataaaggcc tttgccgcgg aaattgacag   3780
aattaaatca tcgggtggat catctcaagg tgaggtcgtc gaaaatgtca ctgcaaatta   3840
tgcggaagac gccaagaaat tggttgagac gctaccaagt tcgtacccct ctcgagaata   3900
ttttgttgaa cctaatagtg ccgaaggaaa aacaacaatt aatgtgtttg ttaccggtgt   3960
cacaggattt ctgggctcct acatccttgc agatttgtta ggacgttctc caaagaacta   4020
cagtttcaaa gtgtttgccc acgtcagggc caaggatgaa gaagctgcat ttgcaagatt   4080
acaaaaggca ggtatcacct atggtacttg gaacgaaaaa tttgcctcaa atattaaagt   4140
tgtattaggc gatttatcta aaagccaatt tggtctttca gatgagaagt ggatggattt   4200
ggcaaacaca gttgatataa ttatccataa tggtgcgtta gttcactggg tttatccata   4260
tgccaaattg agggatccaa atgttatttc aactatcaat gttatgagct tagccgccgt   4320
cggcaagcca aagttctttg actttgtttc ctccacttct actcttgaca ctgaatacta   4380
ctttaatttg tcagataaac ttgttagcga agggaagcca ggcattttag aatcagacga   4440
tttaatgaac tctgcaagcg ggctcactgg tggatatggt cagtccaaat gggctgctga   4500
gtacatcatt agacgtgcag gtgaaagggg cctacgtggg tgtattgtca gaccaggtta   4560
cgtaacaggt gcctctgcca atggttcttc aaacacagat gatttcttat tgagattttt   4620
gaaaggttca gtccaattag gtaagattcc agatatcgaa aattccgtga atatggttcc   4680
agtagatcat gttgctcgtg ttgttgttgc tacgtctttg aatcctccca aagaaaatga   4740
attggccgtt gctcaagtaa cgggtcaccc aagaatatta ttcaaagact acttgtatac   4800
tttacacgat tatggttacg atgtcgaaat cgaaagctat tctaaatgga agaaatcatt   4860
ggaggcgtct gttattgaca ggaatgaaga aaatgcgttg tatcctttgc tacacatggt   4920
cttagacaac ttacctgaaa gtaccaaagc tccagaacta gacgatagga acgccgtggc   4980
atctttaaag aaagacaccg catggacagg tgttgattgg tctaatggaa taggtgttac   5040
tccagaagag gttggtatat atattgcatt tttaaacaag gttggatttt tacctccacc   5100
aactcataat gacaaacttc cactgccaag tatagaacta actcaagcgc aaataagtct   5160
agttgcttca ggtgctggtg ctcgtggaag ctccgcagca gcttaaggtt gagcattacg   5220
tatgatatgt ccatgtacaa taattaaata tgaattagga gaaagactta gcttcttttc   5280
gggtgatgtc acttaaaaac tccgagaata atatataata agagaataaa atattagtta   5340
```

```
ttgaataaga actgtaaatc agctggcgtt agtctgctaa tggcagcttc atcttggttt   5400
attgtagcat gaatcatatt tgcctttttt tcctgtaatt caatgattct tgcttctata   5460
ctatcctcaa tgcaaaacct tgtgatcttc acaggtcgat actgaccaat tctatgaact   5520
ctatcaccac tttgccattc aacactaggg ttccaccatg ggtctgagct ccgcggtacc   5580
tctagaaagg gcgaattcca gcacactggc ggccgcggct acttctcgta ggaacaattt   5640
cgggcccctg cgtgttcttc tgaggttcat cttttacatt tgcttctgct ggataatttt   5700
cagaggcaac aaggaaaaat tagatggcaa aaagtcgtct ttcaaggaaa aatccccacc   5760
atctttcgag atcccctgta acttattggc aactgaaaga atgaaaagga ggaaaataca   5820
aaatatacta gaactgaaaa aaaaaaagta taaatagaga cgatatatgc caatacttca   5880
caatgttcga atctattctt catttgcagc tattgtaaaa taataaaaca tcaagaacaa   5940
acaagctcaa cttgtctttt ctaagaacaa agaataaaca caaaaacaaa aagttttttt   6000
aattctgcag aaaatgccat ctgctacctc taacccagct aacaacacct ctttggaaca   6060
atacgaagtt tggttcttga ctggttctca acacttgtac ggtgaagacg ttttgaagca   6120
agttgctgct caatctcaag aaatcgctaa cgctttgaac gctaactcta acgttccagt   6180
taagttggtt tggaagccag ttttgaccga ctctgacgct atcagaagaa ccgctttgga   6240
agctaacgct gacgactctg ttatcggtgt taccgcttgg atgcacacct tctctccagc   6300
taagatgtgg attcaaggtt tggacgcttt gagaaagcca ttgttgcact tgcacaccca   6360
agctaacaga gacttgccat gggctgacat cgacttcgac ttcatgaact tgaaccaagc   6420
tgctcacggt gacagagaat tcggttacat ccaatccaga ttgggtgttc caagaaagac   6480
cgttgttggt cacgtttcta acccagaagt tgctagacaa gttggtgctt ggcaaagagc   6540
ttctgctggt tgggctgctg ttagaacctt gaagttgacc agattcggtg acaacatgag   6600
aaacgttgct gttaccgaag gtgacaagac cgaagctgaa ttgagattcg gtgtttctgt   6660
caacacctgg tctgttaacg aattggctga cgctgttcac ggtgctgctg aatctgacgt   6720
tgactctttg gttgctgaat acgaaagatt gtacgaagtt gttccagaat tgaagaaggg   6780
tggtgctaga cacgaatctt tgagatactc tgctaagatc gaattgggtt tgagatcctt   6840
cttggaagct aacggttctg ctgctttcac cacctctttc gaagacttgg gtgctttgag   6900
acaattgcca ggtatggctg ttcaaagatt gatggctgaa ggttacggtt tcggtgctga   6960
aggtgactgg aagaccgcta tcttggttag agctgctaag gttatgggtg gtgacttgcc   7020
aggtggtgct tctttgatgg aagactacac ctaccacttg gaaccaggtt ctgaaaagat   7080
cttgggtgct cacatgttgg aagtttgtcc atctttgacc gctaagaagc caagagttga   7140
aatccaccca ttgggtatcg gtggtaagga agacccagtt agaatggttt tcgacaccga   7200
cgctggtcca ggtgttgttg ttgctttgtc tgacatgaga gacagattca gattggttgc   7260
```

```
taacgttgtt gacgttgtcg acttggacca accattgcca aacttgccag ttgctagagc   7320
tttgtgggaa ccaaagccaa acttcgctac ctctgctgct gcttggttga ccgctggtgc   7380
tgctcaccac accgttttgt ctacccaagt tggtttggac gttttcgaag acttcgctga   7440
aatcgctaag accgaattgt tgaccatcga cgaagacacc accatcaagc aattcaagaa   7500
ggaattgaac tggaacgctg cttactacaa gttggctggt ggtttgtaac tcgagacaaa   7560
tcgctcttaa atatatacct aaagaacatt aaagctatat tataagcaaa gatacgtaaa   7620
ttttgcttat attattatac acatatcata tttctatatt tttaagattt ggttatataa   7680
tgtacgtaat gcaaaggaaa taaattttat acattattga acagcgtcca agtaactaca   7740
ttatgtgcac taatagttta gcgtcgtgaa gactttattg tgtcgcgaaa agtaaaaatt   7800
ttaaaaatta gagcaccttg aacttgcgaa aaaggttctc atcaactgtt taaaatcaga   7860
attcgccctt aaagctccac cgcggtggcg gccgccgtac gtgtggaaga acgattacaa   7920
caggtgttgt cctctgagga cataaaatac acaccgagat tcatcaactc attgctggag   7980
ttagcatatc tacaattggg tgaaatgggg agcgatttgc aggcatttgc tcggcatgcc   8040
ggtagaggtg tggtcaataa gagcgacctc atgctatacc tgagaaagca acctgaccta   8100
caggaaagag ttactcaaga ataagaattt tcgttttaaa acctaagagt cactttaaaa   8160
tttgtataca cttatttttt ttataactta tttaataata aaaatcataa atcataagaa   8220
attcgcgcgc ttaagcagaa gcaccttcca aggtagaggc ttgagcagct ggaccgttgt   8280
gagaagaacc ttggatagca gctctttgga tagccttcaa tctgtgcata acgttgttgg   8340
tacctctacc gaagtagtcg tgcaaggttc tgtattcttg gaacaaaact tcgtaagcag   8400
caacgttttc tgggattggg gtgtaaacag cacctggagc agcagccata gaagaagcag   8460
cttctctgat gtccttgtac ttaccagcag caacagcagc gtggatagca gaacccaaag   8520
ctggaccttg ttcagaaccg atggtagaca attgcaaacc agtgatgtca gcgtaaactt   8580
gcatcaagaa cttgttcttc aacaaaccac cagcaacgat gaattcctta actggaacac   8640
cagagtctct gaaagcgtca acgatggttc tggtaccgaa agcggtagct tccaacaaag   8700
ctctgtaggt gtcttctggc ttggtagcca aggtttgacc aacaacaaca ccagacaatt   8760
cgtggtcaac caaaacagat ctgttaccag agtgccagtc caaagcgatc aaaccgtgtt   8820
caccgatagc ttgcttttca gccaattcgg tcaagtattc gtggataccc aaacccttgt   8880
ccttagcagc ttggtggtat tctggtggaa caccgttctt ggtgaaccaa ccgaagatgt   8940
caccaacacc agattgacca gcttcgtaac cccacaaacc gtcaacgata ccaccgtcaa   9000
caacaccaca catacctgga acttctctca agacgtcacc gttcataacg tgacaggtag   9060
aggtacccat gatagcaacc aattgacctg gttcaacagc gttagcagct ggagcagaaa   9120
cgtgagcgtc gacgttacca acagcaacag cgataccagc tggcaaacca gtccaagcag   9180
```

cagcttcttc ggtcaagtaa ccagcagcgt cacccaatct accgatggtg tgttccaact 9240
tttcagaaac gaagtccttg aagtctgggt tcaaagcggt caagaagtct tgagatgggt 9300
acttaccgtc ttggtagata cccttgtaac cagcggtaca agcgtttcta acgtaagaac 9360
cacacaattg ccaaacgatc cagtcagcag cttcaaccca gtgttccata gcaccgtaaa 9420
cttctgggtc ttcttccaac aattgcaaac ccttagcgaa ttcccattca gaagagatca 9480
aaccaccgta tcttggcaac caagattcac ctctttcagc agccaattgg ttgattctgt 9540
cagcttgtgg ttgagcagcg tggtgtctcc acaacttaac gaaagcgtgt ggtctgtcag 9600
cgaatctttc caattcgttc aatggggtac cgtcagcggt ggttggaacc atggtacaag 9660
cggtgaagtc ggtaccgata ccaacaacgt tttctgggtt gataccagcg tcagcaacag 9720
cagctggaac agcgtttctc aaaacgtctc tgtagtcgtt tggaacttgc aaagcccagt 9780
cagctggcaa tctttgagaa gaacctggca agttgtcggt aacaacagcg tgtgggtatt 9840
cgaaaacacc agaaccgatt tcagcaccgt cagaaactct aacaacaaca gctctaccag 9900
acaaggtacc gtagtcaaca ccgataacga attgttcgtc caatgggatg ttttcagagg 9960
tgttcatttt ctagatttat gtatgtgttt tttgtagtta tagatttaag caagaaaaga 10020
atacaaacaa aaaattgaaa aagattgatt tagaattaaa aagaaaaata tttacgtaag 10080
aagggaaaat agtaaatgtt gcaagttcac taaactccta aattatgctg ccctttatat 10140
tccctgttac agcagccgag ccaaaggtat ataggctcct ttgcattagc atgcgtaaca 10200
aaccacctgt cagtttcaac cgaggtggta tccgagagaa ttgtgtgatt gctttaatta 10260
atttcggaga atctcacatg ccactgaaga ttaaaaactg gatgccagaa aaggggtgtc 10320
caggtgtaac atcaatagag gaagctgaaa agtcttagaa cgggtaatct tccaccaacc 10380
tgatgggttc ctagatataa tctcgaaggg aataagtagg gtgataccgc agaagtgtct 10440
gaatgtatta aggtcctcac agtttaaatc ccgctcacac taacgtagga ttattataac 10500
tcaaaaaaat ggcattattc taagtaagtt aaatatccgt aatctttaaa cactatgtag 10560
ttaggtctcc ctcacaatca gtccatttgg gtagcacggt cctcaggacg tatctattga 10620
tggattcgtc cagttccatc accattacgc tcccgttagg aacattggta aacgattcaa 10680
actcttcgta tgtccatcta aaccatttca tcaggaatac tctggaataa ataccatgtg 10740
taactaggac aacaacatct ctgggtcttc tctcttgcct atcatggaag tgcctgaata 10800
aagtctcttg gaaactggcg actctgtcat atacatctgc cgcactttct ccatgaggga 10860
atctgaagaa gaaaccggtt tatcattatc aatactgcca tttcaaagaa tacgtaaata 10920
attaatagta gtgattttcc taactttatt tagtcaaaaa attagccttt taattctgct 10980
gtaacccgta catgcccaaa atagggggcg ggttacacag aatatataac atcgtaggtg 11040
tctgggtgaa cagtttattc ctggcatcca ctaaatataa tggagcccgc tttttaagct 11100

ggcatccaga aaaaaaaaga atcccagcac caaaatattg ttttcttcac caaccatcag 11160

ttcataggtc cattctctta gcgcaactac agagaacagg ggcacaaaca ggcaaaaaac 11220

gggcacaacc tcaatggagt gatgcaacct gcctggagta aatgatgaca caaggcaatt 11280

gacccacgca tgtatctatc tcattttctt acaccttcta ttaccttctg ctctctctga 11340

tttggaaaaa gctgaaaaaa aaggttgaaa ccagttccct gaaattattc ccctacttga 11400

ctaataagta tataaagacg gtaggtattg attgtaattc tgtaaatcta tttcttaaac 11460

ttcttaaatt ctacttttat agttagtctt ttttttagtt ttaaaacacc aagaacttag 11520

tttcgaataa acacacataa agcttaaaat gtcttctttg ttggaatcta tcgctaaggt 11580

tagaagagac gtttgtgact tgcacgctga attgaccaga tacgaattgg ttgtttggac 11640

cgctggtaac gtttctggta gaatcccagg tcacgacttg atggttatca agccatctgg 11700

tgtttcttac gaccaattga ccccagaatt gatggttgtt accgacttgt acggtactcc 11760

agttagaggt atgaacactg gttctgctgg taccgttgac tggggtaacc cagaattgtc 11820

tccatcttct gacaccgctg ctcacgctta cgtttacaga cacatgccag aagttggtgg 11880

tgttgttcac acccactcta cctacgctac cgcttgggct gctagaggtg aagaaatccc 11940

atgtgttttg accatgatgg gtgacgaatt cggtggtcca atcccagttg gtccattcgc 12000

tttgatcggt gacgactcta tcggtcaagg tatcgttgaa accttgaaga actctaactc 12060

tccagctgtt ttgatgcaaa accacggtcc attcaccatc ggtaagtctg ctagagaagc 12120

tgttaaggct gctgttatgt gtgaagaagt tgctagaacc gttcacatct ccagacaatt 12180

gggtgaacca ttgccaatcg accaagctaa gatcgaatct ttgtacaaga gataccaaaa 12240

cgtttacggt agataaggat cccctttcc tttgtcgata tcatgtaatt agttatgtca 12300

cgcttacatt cacgccctcc tcccacatcc gctctaaccg aaaaggaagg agttagacaa 12360

cctgaagtct aggtccctat ttattttttt taatagttat gttagtatta agaacgttat 12420

ttatatttca aatttttctt ttttttctgt acaaacgcgt gtacgcatgt aacattatac 12480

tgaaaacctt gcttgagaag gttttgggac gctcgaaggc ttcctaggtg agaaatgggt 12540

gaatgttgag ataattgttg ggattccatt gttgataaag gctataatat taggtataca 12600

gaatatacta gaagttctcc tcgaggattt aggaatccat aaaaggaaat ctgtaattct 12660

acacaattct ataaatatta ttatcatcgt tttatatgtt aatattcatt gatcctatta 12720

cattatcaat ccttgcgttt cagctcccac taatttagat gactatttct catcatttgc 12780

gtcatcttct aacaccgtat atgataatat actagtaacg taaatactag ttagtagatg 12840

atagttgatt tttattcatg cat 12863

<210> 48
<211> 4638

<212> DNA
<213> Artificial

<220>
<223> pRN914

<400> 48
```
tacattatac gaagttattc tagtaacggc cgccatggaa ttcaaccggt cccacacacc      60

atagcttcaa aatgtttcta ctcctttttt actcttccag attttctcgg actccgcgca     120

tcgccgtacc acttcaaaac acccaagcac agcatactaa atttcccctc tttcttcctc     180

tagggtgtcg ttaattaccc gtactaaagg tttggaaaag aaaaaagaga ccgcctcgtt     240

tctttttctt cgtcgaaaaa ggcaataaaa atttttatca cgtttctttt tcttgaaaat     300

tttttttttt gatttttttc tctttcgatg acctcccatt gatatttaag ttaataaacg     360

gtcttcaatt tctcaagttt cagtttcatt tttcttgttc tattacaact ttttttactt     420

cttgctcatt agaaagaaag catagcaatc taatctaagt ctagaaaaat gtcatttcaa     480

attgaaacgg ttcccaccaa accatatgaa gaccaaaagc ctggtacctc tggtttgcgt     540

aagaagacaa aggtgtttaa agacgaacct aactacacag aaaatttcat tcaatcgatc     600

atggaagcta ttccagaggg ttctaaaggt gccactcttg ttgtcggtgg tgatgggcgt     660

tactacaatg atgtcattct tcataagatt gccgctatcg gtgctgccaa cggtattaaa     720

aagttagtta ttggccagca tggtcttctg tctacgccag ccgcttctca catcatgaga     780

acctacgagg aaaaatgtac tggtggtatt atcttaaccg cctcacataa tccaggtggt     840

ccagaaaatg acatgggtat taagtataac ttatccaatg gggggtcctgc tcctgaatcc     900

gtcacaaatg ctatttggga gatttccaaa aagcttacca gctataagat tatcaaagac     960

ttcccagaac tagacttggg tacgataggc aagaacaaga aatacggtcc attactcgtt    1020

ccggacgtac gtataacttc gtatagcata cattatacga agttattcta gtaacggccg    1080

ccagtgtgct ggaattcgcc cttaaggcct cgtccccgcc gggtcacccg gccagcgaca    1140

tggaggccca gaataccctc cttgacagtc ttgacgtgcg cagctcaggg gcatgatgtg    1200

actgtcgccc gtacatttag cccatacatc cccatgtata atcatttgca tccatacatt    1260

ttgatggccg cacggcgcga agcaaaaatt acggctcctc gctgcagacc tgcgagcagg    1320

gaaacgctcc cctcacagac gcgttgaatt gtccccacgc cgcgcccctg tagagaaata    1380

taaaaggtta ggatttgcca ctgaggttct tctttcatat acttcctttt aaaatcttgc    1440

taggatacag ttctcacatc acatccgaac ataaacaacc atgtaaaatg accactcttg    1500

acgacacggc ttaccggtac cgcaccagtg tcccgggggga cgccgaggcc atcgaggcac    1560

tggatgggtc cttcaccacc gacaccgtct tccgcgtcac cgccaccggg gacggcttca    1620

ccctgcggga ggtgccggtg gacccgcccc tgaccaaggt gttccccgac gacgaatcgg    1680

acgacgaatc ggacgccggg gaggacggcg acccggactc ccggacgttc gtcgcgtacg    1740
```

gggacgacgg cgacctggcg ggcttcgtgg tcgtctcgta ctccggctgg aaccgccggc 1800
tgaccgtcga ggacatcgag gtcgccccgg agcaccgggg gcacggggtc gggcgcgcgt 1860
tgatggggct cgcgacggag ttcgcccgcg agcggggcgc cgggcacctc tggctggagg 1920
tcaccaacgt caacgcaccg gcgatccacg cgtaccggcg gatggggttc accctctgcg 1980
gcctggacac cgccctgtac gacggcaccg cctcggacgg cgagcaggcg ctctacatga 2040
gcatgccctg cccctagtac tgacaataaa aagattcttg ttttcaagaa cttgtcattt 2100
gtatagtttt tttatattgt agttgttcta ttttaatcaa atgttagcgt gatttatatt 2160
ttttttcgcc tcgacatcat ctgcccagat gcgaagttaa gtgcgcagaa agtaatatca 2220
tgcgtcaatc gtatgtgaat gctggtcgct atactgctgt cgattcgata ctaacgccgc 2280
catccagtgt cgacaagggc gaattccagc acactggcgg ccgttactag tggatccgag 2340
ctcggtacca agcttgatgc atagcttgag tattctatag tgtcacctaa atagcttggc 2400
gtaatcatgg tcatagctgt ttcctgtgtg aaattgttat ccgctcacaa ttccacacaa 2460
catacgagcc ggaagcataa agtgtaaagc ctggggtgcc taatgagtga gctaactcac 2520
attaattgcg ttgcgctcac tgcccgcttt ccagtcggga aacctgtcgt gccagctgca 2580
ttaatgaatc ggccaacgcg cggggagagg cggtttgcgt attgggcgct cttccgcttc 2640
ctcgctcact gactcgctgc gctcggtcgt tcggctgcgg cgagcggtat cagctcactc 2700
aaaggcggta atacggttat ccacagaatc aggggataac gcaggaaaga acatgtgagc 2760
aaaaggccag caaaaggcca ggaaccgtaa aaaggccgcg ttgctggcgt ttttccatag 2820
gctccgcccc cctgacgagc atcacaaaaa tcgacgctca agtcagaggt ggcgaaaccc 2880
gacaggacta taaagatacc aggcgtttcc ccctggaagc tccctcgtgc gctctcctgt 2940
tccgaccctg ccgcttaccg gatacctgtc cgcctttctc ccttcgggaa gcgtggcgct 3000
ttctcatagc tcacgctgta ggtatctcag ttcggtgtag gtcgttcgct ccaagctggg 3060
ctgtgtgcac gaaccccccg ttcagcccga ccgctgcgcc ttatccggta actatcgtct 3120
tgagtccaac ccggtaagac acgacttatc gccactggca gcagccactg gtaacaggat 3180
tagcagagcg aggtatgtag gcggtgctac agagttcttg aagtggtggc ctaactacgg 3240
ctacactaga agaacagtat ttggtatctg cgctctgctg aagccagtta ccttcggaaa 3300
aagagttggt agctcttgat ccggcaaaca aaccaccgct ggtagcggtg gtttttttgt 3360
ttgcaagcag cagattacgc gcagaaaaaa aggatctcaa gaagatcctt tgatcttttc 3420
tacggggtct gacgctcagt ggaacgaaaa ctcacgttaa gggattttgg tcatgagatt 3480
atcaaaaagg atcttcacct agatcctttt aaattaaaaa tgaagtttta aatcaatcta 3540
aagtatatat gagtaaactt ggtctgacag ttaccaatgc ttaatcagtg aggcacctat 3600
ctcagcgatc tgtctatttc gttcatccat agttgcctga ctccccgtcg tgtagataac 3660

tacgatacgg gagggcttac catctggccc cagtgctgca atgataccgc gagacccacg 3720

ctcaccggct ccagatttat cagcaataaa ccagccagcc ggaagggccg agcgcagaag 3780

tggtcctgca actttatccg cctccatcca gtctattaat tgttgccggg aagctagagt 3840

aagtagttcg ccagttaata gtttgcgcaa cgttgttgcc attgctacag gcatcgtggt 3900

gtcacgctcg tcgtttggta tggcttcatt cagctccggt tcccaacgat caaggcgagt 3960

tacatgatcc cccatgttgt gcaaaaaagc ggttagctcc ttcggtcctc cgatcgttgt 4020

cagaagtaag ttggccgcag tgttatcact catggttatg gcagcactgc ataattctct 4080

tactgtcatg ccatccgtaa gatgcttttc tgtgactggt gagtactcaa ccaagtcatt 4140

ctgagaatag tgtatgcggc gaccgagttg ctcttgcccg gcgtcaatac gggataatac 4200

cgcgccacat agcagaactt taaaagtgct catcattgga aaacgttctt cggggcgaaa 4260

actctcaagg atcttaccgc tgttgagatc cagttcgatg taacccactc gtgcacccaa 4320

ctgatcttca gcatctttta ctttcaccag cgtttctggg tgagcaaaaa caggaaggca 4380

aaatgccgca aaaaagggaa taagggcgac acggaaatgt tgaatactca tactcttcct 4440

ttttcaattc agaagaactc gtcaagaagg cgatagaagg cgatgcgctg cgaatcggga 4500

gcggcgatac cgtaaagcac gaggaagcgg tcagcccatt cgccgccaag ctcttcagca 4560

atatcacggg tagccaacgc tatgtcctga tagcggtccg ccacacccag ccggacgtac 4620

gtataacttc gtatagca 4638

<210> 49
<211> 33
<212> DNA
<213> Artificial

<220>
<223> primer ugp1f

<400> 49
aactgcagaa aatgtccact aagaagcaca cca 33

<210> 50
<211> 44
<212> DNA
<213> Artificial

<220>
<223> ugp1r

<400> 50
ttgcgcgctc aatgttccaa gatttgcaaa ttaccagtaa cgac 44

<210> 51
<211> 41
<212> DNA
<213> Artificial

<220>
<223> primer gal7f

<400> 51
aatctagaaa atgactgctg aagaatttga tttttctagc c 41

<210> 52
<211> 35
<212> DNA
<213> Artificial

<220>
<223> primer gal7r

<400> 52
tttccggatt ctaagcacca agcttggcca tgtgg 35

<210> 53
<211> 41
<212> DNA
<213> Artificial

<220>
<223> primer Pgm2f

<400> 53
aatctagaaa aatgtcattt caaattgaaa cggttcccac c 41

<210> 54
<211> 24
<212> DNA
<213> Artificial

<220>
<223> primer Pgm2r

<400> 54
tttccggaac gagtaatgga ccgt 24

<210> 55
<211> 2496
<212> DNA
<213> Artificial

<220>
<223> Expression cassette for EC 1.1.1.22 PA3559 with ANB1 promoter

<400> 55
gagctcgtac gcatatgatc atgtgtcgtc gcacacatat atatatgcct gtatgtgtca 60

gcactaaagt tgcctggcca tccacgctat atatacacgc ctggcggatc tgctcgagga 120

ttgcctacgc gtgggcttga tccaccaacc aacgctcgcc aaatgaactg gcgctttggt 180

cttctgccat cgtccgtaaa ccccggccaa agagaccgga aagatcggtg aaaacatctt 240

gatcttgctc ccgggaattt tagattcagg taggaaattg attacatcaa tactgttacc 300

ctgaatcata ttcgacgatg tcgtctcaca cggaaatata attcatttct tggttttcca 360

```
aaaaaatttt cattttttt cactttttg tttcgtcctc cttttttttt tttttttttt    420
atttttttc ctgtgttcac cttttttttt ttcagttgac atctttctgc attcttttct    480
gtgtttttt ttttttttt cgtttttcca ttgttcgttc gttgcctgtt ttttcgccct    540
attgttctcg agcctaaaaa ttttttcctt tcctgctttc ctttcttcgt tcaaagtttc    600
ctattccatt gttctctttg gtaaactcat tgttgtcgga actcagatat attcaggtca    660
atttactgta cttcaattga cttttttctt gaaatttcaa cttgcctttt caacttgttc    720
ttctttttta atcttattct acactttagt tcccttacct tgttcctaat tattgtctag    780
caaaaagaaa acatacacct atttcattca cacactgcag aaaatgaaga tctctgtttt    840
cggttctggt tacgttggtt tggttcaagc tgctgttttg gctgaagttg gtcacgacgt    900
tttgtgtatg gacatcgaca gaaacaaggt tgaaagattg gctcaaggtt tggcttctat    960
ctacgaacca ggtttggacg ctttgttgag agaaggtttg gactctggta gattgagatt   1020
ctcttctgac gctagattgg ctgttgaaca cggtagagtt caattcatcg ctgttggtac   1080
cccaccaggt gaagacggtg ctgctgactt gggtgctgtt ttcgttgttg ctgacgctat   1140
cgctgaacac agaagaaagc cagttatcgt tgttgaaaag tctaccgttc cagttggtac   1200
aggtgacaga ttgagagcac acatcgaaag aagattggaa gctgacggta gagaattgga   1260
atttgaaatc gtttctaacc cagaattttt gaaggaaggt tctgctgttg ctgactgtag   1320
aagaccagac agaatcgttg ttggttgtga caacgaagaa gttagacaag ttatgagaga   1380
actttacgaa ccattcaaca gaaaccacga cagaatgttg ttcatgggtt tgagatctgc   1440
tgaattgacc aagtacgctg ctaacggtat gttggctacc aagatttctt tcatcaacca   1500
aatcgctgaa ttggctgaac acttaggtgc tgacatcgaa gctgttagac aaggtatcgg   1560
tgctgaccca agaatcggtt accacttcat ctacccaggt tgtggttacg gtggttcttg   1620
tttcccaaag gacatgagag ctttgatcca ctctgctgaa caagctcact gttcttctga   1680
cttgttgcaa gctgttgaag ctatcaacag aagacaaaag cacaagttgt tcgaaagaat   1740
cagagttttc tacgacggtg acttgagagg taagaccttc gctttgtggg gtttggcttt   1800
caagccaaac accgacgaca tgagagacgc tccatcaaga gaattgatgg aagctttgtg   1860
gagacacggt gctcaagtta gagcttacga tccagaagct atgcaagaaa cccaaagatt   1920
gtacggtcac gacgaaagat tgtctttgat gggtacccca gaagctacct tgggtggtgc   1980
tgacgctttg gttatctgta ccgaatggca acaattcaag gctccagact tcgaattgtt   2040
gaaggaaaga ttgaaggctc cagttatctt cgacggtaga aacttgtacg acccagaaag   2100
aatggctaga cacggtttcc actactaccc aatgggtaga ggtcaatctt gttctttgcc   2160
aatcaacgag gcttctttgg ctcaagaaga cggtatgaga ttgttgagac aagcttaggg   2220
atccccttttt cctttgtcga tatcatgtaa ttagttatgt cacgcttaca ttcacgccct   2280
```

cctcccacat ccgctctaac cgaaaaggaa ggagttagac aacctgaagt ctaggtccct 2340

atttattttt tttaatagtt atgttagtat taagaacgtt atttatattt caaatttttc 2400

tttttttttct gtacaaacgc gtgtacgcat gtaacattat actgaaaacc ttgcttgaga 2460

aggttttggg acgctcgaag gcttcctagg gtcgac 2496

<210> 56
<211> 10901
<212> DNA
<213> Artificial

<220>
<223> pRN906

<400> 56
ggaatacagg taagcaaatt gatactaatg gctcaacgtg ataaggaaaa agaattgcac 60

tttaacatta atattgacaa ggaggagggc accacacaaa aagttaggtg taacagaaaa 120

tcatgaaact acgattccta atttgatatt ggaggatttt ctctaaaaaa aaaaaaatac 180

aacaaataaa aaacactcaa tgacctgacc atttgatgga gtttaagtca ataccttctt 240

gaaccatttc ccataatggt gaaagttccc tcaagaattt tactctgtca gaaacggcct 300

tacgacgtag tcgatatggt gcactctcag tacaatctgc tctgatgccg catagttaag 360

ccagccccga cacccgccaa cacccgctga cgcgccctga cgggcttgtc tgctcccggc 420

atccgcttac agacaagctg tgaccgtctc cgggagctgc atgtgtcaga ggttttcacc 480

gtcatcaccg aaacgcgcga gacgaaaggg cctcgtgata cgcctatttt tataggttaa 540

tgtcatgata ataatggttt cttagacgtc aggtggcact tttcggggaa atgtgcgcgg 600

aacccctatt tgtttatttt tctaaataca ttcaaatatg tatccgctca tgagacaata 660

accctgataa atgcttcaat aatattgaaa aaggaagagt atgagtattc aacatttccg 720

tgtcgccctt attccctttt ttgcggcatt ttgccttcct gtttttgctc acccagaaac 780

gctggtgaaa gtaaaagatg ctgaagatca gttgggtgca cgagtgggtt acatcgaact 840

ggatctcaac agcggtaaga tccttgagag ttttcgcccc gaagaacgtt ttccaatgat 900

gagcactttt aaagttctgc tatgtggcgc ggtattatcc cgtattgacg ccgggcaaga 960

gcaactcggt cgccgcatac actattctca gaatgacttg gttgagtact caccagtcac 1020

agaaaagcat cttacggatg gcatgacagt aagagaatta tgcagtgctg ccataaccat 1080

gagtgataac actgcggcca acttacttct gacaacgatc ggaggaccga aggagctaac 1140

cgcttttttt cacaacatgg gggatcatgt aactcgcctt gatcgttggg aaccggagct 1200

gaatgaagcc ataccaaacg acgagcgtga caccacgatg cctgtagcaa tggcaacaac 1260

gttgcgcaaa ctattaactg gcgaactact tactctagct tcccggcaac aattaataga 1320

ctggatggag gcggataaag ttgcaggacc acttctgcgc tcggcccttc cggctggctg 1380

```
gtttattgct gataaatctg gagccggtga gcgtgggtct cgcggtatca ttgcagcact   1440
ggggccagat ggtaagccct cccgtatcgt agttatctac acgacgggca gtcaggcaac   1500
tatggatgaa cgaaatagac agatcgctga gataggtgcc tcactgatta agcattggta   1560
actgtcagac caagtttact catatatact ttagattgat ttaaaacttc atttttaatt   1620
taaaaggatc taggtgaaga tcctttttga taatctcatg accaaaatcc cttaacgtga   1680
gttttcgttc cactgagcgt cagaccccgt agaaaagatc aaaggatctt cttgagatcc   1740
tttttttctg cgcgtaatct gctgcttgca aacaaaaaaa ccaccgctac cagcggtggt   1800
ttgtttgccg gatcaagagc taccaactct ttttccgaag gtaactggct tcagcagagc   1860
gcagatacca aatactgtcc ttctagtgta gccgtagtta ggccaccact tcaagaactc   1920
tgtagcaccg cctacatacc tcgctctgct aatcctgtta ccagtggctg ctgccagtgg   1980
cgataagtcg tgtcttaccg ggttggactc aagacgatag ttaccggata aggcgcagcg   2040
gtcgggctga acggggggtt cgtgcacaca gcccagcttg gagcgaacga cctacaccga   2100
actgagatac ctacagcgtg agcattgaga aagcgccacg cttcccgaag ggagaaaggc   2160
ggacaggtat ccggtaagcg gcagggtcgg aacaggagag cgcacgaggg agcttccagg   2220
ggggaacgcc tggtatcttt atagtcctgt cgggtttcgc cacctctgac ttgagcgtcg   2280
atttttgtga tgctcgtcag gggggcgag cctatggaaa aacgccagca acgcggcctt   2340
tttacggttc ctggcctttt gctggccttt tgctcacatg ttctttcctg cgttatcccc   2400
tgattctgtg gataaccgta ttaccgcctt tgagtgagct gataccgctc gccgcagccg   2460
aacgaccgag cgcagcgagt cagtgagcga ggaagcggaa gagcgcccaa tacgcaaacc   2520
gcctctcccc gcgcgttggc cgattcatta atgcagctgg cacgacaggt ttcccgactg   2580
gaaagcgggc agtgagcgca acgcaattaa tgtgagttac ctcactcatt aggcacccca   2640
ggctttacac tttatgcttc cggctcctat gttgtgtgga attgtgagcg gataacaatt   2700
tcacacagga aacagctatg accatgatta cgccaagctc ggaattaacc ctcactaaag   2760
ggaacaaaag ctgggtacgc atatgatcat gtgtcgtcgc acacatatat atatgcctgt   2820
atgtgtcagc actaaagttg cctggccatc cacgctatat atacacgcct ggcggatctg   2880
ctcgaggatt gcctacgcgt gggcttgatc caccaaccaa cgctcgccaa atgaactggc   2940
gctttggtct tctgccatcg tccgtaaacc ccggccaaag agaccggaaa gatcggtgaa   3000
aacatcttga tcttgctccc gggaatttta gattcaggta ggaaattgat tacatcaata   3060
ctgttaccct gaatcatatt cgacgatgtc gtctcacacg gaaatataat tcatttcttg   3120
gttttccaaa aaaattttca tttttttca ctttttgtt tcgtcctcct ttttttttt   3180
ttttttttat tttttttcct gtgttcacct ttttttttt cagttgacat ctttctgcat   3240
tcttttctgt gtttttttt ttttttttcg tttttccatt gttcgttcgt tgcctgtttt   3300
```

ttcgccctat tgttctcgag cctaaaaatt ttttcctttc ctgctttcct ttcttcgttc 3360
aaagtttcct attccattgt tctctttggt aaactcattg ttgtcggaac tcagatatat 3420
tcaggtcaat ttactgtact tcaattgact tttttcttga aatttcaact tgccttttca 3480
acttgttctt cttttttaat cttattctac actttagttc ccttaccttg ttcctaatta 3540
ttgtctagca aaaagaaaac atacacctat ttcattcaca cactgcagaa aatgaagatc 3600
tctgttttcg gttctggtta cgttggtttg gttcaagctg ctgttttggc tgaagttggt 3660
cacgacgttt tgtgtatgga catcgacaga aacaaggttg aaagattggc tcaaggtttg 3720
gcttctatct acgaaccagg tttggacgct ttgttgagag aaggtttgga ctctggtaga 3780
ttgagattct cttctgacgc tagattggct gttgaacacg gtagagttca attcatcgct 3840
gttggtaccc caccaggtga agacggtgct gctgacttgg gtgctgtttt cgttgttgct 3900
gacgctatcg ctgaacacag aagaaagcca gttatcgttg ttgaaaagtc taccgttcca 3960
gttggtacag gtgacagatt gagagcacac atcgaaagaa gattggaagc tgacggtaga 4020
gaattggaat ttgaaatcgt ttctaaccca gaatttttga aggaaggttc tgctgttgct 4080
gactgtagaa gaccagacag aatcgttgtt ggttgtgaca acgaagaagt tagacaagtt 4140
atgagagaac tttacgaacc attcaacaga aaccacgaca gaatgttgtt catgggtttg 4200
agatctgctg aattgaccaa gtacgctgct aacggtatgt tggctaccaa gatttctttc 4260
atcaaccaaa tcgctgaatt ggctgaacac ttaggtgctg acatcgaagc tgttagacaa 4320
ggtatcggtg ctgacccaag aatcggttac cacttcatct acccaggttg tggttacggt 4380
ggttcttgtt tcccaaagga catgagagct ttgatccact ctgctgaaca agctcactgt 4440
tcttctgact tgttgcaagc tgttgaagct atcaacagaa gacaaaagca caagttgttc 4500
gaaagaatca gagttttcta cgacggtgac ttgagaggta agaccttcgc tttgtggggt 4560
ttggctttca agccaaacac cgacgacatg agagacgctc catcaagaga attgatggaa 4620
gctttgtgga gacacggtgc tcaagttaga gcttacgatc cagaagctat gcaagaaacc 4680
caaagattgt acggtcacga cgaaagattg tctttgatgg gtaccccaga agctaccttg 4740
ggtggtgctg acgctttggt tatctgtacc gaatggcaac aattcaaggc tccagacttc 4800
gaattgttga aggaaagatt gaaggctcca gttatcttcg acggtagaaa cttgtacgac 4860
ccagaaagaa tggctagaca cggtttccac tactacccaa tgggtagagg tcaatcttgt 4920
tctttgccaa tcaacgaggc ttctttggct caagaagacg gtatgagatt gttgagacaa 4980
gcttagggat ccccttttcc tttgtcgata tcatgtaatt agttatgtca cgcttacatt 5040
cacgccctcc tcccacatcc gctctaaccg aaaaggaagg agttagacaa cctgaagtct 5100
aggtccctat ttattttttt taatagttat gttagtatta agaacgttat ttatatttca 5160
aatttttctt ttttttctgt acaaacgcgt gtacgcatgt aacattatac tgaaaacctt 5220

gcttgagaag gttttgggac gctcgaaggc ttcctagggt cgacgtacgt gtggaagaac 5280
gattacaaca ggtgttgtcc tctgaggaca taaaatacac accgagattc atcaactcat 5340
tgctggagtt agcatatcta caattgggtg aaatggggag cgatttgcag gcatttgctc 5400
ggcatgccgg tagaggtgtg gtcaataaga gcgacctcat gctatacctg agaaagcaac 5460
ctgacctaca ggaaagagtt actcaagaat aagaattttc gttttaaaac ctaagagtca 5520
ctttaaaatt tgtatacact tatttttttt ataacttatt taataataaa aatcataaat 5580
cataagaaat tcgcgcgctt aagccttgaa gtcgaactta cctggttcag agtacttctt 5640
ttcaacttgt tcaaccttgt tgaacttgaa acctctgatt ctcaaaactt ctggttcaga 5700
agagtccttg tagtcaactg gttccaaagc ccaaccgttg ttttgaacag aaccagaaac 5760
gttaacttca gcgtcgtcaa cagcgtcaac gatcaaagca ccgtcaacag acaagttttc 5820
gatgaagatc tttctaccct tgatagccaa ggtagatctt tgagagatag aacagttacc 5880
agaaacctta gacttaacca aagagaaggt caaaccccac tttggcttcc aggtgattct 5940
tggccaaact tcaacttctt gaccgttgat aacttgcaaa actgggtcag caacttggaa 6000
accagccttc ttcaagatca aagagttagc tctgtagata gccatttcac cagaggtagc 6060
agagtggtat gggttaccct ttggaacctt agcagcgtct tcagcgttgt tcttaactgg 6120
agcgtaagcg aaccaggttt ccataacggt gaaaccaact cttgaagatg gtggcaaggt 6180
ctttgggtag tcttgcatca tacattccaa tctggtagaa gacttgaaag aggtcttaga 6240
agcgtccttg tactttgggt taacaaattc ttggatagca ccaccagtct tagccaattc 6300
ttcgatgtat ggacccaatt ccaagatcaa ttggttgatg ttacctggga atggagagta 6360
accagtttca gagttaacgt caccgtctgg gtaaccagaa gctctcaaca atgggtccaa 6420
ttggttgtat tcaacgttga taaccataga tctaccgtca gagtgggtca atctggtgat 6480
accaccgata gcttccttag cctttcttgg aacagccaaa gagttaacgt ggtattgctt 6540
ggtagaagaa acacccaaag cagatgggat agccttgaac aacaaaccgt tggtgtcttg 6600
gaagaacaaa acccacttca aaccagcgtt gtaccaaacc ttcaagatac cagaagagtg 6660
caacaaagag tgaacgtcac cgtgaccgtg tggcttggtt tgaactctgt atctgttttg 6720
tgggtccaaa gccaatctag cgtcgttgtc ttccaaacaa gcaacctttt cttgcttcaa 6780
caaggtaact tgggttggtt gcataccgaa gtaagagtta gattccaaca agtccaaggt 6840
tctaccgtgg gtgtcgtcag aggtcatgat aacgaatggg atgtgggttt gtccttcacc 6900
ttcagaactt gcttcttgca aagccaagat agattcgatg tagtgttgca agaaacaggt 6960
accagtggtg gtttcagctg gcaaagcaac cttgataccg ttgtaaccca atctttcacc 7020
caaaccacca gcaaccaaaa cgaaagcagc tcttctagct tctctaacac cagcttcttc 7080
gtacttgttg aagttttcgt caccgaactt caaggtttca ccagttggaa cagatggggt 7140

gaaaccgtcg aatgggttgt taccagcctt agagtcagcc aacaatctct tagcgttgtt 7200
gatgtaagca accaaaccac ctgggtaaga agagtccaac aaaaccaatt ggtcgaagaa 7260
agccttcttt tcgtcgtcgt caacacctgg gtttggccag tctctgaaca agtggtcttg 7320
tccgttgtcc aacaacatct taaccaattc tctttgttgt ggagacaaca agttgaagtt 7380
gtcacccaaa gaagaagcca ttttaagctt ctgcagctta gattagattg ctatgctttc 7440
tttctaatga gcaagaagta aaaaaagttg taatagaaca agaaaaatga aactgaaact 7500
tgagaaattg aagaccgttt attaacttaa atatcaatgg gaggtcatcg aaagagaaaa 7560
aaatcaaaaa aaaaaatttt caagaaaaag aaacgtgata aaaattttta ttgccttttt 7620
cgacgaagaa aaagaaacga ggcggtctct tttttctttt ccaaaccttt agtacgggta 7680
attaacgaca ccctagagga agaaagaggg gaaatttagt atgctgtgct tgggtgtttt 7740
gaagtggtac ggcgatgcgc ggagtccgag aaaatctgga agagtaaaaa aggagtagaa 7800
acattttgaa gctatggtgt gtgggaccgg attatcgatg ataagctgtc aaagatgaga 7860
attaattcca cggactatag actatactag atactccgtc tactgtacga tacacttccg 7920
ctcaggtcct tgtcctttaa cgaggcctta ccactctttt gttactctat tgatccagct 7980
cagcaaaggc agtgtgatct aagattctat cttcgcgatg tagtaaaact agctagaccg 8040
agaaagagac tagaaatgca aaaggcactt ctacaatggc tgccatcatt attatccgat 8100
gtgacgctgc agcttctcaa tgatattcga atacgctttg aggagataca gcctaatatc 8160
cgacaaactg ttttacagat ttacgatcgt acttgttacc catcattgaa ttttgaacat 8220
ccgaacctgg gagttttccc tgaaacagat agtatatttg aacctgtata ataatatata 8280
gtctagcgct ttacggaaga caatgtatgt atttcggttc ctggagaaac tattgcatct 8340
attgcatagg taatcttgca cgtcgcatcc ccggttcatt ttctgcgttt ccatcttgca 8400
cttcaatagc atatctttgt taacgaagca tctgtgcttc attttgtaga acaaaaatgc 8460
aacgcgagag cgctaatttt tcaaacaaag aatctgagct gcatttttac agaacagaaa 8520
tgcaacgcga aagcgctatt ttaccaacga agaatctgtg cttcattttt gtaaaacaaa 8580
aatgcaacgc gacgagagcg ctaatttttc aaacaaagaa tctgagctgc atttttacag 8640
aacagaaatg caacgcgaga gcgctatttt accaacaaag aatctatact tcttttttgt 8700
tctacaaaaa tgcatcccga gagcgctatt tttctaacaa agcatcttag attacttttt 8760
ttctcctttg tgcgctctat aatgcagtct cttgataact ttttgcactg taggtccgtt 8820
aaggttagaa gaaggctact ttggtgtcta ttttctcttc cataaaaaaa gcctgactcc 8880
acttccccgcg tttactgatt actagcgaag ctgcgggtgc attttttcaa gataaaggca 8940
tccccgatta tattctatac cgatgtggat tgcgcatact ttgtgaacag aaagtgatag 9000
cgttgatgat tcttcattgg tcagaaaatt atgaacggtt tcttctattt tgtctctata 9060

tactacgtat aggaaatgtt tacattttcg tattgttttc gattcactct atgaatagtt 9120
cttactacaa tttttttgtc taaagagtaa tactagagat aaacataaaa aatgtagagg 9180
tcgagtttag atgcaagttc aaggagcgaa aggtggatgg gtaggttata tagggatata 9240
gcacagagat atatagcaaa gagatacttt tgagcaatgt ttgtggaagc ggtattcgca 9300
atgccggagc tcgtacgttc gaacttaagg cctcgtcccc gccgggtcac ccggccagcg 9360
acatggaggc ccagaatacc ctccttgaca gtcttgacgt gcgcagctca ggggcatgat 9420
gtgactgtcg cccgtacatt tagcccatac atccccatgt ataatcattt gcatccatac 9480
attttgatgg ccgcacggcg cgaagcaaaa attacggctc ctcgctgcag acctgcgagc 9540
agggaaacgc tcccctcaca gacgcgttga attgtcccca cgccgcgccc ctgtagagaa 9600
atataaaagg ttaggatttg ccactgaggt tcttctttca tatacttcct tttaaaatct 9660
tgctaggata cagttctcac atcacatccg aacataaaca accatgggta aggaaaagac 9720
tcacgtttcg aggccgcgat taaattccaa catggatgct gatttatatg ggtataaatg 9780
ggctcgcgat aatgtcgggc aatcaggtgc gacaatctat cgattgtatg ggaagcccga 9840
tgcgccagag ttgtttctga aacatggcaa aggtagcgtt gccaatgatg ttacagatga 9900
gatggtcaga ctaaactggc tgacggaatt tatgcctctt ccgaccatca agcattttat 9960
ccgtactcct gatgatgcat ggttactcac cactgcgatc cccggcaaaa cagcattcca 10020
ggtattagaa gaatatcctg attcaggtga aaatattgtt gatgcgctgg cagtgttcct 10080
gcgccggttg cattcgattc ctgtttgtaa ttgtcctttt aacagcgatc gcgtatttcg 10140
tctcgctcag gcgcaatcac gaatgaataa cggtttggtt gatgcgagtg attttgatga 10200
cgagcgtaat ggctggcctg ttgaacaagt ctggaaagaa atgcataagc ttttgccatt 10260
ctcaccggat tcagtcgtca ctcatggtga tttctcactt gataacctta tttttgacga 10320
ggggaaatta ataggttgta ttgatgttgg acgagtcgga atcgcagacc gataccagga 10380
tcttgccatc ctatggaact gcctcggtga gttttctcct tcattacaga aacggctttt 10440
tcaaaaatat ggtattgata atcctgatat gaataaattg cagtttcatt tgatgctcga 10500
tgagtttttc taatcagtac tgacaataaa aagattcttg ttttcaagaa cttgtcattt 10560
gtatagtttt tttatattgt agttgttcta ttttaatcaa atgttagcgt gatttatatt 10620
tttttttcgcc tcgacatcat ctgcccagat gcgaagttaa gtgcgcagaa agtaatatca 10680
tgcgtcaatc gtatgtgaat gctggtcgct atactgctgt cgattcgata ctaacgccgc 10740
catccagtgt cgacggatcc taggtgtaca taaactttat aaatgaaatt cataatagaa 10800
acgacacgaa attacaaaat ggaatatgtt catagggtag acgaaactat atacgcaatc 10860
tacatacatt tatcaagaag gagaaaaagg aggatagtaa a 10901

<210> 57

<211> 9948
<212> DNA
<213> Artificial

<220>
<223> pRN907

<400> 57
tctcagaatg acttggttga gtactcacca gtcacagaaa agcatcttac ggatggcatg 60

acagtaagag aattatgcag tgctgccata accatgagtg ataacactgc ggccaactta 120

cttctgacaa cgatcggagg accgaaggag ctaaccgctt ttttcacaa catgggggat 180

catgtaactc gccttgatcg ttgggaaccg gagctgaatg aagccatacc aaacgacgag 240

cgtgacacca cgatgcctgt agcaatggca acaacgttgc gcaaactatt aactggcgaa 300

ctacttactc tagcttcccg gcaacaatta atagactgga tggaggcgga taaagttgca 360

ggaccacttc tgcgctcggc ccttccggct ggctggttta ttgctgataa atctggagcc 420

ggtgagcgtg ggtctcgcgg tatcattgca gcactggggc cagatggtaa gccctcccgt 480

atcgtagtta tctacacgac gggcagtcag gcaactatgg atgaacgaaa tagacagatc 540

gctgagatag gtgcctcact gattaagcat tggtaactgt cagaccaagt ttactcatat 600

atactttaga ttgatttaaa acttcatttt taatttaaaa ggatctaggt gaagatcctt 660

tttgataatc tcatgaccaa aatcccttaa cgtgagtttt cgttccactg agcgtcagac 720

cccgtagaaa agatcaaagg atcttcttga gatccttttt ttctgcgcgt aatctgctgc 780

ttgcaaacaa aaaaaccacc gctaccagcg gtggtttgtt tgccggatca agagctacca 840

actctttttc cgaaggtaac tggcttcagc agagcgcaga taccaaatac tgtccttcta 900

gtgtagccgt agttaggcca ccacttcaag aactctgtag caccgcctac atacctcgct 960

ctgctaatcc tgttaccagt ggctgctgcc agtggcgata agtcgtgtct taccgggttg 1020

gactcaagac gatagttacc ggataaggcg cagcggtcgg gctgaacggg gggttcgtgc 1080

acacagccca gcttggagcg aacgacctac accgaactga gatacctaca gcgtgagcat 1140

tgagaaagcg ccacgcttcc cgaagggaga aaggcggaca ggtatccggt aagcggcagg 1200

gtcggaacag gagagcgcac gagggagctt ccagggggga acgcctggta tctttatagt 1260

cctgtcgggt ttcgccacct ctgacttgag cgtcgatttt tgtgatgctc gtcagggggg 1320

cggagcctat ggaaaaacgc cagcaacgcg gcctttttac ggttcctggc cttttgctgg 1380

ccttttgctc acatgttctt tcctgcgtta tcccctgatt ctgtggataa ccgtattacc 1440

gcctttgagt gagctgatac cgctcgccgc agccgaacga ccgagcgcag cgagtcagtg 1500

agcgaggaag cggaagagcg cccaatacgc aaaccgcctc tccccgcgcg ttggccgatt 1560

cattaatgca gctggcacga caggtttccc gactggaaag cgggcagtga gcgcaacgca 1620

attaatgtga gttacctcac tcattaggca ccccaggctt tacactttat gcttccggct 1680

cctatgttgt gtggaattgt gagcggataa caatttcaca caggaaacag ctatgaccat 1740

gattacgcca agctcggaat taaccctcac taaagggaac aaaagctggg tacctaggac 1800

cggtttatca ttatcaatac tgccatttca aagaatacgt aaataattaa tagtagtgat 1860

tttcctaact ttatttagtc aaaaaattag ccttttaatt ctgctgtaac ccgtacatgc 1920

ccaaaatagg gggcgggtta cacagaatat ataacatcgt aggtgtctgg gtgaacagtt 1980

tattcctggc atccactaaa tataatggag cccgcttttt aagctggcat ccagaaaaaa 2040

aaagaatccc agcaccaaaa tattgttttc ttcaccaacc atcagttcat aggtccattc 2100

tcttagcgca actacagaga acaggggcac aaacaggcaa aaaacgggca caacctcaat 2160

ggagtgatgc aacctgcctg gagtaaatga tgacacaagg caattgaccc acgcatgtat 2220

ctatctcatt ttcttacacc ttctattacc ttctgctctc tctgatttgg aaaaagctga 2280

aaaaaaaggt tgaaaccagt tccctgaaat tattccccta cttgactaat aagtatataa 2340

agacggtagg tattgattgt aattctgtaa atctatttct taaacttctt aaattctact 2400

tttatagtta gtcttttttt tagttttaaa acaccaagaa cttagtttcg aataaacaca 2460

cataaagaat tcaaaatgtc ttggccaacc gactctgaat tgaactctat caaggaagct 2520

gttgctcaaa tgtctggtag agacaagggt gaagttagag ttgttgttgc tccatacaga 2580

atctgtccat tgggtgctca catcgaccac caaggtggta ctgtttctgc tatgaccatc 2640

aacaagggta tcttgttggg tttcgttcca tctggtgaca cccaagttca attgagatct 2700

gctcaattcg aaggtgaagt ttgtttcaga gttgacgaaa tccaacaccc aatcggtttg 2760

gctaacaaga acggtgcttc taccccatct ccatctaagg aaaagtctat ctggggtact 2820

tacgctagag gtgctgttta cgctttgcaa tcttctaaga agaacttgaa gcaaggtatc 2880

atcggttact tgtctggttc taacggtttg gactcttcag gtttatcttc ttctgctgct 2940

gttggtgttg cttacttgtt agctttggaa aacgctaacg aattgaccgt ttctccaacc 3000

gaaaacatcg aatacgacag attgatcgaa aacggttact tgggtttgag aaacggtatc 3060

ttggaccaat ctgctatctt gttgtctaac tacggttgtt tgacctacat ggactgtaag 3120

accttggacc acgaattggt tcaagctcca gaattggaaa agccattcag aatcttgttg 3180

gctttctctg gtttgagaca agctttgacc accaacccag gtttcaactt gagagtttct 3240

gaatgtcaag aagctgctaa ggttttgttg accgcttcag gtaactctga attggaacca 3300

accttgtgta acgttgaaca cgctgtttac gaagctcaca agcacgaatt gaagcctgtt 3360

ttggctaaga gagctgaaca ctacttctct gaaaacatga gagttatcaa gggtagagaa 3420

gcttgggctt ctggtaactt ggaagaattt ggtaagttga tctctgcttc tggtttgtct 3480

tctatcgaaa actacgaatg tggtgctgaa ccattgatcc aattgtacaa gattttgttg 3540

aaggctccag gtgtttacgg tgctagattc tctggtgctg gtttcagagg ttgttgtttg 3600

gctttcgttg acgctgaaaa ggctgaagct gctgcttctt acgttaagga cgaatacgaa 3660

aaggctcaac cagaatttgc taacaacttg aacggtggta agccagtttt gatctgtgaa 3720
gctggtgacg ctgctagagt tttgttgtaa ggatcccctt ttcctttgtc gatatcatgt 3780
aattagttat gtcacgctta cattcacgcc ctcctcccac atccgctcta accgaaaagg 3840
aaggagttag acaacctgaa gtctaggtcc ctatttattt tttttaatag ttatgttagt 3900
attaagaacg ttatttatat ttcaaatttt tctttttttt ctgtacaaac gcgtgtacgc 3960
atgtaacatt atactgaaaa ccttgcttga gaaggttttg ggacgctcga aggcttccta 4020
ggctcgagcc taggcgtgtg gaagaacgat tacaacaggt gttgtcctct gaggacataa 4080
aatacacacc gagattcatc aactcattgc tggagttagc atatctacaa ttgggtgaaa 4140
tggggagcga tttgcaggca tttgctcggc atgccggtag aggtgtggtc aataagagcg 4200
acctcatgct atacctgaga aagcaacctg acctacagga aagagttact caagaataag 4260
aattttcgtt ttaaaaccta agagtcactt taaaatttgt atacacttat ttttttttata 4320
acttatttaa taataaaaat cataaatcat aagaaattcg cgcgctcaat gttccaagat 4380
ttgcaaatta ccagtaacga caacattttc caatatggag ccgtttggaa tatcgatttt 4440
atgaccgtcg gagcaaacga tgatgacagt acccctcaaa gtgacatctt tacctaaaaa 4500
gacgttacca gtgatggtca aatgatctag ctcgacgatt tttgggatgt gagggattct 4560
tgcgttaaaa ccagaaacct ttttgaaatg cgagcccaac ttgattaatg ggtttggacc 4620
aaaacgggat gggtctaact tcaaagaacc gtgttccaga cggaatagat ctgatttaac 4680
cagcaacaaa tcggaacagg tcttgacagg caagaatctt gatcttggaa cgacaacacc 4740
gtgagcacca tcaaaatgcc tgatagcagc accacaagcg gtttctaatt gtaagacatt 4800
aatttcatga ccgtctcttg ttatagtttt ttggtttgga atgatttcca tctccaaatt 4860
gctcgattcg atcaacctct ttactgcttt cagattgatc cataagttat tcgtgttgaa 4920
gttggtaaac tttctgatat ttttgaattc gtcaatgtgt tcttttggaa cttgggcgac 4980
ttccaataaa cggacttgac catcgtaaga aatcaaagta ccaccttttaa catcggctct 5040
ggtcttatca gtcaattcca ttatatattc ggcaccagtc tcgatcatgt ggtttaaaat 5100
ttttaagtcg acggtagcac ccaagttgtc accgttagaa acaaataata tttctcttcc 5160
ttgggcaatt aaggcatcca gttcaccaga tacgtgtaaa gattcaaaca aatcaccgtg 5220
acctggtgga taccaagcat ccagtggaga atcgtattcg gtggggacag gcaataaaga 5280
atccttgtag actcttggga acctggattg attgaaagat ctgattctga ttctgttagc 5340
ggaatacttc ttaatcaagt gttccgtatc cttgtcagtg ttgaaagaat tcatcaataa 5400
caatggcacg tcgctatcgt actgtctgtt caagtattca atttgacgaa cagacaaatc 5460
caaaaaggtg tttccctctc tcacttcaat aacagattta gggccaacgc agcccatgga 5520
ggtacccagc ccaccgttca acttcaaaac agccaatttg gaaaggtttg agacattctc 5580

gggctgctga gaaataattt catacttaac cacttcatcc gggttgggag acttgatctt 5640
gtcccattcc aaggtggttc tagaagactt ctctaccaaa tatctcctga aaagcgtgaa 5700
aaacgaatcc agttcgttct caaacttagc gcgagcagca tcgtcaagtt tactagagtc 5760
cgccaacttg tttaaggcgt ttctcatttg tgaggcagca acgctgtttg tgttgctctc 5820
gaatgcataa gtggaatgtg ttttggtgtg cttcttagtg gacattttct gcagtctaga 5880
tatatttgtt gtaaaaagta gataattact tccttgatga tctgtaaaaa agagaaaaag 5940
aaagcatcta agaacttgaa aaactacgaa ttagaaaaga ccaaatatgt atttcttgca 6000
ttgaccaatt tatgcaagtt tatatatatg taaatgtaag tttcacgagg ttctactaaa 6060
ctaaaccacc cccttggtta gaagaaaaga gtgtgtgaga acaggctgtt gttgtcacac 6120
gattcggaca attctgtttg aaagagagag agtaacagta cgatcgaacg aactttgctc 6180
tggagatcac agtgggcatc atagcatgtg gtactaaacc ctttcccgcc attccagaac 6240
cttcgattgc ttgttacaaa acctgtgagc cgtcgctagg accttgttgt gtgacgaaat 6300
tggaagctgc aatcaatagg aagacaggaa gtcgagcgtg tctgggtttt ttcagttttg 6360
ttctttttgc aaacaaatca cgagcgacgg taatttcttt ctcgataaga ggccacgtgc 6420
tttatgaggg taacatcaat tcaagaagga gggaaacact tcctttttct ggccctgata 6480
atagtatgag ggtgaagcca aaataaagga ttcgcgccca aatcggcatc tttaaatgca 6540
ggtatgcgat agttcctcac tctttcctta ctcacgagta attcttgcaa atgcctatta 6600
tgcagatgtt ataatatctg tgcgtcttga gttgaagagc tcgtacgttc gaacttaagg 6660
cctcgtcccc gccgggtcac ccggccagcg acatggaggc ccagaatacc ctccttgaca 6720
gtcttgacgt gcgcagctca ggggcatgat gtgactgtcg cccgtacatt tagcccatac 6780
atccccatgt ataatcattt gcatccatac attttgatgg ccgcacggcg cgaagcaaaa 6840
attacggctc ctcgctgcag acctgcgagc agggaaacgc tcccctcaca gacgcgtgaa 6900
ttgtccccac gccgcgcccc tgtagagaaa tataaaaggt taggatttgc cactgaggtt 6960
cttctttcat atacttcctt ttaaaatctt gctaggatac agttctcaca tcacatccga 7020
acataaacaa ccatgggtaa aaagcctgaa ctcaccgcga cgtctgtcga gaagtttctg 7080
atcgaaaagt tcgacagcgt ctccgacctg atgcagctct cggagggcga agaatctcgt 7140
gctttcagct tcgatgtagg agggcgtgga tatgtcctgc gggtaaatag ctgcgccgat 7200
ggtttctaca aagatcgtta tgtttatcgg cactttgcat cggccgcgct cccgattccg 7260
gaagtgcttg acattgggga attcagcgag agcctgacct attgcatctc ccgccgtgca 7320
cagggtgtca cgttgcaaga cctgcctgaa accgaactgc ccgctgttct gcagccggtc 7380
gcggaggcca tggatgcgat cgctgcggcc gatcttagcc agacgagcgg gttcggccca 7440
ttcggaccgc aaggaatcgg tcaatacact acatggcgtg atttcatatg cgcgattgct 7500

gatccccatg tgtatcactg gcaaactgtg atggacgaca ccgtcagtgc gtccgtcgcg 7560
caggctctcg atgagctgat gctttgggcc gaggactgcc ccgaagtccg gcacctcgtg 7620
cacgcggatt tcggctccaa caatgtcctg acggacaatg gccgcataac agcggtcatt 7680
gactggagcg aggcgatgtt cggggattcc caatacgagg tcgccaacat cttcttctgg 7740
aggccgtggt tggcttgtat ggagcagcag acgcgctact tcgagcggag gcatccggag 7800
cttgcaggat cgccgcggct ccgggcgtat atgctccgca ttggtcttga ccaactctat 7860
cagagcttgg ttgacggcaa tttcgatgat gcagcttggg cgcagggtcg atgcgacgca 7920
atcgtccgat ccggagccgg gactgtcggg cgtacacaaa tcgcccgcag aagcgcggcc 7980
gtctggaccg atggctgtgt agaagtactc gccgatagtg gaaaccgacg ccccagcact 8040
cgtccgaggg caaaggaata atcagtactg acaataaaaa gattcttgtt ttcaagaact 8100
tgtcatttgt atagtttttt tatattgtag ttgttctatt ttaatcaaat gttagcgtga 8160
tttatatttt ttttcgcctc gacatcatct gcccagatgc gaagttaagt gcgcagaaag 8220
taatatcatg cgtcaatcgt atgtgaatgc tggtcgctat actgctgtcg attcgatact 8280
aacgccgcca tccagtgtcg acggatccta ggtgtacata aactttataa atgaaattca 8340
taatagaaac gacacgaaat tacaaaatgg aatatgttca tagggtagac gaaactatat 8400
acgcaatcta catacattta tcaagaagga gaaaaaggag gatagtaaag gaatacaggt 8460
aagcaaattg atactaatgg ctcaacgtga taaggaaaaa gaattgcact ttaacattaa 8520
tattgacaag gaggagggca ccacacaaaa agttaggtgt aacagaaaat catgaaacta 8580
cgattcctaa tttgatattg gaggattttc tctaaaaaaa aaaaaataca acaaataaaa 8640
aacactcaat gacctgacca tttgatggag tttaagtcaa taccttcttg aaccatttcc 8700
cataatggtg aaagttccct caagaatttt actctgtcag aaacggcctt acgacgtagt 8760
cgatatggtg cactctcagt acaatctgct ctgatgccgc atagttaagc cagccccgac 8820
acccgccaac acccgctgac gcgccctgac gggcttgtct gctcccggca tccgcttaca 8880
gacaagctgt gaccgtctcc gggagctgca tgtgtcagag gttttcaccg tcatcaccga 8940
aacgcgcgag acgaaagggc ctcgtgatac gcctattttt ataggttaat gtcatgataa 9000
taatggtttc ttaggacgga tcgcttgcct gtaacttaca cgcgcctcgt atcttttaat 9060
gatggaataa tttgggaatt tactctgtgt ttatttattt ttatgttttg tatttggatt 9120
ttagaaagta aataaagaag gtagaagagt tacggaatga agaaaaaaaa ataaacaaag 9180
gtttaaaaaa tttcaacaaa aagcgtactt tacatatata tttattagac aagaaaagca 9240
gattaaatag atatacattc gattaacgat aagtaaaatg taaaatcaca ggattttcgt 9300
gtgtggtctt ctacacagac aagatgaaac aattcggcat taatacctga gagcaggaag 9360
agcaagataa aaggtagtat ttgttggcga tccccctaga gtcttttaca tcttcggaaa 9420

acaaaaacta ttttttcttt aatttctttt tttactttct atttttaatt tatatattta 9480

tattaaaaaa tttaaattat aattattttt atagcacgtg atgaaaagga cccaggtggc 9540

acttttcggg gaaatgtgcg cggaacccct atttgtttat ttttctaaat acattcaaat 9600

atgtatccgc tcatgagaca ataaccctga taaatgcttc aataatattg aaaaaggaag 9660

agtatgagta ttcaacattt ccgtgtcgcc cttattccct tttttgcggc attttgcctt 9720

cctgtttttg ctcacccaga aacgctggtg aaagtaaaag atgctgaaga tcagttgggt 9780

gcacgagtgg gttacatcga actggatctc aacagcggta agatccttga gagttttcgc 9840

cccgaagaac gttttccaat gatgagcact tttaaagttc tgctatgtgg cgcggtatta 9900

tcccgtattg acgccgggca agagcaactc ggtcgccgca tacactat 9948

<210> 58
<211> 2573
<212> DNA
<213> Artificial

<220>
<223> GDP1 disruption

<400> 58
ggtacccccg ccttgcttct ctccccttcc ttttcttttt ccagttttcc ctattttgtc 60

cctttttccg cacaacaagt atcagaatgg gttcatcaaa tctatccaac ctaattcgca 120

cgtagactgg cttggtattg gcagtttcgt agttatatat atactaccat gagtgaaact 180

gttacgttac cttaaattct ttctcccttt aattttcttt tatcttactc tcctacataa 240

gacatcaaga aacaattgta tattgtacac cccccccctc cacaaacaca aatattgata 300

atataaagat gtctgctgct gctgatagat taaacttaac ttccggccac ttgaatctag 360

atgcatgctc gagcggccgc cagtgtgatg gatatctgca gaattcgccc ttttgggccc 420

tgtacaccta ggatccgtcg acactggatg gcggcgttag tatcgaatcg acagcagtat 480

agcgaccagc attcacatac gattgacgca tgatattact ttctgcgcac ttaacttcgc 540

atctgggcag atgatgtcga ggcgaaaaaa aatataaatc acgctaacat ttgattaaaa 600

tagaacaact acaatataaa aaaactatac aaatgacaag ttcttgaaaa caagaatctt 660

tttattgtca gtactgatta ttcctttgcc ctcggacgag tgctggggcg tcggtttcca 720

ctatcggcga gtacttctac acagccatcg gtccagacgg ccgcgcttct gcgggcgatt 780

tgtgtacgcc cgacagtccc ggctccggat cggacgattg cgtcgcatcg accctgcgcc 840

caagctgcat catcgaaatt gccgtcaacc aagctctgat agagttggtc aagaccaatg 900

cggagcatat acgcccggag ccgcggcgat cctgcaagct ccggatgcct ccgctcgaag 960

tagcgcgtct gctgctccat acaagccaac cacggcctcc agaagaagat gttggcgacc 1020

tcgtattggg aatccccgaa catcgcctcg ctccagtcaa tgaccgctgt tatgcggcca 1080

ttgtccgtca ggacattgtt ggagccgaaa tccgcgtgca cgaggtgccg gacttcgggg 1140

cagtcctcgg cccaaagcat cagctcatcg agagcctgcg cgacggacgc actgacggtg 1200

tcgtccatca cagtttgcca gtgatacaca tggggatcag caatcgcgca tatgaaatca 1260

cgccatgtag tgtattgacc gattccttgc ggtccgaatg ggccgaaccc gctcgtctgg 1320

ctaagatcgg ccgcagcgat cgcatccatg gcctccgcga ccggctgcag aacagcgggc 1380

agttcggttt caggcaggtc ttgcaacgtg acaccctgtg cacggcggga gatgcaatag 1440

gtcaggctct cgctgaattc cccaatgtca agcacttccg gaatcgggag cgcggccgat 1500

gcaaagtgcc gataaacata acgatctttg tagaaaccat cggcgcagct atttacccgc 1560

aggacatatc cacgccctcc tacatcgaag ctgaaagcac gagattcttc gccctccgag 1620

agctgcatca ggtcggagac gctgtcgaac ttttcgatca gaaacttctc gacagacgtc 1680

gcggtgagtt caggcttttt acccatggtt gtttatgttc ggatgtgatg tgagaactgt 1740

atcctagcaa gattttaaaa ggaagtatat gaaagaagaa cctcagtggc aaatcctaac 1800

cttttatatt tctctacagg ggcgcggcgt ggggacaatt cacgcgtctg tgaggggagc 1860

gtttccctgc tcgcaggtct gcagcgagga gccgtaattt ttgcttcgcg ccgtgcggcc 1920

atcaaaatgt atggatgcaa atgattatac atggggatgt atgggctaaa tgtacgggcg 1980

acagtcacat catgcccctg agctgcgcac gtcaagactg tcaaggaggg tattctgggc 2040

ctccatgtcg ctggccgggt gacccggcgg ggacgaggcc ttaagttcga acgtacggag 2100

ttgttgaatg gccaatccgc tcaaggttta attacctgca aagaagttca cgaatggttg 2160

gaaacatgtg gctctgtcga agacttccca ttatttgaag ccgtatacca aatcgtttac 2220

aacaactacc caatgaagaa cctgccggac atgattgaag aattagatct acatgaagat 2280

tagatttatt ggagaaagat aacatatcat actttccccc actttttttcg aggctcttct 2340

atatcatatt cataaattag cattatgtca tttctcataa ctactttatc acgttagaaa 2400

ttacttatta ttattaaatt aatacaaaat ttagtaacca aataaatata aataaatatg 2460

tatatttaaa ttttaaaaaa aaaatcctat agagcaaaag gattttccat tataatatta 2520

gctgtacacc tcttccgcat tttttgaggg tggttacaac accactcggt acc 2573

<210> 59
<211> 2058
<212> DNA
<213> Artificial

<220>
<223> GPD2 disruption

<400> 59
agatcttttg cggcgaggtg ccgatgggtt gctgagggga agagtgttta gcttacggac 60

ctattgccat tgttattccg attaatctat tgttcagcag ctcttctcta ccctgtcatt 120

ctagtatttt tttttttttt ttttggtttt actttttttt cttcttgcct ttttttcttg 180

ttactttttt tctagttttt tttccttcca ctaagctttt tccttgattt atccttgggt 240

tcttctttct actcctttag attttttttt tatatattaa tttttaagtt tatgtatttt 300

ggtagattca attctctttc cctttccttt tccttcgctc cccttcctta tcaatgcttg 360

ctgtcagaag attaacaaga tacacattcc ttaaggcctc gtccccgccg ggtcacccgg 420

ccagcgacat ggaggcccag aataccctcc ttgacagtct tgacgtgcgc agctcagggg 480

catgatgtga ctgtcgcccg tacatttagc ccatacatcc ccatgtataa tcatttgcat 540

ccatacattt tgatggccgc acggcgcgaa gcaaaaatta cggctcctcg ctgcagacct 600

gcgagcaggg aaacgctccc ctcacagacg cgttgaattg tccccacgcc gcgcccctgt 660

agagaaatat aaaaggttag gatttgccac tgaggttctt ctttcatata cttcctttta 720

aaatcttgct aggatacagt tctcacatca catccgaaca taaacaacca tgtaaaatga 780

ccactcttga cgacacggct taccggtacc gcaccagtgt cccgggggac gccgaggcca 840

tcgaggcact ggatgggtcc ttcaccaccg acaccgtctt ccgcgtcacc gccaccgggg 900

acggcttcac cctgcgggag gtgccggtgg acccgcccct gaccaaggtg ttccccgacg 960

acgaatcgga cgacgaatcg gacgccgggg aggacggcga cccggactcc cggacgttcg 1020

tcgcgtacgg ggacgacggc gacctggcgg gcttcgtggt cgtctcgtac tccggctgga 1080

accgccggct gaccgtcgag gacatcgagg tcgccccgga gcaccggggg cacggggtcg 1140

ggcgcgcgtt gatggggctc gcgacggagt tcgcccgcga gcggggcgcc gggcacctct 1200

ggctggaggt caccaacgtc aacgcaccgg cgatccacgc gtaccggcgg atggggttca 1260

ccctctgcgg cctggacacc gccctgtacg acggcaccgc ctcggacggc gagcaggcgc 1320

tctacatgag catgccctgc ccctagtact gacaataaaa agattcttgt tttcaagaac 1380

ttgtcatttg tatagttttt ttatattgta gttgttctat tttaatcaaa tgttagcgtg 1440

atttatattt tttttcgcct cgacatcatc tgcccagatg cgaagttaag tgcgcagaaa 1500

gtaatatcat gcgtcaatcg tatgtgaatg ctggtcgcta tactgctgtc gattcgatac 1560

taacgccgcc atccagtgtc gacggatcct aggtgtacag ggcccaaaag ggcgaattct 1620

gcagatatcc atcacactgg cggccgctcg aggatagtct acaacaacgt ccgcatggaa 1680

gacctaccgg agatgattga agagctagac atcgatgacg aatagacact ctcccccccc 1740

ctccccctct gatctttcct gttgcctctt tttcccccaa ccaatttatc attatacaca 1800

agttctacaa ctactactag taacattact acagttatta taatttttcta ttctcttttt 1860

ctttaagaat ctatcattaa cgttaatttc tatatataca taactaccat tatacacgct 1920

attatcgttt acatatcaca tcaccgttaa tgaaagatac gacaccctgt acactaacac 1980

aattaaataa tcgccataac cttttctgtt atctatagcc cttaaagctg tttcttcgag 2040

```
ctttttcact gcagatct                                                  2058

<210>  60
<211>  826
<212>  DNA
<213>  Saccharomyces cerevisiae

<400>  60
gagctcgtac gcatatgatc atgtgtcgtc gcacacatat atatatgcct gtatgtgtca     60

gcactaaagt tgcctggcca tccacgctat atatacacgc ctggcggatc tgctcgagga    120

ttgcctacgc gtgggcttga tccaccaacc aacgctcgcc aaatgaactg gcgctttggt    180

cttctgccat cgtccgtaaa ccccggccaa agagaccgga aagatcggtg aaaacatctt    240

gatcttgctc ccgggaattt tagattcagg taggaaattg attacatcaa tactgttacc    300

ctgaatcata ttcgacgatg tcgtctcaca cggaaatata attcatttct tggttttcca    360

aaaaaatttt catttttttt cacttttttg tttcgtcctc cttttttttt tttttttttt    420

attttttttc ctgtgttcac cttttttttt ttcagttgac atctttctgc attcttttct    480

gtgttttttt tttttttttt cgtttttcca ttgttcgttc gttgcctgtt ttttcgccct    540

attgttctcg agcctaaaaa ttttttcctt tcctgctttc ctttcttcgt tcaaagtttc    600

ctattccatt gttctctttg gtaaactcat tgttgtcgga actcagatat attcaggtca    660

atttactgta cttcaattga ctttttctt gaaatttcaa cttgcctttt caacttgttc    720

ttctttttta atcttattct acactttagt tcccttacct tgttcctaat tattgtctag    780

caaaaagaaa acatacacct atttcattca cacactgcag aaaatg                   826
```

## Description

Field of the invention

The present invention relates to metabolic engineering in microorganisms such as yeast. In particular the invention relates to yeast strains that have been engineered to produce fermentation products from uronic acids such as galacturonic acid or glucuronic acid. These strains have retained their natural ability to ferment hexoses (glucose, fructose, galactose, etc) and may further comprise an engineered ability to ferment pentoses like xylose and arabinose. The invention further relates to the processes wherein the engineered strains of the invention produce fermentation products such as ethanol from uronic acids, either as main fermentation feedstock, or concomitantly with one or more of hexoses, pentoses and glycerol.

Background of the invention

There is a need for bioproduction of fuels or chemicals from second generation feedstocks. Second generation feedstocks may contain, apart from cellulose, also hemicelluloses and/or pectin. Hemicellulose contains mainly the pentose sugars xylose and arabinose, but also glucuronate. Pectin contains apart from various sugars, vast quantities of galacturonate. Pectin-rich residues become available as by-product when sugar is extracted from e.g. sugar beet or when juices are produced from citrus fruits. The uronic acids glucuronate and galacturonate are thus available as important constituents in second generation feedstocks, however, they cannot be metabolized by industrially important yeasts including *Saccharomyces cerevisiae* e.g. for the production of fuel ethanol.

No suggestions have been reported for engineering *S. cerevisiae* to introduce the ability to ferment glucuronate into ethanol. For galacturonate, however, three routes have been suggested that potentially may result in the formation of ethanol from galacturonate in *S. cerevisiae* (Richard and Hilditch, 2009, Appl Microbiol Biotechnol 82:597-604; Hilditch, Thesis 2010, VTT publications 739, ISBN 978-951-38-7398-1): two bacterial routes and one fungal route. However, for none of these three routes, it has been demonstrated that ethanol is actually formed by *S. cerevisiae* that is transformed with the respective sets of genes.

There is therefore still a need in the art for yeasts engineered to produce fermentation products such as ethanol from uronic acids such as galacturonic acid or glucuronic acid. It is an object of the invention to provide such yeasts, as well as processes wherein they are used to produce fermentation products such as ethanol from these uronic acids.

Summary of the invention

In a first embodiment, the invention relates to a yeast cell having the ability of metabolizing a uronic acid. For this purpose the yeast cell has been genetically modified to comprise and express genes coding for: a) a uronokinase (EC 2.7.1.43 and/or EC 2.7.1.44); b) a UTP-monosaccharide-1-phosphate uridylyltransferase (EC 2.7.7.64); and, c) a UDP-glucose 6-dehydrogenase (EC 1.1.1.22). Preferably the yeast cell of the invention further comprises and expresses a gene coding for: d) a UDP-glucuronate-4-epimerase (EC 5.1.3.6).

In another embodiment, the yeast cell of the invention further expresses or overexpresses genes coding for at least one of: i) a galactose-1-phosphate uridyltransferase (EC 2.7.7.12); and ii) a UDP-glucose-4-epimerase (EC 5.1.3.2). Preferably, the galactose-1-phosphate uridyltransferase and the UDP-glucose-4-epimerase are expressed in a galactose-independent manner.

In another embodiment, the yeast cell of the invention further comprises a genetic modification that increases the specific activity of at least one of a UDP-glucose pyrophosphorylase (EC 2.7.7.9) and a phosphoglucomutase (EC 5.4.2.2). Preferably, the phosphoglucomutase is expressed in a galactose-independent manner.

In a another embodiment, the yeast cell of the invention can further comprises a genetic modification that increases at least one of: i) the specific activity of glycerol dehydrogenase; ii) the specific activity of dihydroxyacetone kinase; and, iii) transport of glycerol into the cell.

In a another embodiment, the yeast cell of the invention can further comprises a genetic modification that inactivates or reduces the specific activity of one or more enzymes required for NADH-dependent synthesis of glycerol, compared to a corresponding wild-type cell.

In a another embodiment, the yeast cell of the invention further preferably comprises genes coding for enzymes with at least one of the abilities to: i) directly isomerise xylose into xylulose (EC 5.3.1.5); and, ii) convert L-arabinose into D-xylulose 5-phosphate, wherein the enzymes comprise a L-arabinose isomerase (EC 5.3.1.3), a L-ribulokinase (EC 2.7.1.16) and a L-ribulose-5-phosphate 4-epimerase (EC 5.1.3.4).

A yeast cell according to the invention, preferably is a yeast cell selected from the genera *Saccharomyces, Kluyveromyces, Candida, Pichia, Schizosaccharomyces, Hansenula, Kloeckera, Schwanniomyces, Brettanomyces*, and *Yarrowia*, in particular the cell is a yeast cell selected from the species *S. cerevisiae, S. exiguus, S. bayanus, K. lactis,*

*K. marxianus* and *Schizosaccharomyces pombe*.

In a another embodiment, the invention relates to use of a yeast cell according to the invention for the preparation of a fermentation product, wherein preferably the fermentation product selected from the group consisting of ethanol, lactic acid, 3-hydroxy-propionic acid, acrylic acid, 1,3-propane-diol, butanols and isoprenoid-derived products.

In a another embodiment, the invention relates to a process for producing a fermentation product, whereby the process comprises the steps of: a) fermenting a medium with a yeast cell of the invention, whereby the medium contains or is fed with a source of a uronic acid and whereby the yeast cell ferments the uronic acid to the fermentation product; and optionally, b) recovery of the fermentation product, wherein preferably the fermentation product selected from the group consisting of ethanol, lactic acid, 3-hydroxy-propionic acid, acrylic acid, 1,3-propane-diol, a butanol and an isoprenoid-derived product. Preferably, in the process of the invention, the uronic acid is at least one of galacturonic acid and glucuronic acid.

In other embodiments of the process of the invention, medium further contains or is fed with a carbon source having a more reduced state than glucose, and/or the medium contains or is fed with a source of at least one of a hexose, a pentose, and acetic acid.

In a preferred embodiment of the process of the invention, the yeast cell ferments under anaerobic conditions or under oxygen-limited aerobic conditions.

In a further preferred embodiment of the process of the invention, the fermentation product is ethanol.

Description of the invention

Definitions

Sequence identity is herein defined as a relationship between two or more amino acid (polypeptide or protein) sequences or two or more nucleic acid (polynucleotide) sequences, as determined by comparing the sequences. In the art, "identity" also means the degree of sequence relatedness between amino acid or nucleic acid sequences, as the case may be, as determined by the match between strings of such sequences. "Similarity" between two amino acid sequences is determined by comparing the amino acid sequence and its conserved amino acid substitutes of one polypeptide to the sequence of a second polypeptide. "Identity" and "similarity" can be readily calculated by known methods. The terms "sequence identity" or "sequence similarity" means that two (poly)peptide or two nucleotide sequences, when optimally aligned, preferably over the entire length (of at least the shortest sequence in the comparison) and maximizing the number of matches and minimizes the number of gaps such as by the programs ClustalW (1.83), GAP or BESTFIT using default parameters, share at least a certain percentage of sequence identity as defined elsewhere herein. GAP uses the Needleman and Wunsch global alignment algorithm to align two sequences over their entire length, maximizing the number of matches and minimizes the number of gaps. Generally, the GAP default parameters are used, with a gap creation penalty = 50 (nucleotides) / 8 (proteins) and gap extension penalty = 3 (nucleotides) / 2 (proteins). For nucleotides the default scoring matrix used is nwsgapdna and for proteins the default scoring matrix is Blosum62 (Henikoff & Henikoff, 1992, PNAS 89, 915-919). A preferred multiple alignment program for aligning protein sequences of the invention is ClustalW (1.83) using a blosum matrix and default settings (Gap opening penalty:10; Gap extension penalty: 0.05). It is clear than when RNA sequences are said to be essentially similar or have a certain degree of sequence identity with DNA sequences, thymine (T) in the DNA sequence is considered equal to uracil (U) in the RNA sequence. Sequence alignments and scores for percentage sequence identity may be determined using computer programs, such as the GCG Wisconsin Package, Version 10.3, available from Accelrys Inc., 9685 Scranton Road, San Diego, CA 92121-3752 USA or the open-source software Emboss for Windows (current version 2.10.0-0.8). Alternatively percent similarity or identity may be determined by searching against databases such as FASTA, BLAST, etc.

A variant of a nucleotide or amino acid sequence disclosed herein may also be defined as a nucleotide or amino acid sequence having one or several substitutions, insertions and/or deletions as compared to the nucleotide or amino acid sequence specifically disclosed herein (e.g. in de the sequence listing).

Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alanine-valine, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for each of the naturally occurring amino acids are as follows: Ala to ser; Arg to lys; Asn to gln or his; Asp to glu; Cys to ser or ala; Gln to asn; Glu to asp; Gly to pro; His to asn or gln; Ile to leu or val; Leu to ile or val; Lys to arg; gln or glu; Met to leu or ile; Phe to met, leu or tyr; Ser to thr; Thr to ser; Trp to tyr; Tyr to trp or phe; and, Val to ile or leu.

Nucleotide sequences of the invention may also be defined by their capability to hybridize with parts of specific nucleotide sequences disclosed herein, respectively, under moderate, or preferably under stringent hybridization conditions. Stringent hybridization conditions are herein defined as conditions that allow a nucleic acid sequence of at least about 25, preferably about 50 nucleotides, 75 or 100 and most preferably of about 200 or more nucleotides, to hybridize at a temperature of about 65°C in a solution comprising about 1 M salt, preferably 6 x SSC or any other solution having a comparable ionic strength, and washing at 65°C in a solution comprising about 0.1 M salt, or less, preferably 0.2 x SSC or any other solution having a comparable ionic strength. Preferably, the hybridization is performed overnight, i.e. at least for 10 hours and preferably washing is performed for at least one hour with at least two changes of the washing solution. These conditions will usually allow the specific hybridization of sequences having about 90% or more sequence identity.

Moderate conditions are herein defined as conditions that allow a nucleic acid sequences of at least 50 nucleotides, preferably of about 200 or more nucleotides, to hybridize at a temperature of about 45°C in a solution comprising about 1 M salt, preferably 6 x SSC or any other solution having a comparable ionic strength, and washing at room temperature in a solution comprising about 1 M salt, preferably 6 x SSC or any other solution having a comparable ionic strength. Preferably, the hybridization is performed overnight, i.e. at least for 10 hours, and preferably washing is performed for at least one hour with at least two changes of the washing solution. These conditions will usually allow the specific hybridization of sequences having up to 50% sequence identity. The person skilled in the art will be able to modify these hybridization conditions in order to specifically identify sequences varying in identity between 50% and 90%.

A "nucleic acid construct" or "nucleic acid vector" is herein understood to mean a man-made nucleic acid molecule resulting from the use of recombinant DNA technology. The term "nucleic acid construct" therefore does not include naturally occurring nucleic acid molecules although a nucleic acid construct may comprise (parts of) naturally occurring nucleic acid molecules. The terms "expression vector" or expression construct" refer to nucleotide sequences that are capable of affecting expression of a gene in host cells or host organisms compatible with such sequences. These expression vectors typically include at least suitable transcription regulatory sequences and optionally, 3' transcription termination signals. Additional factors necessary or helpful in effecting expression may also be present, such as expression enhancer elements. The expression vector will be introduced into a suitable host cell and be able to effect expression of the coding sequence in an in vitro cell culture of the host cell. The expression vector will be suitable for replication in the host cell or organism of the invention.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter. A "constitutive" promoter is a promoter that is active in most tissues under most physiological and developmental conditions. An "inducible" promoter is a promoter that is physiologically or developmentally regulated, e.g. by the application of a chemical inducer.

The term "selectable marker" is a term familiar to one of ordinary skill in the art and is used herein to describe any genetic entity which, when expressed, can be used to select for a cell or cells containing the selectable marker. The term "reporter" may be used interchangeably with marker, although it is mainly used to refer to visible markers, such as green fluorescent protein (GFP). Selectable markers may be dominant or recessive or bidirectional.

As used herein, the term "operably linked" refers to a linkage of polynucleotide elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. Operably linked means that the DNA sequences being linked are typically contiguous and, where necessary to join two protein encoding regions, contiguous and in reading frame.

The terms "protein" or "polypeptide" are used interchangeably and refer to molecules consisting of a chain of amino acids, without reference to a specific mode of action, size, 3-dimensional structure or origin.

"Fungi" (singular fungus) are herein understood as heterotrophic eukaryotic microorganism that digest their food externally, absorbing nutrient molecules into their cells. Fungi are a separate kingdom of eukaryotic organisms and include yeasts, molds, and mushrooms. The terms fungi, fungus and fungal as used herein thus expressly includes yeasts as well as filamentous fungi.

The term "gene" means a DNA fragment comprising a region (transcribed region), which is transcribed into an RNA molecule (e.g. an mRNA) in a cell, operably linked to suitable regulatory regions (e.g. a promoter). A gene will usually comprise several operably linked fragments, such as a promoter, a 5' leader sequence, a coding region and a 3'nontranslated sequence (3'end) comprising a polyadenylation site. The coding region of the gene may still comprise intron but usually will comprise an uninterrupted open reading frame, such as e.g. a cDNA. "Expression of a gene" refers to the process wherein a DNA region which is operably linked to appropriate regulatory regions, particularly a promoter, is transcribed into an RNA, which is biologically active, i.e. which is capable of being translated into a biologically active protein or peptide.

The term "homologous" when used to indicate the relation between a given (recombinant) nucleic acid or polypeptide molecule and a given host organism or host cell, is understood to mean that in nature the nucleic acid or polypeptide molecule is produced by a host cell or organisms of the same species, preferably of the same variety or strain. If homologous to a host cell, a nucleic acid sequence encoding a polypeptide will typically (but not necessarily) be operably linked to another (heterologous) promoter sequence and, if applicable, another (heterologous) secretory signal sequence and/or terminator sequence than in its natural environment. It is understood that the regulatory sequences, signal sequences, terminator sequences, etc. may also be homologous to the host cell. In this context, the use of only "homologous" sequence elements allows the construction of "self-cloned" genetically modified organisms (GMO's) (self-cloning is defined herein as in European Directive 98/81/EC Annex II). When used to indicate the relatedness of two nucleic acid sequences the term "homologous" means that one single-stranded nucleic acid sequence may hybridize to a complementary single-stranded nucleic acid sequence. The degree of hybridization may depend on a number of factors including the amount of identity between the sequences and the hybridization conditions such as temperature and salt concentration as discussed later.

The terms "heterologous" and "exogenous" when used with respect to a nucleic acid (DNA or RNA) or protein refers to a nucleic acid or protein that does not occur naturally as part of the organism, cell, genome or DNA or RNA sequence in which it is present, or that is found in a cell or location or locations in the genome or DNA or RNA sequence that differ from that in which it is found in nature. Heterologous and exogenous nucleic acids or proteins are not endogenous to the cell into which it is introduced, but have been obtained from another cell or synthetically or recombinantly produced. Generally, though not necessarily, such nucleic acids encode proteins, i.e. exogenous proteins, that are not normally produced by the cell in which the DNA is transcribed or expressed. Similarly exogenous RNA encodes for proteins not normally expressed in the cell in which the exogenous RNA is present. Heterologous or exogenous nucleic acids and proteins may also be referred to as foreign nucleic acids or proteins. Any nucleic acid or protein that one of skill in the art would recognize as foreign to the cell in which it is expressed is herein encompassed by the term heterologous or exogenous nucleic acid or protein. The terms heterologous and exogenous also apply to non-natural combinations of nucleic acid or amino acid sequences, i.e. combinations where at least two of the combined sequences are foreign with respect to each other.

The "specific activity" of an enzyme is herein understood to mean the amount of activity of a particular enzyme per amount of total host cell protein, usually expressed in units of enzyme activity per mg total host cell protein. In the context of the present invention, the specific activity of a particular enzyme may be increased or decreased as compared to the specific activity of that enzyme in an (otherwise identical) wild type host cell.

"Anaerobic conditions" or an anaerobic fermentation process is herein defined as conditions or a fermentation process run in the absence of oxygen or in which substantially no oxygen is consumed, preferably less than 5, 2.5 or 1 mmol/L/h, more preferably 0 mmol/L/h is consumed (i.e. oxygen consumption is not detectable), and wherein organic molecules serve as both electron donor and electron acceptors. It is understood that the term "anaerobic" is interchangeable with the term "anoxic".

When an enzyme is mentioned with reference to an enzyme class (EC), the enzyme class is a class wherein the enzyme is classified or may be classified, on the basis of the Enzyme Nomenclature provided by the Nomenclature Committee of the International Union of Biochemistry and Molecular Biology (NC-IUBMB), which nomenclature may be found at http://www.chem.qmul.ac.uk/iubmb/enzyme/. Other suitable enzymes that have not (yet) been classified in a specified class but may be classified as such, are meant to be included.

If referred herein to a protein or a nucleic acid sequence, such as a gene, by reference to an accession number, this number in particular is used to refer to a protein or nucleic acid sequence (gene) having a sequence as can be found via www.ncbi.nlm.nih.gov/ and refers to the version of that sequence as available on 14 March 2011, unless specified otherwise.

<u>Detailed description of the invention</u>

In a first aspect the invention relates to a yeast cell that has the ability of metabolizing uronic acids. A uronic acid is herein understood to refer to a sugar acid with both a carbonyl and a carboxylic acid function. Preferred uronic acids in the context of the invention are glucuronic acid and galacturonic acid. It is further understood herein that the terms "uronic acid", "glucuronic acid" and "galacturonic acid" as used herein are interchangeable with the term "uronate", "glucuronate" and "galacturonate", respectively. A yeast cell that has the ability of metabolizing uronic acids preferably is a yeast that can grow at the expense of the uronic acid, more preferably the yeast cell has the ability to grow on the uronic acid as sole carbon source, preferably at least under aerobic conditions. More preferably the yeast cell has the ability to grow at the expense of the uronic acid also under oxygen-limited aerobic conditions. Most preferably the yeast cell has the ability to grow at the expense of the uronic acid also under anaerobic conditions. For a yeast cell of the invention to grow at the expense of the uronic acid under anaerobic conditions it can be useful that the yeast cell simultaneously consumed another carbon source than the uronic acid that is in a less oxidised state than the uronic acid, preferably in a less oxidised state than glucose, including e.g. alkanols such as propanol and butanol; polyols such as 1,3-propane-diol, butandiol, glycerol, mannitol and xylitol. More preferably the yeast cell has the ability to ferment the uronic acid, together with less oxidised carbon sources, under anaerobic conditions to fermentation products such as ethanol or lactic acid.

A yeast cell of the invention that has the ability of metabolizing a uronic acid preferably is a yeast that has been engineered to have this metabolic ability. Preferably the yeast cell has been engineered by introducing one or more expression constructs for the expression of one or more nucleotide sequences (genes) coding for enzymes that are required for metabolizing a uronic acid, i.e. the cell has been transformed with the corresponding expression constructs.

Thus, in one embodiment, a yeast cell of the invention comprises genes coding for: a) a uronokinase (EC 2.7.1.43 and/or EC 2.7.1.44); b) a UTP-monosaccharide-1-phosphate uridylyltransferase (EC 2.7.7.64); and, c) a UDP-glucose 6-dehydrogenase (EC 1.1.1.22). The genes in a), b) and c) will usually be genes that are exogenous to the yeast cell but they can also be endogenous to the yeast cell. Genes coding for the enzymes specified in a), b) and c) are further specified herein below under "Enzyme activities introduced into the yeast cell of the invention", in paragraphs A) to C) herein below.

In one embodiment the yeast cell is a cell that has the ability of metabolizing glucuronic acid. Such a yeast cell will at least comprise genes coding for a) a glucuronokinase (EC 2.7.1.43) or a modified galacturonokinase (EC 2.7.1.44) having a broader substrate specificity as described in A) herein below; b) a UTP-monosaccharide-1-phosphate uridylyltransferase (EC 2.7.7.64) as described in B); and a UDP-glucose 6-dehydrogenase (EC 1.1.1.22) as described in C) herein below. The expression of these genes in the yeast cell confers to the yeast cell the ability of metabolizing glucuronic acid (see Figure 1).

In another embodiment the yeast cell is a cell that has the ability of metabolizing galacturonic acid. Such a yeast cell will at least comprise genes coding for a) a galacturonokinase (EC 2.7.1.44) as described in A) herein below; b) a UTP-monosaccharide-1-phosphate uridylyltransferase (EC 2.7.7.64) as described in B) herein below; c) a UDP-glucose 6-dehydrogenase (EC 1.1.1.22) as described in C) herein below; and preferably d) a UDP-glucuronate-4-epimerase (EC 5.1.3.6) as described in D) herein below. The expression of these genes in the yeast cell confers to the yeast cell the ability of metabolizing galacturonic acid (see Figure 2).

In another embodiment the yeast cell is a cell that has the ability of metabolizing galacturonic acid. Such a yeast cell will at least comprise genes coding for a) a galacturonokinase (EC 2.7.1.44) as described in A) herein below; b) a UTP-monosaccharide-1-phosphate uridylyltransferase (EC 2.7.7.64) as described in B) herein below; c) a UDP-glucose 6-dehydrogenase (EC 1.1.1.22) as described in C) herein below. Such a yeast cell further preferably expresses at least one of e) a galactose-1-phosphate uridyltransferase (EC 2.7.7.12; GAL7) and a UDP-glucose-4-epimerase (EC 5.1.3.2; GAL10), as described in E) herein below. The yeast cell preferably (over)expresses the galactose-1-phosphate uridyltransferase and UDP-glucose-4-epimerase in a galactose-independent manner, i.e. in the absence of galactose. The yeast cell further preferably (over)expresses at least one of a UDP-glucose pyrophosphorylase (EC 2.7.7.9; UGP1) and a phosphoglucomutase (EC 5.4.2.2; PGM1 or PGM2), as described in F) herein below. The yeast cell preferably (over)expresses the UDP-glucose pyrophosphorylase and phosphoglucomutase in a galactose-independent manner, i.e. in the absence of galactose. The expression of these genes in the yeast cell confers to the yeast cell the ability of metabolizing galacturonic acid (see Figure 3).

In preferred embodiment the yeast cell is a cell that has the ability of metabolizing both galacturonic acid and glucuronic acid. Such a yeast cell will at least comprise genes coding for a) a galacturonokinase (EC 2.7.1.44) and a glucuronokinase (EC 2.7.1.43) or a modified galacturonokinase (EC 2.7.1.44) having a broader substrate specificity as described in A) herein below; b) a UTP-monosaccharide-1-phosphate uridylyltransferase (EC 2.7.7.64) as described in B) herein below; c) a UDP-glucose 6-dehydrogenase (EC 1.1.1.22) as described in C) herein below; and preferably d) a UDP-glucuronate-4-epimerase (EC 5.1.3.6) as described in D) herein below an/or e) a galactose-1-phosphate uridyltransferase (EC 2.7.7.12; GAL7) and a UDP-glucose-4-epimerase (EC 5.1.3.2; GAL10), as described in E) herein below. The expression of these genes in the yeast cell confers to the yeast cell the ability of metabolizing both galacturonic acid and glucuronic acid.

In the above embodiments wherein the yeast cell has the ability of metabolizing galacturonic acid or both galacturonic acid and glucuronic acid, the yeast cell optionally, though preferably, comprises a gene coding for a UDP-glucuronate-4-epimerase (EC 5.1.3.6). In case the gene encoding the UDP-glucose 6-dehydrogenase (EC 1.1.1.22) encodes an enzyme with a broad substrate specificity, which also has the ability to convert UDP-D-galacturonate into UDP-D-galactose, a UDP-glucuronate-4-epimerase would strictly not be required. However, the activity of the UDP-glucose 6-dehydrogenase on UDP-D-galacturonate substrate may be suboptimal as compared to its activity on UDP-glucuronate. It may therefore be advantageous to also express a UDP-glucuronate-4-epimerase to convert UDP-D-galacturonate into UDP-glucuronate, which is then more efficiently metabolised by the UDP-glucose 6-dehydrogenase as compared to UDP-D-galacturonate.

Enzyme activities introduced into and/or modified in the yeast cell of the invention

A) Uronokinases (2.7.1.44 and 2.7.1.43)

A yeast cell of the invention preferably comprises a gene coding for a enzyme with the ability to convert a uronic acid into a 1-P-uronic acid. Preferably the enzyme is a galacturonokinase (EC 2.7.1.44) or a glucuronokinase (EC 2.7.1.43). A galacturonokinase is herein understood as an enzyme that catalyses the ATP-dependent conversion of α-D-galacturonic acid to α-D-galacturonic acid-1-phosphate. Likewise, a glucuronokinase is herein understood as an enzyme that catalyzes the ATP-dependent conversion of α-D-glucuronic acid to α-D-glucuronic acid-1-phosphate. A preferred gene coding for a uronokinase is an exogenous gene, preferably a gene encoding an enzyme of plant origin or a variant thereof.

An example of a suitable gene coding for a uronokinase is e.g. the GalAK gene (cDNA) encoding the galacturokinase of *Arabidopsis thaliana* as described by Yang et al. (2009, J. Biol Chem. 284: 21526-21535; accession no.: At3g10700). A gene coding for an enzyme with galacturokinase activity preferably comprises a nucleotide sequence coding for an amino acid sequence with at least 45, 50, 60, 65, 70, 75, 80, 85, 90, 95, 98, 99% amino acid sequence identity with SEQ ID NO: 1. A gene coding for an enzyme with galacturokinase activity may also comprises a nucleotide sequence coding for an amino acid sequence having one or several substitutions, insertions and/or deletions as compared to the amino acid sequence of SEQ ID NO: 1. Preferably the amino acid sequence has no more than 210, 200, 150, 100, 75, 50, 40, 30, 20, 10 or 5 amino acid substitutions, insertions and/or deletions as compared to SEQ ID NO: 1. Other suitable and available galacturokinases are from *Oryza sativa, Populus trichocarpa, Vitis vinifera, Sorghum bicolor* and *Selaginella moellendorffii* (accession no.'s: *Oryza sativa* GalAK: Os4g51880; *Populus trichocarpa* GalAK: JGI: 427630; *Vitis vinifera* GalAK: GSVIVT00007137001; *Sorghum bicolor* GalAK: Sb06g027910; *Selaginella moellendorffii* GalAK: SmGalAK, JGI:82393). The amino acid sequences of these enzymes are available in public databases and can be used by the skilled person to design codon-optimized nucleotide sequences coding for the corresponding enzyme. A preferred codon-optimized nucleotide sequences coding for a galacturokinase is a nucleotide sequence with at least 80, 85, 90, 95, 98, 99% sequence identity with the open reading frame in SEQ ID NO: 19 (see Table 1).

A particularly preferred uronokinase is a uronokinase having substrate specificity for both glucuronic acid and galacturonic acid. An example thereof is a galacturokinase engineered for broader substrate specificity, such as e.g. the $GalAK^{Y250F}$ variant described by Yang et al. (2009, *supra),* which also phosphorylates α-D-glucuronic acid.

An example of a suitable gene coding for a uronokinase is e.g. the GlcAK gene (cDNA) encoding the glucurokinase of *Arabidopsis thaliana* as described by Pieslinger et al. (2010, J. Biol Chem. 285: 2902-2910; accession no.'s: At3g01640; At5g14470). A gene coding for an enzyme with glucurokinase activity preferably comprises a nucleotide sequence coding for an amino acid sequence with at least 45, 50, 60, 65, 70, 75, 80, 85, 90, 95, 98, 99% amino acid sequence identity with SEQ ID NO: 2. A gene coding for an enzyme with glucurokinase activity may also comprises a nucleotide sequence coding for an amino acid sequence having one or several substitutions, insertions and/or deletions as compared to the amino acid sequence of SEQ ID NO: 2. Preferably the amino acid sequence has no more than 180, 150, 100, 75, 50, 40, 30, 20, 10 or 5 amino acid substitutions, insertions and/or deletions as compared to SEQ ID NO: 2. Other suitable and available glucurokinases are from *Zea mays, Oryza sativa, Populus trichocarpa, Vitis vinifera, Sorghum bicolor* and *Physcomitrella patens* (accession no.'s: Os11g11060 and Os11g0217300 from rice; *Vitis vinifera,* CAO14683; *Sorghum bicolour*, EES15489; *Zea mays*, ACG36196; *Populus trichocarpa,* XM_002298517; *Physcomitrella patens* subsp. *patens*, EDQ81053). The amino acid sequences of these enzymes are available in public databases and can be used by the skilled person to design codon-optimized nucleotide sequences coding for the corresponding enzyme. A preferred codon-optimized nucleotide sequences coding for a glucurokinase is a nucleotide sequence with at least 80, 85, 90, 95, 98, 99% sequence identity with the open reading frame in SEQ ID NO: 20 (see Table 1).

A yeast cell of the invention with the ability to grow on galacturonic acid contains at least a gene coding for a galacturonokinase. A yeast cell of the invention with the ability to grow glucuronic acid contains at least a gene coding for a glucuronokinase. A yeast cell of the invention with the ability to grow on both galacturonic acid and glucuronic acid, contains either a gene coding for a galacturokinase engineered for broader substrate specificity, such as e.g. the $GalAK^{Y250F}$ variant described above, or it contains a gene coding for a galacturokinase and a gene coding for a glucuronokinase.

B) Uridylyltransferase/UDP-sugar pyrophosphorylase (EC 2.7.7.64)

A yeast cell of the invention further preferably comprises a gene coding for a enzyme with the ability to convert a D-uronate-1P into UDP-D-uronate. The enzyme preferably has to ability to convert D-galacturonate-1P into UDP-D-galacturonate and/or to convert D-glucuronate-1P into UDP-D-glucuronate. A suitable enzyme for catalyzing this reaction is an UTP-monosaccharide-1-phosphate uridylyltransferase (EC 2.7.7.64), also referred to as a UDP-sugar pyrophosphorylase. A preferred gene coding for a uridylyltransferase is an exogenous gene, preferably a gene encoding an enzyme of plant origin or a variant thereof. Plant UDP-sugar pyrophosphorylase/ uridylyltransferase (EC 2.7.7.64) are a novel class of nucleotide sugar pyrophosphorylases with broad substrate specificity toward monosaccharide 1-Ps, which at least catalyze the formation of UDP-D-galacturonate and UDP-D-glucuronate from the respective 1-P uronic acids.

Examples of a suitable genes coding for a uridylyltransferase are e.g. the PsUSP gene (cDNA) of *Pisum sativum* as described by Kotake et al. (2004, J. Biol. Chem. 279: 45728-45736; accession no.: AB178642) or the AtUSP gene (cDNA) encoding the UDP-sugar pyrophosphorylase of *Arabidopsis thaliana* with accession no.: At5g52560 as described by Litterer et al. (2006, Plant Physiol. Biochem. 44: 171-180) and by Kotake et al. (2007, Biosci. Biotechnol. Biochem. 71: 761-771). A gene coding for an enzyme with uridylyltransferase activity preferably comprises a nucleotide sequence coding for an amino acid sequence with at least 45, 50, 60, 65, 70, 75, 80, 85, 90, 95, 98, 99% amino acid sequence identity with at least one of SEQ ID NO: 3 and 4. A gene coding for an enzyme with uridylyltransferase activity may also comprises a nucleotide sequence coding for an amino acid sequence having one or several substitutions, insertions and/or deletions as compared to the amino acid sequence of at least one of SEQ ID NO: 3 and 4. Preferably the amino acid sequence has no more than 300, 250, 200, 150, 100, 75, 50, 40, 30, 20, 10 or 5 amino acid substitutions, insertions and/or deletions as compared to at least one of SEQ ID NO: 3 and 4. Another suitable and available uridylyltransferase is from *Oryza sativa* (accession no. rice: AK064009). The amino acid sequences of this enzyme is available in public databases and can be used by the skilled person to design codon-optimized nucleotide sequences coding for the corresponding enzyme. A preferred codon-optimized nucleotide sequences coding for a uridylyltransferase is a nucleotide sequence with at least 80, 85, 90, 95, 98, 99% sequence identity with the open reading frames in at least one of SEQ ID NO's: 21 and 22 (see Table 1).

C) UDP-glucose 6-dehydrogenase (EC 1.1.1.22)

A yeast cell of the invention further preferably comprises a gene coding for a enzyme with the ability to convert a UDP-D-uronate into the corresponding UDP-D-hexose. In particular the enzyme has the ability to reduce UDP-glucuronate into UDP-glucose with the concomitant oxidation of NADH to $NAD^+$. Thus the enzymes catalyzes the reaction:

$$\text{UDP-glucuronate} + 2\ \text{NADH} + 2\ H^+ \leftrightarrow \text{UDP-glucose} + 2\ NAD^+ + H_2O$$

The enzyme preferably has a broader substrate specificity and therefore also has the ability to convert UDP-D-galacturonate into UDP-D-galactose. A suitable enzyme for catalyzing this reaction is a UDP-glucose 6-dehydrogenase (EC 1.1.1.22), also referred to as a UDPglucose:$NAD^+$ oxidoreductase. A preferred gene coding for a uridylyltransferase is an exogenous gene, preferably a gene encoding an enzyme of plant or bacterial origin or a variant thereof.

Examples of a suitable genes coding for a UDP-glucose 6-dehydrogenase are e.g. the PA2022 and PA3559 genes (cDNA) of *Pseudomonas aeruginosa* as described by Hung et al. (2007, J. Biol. Chem., 282 17738-17748; accession no.'s: NP_250712 and NP_252249, respectively). The UDP-glucose 6-dehydrogenase encoded by the PA3559 gene has a higher activity on UDP-D-galactose as compared to the enzyme encoded by the PA2022 gene. A gene coding for an enzyme with UDP-glucose 6-dehydrogenase activity preferably comprises a nucleotide sequence coding for an amino acid sequence with at least 45, 50, 60, 65, 70, 75, 80, 85, 90, 95, 98, 99% amino acid sequence identity with at least one of SEQ ID NO: 5 and 6 (the gene products of PA2022 and PA3559, respectively). A gene coding for an enzyme with UDP-glucose 6-dehydrogenase activity may also comprises a nucleotide sequence coding for an amino acid sequence having one or several substitutions, insertions and/or deletions as compared to the amino acid sequence of at least one of SEQ ID NO: 5 and 6. Preferably the amino acid sequence has no more than 250, 200, 150, 100, 75, 50, 40, 30, 20, 10 or 5 amino acid substitutions, insertions and/or deletions as compared to at least one of SEQ ID NO: 5 and 6. A preferred codon-optimized nucleotide sequences coding for a UDP-glucose 6-dehydrogenase is a nucleotide sequence with at least 80, 85, 90, 95, 98, 99% sequence identity with the open reading frames in at least one of SEQ ID NO's: 23 and 24 (see Table 1).

Further suitable and available genes coding for a UDP-glucose 6-dehydrogenase include the SpUGDH gene (cDNA) of *Streptococcus pyogenes* as described by Campbell et al. (2000, Biochemistry 39: 7012-7023; accession no.: NP_270108) and the genes listed in the Kyoto encyclopedia of genes and genomes (Kegg Database - Orthology K00012 UDPglucose 6-dehydrogenase EC 1.1.1.22 - http://www.genome.jp/dbget-bin/www_bget?ko:K00012) and genes (cDNAs) coding for the UDP-glucose 6-dehydrogenases of *Arabidopsis thaliana* (UGD1), *Burkholderia cenocepaci* (UGD) and Xenopus leavis (UGDH-a), with respectively GenBank accession no.'s: AT1G26570, BCAL2946 and AY762616.

D) UDP-glucuronate-4-epimerase (EC 5.1.3.6)

A yeast cell of the invention further preferably comprises a gene coding for a enzyme with the ability to epimerize UDP-D-glucuronate into UDP-D-galacturonate and vice versa. A suitable enzyme for catalyzing this reaction is a UDP-D-glucuronate 4-epimerase (EC 5.1.3.6). A preferred gene coding for a UDP-D-glucuronate 4-epimerase is an exogenous gene, more preferably a gene encoding an enzyme of plant origin or a variant thereof. However, other preferred genes coding for the UDP-D-glucuronate 4-epimerase are genes encoding enzymes of bacterial origin or variants thereof, such as e.g. the CaplJ gene from *S. pneumoniae* as described by Muñoz et al. (1999, Mol. Microbiol. 31: 703-713).

An example of a suitable gene coding for a UDP-D-glucuronate 4-epimerase is e.g. the GAE1 gene (cDNA) encoding the UDP-D-glucuronate 4-epimerase of *Arabidopsis thaliana* as described by Mølhøj et al. (2004, Plant Physiol. 135: 1221-1230; accession no.: At4g30440). A gene coding for an enzyme with UDP-D-glucuronate 4-epimerase activity preferably comprises a nucleotide sequence coding for an amino acid sequence with at least 45, 50, 60, 65, 70, 75, 80, 85, 90, 95, 98, 99% amino acid sequence identity with SEQ ID NO: 7. A gene coding for an enzyme with UDP-D-glucuronate 4-epimerase activity may also comprises a nucleotide sequence coding for an amino acid sequence having one or several substitutions, insertions and/or deletions as compared to the amino acid sequence of SEQ ID NO: 7. Preferably the amino acid sequence has no more than 200, 150, 100, 75, 50, 40, 30, 20, 10 or 5 amino acid substitutions, insertions and/or deletions as compared to SEQ ID NO: 7.

A preferred codon-optimized nucleotide sequences coding for a UDP-D-glucuronate 4-epimerase is a nucleotide sequence with at least 80, 85, 90, 95, 98, 99% sequence identity with the open reading frame in SEQ ID NO: 25 (see Table 1).

E) Enzymes for converting UDP-D-galactose to UDP-glucose

A yeast cell of the invention further preferably comprises a gene coding for an enzyme with the ability to convert UDP-D-galactose to UDP-glucose. Two types of enzymes have this ability: galactose-1-phosphate uridyltransferase (EC 2.7.7.12) and UDP-glucose-4-epimerase (EC 5.1.3.2). A galactose-1-phosphate uridyltransferase (EC 2.7.7.12) is understood as an enzyme with the ability to convert UDP-D-glucose and alpha-D-galactose-1-phosphate into glucose-1-phosphate and UDP-galactose and *vice versa*. A UDP-glucose-4-epimerase (EC 5.1.3.2) is understood as an enzyme with the ability to convert UDP-galactose into UDP-D-glucose and *vice versa*.

Yeast cells usually have endogenous genes coding for galactose-1-phosphate uridyltransferase and UDP-glucose-4-epimerase, such as e.g. the *GAL7* and *GAL10* genes in *S. cerevisiae*, respectively, which are regulated by galactose inducible promoters. A preferred yeast cell of the invention expresses at least one of a galactose-1-phosphate uridyltransferase and a UDP-glucose-4-epimerase. More preferably, the yeast cell comprises a genetic modification that causes at least one of the galactose-1-phosphate uridyltransferase and the UDP-glucose-4-epimerase to be expressed or overexpressed in a galactose-independent manner, which is understood to mean that the yeast cell does not require galactose in the culture medium for expression of the transferase and/or epimerase. Galactose-independent (over) expression of the galactose-1-phosphate uridyltransferase and the UDP-glucose-4-epimerase may be achieved by operably linking the coding sequences of these enzymes to a promoter described herein below for overexpression of enzymes in yeast, e.g. a constitutive such as a glycolytic promoter.

Thus, in one embodiment a yeast cell of the invention preferably, contains (endogenous) galactose-1-phosphate uridyltransferase activity. More preferably, the cell comprises a genetic modification that increases the specific galactose-1-phosphate uridyltransferase activity. Preferably the genetic modification causes overexpression of the galactose-1-phosphate uridyltransferase in a galactose-independent manner, e.g. by overexpression of a nucleotide sequence encoding a galactose-1-phosphate uridyltransferase. The gene encoding the galactose-1-phosphate uridyltransferase may be endogenous to the cell or may be a gene that is heterologous to the cell. A suitable nucleotide sequence for (over) expression of galactose-1-phosphate uridyltransferase activity in the cells of the invention is e.g. a nucleotide sequence encoding an amino acid sequence with at least 45, 50, 60, 65, 70, 75, 80, 85, 90, 95, 98, 99% amino acid sequence identity with SEQ ID NO: 8 and having galactose-1-phosphate uridyltransferase activity, such as the *GAL7* gene of *S. cerevisiae* (GAL7-YBR018c) as depicted in SEQ ID NO: 8.

Thus, in another embodiment a yeast cell of the invention preferably, contains (endogenous) UDP-glucose-4-epimerase activity. More preferably, the cell comprises a genetic modification that increases the specific UDP-glucose-4-epimerase activity. Preferably the genetic modification causes overexpression of the UDP-glucose-4-epimerase in a galactose-independent manner, e.g. by overexpression of a nucleotide sequence encoding a UDP-glucose-4-epimerase. The gene encoding the UDP-glucose-4-epimerase may be endogenous to the cell or may be a gene that is heterologous to the cell. A suitable nucleotide sequence for (over)expression of UDP-glucose-4-epimerase activity in the cells of the invention is e.g. a nucleotide sequence encoding an amino acid sequence with at least 45, 50, 60, 65, 70, 75, 80, 85, 90, 95, 98, 99% amino acid sequence identity with SEQ ID NO: 9 and having UDP-glucose-4-epimerase activity, such as the *GAL10* gene of *S. cerevisiae* (GAL10-YBR019c) as depicted in SEQ ID NO:9.

A genetic modification that increases the specific galactose-1-phosphate uridyltransferase and/or UDP-glucose-4-epimerase activity in the cell of the invention may be achieved as described under "Construction of yeast cells of the invention". In the cells of the invention, the specific galactose-1-phosphate uridyltransferase and/or UDP-glucose-4-epimerase activity is preferably increased by at least a factor 1.1, 1.2, 1.5, 2, 5, 10 or 20 as compared to a strain which is genetically identical except for the genetic modification causing the increase. Preferably, the specific galactose-1-phosphate uridyltransferase and/or UDP-glucose-4-epimerase activity is increased under anaerobic conditions or oxygen-limited aerobic conditions by at least a factor 1.1, 1.2, 1.5, 2, 5, 10 or 20 as compared to a strain which is genetically identical except for the genetic modification causing the increase. It is to be understood that these increased levels of specific activity may be determined by measuring the steady state level of the enzyme's activity (specific activity in the cell), the steady state level of the enzyme's protein as well as to the steady state level of the transcript coding for the enzyme in the cell.

F) Increased activity of UDP-glucose pyrophosphorylase (EC 2.7.7.9) and phosphoglucomutase (EC 5.4.2.2) enzymes

A yeast cell of the invention further preferably comprises a genetic modification that increases the specific activity of at least one of a UDP-glucose pyrophosphorylase (EC 2.7.7.9) and a phosphoglucomutase (EC 5.4.2.2). Preferably, the genetic modification that increases the specific activity of at least one of the UDP-glucose pyrophosphorylase and the phosphoglucomutase is overexpression of a nucleotide sequence encoding at least one of the UDP-glucose pyrophosphorylase and the phosphoglucomutase.

More preferably, the yeast cell comprises a genetic modification that causes at least one of the UDP-glucose pyrophosphorylase and the phosphoglucomutase to be expressed or overexpressed in a galactose-independent manner, which is understood to mean that the yeast cell does not require galactose in the culture medium for expression of the pyrophosphorylase and/or mutase. Galactose-independent (over)expression of the UDP-glucose pyrophosphorylase and the phosphoglucomutase may be achieved by operably linking the coding sequences of these enzymes to a promoter described herein below for overexpression of enzymes in yeast, e.g. a constitutive such as a glycolytic promoter or a promoter of a translation elongation factor such as the *TEF1* promoter. Most preferred is a yeast cell comprising a genetic modification that causes at least a *PGM2* phosphoglucomutase to be expressed or overexpressed in a galactose-independent manner. A *PGM2* phosphoglucomutase is herein understood as a phosphoglucomutase having the amino acid sequence of the *PGM2* phosphoglucomutase of *S. cerevisiae* (SEQ ID NO: 39) or an orthologue thereof from other yeast species (e.g. a phosphoglucomutase having an amino acid sequence with at least 70, 75, 80, 85, 90, 95, 98 or 99% sequence identity to SEQ ID NO: 39).

However, alternatively, the specific activity of the UDP-glucose pyrophosphorylase and/or the phosphoglucomutase may be increased by expressing an enzyme having increased activity as compared to the endogenous wild type enzyme of the host cell, in addition to, or as a replacement for the wild type enzyme.

A genetic modification that increases the specific UDP-glucose pyrophosphorylase and/or the phosphoglucomutase activity in the cell of the invention may be achieved as described under "Construction of yeast cells of the invention". In the cells of the invention, the specific UDP-glucose pyrophosphorylase and/or the phosphoglucomutase activity is preferably increased by at least a factor 1.1, 1.2, 1.5, 2, 5, 10 or 20 as compared to a strain which is genetically identical except for the genetic modification causing the increase. Preferably, the specific UDP-glucose pyrophosphorylase and/or the phosphoglucomutase activity is increased under anaerobic conditions or oxygen-limited aerobic conditions by at least a factor 1.1, 1.2, 1.5, 2, 5, 10 or 20 as compared to a strain which is genetically identical except for the genetic modification causing the increase. It is to be understood that these increased levels of specific activity may be determined by measuring the steady state level of the enzyme's activity (specific activity in the cell), the steady state level of the enzyme's protein as well as to the steady state level of the transcript coding for the enzyme in the cell.

Preferably the genetic modification causes overexpression of a UDP-glucose pyrophosphorylase, e.g. by overexpression of a nucleotide sequence encoding a UDP-glucose pyrophosphorylase. The nucleotide sequence encoding the UDP-glucose pyrophosphorylase may be endogenous to the cell or may be a UDP-glucose pyrophosphorylase that is heterologous to the cell. A nucleotide sequence that may be used for overexpression of UDP-glucose pyrophosphorylase in the cells of the invention is e.g. the *UGP1* UDP-glucose pyrophosphorylase gene from *S. cerevisiae*.

Preferably, the nucleotide sequence encoding the UDP-glucose pyrophosphorylase comprises a nucleotide sequence coding for an amino acid sequence with at least 45, 50, 60, 65, 70, 75, 80, 85, 90, 95, 98, 99% amino acid sequence identity with SEQ ID NO: 10 and having UDP-glucose pyrophosphorylase activity. In a preferred embodiment a codon-optimised (see below) nucleotide sequence encoding the UDP-glucose pyrophosphorylase is overexpressed.

Preferably the genetic modification causes overexpression of a phosphoglucomutase, e.g. by overexpression of a nucleotide sequence encoding a phosphoglucomutase. The nucleotide sequence encoding the phosphoglucomutase may be endogenous to the cell or may be a phosphoglucomutase that is heterologous to the cell. Nucleotide sequences that may be used for overexpression of phosphoglucomutase in the cells of the invention are e.g. the *PGM1* and *PGM2* phosphoglucomutase genes from *S. cerevisiae*, or a nucleotide sequence coding for an amino acid sequence with at least 45, 50, 60, 65, 70, 75, 80, 85, 90, 95, 98, 99% amino acid sequence identity with the amino acid sequence encoded by at least of one of the *PGM1* and *PGM2* (SEQ ID NO: 39) genes from *S. cerevisiae*.

G) Enzymes for fermenting pentoses

A yeast cell of the invention further preferably comprises genes coding for enzymes with the ability to convert a pentose into at least one of xylulose and xylulose 5-phosphate. These genes are preferably selected from the group consisting of genes coding for enzymes with the ability to i) directly isomerise xylose into xylulose, and, ii) convert L-arabinose into D-xylulose 5-phosphate.

In a further preferred embodiment, the yeast cell of the invention has at least one of: a) the ability of isomerising xylose to xylulose (a xylose isomerase; EC 5.3.1.5); and, b) the ability to convert L-arabinose into D-xylulose 5-phosphate. For a) the cell preferably has a functional xylose isomerase gene, which gene confers to the cell the ability to isomerise xylose into xylulose. The xylose isomerase preferably is an exogenous gene. For b) the cell preferably has functional exogenous genes coding for a L-arabinose isomerase (EC 5.3.1.3), a L-ribulokinase (EC 2.7.1.16) and a L-ribulose-5-phosphate 4-epimerase (EC 5.1.3.4), which genes together confers to the cell the ability to isomerise convert L-arabinose into D-xylulose 5-phosphate. The genes conferring to the cell the ability to isomerise convert L-arabinose into D-xylulose 5-phosphate are preferably exogenous genes.

Yeast cells having the ability of isomerising xylose to xylulose as e.g. described in WO 03/0624430, WO 06/009434, and WO 10/074577. The ability of isomerising xylose to xylulose is preferably conferred to the cell by transformation with a nucleic acid construct comprising a nucleotide sequence encoding a xylose isomerase. Preferably the cell thus acquires the ability to directly isomerise xylose into xylulose. More preferably the cell thus acquires the ability to grow aerobically and/or anaerobically on xylose as sole energy and/or carbon source though direct isomerisation of xylose into xylulose (and further metabolism of xylulose). It is herein understood that the direct isomerisation of xylose into xylulose occurs in a single reaction catalyzed by a xylose isomerase, as opposed to the two step conversion of xylose into xylulose via a xylitol intermediate as catalyzed by xylose reductase and xylitol dehydrogenase, respectively.

Several xylose isomerases (and their amino acid and coding nucleotide sequences) that may be successfully used to confer to the cell of the invention the ability to directly isomerise xylose into xylulose have been described in the art. These include the xylose isomerases of *Piromyces* sp. and of other anaerobic fungi that belongs to the families *Neocallimastix, Caecomyces, Piromyces* or *Ruminomyces* (WO 03/0624430), *Cyllamyces aberensis* (US 20060234364), *Orpinomyces* (Madhavan et al., 2008, DOI 10.1007/s00253-008-1794-6), the xylose isomerase of the bacterial genus *Bacteroides*, including e.g. *B. thetaiotaomicron* (WO 06/009434), *B.fragilis* and *B. uniformis* (WO 09/109633), the xylose isomerase of the anaerobic bacterium *Clostridium phytofermentans* (Brat et al., 2009, Appl. Environ. Microbiol. 75: 2304-2311), *Lactococcal* xylose isomerases (WO 10/070549), the xylose isomerases of *Clostridium difficile, Ciona intestinales* and *Fusobacterium mortiferum* (WO 10/074577) and *Ruminococcal* xylose isomerases and chimeric isomerases therewith (WO 11/006136).

Yeast cells having the ability to convert L-arabinose into D-xylulose 5-phosphate as e.g. described in Wisselink et al. (2007, AEM Accepts, published online ahead of print on 1 June 2007; Appl. Environ. Microbiol. doi:10.1128/AEM. 00177-07) and in EP 1 499 708. The ability of to converting L-arabinose into D-xylulose 5-phosphate is preferably conferred to the cell by transformation with a nucleic acid construct(s) comprising nucleotide sequences encoding a) an arabinose isomerase; b) a ribulokinase, preferably a L-ribulokinase a xylose isomerase; and c) a ribulose-5-P-4-epimerase, preferably a L-ribulose-5-P-4-epimerase. Preferably, in the cells of the invention, the ability to convert L-arabinose into D-xylulose 5-phosphate is the ability to convert L-arabinose into D-xylulose 5-phosphate through the subsequent reactions of 1) isomerisation of arabinose into ribulose; 2) phosphorylation of ribulose to ribulose 5-phosphate; and, 3) epimerisation of ribulose 5-phosphate into D-xylulose 5-phosphate. Suitable nucleotide sequences encoding arabinose isomerases, a ribulokinases and ribulose-5-P-4-epimerases may be obtained from *Bacillus subtilis, Escherichia coli* (see e.g. EP 1 499 708), *Lactobacilli*, e.g. *Lactobacillus plantarum* (see e.g. Wisselink et al. *supra),* or species of *Clavibacter, Arthrobacter* and *Gramella*, of which preferably *Clavibacter michiganensis, Arthrobacter aurescens* and *Gramella forsetii* (see WO2009/011591).

H) Genetic modifications for improved utilization of glycerol

In a further embodiment, the host cell of the invention further comprises a genetic modification that increases at least one of: i) the specific activity of glycerol dehydrogenase; ii) the specific activity of dihydroxyacetone kinase; and, iii) transport of glycerol into the cell.

Preferably, the genetic modification that increases the specific activity of at least one of glycerol dehydrogenase and dihydroxyacetone kinase is overexpression of a nucleotide sequence encoding at least one of a glycerol dehydrogenase and dihydroxyacetone kinase. However, alternatively, the specific activity of the glycerol dehydrogenase and/or dihydroxyacetone kinase may be increased by expressing an enzyme having increased activity as compared to the endogenous wild type enzyme of the host cell, in addition to, or as a replacement for the wild type enzyme.

A glycerol dehydrogenase is herein understood as an enzyme that catalyzes the chemical reaction (EC 1.1.1.6):

$$\text{glycerol} + NAD^{+} \leftrightarrow \text{glycerone} + NADH + H^{+}$$

Other names in common use include glycerin dehydrogenase, $NAD^+$-linked glycerol dehydrogenase and glycerol:NAD+ 2-oxidoreductase. Preferably the genetic modification causes overexpression of a glycerol dehydrogenase, e.g. by overexpression of a nucleotide sequence encoding a glycerol dehydrogenase. The nucleotide sequence encoding the glycerol dehydrogenase may be endogenous to the cell or may be a glycerol dehydrogenase that is heterologous to the cell. Nucleotide sequences that may be used for overexpression of glycerol dehydrogenase in the cells of the invention are e.g. the glycerol dehydrogenase gene from *S. cerevisiae* (*GCYl*) as e.g. described by Oechsner et al. (1988, FEBS Lett. 238: 123-128) or Voss et al. (1997, Yeast 13: 655-672). Preferably, the nucleotide sequence encoding the glycerol dehydrogenase comprises a nucleotide sequence coding for an amino acid sequence with at least 45, 50, 60, 65, 70, 75, 80, 85, 90, 95, 98, 99% amino acid sequence identity with SEQ ID NO: 11. In a preferred embodiment a codon-optimised (see above) nucleotide sequence encoding the glycerol dehydrogenase is overexpressed, such as e.g. a codon optimised nucleotide sequence encoding the glycerol dehydrogenase of SEQ ID NO: 11.

A genetic modification that increases the specific glycerol dehydrogenase activity in the cell of the invention may be achieved as described under "Construction of yeast cells of the invention", e.g. by overexpression of a nucleotide sequence encoding the glycerol dehydrogenase. In the cells of the invention, the specific glycerol dehydrogenase activity is preferably increased by at least a factor 1.1, 1.2, 1.5, 2, 5, 10 or 20 as compared to a strain which is genetically identical except for the genetic modification causing the increase. Preferably, the specific glycerol dehydrogenase activity is increased under anaerobic conditions or oxygen-limited aerobic conditions by at least a factor 1.1, 1.2, 1.5, 2, 5, 10 or 20 as compared to a strain which is genetically identical except for the genetic modification causing the increase. It is to be understood that these increased levels of specific activity may be determined by measuring the steady state level of the enzyme's activity (specific activity in the cell), the steady state level of the enzyme's protein as well as to the steady state level of the transcript coding for the enzyme in the cell. A genetic modification that increases the glycerol dehydrogenase specific activity in the cell produces a specific glycerol dehydrogenase activity of at least 0.2, 0.5, 1.0, 2.0, or 5.0 U $min^{-1}$ (mg $protein)^{-1}$, preferably determined in cell extracts of the transformed host cells at 30 °C, e.g. as described in the Examples herein.

A dihydroxyacetone kinase is herein understood as an enzyme that catalyzes the chemical reaction ((EC 2.7.1.29):

$$\text{ATP} + \text{glycerone} \leftrightarrow \text{ADP} + \text{glycerone phosphate}$$

Other names in common use include glycerone kinase, ATP:glycerone phosphotransferase and (phosphorylating) acetol kinase. It is understood that glycerone and dihydroxyacetone are the same molecule. Preferably the genetic modification causes overexpression of a dihydroxyacetone kinase, e.g. by overexpression of a nucleotide sequence encoding a dihydroxyacetone kinase. The nucleotide sequence encoding the dihydroxyacetone kinase may be endogenous to the cell or may be a dihydroxyacetone kinase that is heterologous to the cell. Nucleotide sequences that may be used for overexpression of dihydroxyacetone kinase in the cells of the invention are e.g. the dihydroxyacetone kinase genes from *S. cerevisiae* (*DAK1*) and (*DAK2*) as e.g. described by Molin et al. (2003, J. Biol. Chem. 278: 1415-1423). Preferably, the nucleotide sequence encoding the dihydroxyacetone kinase comprises an amino acid sequence with at least 45, 50, 60, 65, 70, 75, 80, 85, 90, 95, 98, 99% amino acid sequence identity with at least one of SEQ ID NO's: 12 and 13. In a preferred embodiment a codon-optimised (see above) nucleotide sequence encoding the dihydroxyacetone kinase is overexpressed, such as e.g. a codon optimised nucleotide sequence encoding the dihydroxyacetone kinase of SEQ ID NO: 12 or a codon optimised nucleotide sequence encoding the dihydroxyacetone kinase of SEQ ID NO: 13. A preferred nucleotide sequence for overexpression of a dihydroxyacetone kinase is a nucleotide sequence encoding a dihydroxyacetone kinase comprises an amino acid sequence with at least 45, 50, 60, 65, 70, 75, 80, 85, 90, 95, 98, 99% amino acid sequence identity with at least one of SEQ ID NO's: 12 (*S. cerevisiae* (*DAK1*).

A genetic modification that increases the specific dihydroxyacetone kinase activity in the cell of the invention may be achieved as described under "Construction of yeast cells of the invention", e.g. by overexpression of a nucleotide sequence encoding the dihydroxyacetone kinase. In the cells of the invention, the specific dihydroxyacetone kinase activity is preferably increased by at least a factor 1.1, 1.2, 1.5, 2, 5, 10 or 20 as compared to a strain which is genetically identical except for the genetic modification causing the increase. Preferably, the specific dihydroxyacetone kinase activity is increased under anaerobic conditions or oxygen-limited aerobic conditions by at least a factor 1.1, 1.2, 1.5, 2, 5, 10 or 20 as compared to a strain which is genetically identical except for the genetic modification causing the increase. It is to be understood that these increased levels of specific activity may be determined by measuring the steady state level of the enzyme's activity (specific activity in the cell), the steady state level of the enzyme's protein as well as to the steady state level of the transcript coding for the enzyme in the cell. A genetic modification that increases the specific dihydroxyacetone kinase activity in the cell produces a specific dihydroxyacetone kinase activity of at least 0.002, 0.005, 0.01, 0.02 or 0.05 U $min^{-1}$ (mg $protein)^{-1}$, preferably determined in cell extracts of the transformed host cells at 30 °C, e.g. as described in the Examples herein.

Preferably, the genetic modification that increases transport of glycerol into the cell preferably is a genetic modification that causes overexpression of a nucleotide sequence encoding at least one of a glycerol uptake protein and a glycerol channel.

A glycerol uptake protein is herein understood as a multimembrane-spanning protein that belongs to the included in the membrane bound O-acyltransferases (MBOAT) superfamily including e.g. the *S. cerevisiae* glycerol uptake proteins encoded by the *GUP1* and *GUP2* genes. Preferably the genetic modification causes overexpression of a glycerol uptake protein, e.g. by overexpression of a nucleotide sequence encoding a glycerol uptake protein. The nucleotide sequence encoding the glycerol uptake protein may be endogenous to the cell or may be a glycerol uptake protein that is heterologous to the cell. Nucleotide sequences that may be used for overexpression of glycerol uptake protein in the cells of the invention are e.g. the glycerol uptake protein genes from *S. cerevisiae* (*GUP1*) and (*GUP2*) and orthologues thereof as e.g. described by Neves et al. (2004, FEMS Yeast Res. 5:51-62). Preferably, the nucleotide sequence encoding the glycerol uptake protein comprises a nucleotide sequence coding for an amino acid sequence with at least 45, 50, 60, 65, 70, 75, 80, 85, 90, 95, 98, 99% amino acid sequence identity with at least one of SEQ ID NO's: 14 (Gup1p) and 15 (Gup2p). In a preferred embodiment a codon-optimised (see above) nucleotide sequence encoding the glycerol uptake protein is overexpressed, such as e.g. a codon optimised nucleotide sequence encoding the glycerol uptake protein SEQ ID NO: 14 or a codon optimised nucleotide sequence encoding the glycerol uptake protein of SEQ ID NO: 15. Although the exact nature of the influence of GUP1 on glycerol transport is not yet clear, Yu et al. (2010, *supra*) have shown that overexpression of GUP1 in *S.cerevisiae* improves ethanol production on glycerol grown cells. A preferred nucleotide sequence for overexpression of a glycerol uptake protein is therefore a nucleotide sequence encoding a glycerol uptake protein that is capable of rescuing salt stress-associated phenotype of a *S. cerevisiae gup1*Δ mutant by complementation as described by Neves et al. (2004, *supra*). Such complementing orthologues of *S. cerevisiae GUP1* include nucleotide sequences encoding amino acid sequences having at least 60, 68, 72, 75, 80, 85, 90, 95, 98, 99% identity with the amino acid sequence of SEQ ID NO: 14 and may be obtained from yeast species belonging to the genera of *Saccharomyces, Zygosaccharomyces, Kluyveromyces, Candida, Pichia, Hansenula, Kloeckera, Schwanniomyces*, and *Yarrowia*.

A glycerol channel is herein understood as a member of the MIP family of channel proteins reviewed by Reizer et al. (1993, CRC Crit. Rev. Biochem. Mol. Biol., 28: 235-257), which channel proteins comprise a 250 -280 amino acid transmembrane domain consisting of six membrane-spanning domains and have at least 30, 35, 40, 45, 50, 60, 70, 80, 90, 95, 98 or 99 % amino acid identity, or at least 55, 60, 65, 70, 80, 90, 95, 98 or 99% amino acid similarity with the amino acid sequence between amino acids 250 and 530 of SEQ ID NO: 16, the *S. cerevisiae* FPS1 aquaglyceroporin. Nucleotide sequences that may be used for overexpression of a glycerol channel in the cells of the invention include nucleotide sequences encoding the yeast aquaglyceroporin FPS 1 gene from e.g. *S. cerevisiae* (Van Aelst et al., 1991, EMBO J. 10:2095-2104) and orthologues thereof from other yeasts including *Kluyveromyces lactis, Kluyveromyces marxianus* and *Zygosaccharomyces rouxii* as e.g. described by Neves et al. (2004, *supra).* However, the use of bacterial or plant glycerol channels is not excluded as e.g. Luyten et al. (1995, EMBO J. 14:1360-1371) have shown that the *E.coli* glycerol facilitator, having only 30% sequence identity with the amino acid sequence between amino acids 250 and 530 of the *S. cerevisiae* FPS1 aquaglyceroporin, can complement glycerol uptake in a *S. cerevisiae fps1*Δ mutant. The nucleotide sequence encoding the glycerol channel may be endogenous to the cell or may be a glycerol channel that is heterologous to the cell. In a preferred embodiment a codon-optimised (see above) nucleotide sequence encoding the glycerol channel is overexpressed, such as e.g. a codon optimised nucleotide sequence encoding the aquaglyceroporin of SEQ ID NO: 16.

For overexpression of the nucleotide sequence encoding the glycerol uptake protein and/or the glycerol channel protein, the nucleotide sequence (to be overexpressed) is placed in an expression construct wherein it is operably linked to suitable expression regulatory regions/sequences to ensure overexpression of the glycerol uptake protein and/or the glycerol channel protein upon transfomation of the expression construct into the host cell of the invention (see above). Suitable promoters for (over)expression of the nucleotide sequence coding for the glycerol uptake protein and/or the glycerol channel protein include promoters that are preferably insensitive to catabolite (glucose) repression, that are active under anaerobic conditions and/or that preferably do not require xylose or arabinose for induction. Examples of such promoters are given above. In the cells of the invention, a glycerol uptake protein and/or a glycerol channel protein to be overexpressed are preferably overexpressed by at least a factor 1.1, 1.2, 1.5, 2, 5, 10 or 20 as compared to a strain which is genetically identical except for the genetic modification causing the overexpression. Preferably, the glycerol uptake protein and/or the glycerol channel protein are overexpressed under anaerobic conditions by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity (specific activity in the cell), the steady state level of the enzyme's protein as well as to the steady state level of the transcript coding for the enzyme in the cell.

l) Genetic modifications that reduce glycerol production

In a further embodiment, the host cell of the invention further comprises a genetic modification that reduce or prevent the production of glycerol. Waks and Silver (2009, Appl. Environ. Microbiol. 75: 1867-1875) have suggested that expression of an $NAD^+$-dependent acetylating acetaldehyde dehydrogenase (EC 1.2.1.10) presents an alternative to glycerol synthesis as a means of recycling excess NADH in anaerobically grown yeasts. The cells of the present invention express an $NAD^+$-dependent UDP-glucose 6-dehydrogenase (EC 1.1.1.22; see above) that is likewise capable of an alternative to glycerol synthesis as a means of recycling excess NADH in anaerobically grown yeasts. Preferably therefore, also the cells of the invention preferably lack enzymatic activity required for NADH-dependent synthesis of glycerol or comprise a genetic modification that reduces the specific activity of one or more enzymes required for NADH-dependent synthesis of glycerol, compared to its corresponding wild-type cell. Preferably therefore, one or more genes involved in glycerol formation, such as the *S. cerevisiae* glycerol-phosphate dehydrogenase (GPD1 and/or GPD2) genes and/or glycerol-phosphate phosphatase (GPP1 and/or GPP2) genes, are inactivated or reduced in expression as e.g. described in WO 11/01092.

Thus, in the cells of the invention, the at least one of the specific glycerolphosphate dehydrogenase activity and the specific glycerol-phosphate phosphatase activity is preferably reduced by at least a factor 0.8, 0.5, 0.3, 0.1, 0.05 or 0.01 as compared to a strain which is genetically identical except for the genetic modification causing the overexpression, preferably under anaerobic conditions.

Preferably, glycerolphosphate dehydrogenase and/or phosphatase activities are reduced in the host cell by one or more genetic modifications that reduce the expression of or inactivates a gene encoding an glycerolphosphate dehydrogenase and/or phosphatase. Preferably, the genetic modifications reduce or inactivate the expression of each endogenous copy of the gene encoding a specific glycerolphosphate dehydrogenase and/or phosphatase in the cell's genome. A given cell may comprise multiple copies of the gene encoding a specific glycerolphosphate dehydrogenase and/or phosphatase with one and the same amino acid sequence as a result of di-, poly- or aneu-ploidy. In such instances preferably the expression of each copy of the specific gene that encodes the glycerolphosphate dehydrogenase and/or phosphatase is reduced or inactivated. Alternatively, a cell may contain several different (iso)enzymes with glycerolphosphate dehydrogenase and/or phosphatase activities that differ in amino acid sequence and that are each encoded by a different gene. In such instances, in some embodiments of the invention it is preferred that only certain types of the isoenzymes are reduced or inactivated while other types remain unaffected (see below). Preferably, the gene is inactivated by deletion of at least part of the gene or by disruption of the gene, whereby in this context the term gene also includes any non-coding sequence up- or down-stream of the coding sequence, the (partial) deletion or inactivation of which results in a reduction of expression of glycerolphosphate dehydrogenase and/or phosphatase activities in the host cell.

In a preferred embodiment of the invention, the host cell of the invention comprises a functional high-osmolarity glycerol response pathway. Preferably therefore, only the activity of the gene(s) encoding the *S. cerevisiae GPD2* gene as described by Eriksson et al. (1995, Mol. Microbiol. 17: 95-107; SEQ ID NO: 17), or an orthologue thereof in other yeast species (e.g. a glycerolphosphate dehydrogenase having an amino acid sequence with at least 70, 75, 80, 85, 90, 95, 98 or 99% sequence identity to SEQ ID NO: 17) are reduced or inactivated; while at least one endogenous gene encoding the *S. cerevisiae GPD1* gene as described by Albertyn et al. (1994, Mol. Cell. Biol. 14: 4135-4144; SEQ ID NO: 18) or an orthologue thereof in other yeast species (e.g. a glycerolphosphate dehydrogenase having an amino acid sequence with at least 70, 75, 80, 85, 90, 95, 98 or 99% sequence identity to SEQ ID NO: 18) is functional. The *S. cerevisiae GPD1* gene is the stress-induced glycerolphosphate dehydrogenase of *S. cerevisiae*, which is important for growth under osmotic stress as may occur under industrial fermentations conditions. Its expression is *inter alia* regulated by the high-osmolarity glycerol response pathway. It is therefore advantageous that a host cell of the invention has at least one functional copy of a endogenous *S. cerevisiae GPD1* gene or an orthologue thereof in other yeast species.

DNA sequences of genetic constructs for disruption of *GPD1* and *GPD2* genes in *S. cerevisiae* are provided SEQ ID NO.'s: 58 and 59, respectively.

Construction of yeast cells of the invention

In a second aspect the invention relates to methods for preparing or constructing the yeast cells of the invention. For this purpose standard genetic and molecular biology techniques are used that are generally known in the art and have e.g. been described by Sambrook and Russell (2001, "Molecular cloning: a laboratory manual" (3rd edition), Cold Spring Harbor Laboratory, Cold Spring Harbor Laboratory Press) and Ausubel et al. (1987, eds., "Current protocols in molecular biology", Green Publishing and Wiley Interscience, New York). Furthermore, the construction of mutated host yeast strains is carried out by genetic crosses, sporulation of the resulting diploids, tetrad dissection of the haploid spores containing the desired auxotrophic markers, and colony purification of such haploid host yeasts in the appropriate selection medium. All of these methods are standard yeast genetic methods known to those in the art. See, for example, Sherman et al., Methods Yeast Genetics, Cold Spring Harbor Laboratory, NY (1978) and Guthrie et al. (Eds.) Guide To Yeast Genetics and Molecular Biology Vol. 194, Academic Press, San Diego (1991).

The exogenous and/or endogenous genes coding for the above described enzymes that are be introduced into the yeast cell of the invention, preferably are expression constructs comprising the nucleotide sequence coding for the enzymes operably linked to suitable expression regulatory regions/sequences to ensure expression of the enzymes upon transformation of the expression constructs into the host cell of the invention. Thus, the gene or expression construct will at least comprise a promoter that is functional in the host cell operably linked to the coding sequence. The gene or construct may further comprise a 5' leader sequence upstream of the coding region and a 3'-nontranslated sequence (3'end) comprising a polyadenylation site and a transcription termination site downstream of the coding sequence. It is understood that the nucleotide sequences coding for the various enzymes to be introduced into the yeast cell of the invention, may be present together on a single expression construct, they may be present on two or more different expression constructs or each enzyme may be present on a separate expression construct.

There are various means available in the art for overexpression of enzymes in the yeast cells of the invention. In particular, an enzyme may be overexpressed by increasing the copynumber of the gene coding for the enzyme in the cell, e.g. by integrating additional copies of the gene in the cell's genome, by expressing the gene from an episomal multicopy expression vector or by introducing a episomal expression vector that comprises multiple copies of the gene. The coding sequence used for overexpression of the enzymes preferably is homologous to the yeast cell of the invention. However, coding sequences that are heterologous to the yeast cell of the invention may likewise be applied. Alternatively overexpression of enzymes in the cells of the invention may be achieved by using a promoter that is not native to the sequence coding for the enzyme to be overexpressed, i.e. a promoter that is heterologous to the coding sequence to which it is operably linked. Although the promoter preferably is heterologous to the coding sequence to which it is operably linked, it is also preferred that the promoter is homologous, i.e. endogenous to the cell of the invention. Preferably the heterologous promoter is capable of producing a higher steady state level of the transcript comprising the coding sequence (or is capable of producing more transcript molecules, i.e. mRNA molecules, per unit of time) than is the promoter that is native to the coding sequence, preferably under the fermentation conditions of the processes of the invention (see below).

Suitable promoters for expression of the nucleotide sequences coding for the enzymes to be introduced into the yeast cell of the invention include promoters that are preferably insensitive to catabolite (glucose) repression, that are active under anaerobic conditions and/or that preferably do not require xylose or arabinose for induction. Promoters having these characteristics are widely available and known to the skilled person. Suitable examples of such promoters include e.g. promoters from glycolytic genes such as the phosphofructokinase (*PPK*), triose phosphate isomerase (*TPI*), glyceraldehyde-3-phosphate dehydrogenase (*GPD, TDH3* or *GAPDH*), pyruvate kinase (*PYK*), phosphoglycerate kinase (*PGK*), glucose-6-phosphate isomerase promoter (*PGI1*) promoters from yeasts. More details about such promoters from yeast may be found in (WO 93/03159). Other useful promoters are promoters of gene encoding ribosomal proteins such as translation elongation factors (e.g. *TEF1*), the lactase gene promoter (*LAC4*), alcohol dehydrogenase promoters (*ADH1, ADH4*, and the like), the enolase promoter (*ENO*) and the hexose (glucose) transporter promoter (*HXT7*). Alternatively, a nucleotide sequences encoding an enzyme to be introduced into the yeast cell of the invention can be expressed under anaerobic conditions by using an anoxic promoter such as e.g. the *S. cerevisiae ANB1* promoter (SEQ ID NO: 60). Other promoters, both constitutive and inducible, and enhancers or upstream activating sequences will be known to those of skill in the art. Preferably the promoter that is operably linked to nucleotide sequence as defined above is homologous to the host cell. Suitable terminator sequences are e.g. obtainable from the cytochrome c1 (*CYC1*) gene or an alcohol dehydrogenase gene (e.g. *ADH1*).

To increase the likelihood that the enzymes to be introduced into the yeast cell of the invention are expressed at sufficient levels and in active form in the transformed yeast cells, the nucleotide sequence encoding these enzymes are preferably adapted to optimize their codon usage to that of the yeast cell in question. The adaptiveness of a nucleotide sequence encoding an enzyme to the codon usage of a host cell may be expressed as codon adaptation index (CAI). The codon adaptation index is herein defined as a measurement of the relative adaptiveness of the codon usage of a gene towards the codon usage of highly expressed genes in a particular host cell or organism. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (see Sharp and Li , 1987, Nucleic Acids Research 15: 1281-1295; also see: Jansen et al., 2003, Nucleic Acids Res. 31(8):2242-51). An adapted nucleotide sequence preferably has a CAI of at least 0.2, 0.3, 0.4, 0.5, 0.6, 0.7, 0.8 or 0.9. Most preferred are the sequences which have been codon optimized for expression in the yeast host cell in question such as e.g. *S. cerevisiae* cells.

The yeast cell transformed with the nucleic acid construct(s) comprising the nucleotide sequence encoding the enzymes to be introduced into the yeast cell of the invention preferably is a yeast cell capable of passive or active uronic acid transport into the cell. The yeast cell of the invention, further preferably is a cell capable of active or passive pentose (xylose and preferably also arabinose) transport into the cell. The yeast cell preferably contains active glycolysis. The yeast cell may further preferably contain an endogenous pentose phosphate pathway and may contain endogenous xylulose kinase activity so that xylulose isomerised from xylose may be metabolized to pyruvate. The yeast further preferably contains enzymes for conversion of a pentose (preferably through pyruvate) to a desired fermentation product such as ethanol, lactic acid, 3-hydroxy-propionic acid, acrylic acid, 1,3-propane-diol, butanols (1-butanol, 2-butanol, isobutanol) isoprenoid-derived products. A particularly preferred yeast cell is a yeast cell that is naturally capable of alcoholic fermentation, preferably, anaerobic alcoholic fermentation. The yeast cell further preferably has a high tolerance to ethanol, a high tolerance to low pH (i.e. capable of growth at a pH lower than 5, 4, or 3) and towards organic acids like lactic acid, acetic acid or formic acid and sugar degradation products such as furfural and hydroxy-methylfurfural, and a high tolerance to elevated temperatures. Any of these characteristics or activities of the yeast cell may be naturally present in the yeast cell or may be introduced or modified by genetic modification, preferably by self cloning or by the methods of the invention described below. A suitable cell is a cultured cell, a cell that may be cultured in fermentation process e.g. in submerged or solid state fermentation.

Yeasts are herein defined as eukaryotic microorganisms and include all species of the subdivision Eumycotina (Yeasts: characteristics and identification, J.A. Barnett, R.W. Payne, D. Yarrow, 2000, 3rd ed., Cambridge University Press, Cambridge UK; and, The yeasts, a taxonomic study, C.P. Kurtzman and J.W. Fell (eds) 1998, 4th ed., Elsevier Science Publ. B.V., Amsterdam, The Netherlands) that predominantly grow in unicellular form. Yeasts may either grow by budding of a unicellular thallus or may grow by fission of the organism. Preferred yeasts cells for use in the present invention belong to the genera *Saccharomyces, Kluyveromyces, Candida, Pichia, Schizosaccharomyces, Hansenula, Kloeckera, Schwanniomyces, Brettanomyces*, and *Yarrowia*. Preferably the yeast is capable of anaerobic fermentation, more preferably anaerobic alcoholic fermentation. Over the years suggestions have been made for the introduction of various organisms for the production of bio-ethanol from crop sugars. In practice, however, all major bio-ethanol production processes have continued to use the yeasts of the genus *Saccharomyces* as ethanol producer. This is due to the many attractive features of *Saccharomyces* species for industrial processes, i.e., a high tolerance for acidity, ethanol and high osmolarity, capability of anaerobic growth, and of course its high alcoholic fermentative capacity. Preferred yeast species for use in the present invention include *S. cerevisiae, S. exiguus, S. bayanus, K. lactis, K. marxianus* and *Schizosaccharomyces pombe*.

A yeast cell of the invention may be a haploid cell, however, preferably the yeast cell is a non-haploid cell such as e.g. a diploid, aneuploid or polyploid cell. Thus, the yeast cell preferably has a greater number of chromosomes than the haploid number (n) of chromosomes in a gamete, i.e. yeast spore. Non-haploid yeast strains are more robust and more stable compared to haploid strains with the same genotype and are therefore preferably used for industrial fermentation processes, including the production of ethanol.

A yeast cell of the invention further preferably comprises xylulose kinase activity so that xylulose isomerised from xylose may be metabolized to pyruvate. Preferably, the cell contains endogenous xylulose kinase activity. More preferably, a cell of the invention comprises a genetic modification that increases the specific xylulose kinase activity. Preferably the genetic modification causes overexpression of a xylulose kinase, e.g. by overexpression of a nucleotide sequence encoding a xylulose kinase. The gene encoding the xylulose kinase may be endogenous to the cell or may be a xylulose kinase that is heterologous to the cell. A nucleotide sequence that may be used for overexpression of xylulose kinase in the cells of the invention is e.g. the xylulose kinase gene from *S. cerevisiae* (*XKS1*) as described by Deng and Ho (1990, Appl. Biochem. Biotechnol. 24-25: 193-199). Another preferred xylulose kinase is a xylose kinase that is related to the xylulose kinase from *Piromyces* (*xylB*; see WO 03/0624430). In the cells of the invention, a xylulose kinase to be overexpressed is overexpressed by at least a factor 1.1, 1.2, 1.5, 2, 5, 10 or 20 as compared to a strain which is genetically identical except for the genetic modification causing the overexpression. It is to be understood that these levels of overexpression may apply to the steady state level of the enzyme's activity, the steady state level of the enzyme's protein as well as to the steady state level of the transcript coding for the enzyme.

A yeast cell of the invention further preferably comprises a genetic modification that increases the flux of the pentose phosphate pathway as described in WO 06/009434. In particular, the genetic modification causes an increased flux of the non-oxidative part pentose phosphate pathway. A genetic modification that causes an increased flux of the non-oxidative part of the pentose phosphate pathway is herein understood to mean a modification that increases the flux by at least a factor 1.1, 1.2, 1.5, 2, 5, 10 or 20 as compared to the flux in a strain which is genetically identical except for the genetic modification causing the increased flux. The flux of the non-oxidative part of the pentose phosphate pathway may be measured as described in WO 06/009434. Genetic modifications that increase the flux of the pentose phosphate pathway may be introduced in the cells of the invention in various ways. These including e.g. achieving higher steady state activity levels of xylulose kinase and/or one or more of the enzymes of the non-oxidative part pentose phosphate pathway and/or a reduced steady state level of unspecific aldose reductase activity. These changes in steady state activity levels may be effected by selection of mutants (spontaneous or induced by chemicals or radiation) and/or by recombinant DNA technology e.g. by overexpression or inactivation, respectively, of genes encoding the enzymes or factors regulating these genes. In a preferred cell of the invention, the genetic modification comprises overexpression of at least one enzyme of the (non-oxidative part) pentose phosphate pathway. Preferably the enzyme is selected from the group consisting of the enzymes encoding for ribulose-5-phosphate isomerase, ribulose-5-phosphate 3-epimerase, transketolase and transaldolase.

A further preferred yeast cell of the invention comprises a genetic modification that reduces unspecific aldose reductase activity in the cell. Preferably, unspecific aldose reductase activity is reduced in the yeast cell by one or more genetic modifications that reduce the expression of or inactivates a gene encoding an unspecific aldose reductase. Preferably, the genetic modifications reduce or inactivate the expression of each endogenous copy of a gene encoding an unspecific aldose reductase that is capable of reducing an aldopentose, including, xylose, xylulose and arabinose, in the cell's genome. A given cell may comprise multiple copies of genes encoding unspecific aldose reductases as a result of di-, poly- or aneu-ploidy, and/or a cell may contain several different (iso)enzymes with aldose reductase activity that differ in amino acid sequence and that are each encoded by a different gene. Also in such instances preferably the expression of each gene that encodes an unspecific aldose reductase is reduced or inactivated. Preferably, the gene is inactivated by deletion of at least part of the gene or by disruption of the gene, whereby in this context the term gene also includes any non-coding sequence up- or down-stream of the coding sequence, the (partial) deletion or inactivation of which results in a reduction of expression of unspecific aldose reductase activity in the yeast cell. A nucleotide sequence encoding an aldose reductase whose activity is to be reduced in the cell of the invention and amino acid sequences of such aldose reductases are described in WO 06/009434 and include e.g. the (unspecific) aldose reductase genes of *S. cerevisiae GRE3* gene (Träff et al., 2001, Appl. Environm. Microbiol. 67: 5668-5674) and orthologues thereof in other species.

A further preferred transformed yeast cell according to the invention may comprises further genetic modifications that result in one or more of the characteristics selected from the group consisting of (a) increased transport of xylose and/or arabinose into the cell; (b) decreased sensitivity to catabolite repression; (c) increased tolerance to ethanol, osmolarity or organic acids; and, (d) reduced production of by-products. By-products are understood to mean carbon-containing molecules other than the desired fermentation product and include e.g. xylitol, arabinitol, glycerol and/or acetic acid.

Any genetic modification described herein can be introduced by classical mutagenesis and screening and/or selection for the desired mutant, or simply by screening and/or selection for the spontaneous mutants with the desired characteristics.

Alternatively, the genetic modifications may consist of overexpression of endogenous genes and/or the inactivation of endogenous genes. Genes the overexpression of which is desired for increased transport of arabinose and/or xylose into the cell are preferably chosen form genes encoding a hexose or pentose transporter. In *S. cerevisiae* and other yeasts these genes include *HXT1, HXT2, HXT3, HXT4, HXT5, HXT7* and *GAL2*, of which *HXT7, HXT5* and *GAL2* are most preferred (see Sedlack and Ho, Yeast 2004; 21: 671-684). Another preferred transporter for expression in yeast is the glucose transporter encoded by the *P. stipitis SUT1* gene (Katahira et al., 2008, Enzyme Microb. Technol. 43: 115-119). Similarly orthologues of these transporter genes in other species may be overexpressed. Other genes that may be overexpressed in the cells of the invention include genes coding for glycolytic enzymes and/or ethanologenic enzymes such as alcohol dehydrogenases. Preferred endogenous genes for inactivation include hexose kinase genes e.g. the *S. cerevisiae HXK2* gene (see Diderich et al., 2001, Appl. Environ. Microbiol. 67: 1587-1593); the S. *cerevisiae MIG1* or *MIG2* genes; or (hybridizing) orthologues of these genes in other species. Other preferred further modifications of yeast cells for xylose fermentation are described in van Maris et al. (2006, Antonie van Leeuwenhoek 90:391-418), WO2006/009434, WO2005/023998, WO2005/111214, and WO2005/091733. Any of the genetic modifications of the cells of the invention as described herein are, in as far as possible, preferably introduced or modified by self cloning genetic modification.

In a preferred embodiment of a method for preparing or constructing the yeast cells of the invention, a yeast of the invention is further improved by evolutionary engineering. Thus, a yeast of the invention that has the ability of metabolizing uronic acids as described herein above, can be further subjected to a method for identifying a yeast cell/ strain that shows improved consumption ofuronic acids in comparison to the starting or reference yeast cell/strain to which the method is applied. The method may thus be used to improve the performance of an existing strain of yeast cells of the invention with respect to its ability to consume a uronic acid such as galacturonic acid or glucuronic acid, e.g. by selecting a strain of the organism which shows faster consumption of the uronic acid(s). Preferably, the method is used to select a strain which has improved consumption on a carbon source comprising a uronic acid so that it shows improved fermentation characteristics. Thus, a strain which has been selected according to the invention may show improved performance in terms of increased productivity, e.g. on a volumetric basis, of the fermentation product in question. Also, or alternatively, a strain selected using this method may show an increase in yield of the fermentation product (in comparison to the starting strain from which it was selected). In the method of the invention, a population of the yeast cell of the invention is grown, that is to say selected, in the presence a uronic acid, preferably at least one of galacturonic acid and glucuronic acid. Preferably the yeast cell of the invention is grown/ selected on the uronic acid(s) as sole carbon source. Growth of the population of the yeast cell on the indicated carbon sources exerts selection pressure on the population. Thus, mutants in the population may be selected for with an increased maximum specific growth rate ($\mu$max) on the uronic acid(s) carbon source. If the selection pressure is maintained, e.g. by sequentially transferring batch-wise grown cultures to new batches, eventually (mutant) cells with a higher specific growth rate will overgrow all other cells with a lower specific growth rate. The process of growing the yeast cells may e.g. be operated in batch culture, as a fed batch fermentation with constant feed or as a continuous fermentation, e.g. in a chemostat. A method for selecting a strain the yeast cell of the invention capable of improved consumption of a uronic acid carbon sources as compared to a reference strain of the yeast cell, which method comprises: a) growing a population of the reference strain of the yeast cell in the presence of a uronic acid carbon source and b) selecting from the population a resulting strain of the yeast cell capable of improved consumption of a uronic acid carbon source as compared to the reference strain of the organism. Preferably the method is a method, wherein the growth of the population of the yeast cell is carried out by cultivation in sequential batch reactors (SBR). The method can be carried out under anaerobic conditions or the method can be carried out under aerobic conditions, preferably under oxygen limited conditions aerobic conditions.

A preferred yeast cell according to the invention has the ability to grow on at least one of glucuronic acid and galacturonic acid as carbon/energy source, preferably as sole carbon/energy source, and preferably under oxygen-limited aerobic conditions, more preferably under anaerobic conditions. Oxygen-limited aerobic conditions and anaerobic conditions as herein defined below.

A preferred yeast cell of the invention has the ability to grow on at least one of a hexose, a pentose, a uronic acid and combinations thereof at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under oxygen-limited aerobic or anaerobic conditions. Therefore, preferably the yeast cell has the ability to grow on at least one of glucuronic acid and galacturonic acid as sole carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under oxygen-limited aerobic or anaerobic conditions. More preferably, the yeast cell has the ability to grow on a mixture of a hexose (e.g. sucrose, glucose, fructose or galactose) and at least on one of xylose and arabinose and at least on one of glucuronic acid and galacturonic acid as carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under oxygen-limited aerobic or anaerobic conditions. Most preferably, the yeast cell has the ability to grow on a mixture of a hexoses (e.g. sucrose, glucose, fructose or galactose) and at least on one of xylose and arabinose and at least on one of glucuronic acid and galacturonic acid as carbon/energy source at a rate of at least 0.01, 0.02, 0.05, 0.1, 0.2, 0,25 or 0,3 $h^{-1}$ under aerobic conditions, or, more preferably, at a rate of at least 0.005, 0.01, 0.02, 0.05, 0.08, 0.1, 0.12, 0.15 or 0.2 $h^{-1}$ under oxygen-limited aerobic or anaerobic conditions.

## Further aspects of the invention

In a third aspect, the invention relates to the use of a yeast cell according to the invention for the preparation of a fermentation product selected from the group consisting of ethanol, lactic acid, 3-hydroxy-propionic acid, acrylic acid, 1,3-propane-diol, butanols and isoprenoid-derived products. Preferably the yeast cell is used for the preparation of a fermentation product from a uronic acid as carbon source, more preferably at least one of galacturonic acid and glucuronic acid as carbon source.

In another aspect the invention relates to a process for producing a fermentation product selected from the group consisting of ethanol, lactic acid, 3-hydroxy-propionic acid, acrylic acid, 1,3-propane-diol, butanols (1-butanol, 2-butanol, isobutanol) and isoprenoid-derived products. The process preferably comprises the step of: a) fermenting a medium with a yeast cell, whereby the medium contains or is fed with: a) a source of a uronic acid and whereby the yeast cell ferments the uronic acid to the fermentation product. The source of the uronic acid preferably is a source of at least one of galacturonic acid and glucuronic acid. The yeast cell preferably is a yeast cell as herein defined above. The process preferably comprises one or more further steps wherein the fermentation product is recovered. The process may be a batch process, a fed-batch process or a continuous process as are well known in the art.

In a preferred process the medium further contains or is fed with a source of at least one of a hexose, a pentose and glycerol. A source of at least one of a hexose, a pentose and glycerol comprises or consist of: hexose and pentose; hexose and glycerol; pentose and glycerol; hexose, pentose and glycerol. In a further preferred process, the medium further contains or is fed with a source of acetic acid. In the process of the invention, the source of glycerol may be a any carbon source that has a more reduced state than glucose. A carbon source having a more reduced state than glucose is understood as a carbon source of which the average reduction state per C-mol (of the compounds therein) is higher than the reduction state per C-mol of glucose. Examples of carbon sources having a more reduced state than glucose include e.g. alkanols such as propanol and butanol; polyols such as 1,3-propane-diol, butandiol, glycerol, mannitol and xylitol.

Preferably, the medium fermented by the cells of the invention comprises or is fed with (fractions of) hydrolyzed biomass comprising the uronic acid, preferably at least one of galacturonic acid and glucuronic acid. Suitable sources of uronic acids are pectin-rich residues which accumulate when sugar is extracted from sugar beet, i.e. sugar beet pulp, and when juices are produced from fruits such as citrus and apple, i.e. apple or citrus fruit pulp, peel, and/or rag. Another suitable source of uronic acids are pectins in hemicellulose, e.g. as present in lignocellulosic biomass.

Pectins are complex and heterogeneous polymers that primarily act as hydrating and cementing agents for the cellulosic matrix of plant cell walls. The principal unit in pectin chains is $\alpha$-(1-4) linked galacturonic acid. The galacturonic acid residues can be esterified with methyl and acetyl groups. Additionally, pectin contains the branched polysaccharides rhamnogalacturonan I, rhamnogalacturonan II and xylogalacturonan (see Richard and Hilditch, 2009, *supra).* The various pectins or pectic substances can be hydrolyzed by simply boiling in 2.5% sulphuric acid. However, preferably they are hydrolyzed by pectinolytic enzymes as are known in the art. Hydrolysis of pectin can e.g. be accomplished by a mixture of enzymes, comprising one or more of a pectin methyl esterase, a pectin acetyl esterase, an endo-polygalacturonase, an endo-pectin lyase, a rhamnogalacturonan hydrolyase, a rhamnogalacturonan lyase, a rhamnogalacturonan acetyl esterase, an arabinofuranosidase, an endo-arabinose, an endo-galactanase and $\beta$-galactosidase see e.g. Kashyap et al. (2001, Bioresour Technol. 77:215-227), Hoondal et al. (2002, Appl Microbiol Biotechnol 59:409-418), Baciu and Jördening (2004, Enzyme Microb. Technol. 34:505-512), Jayani et al. (2005, Process Biochem 40:2931-2944) and references therein. In addition to galacturonic acid the (fractions of) hydrolyzed pectic substances acid can also comprise glucuronic acid, galactose, xylose, arabinose, acetic acid (or a salt thereof), all of which can be consumed by the yeast cells of the invention.

In the process of the invention, the sources of uronic acid may be galacturonic acid and glucuronic acid as such (i.e. as monomeric uronic acids) or they may be in a polymeric form such as e.g. above described pectic substances. For release of the various monomeric units from the polymeric pectic substances, appropriate pectinolytic enzymes may be added to the fermentation medium or may be produced by a yeast cell of the invention. In the latter case the yeast cell may be genetically engineered to produce and excrete such pectinolytic enzymes.

In the process of the invention, the medium further preferably comprises and/or is fed a source of glycerol. Glycerol for use in the process of the present invention may advantageously be glycerol that is generated as a by-product in biodiesel production from transesterification reactions using vegetable oils or animal fats and an alcohol.

In a preferred process the medium fermented by the cells of the invention comprises or is fed with, in addition to the above-described sources of uronic acid, sources of hexoses and/or pentoses. The source of hexose comprises or consists of at least one of glucose, fructose, sucrose, maltose, galactose and lactose. Preferably the source of pentose comprises or consists of at least one of xylose and arabinose, of which xylose is preferred. Preferably, the medium fermented by the cells of the invention comprises or is fed with (fractions of) hydrolyzed biomass comprising at least one at least one of a hexose and a pentose such as glucose, xylose and/or arabinose. The (fractions of) hydrolyzed biomass comprising the hexoses and pentose will usually also comprise acetic acid (or a salt thereof). An example of hydrolyzed biomass to be fermented in the processes of the invention is e.g. hydrolyzed lignocellulosic biomass. Examples of lignocellulosic biomass to be hydrolyzed for use in the present invention include agricultural residues (including e.g. empty fruit bunches (EFB) of oil palm, corn stover and sugarcane bagasse), wood residues (including sawmill and paper mill discards and (municipal) paper waste. Methods for hydrolysis of biomass such as lignocelluloses are known in the art per se and include e.g. acids, such as sulphuric acid and enzymes such as cellulases and hemicellulases. In the process of the invention, the sources of xylose, glucose and arabinose may be xylose, glucose and arabinose as such (i.e. as monomeric sugars) or they may be in the form of any carbohydrate oligo- or polymer comprising xylose, glucose and/or arabinose units, such as e.g. lignocellulose, arabinans, xylans, cellulose, starch and the like. For release of xylose, glucose and/or arabinose units from such carbohydrates, appropriate carbohydrases (such as arabinases, xylanases, glucanases, amylases, cellulases, glucanases and the like) may be added to the fermentation medium or may be produced by the modified host cell. In the latter case the modified host cell may be genetically engineered to produce and excrete such carbohydrases. An additional advantage of using oligo- or polymeric sources of glucose is that it enables to maintain a low(er) concentration of free glucose during the fermentation, e.g. by using rate-limiting amounts of the carbohydrases preferably during the fermentation. This, in turn, will prevent repression of systems required for metabolism and transport of non-glucose sugars such as xylose and arabinose. In a preferred process the modified host cell ferments both the glucose and at least one of xylose and arabinose, preferably simultaneously in which case preferably a modified host cell is used which is insensitive to glucose repression to prevent diauxic growth. In addition to a source of at least one of a hexose, a pentose and glycerol, as carbon source, the fermentation medium will further comprise the appropriate ingredients required for growth of the modified host cell. Compositions of fermentation media for growth of eukaryotic microorganisms such as yeasts are well known in the art.

The fermentation process may be an aerobic or an anaerobic fermentation process. An anaerobic fermentation process is herein defined as a fermentation process run in the absence of oxygen or in which substantially no oxygen is consumed, preferably less than 5, 2.5 or 1 mmol/L/h, more preferably 0 mmol/L/h is consumed (i.e. oxygen consumption is not detectable), and wherein organic molecules serve as both electron donor and electron acceptors. In the absence of oxygen, NADH produced in glycolysis and biomass formation, cannot be oxidized by oxidative phosphorylation. To solve this problem many microorganisms use pyruvate or one of its derivatives as an electron and hydrogen acceptor thereby regenerating $NAD^+$. Thus, in a preferred anaerobic fermentation process pyruvate is used as an electron (and hydrogen acceptor) and is reduced to fermentation products such as ethanol, as well as non-ethanol fermentation products such as lactic acid, 3-hydroxy-propionic acid, acrylic acid, 1,3-propane-diol, butanols (1-butanol, 2-butanol, isobutanol) and isoprenoid-derived products, preferably under concomitant production of formate. Anaerobic processes of the invention are preferred over aerobic processes because anaerobic processes do not require investments and energy for aeration and in addition, anaerobic processes produce higher product yields than aerobic processes. Alternatively, the fermentation process of the invention may be run under aerobic oxygen-limited conditions. Preferably, in an aerobic process under oxygen-limited conditions, the rate of oxygen consumption is at least 5.5, more preferably at least 6 and even more preferably at least 7 mmol/L/h. In a preferred aerobic oxygen-limited fermentation process of the invention, the yeast cell of the invention consumes less than 30, 20, 18, 15, 12, 10, 8 or 5% of the amount of oxygen on a C-molar basis related to the carbon source consumed during the conversion of the carbon source into the fermentation product. The conversion coefficient of oxygen consumed over substrate utilised on a C-molar basis ($C_{OS}$) is herein understood to mean mol $O_2$ used per C-mol carbon source consumed. Thus, a process of the invention can be carried out under strict anaerobic conditions (i.e. $C_{OS}$ = 0.0), or the process of the invention can be carried out under aerobic, preferably oxygen-limited conditions wherein the $C_{OS}$ is preferably less than 0.3, 0.2, 0.18, 0.15, 0.12, 0.1, 0.08, or 0.05.

The fermentation process is preferably run at a temperature that is optimal for the modified cells of the invention. Thus, for most yeasts cells, the fermentation process is performed at a temperature which is less than 42°C, preferably less than 38°C. For yeast cells, the fermentation process is preferably performed at a temperature which is lower than 35, 33, 30 or 28°C and at a temperature which is higher than 20, 22, or 25°C.

The fermentation process is preferably run at a pH that is acceptable to the yeast cell that is used in the process, based on common general knowledge or as can be routinely determined. Usually fermentation process is run at a neutral or acidic pH, preferably at a pH less than pH, 7.0, 6.0, 5.5, 5.0 or 4.5 and at pH higher than pH 2.0, 3.0, 3.5 or 4.0.

A preferred fermentation process according to the invention is a process for the production of ethanol, whereby the process comprises the step of fermenting a medium with a yeast cell, whereby the medium contains or is fed with a source of a uronic acid and optionally a source of at least one of a hexose, a pentose and glycerol and whereby the yeast cell ferments the uronic acid and optionally the at least one of a hexose, pentose and glycerol to ethanol. Optionally the process comprises the step of recovery of at least one of ethanol. The fermentation may further be performed as described above. In the process the volumetric ethanol productivity is preferably at least 0.5, 1.0, 1.5, 2.0, 2.5, 3.0, 5.0 or 10.0 g ethanol per liter per hour. The ethanol yield on the uronic acid, and optionally the hexose and/or pentose and/or glycerol (and/or acetate) in the process preferably is at least 50, 60, 70, 80, 90, 95 or 98%. The ethanol yield is herein defined as a percentage of the theoretical maximum yield, which, for xylose, glucose and arabinose is 0.51 g. ethanol per g. hexose or pentose. For glycerol the theoretical maximum yield is 0.50 g. ethanol per g. glycerol and for acetic acid the theoretical maximum yield is 0.77 g. ethanol per g. acetic acid. For the uronic acids galacturonic acid and glucuronic acid the theoretical maximum yield is 0.39 g. ethanol per g. uronic acid.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The following examples are offered for illustrative purposes only, and are not intended to limit the scope of the present invention in any way.

Figure 1. Schematic depiction of an engineered metabolic pathway introduced into yeast for converting glucuronic acid into glucose-6P. The relevant enzymes of the pathways are indicated by the corresponding EC numbers. Enzymes that are exogenous to yeast are indicated with EC numbers in bold italics and with open or thin arrows. Enzymes that are endogenous to yeast are indicated with with EC numbers in normal letters and with filled arrows. Yeast strains RN1136, RN1137, RN1138, RN1139, RN1160 and RN1161 described in the Examples herein use this pathway.

Figure 2. Schematic depiction of an engineered metabolic pathway introduced into yeast for converting galacturonic acid into glucose-6P. The relevant enzymes of the pathways are indicated by the corresponding EC numbers. Enzymes that are exogenous to yeast are indicated with EC numbers in bold italics and with open or thin arrows. Enzymes that are endogenous to yeast are indicated with with EC numbers in normal letters and with filled arrows. Yeast strains RN 1140 and RN 1162 described in the Examples herein use this pathway.

Figure 3. Schematic depiction of an engineered metabolic pathway introduced into yeast for converting galacturonic acid into glucose-6P. The relevant enzymes of the pathways are indicated by the corresponding EC numbers. Enzymes that are exogenous to yeast are indicated with EC numbers in bold italics and with open arrows. Enzymes that are endogenous to yeast are indicated with with EC numbers in normal letters and with filled arrows.. Yeast strains RN1141, RN1142, RN1143 and RN1163 described in the Examples herein use this pathway.

## Examples

### 1. Construction of yeast cells of the invention

All promoter and terminator sequences used in the construction of expression cassettes are publicly available and are their sequences are indicated in the sequence listing. Synthetic DNA fragments were prepared comprising codon optimized (for expression in *S. cerevisiae*) open reading frames coding for the enzymes to be expressed in the yeast cells of the invention. In Table 1 the Codon Adaptation Index (CAI) is given for the various coding sequences including references to the sequence listing.

Table 1: Overview of the codon optimized synthetic DNA fragments comprising the open reading frames coding for the various enzymes identified by their respective EC numbers. SEQ ID NO.'s for the corresponding amino acid sequences (aa) and nucleotide sequences (nt) are also given.

| Enzyme | CAI | L_aa | SEQ ID NO: aa | SEQ ID NO: nt | ORF |
|---|---|---|---|---|---|
| 2.7.1.44_ | 0.956 | 424 | 1 | 19 | 10-1284 |
| 2.7.1.43 | 0.968 | 362 | 2 | 20 | 10-1098 |
| 2.7.7.64_(Ps) | 0.956 | 600 | 3 | 21 | 10-1812 |
| 2.7.7.64_(At) | 0.996 | 614 | 4 | 22 | 10-1854 |
| 1.1.1.22 PA2022 | 0.963 | 453 | 5 | 23 | 10-1368 |
| 1.1.1.22 PA3559 | 0.938 | 464 | 6 | 24 | 10-1401 |
| 5.1.3.6 | 1.000 | 429 | 7 | 25 | 11-1300 |

### 1.1 Construction of expression vector plasmids pRN830, pRN831 and pRN906

First, expression cassettes for the expression of the genes encoding the enzymes EC 2.7.7.64 (based on *Pisum sativum* USP - AB178642) and EC 1.1.1.22 (based on *Pseudomonas aeruginosa* PAO1 UGDH PA2022 - NP_250712) are made. The plasmids are based on pRN599, a shuttle vector plasmid with a 2μ origin of replication and a kanMX selection marker encoding G418 resistance (SEQ ID NO: 26).

The EC 2.7.7.64 Ps expression cassette (SEQ ID NO: 27) is prepared by ligating the TEF1 promoter (cut with the restriction enzymes *Age*I and *Hind*III), the synthetic ORF (cut with *Hind*III and *Bss*HII) and the ADH1 terminator (cut with *Bss*HII and *Sal*I) together in pCRII (InVitrogen) to yield plasmid pPS27764.

Next, ANB1- and ADH1-expression cassettes are made for the *Pseudomonas aeruginosa* PAO1 UGDH PA2022 coding sequence (SEQ ID NO: 23). The EC 1.1.1.22 PA2022 Pa-anb expression cassette (SEQ ID NO: 28) is prepared by ligating the ANB1 promoter (cut with the restriction enzymes *Bsi*WI and *Pst*I), the synthetic ORF (CAI = 0.963, cut with *Pst*I and *Bam*HI) and the CYC1 terminator (cut with *Bam*HI and *Sal*I) together in pCRII (invitrogen) to yield plasmid pPA11122ANB.

The EC 1.1.1.22 PA2022 Pa-adh expression cassette (SEQ ID NO: 29) is prepared by ligating the ADH1 promoter (cut with the restriction enzymes *Bsi*WI and *Pst*I), the synthetic ORF (CAI = 0.963, cut with *Pst*I and *Bam*HI) and the CYC1 terminator (cut with *Bam*HI and *Sal*I) together in pCRII (invitrogen) to yield plasmid pPA11122ADH.

Similar ANB1- and ADH1-expression cassettes are made for the *Pseudomonas aeruginosa* PAO1 UGDH PA3559 coding sequence (SEQ ID NO: 24).

Next, expression vector plasmids pRN830 and pR831 are constructed as follows. For the final construction of pRN830, the shuttle vector pRN599 is cut with *Acc*65I en *BspEI,* this vector is combined with the inserts from pPA11122ANB cut with *Sal*I en *BsiWI* (2458bp) and pPS27764 cut with *Age*I en *Sal*I (2577bp) to produce pRN830 (SEQ ID NO: 30). For the final construction of pRN831, the shuttlevector pRN599 is cut with *Acc*65I en *Bsp*EI*,* this vector is combined with the inserts from pPA11122ADH cut with *Sal*I en *Bsi*WI (2369bp) and pPS27764 cut with *Age*I en *Sal*I (2577bp) to produce pRN831 (SEQ ID NO: 31).

The EC 1.1.1.22 PA3559-anb expression cassette (SEQ ID NO: 55) is prepared by ligating the ANB1 promoter (cut with the restriction enzymes *BsiWI* and *Pst*I), the synthetic ORF (CAI = 0.938, cut with *Pst*I and *Bam*HI) and the CYC1 terminator (cut with *Bam*HI and *Sal*I) together in pCRII (invitrogen) to yield plasmid pPA111223559ANB. For the final construction of pRN906, the shuttle vector pRN599 is cut with *Acc*65I en *Bsp*EI*,* this vector is combined with the inserts from pPA111223559ANB cut with *Sal*I en *Bsi*W*I* (2491bp) and pPS27764 cut with *Age*I en *Sal*I (2577bp) to produce pRN906 (SEQ ID NO: 56).

<u>1.2 Construction of expression vector plasmids pRN761, pRN762, pRN832, pRN833, pRN834 and pRN907</u>

First, expression cassettes for the expression of the genes encoding the enzymes EC 2.7.1.43 (based on *Pisum sativum* USP - AB178642), EC2.7.1.44 (based on *Arabidopsis thaliana* GalAK -Y250F FJ439676) and EC 2.7.7.9 (*Saccharomyces cerevisiae* UGP1) are made. The plasmids are based on pRN600, a shuttle vector plasmid with a CEN/ARS sequence and a hphMX selection marker encoding hygromycin resistance (SEQ ID NO: 32).

<u>Construction of expression vector plasmid pRN761</u>

Expression cassettes for the expression of the genes encoding the enzymes EC 2.7.1.44 Y250F mutant (Yang *et al.*, 2009, *supra*; based on *Arabidopsis thaliana* GalAK- FJ439676) and EC 5.1.3.6 (based on *Arabidopsis thaliana* GAE1 - AT4G30440) are prepared as follows.

The plasmid is based on pRN600, a shuttle vector plasmid with a CENIV/ARS origin of replication and a hphMX selection marker encoding hygromycine resistance (SEQ ID NO: 32).

The EC 2.7.1.44 At Y250F expression cassette (SEQ ID NO: 33) is prepared by ligating the TDH3 promoter (cut with the restriction enzymes *Acc65I* and *Eco*RI), the synthetic ORF (cut with *Eco*RI and *Bam*HI) and the CYC1 terminator (cut with *Bam*HI and *Xho*I) together in pCRII (invitrogen) to yield plasmid pAT27144.

The EC 5.1.3.6 At expression cassette (SEQ ID NO: 34) is prepared by ligating the ACT 1 promoter (cut with the restriction enzymes *Spe*I and *Pst*I), the synthetic ORF (cut with *Nsi*I and *Bss*HII) and the ADH1 terminator (cut with *Bss*HII and *Bsi*WI) together in pCRII (invitrogen) to yield plasmid pAT5136.

For the final construction of pRN761, the shuttle vector pRN600 is cut with *Xho*I and *Spe*I, this vector is combined with the inserts from pAT5136 cut with *Spe*I and *Bsi*WI (2242bp) and pAT27144 cut with *Acc*65I and *Xho*I (2189bp) to produce pRN761 (SEQ ID NO: 35).

<u>Construction of expression vector plasmid pRN762</u>

An expression cassette for the expression of the gene encoding the enzyme EC 2.7.1.43 (based on Arabidopsis thaliana GlcAK - Q93ZC9) is prepared as follows.

The plasmid is based on pRN615, a shuttle vector plasmid with a 2μ origin of replication and a zeoMX selection marker encoding phleomycine resistance (SEQ ID NO: 36).

The EC 2.7.1.43 At expression cassette (SEQ ID NO: 37) is prepared by ligating the TDH3 promoter (cut with the restriction enzymes Acc65I and *Eco*RI), the synthetic ORF (cut with *Eco*RI and *Bam*HI) and the CYC1 terminator (cut with *Bam*HI and *Xho*I) together in pCRII (invitrogen) to yield plasmid pAT27143.

For the final construction of pRN762, the shuttle vector pRN615 is cut with *Acc65I* and *XhoI,* this vector is combined with the insert from pAT27143 cut with *Acc65I* and *XhoI* (2056bp) to produce pRN762 (SEQ ID NO: 38).

The EC2.7.7.9 Sc UGP1 overexpression cassette (SEQ ID NO: 40) is prepared by ligating the PGK1 promoter (cut with *SacI* en *Pst*I), the UGP1 ORF (PCR with primers ugp1f and ugp1r (see Table 5) - the PCR product is cut with *Pst*I and *Bss*HII) and the ADH1 terminator (cut with *Bss*HII en *Xho*I) together in pCRII (invitrogen) to yield plasmid pUGP1.

For the final construction of pRN832, the shuttle vector pRN600 is cut with *KpnI* and-XhoI. This vector is combined with the insert from pAT27144 cut with *KpnI* and *XhoI* (2240bp) to produce pRN832 (SEQ ID NO: 41).

For the final construction of pRN833, the shuttle vector pRN600 is cut with *Xba*I and *Kpn*I, this vector is combined with the EC 2.7.1.43 At expression cassette from pRN762 cut with *Xba*I and *Kpn*I (2119bp) to produce pRN833 (SEQ ID NO: 42).

For the final construction of pRN834, the shuttle vector pRN833 is cut with *Xho*I and *SacI,* this vector is combined with the inserts from pUGP1 cut with *SacI* en *XhoI* to produce pRN834 (SEQ ID NO: 43).

For the final construction of pRN907, the shuttle vector pRN600 is cut with *KpnI*I and *Sac*I, in this vector the inserts of pUGP1 cut with *Sac*I en *Xho*I (2620 bp) and pAT27144 (Y250F) cut with *Kpn*I and *Xho*I (2240bp) are ligated to produce pRN907 (SEQ ID NO: 57).

1.3 Construction of overexpression integration plasmid pRN835

The integration construct is prepared by amplifying a 570 bp N-terminal fragment from the GAL7 ORF (the complete ORF is 1101bp) with PCR with primers gal7f and gal7r (see Table 5). The PCR product is cut with *Xba*I and *Bsp*EI and ligating to pRN793 cut with *Xba*I and *Bsp*EI to produce pRN835 (SEQ ID NO: 44). For integration of pRN835 into the genome the construct is linearised with *Bgl*II.

Upon correct integration the TEF1 promoter drives the expression of GAL7, overexpression is verified with Q-PCR.

1.4 Construction of *S.cerevisiae* strains RN1135 and RN1159 transformation with expression constructs

RN1070 is constructed from RN1001. RN1001 is derived from RN1000 (genotype described in WO 2010/074577), has a CEN.PK102-3A background and further has the genotype: *MAT a, ura3-52, leu2-112, gre3::loxP, loxP-Ptpi::TAL1, loxP-Ptpi::RKI1, loxP-Ptpi-TKL1, loxP-Ptpi-RPE1, delta::Padh1XKS1Tcyc1-LEU2, delta::URA3-Ptpi-xylA-Tcyc1.*

RN1070 was obtained as an autodiploid of RN1001 by ectopic expression of the HO gene using plasmid pFL39 KanMX-GAL1HO (comprising a G418 resistance marker and the GAL1 promoter fused to HO as described in Teunissen et al. (2002, Appl. Environ. Microbiol. 68: 4780-4787). The forced mating type switch followed by mating with the original cells results in diploid MATa/MATα cells. The diploid nature was confirmed by specific PCR on the MAT locus. The pFL39 plasmid is cured from the cells by cultivation (>10 generations) in non selective medium (xylose as carbon source) and testing for G418 sensitivity. The genotype of RN1070 is: *MATa /MATα, ura3-52/ ura3-52, leu2-112/leu2-112, gre3:: loxP/ gre3::loxP, loxP-Ptpi::TAL1/ loxP-Ptpi::TAL1, loxP-Ptpi::RKI1/ loxP-Ptpi::RKI1, loxP-Ptpi-TKL1/ loxP-Ptpi-TKL1, loxP-Ptpi-RPE1/ loxP-Ptpi-RPE1, delta::Padh1XKS1Tcyc1-LEU2/ delta::Padh1XKS1Tcyc1-LEU2, delta:: URA3-Ptpi-xylA-Tcyc1 delta:: URA3-Ptpi-xylA-Tcyc1*

RN1030 (n) and RN1100 (2n) were constructed from CEN.PK113-7A. In the haploid laboratory strain CEN.PK113-7A the HIS3 gene is deleted by insertion of the loxP sequence (one step gene deletion method: the loxP-KanMX -loxP construct is flanked by his3 up and downstream sequences). This fragment is integrated into the genome (selected for G418 resistance) CRE mediated recombination resulted in marker removal leaving a footprint of one loxP site). The same method is used to delete in this strain also the LYS2 gene. Deletions are PCR verified. The resulting strain requires both histidine and lysine for growth.

Plasmid pRN792 (SEQ ID NO: 45) contains the N-terminal part of the GAL2 gene fused to the TPI1 promoter sequence. This plasmid contains the kanMX dominant marker. The plasmid lacks 2μ, ARS or CEN sequences and therefore not able to replicate in yeast. By restriction enzyme digestion with *Bst*BI (unique in pRN792) the plasmid is linearized. Transformation of yeast with this fragment results in forced integration in the GAL2 locus by homologous recombination. Cre mediated recombination results in removal of both marker and plasmid sequences. The resulting yeast displays a TPI1 promoter driven GAL2 (over-)expression (qPCR verified).

Next, two Ty constructs were used to transform the GAL2 overexpressing yeast strain. The first construct (the insert of plasmid pRN693, linearized by restriction digestion with *Bam*HI; see SEQ ID NO 46) contains the overexpression constructs for the oxidative part of the pentose phosphate pathway (Ptpi1-TAL1-Tadh1, Ptef1-TKL1-Tpgi1, Ptdh3- RPE1-Tpgi1 and Ptdh3-RKI1-Tcyc1), in addition to the HIS3 gene. This construct is integrated by homologous recombination at one of the endogenous TY1 elements. Transformants are selected by complementation of the histidine auxotrophy. Similarly the second construct, the insert of pRN755 (linearized by restriction digestion with *Nsi*I; see SEQ ID NO: 47) is integrated by selection for complementation of the lysine auxotrophy. This insert of pRN755 contains, in addition to the LYS2 gene, expression cassettes for the araA, -B and -D genes of *Arthrobacter aurescens* as are described in WO 2009/011591 (Phxt7-araA-Tpgi1; Ptpi1-araB-Tadh1; Ptdh3-araD-Tcyc1). Transformants were selected on mineral medium with arabinose as single carbon source. The best growing transformant RN1030 was selected for further modification. The genotype of RN1030 is: *MATa, his3::loxP, lys2::loxP, loxP-Ptpi-GAL2, delta::araA-araB-araD-LYS2, delta: : Ptpi1-TAL1-Tadh1+Ptef1-TKL1-Tpgi1 +Ptdh3- RPE1-Tpgi1 +Ptdh3-RKII-Tcycl +HIS3.*

An autodiploid of RN1030 is obtained by ectopic expression of the HO gene using pFL39 KanMX-GAL1 HO as described above (Teunissen et al., 2002, *supra*). The forced mating type switch followed by mating with the original cells results in diploid MATa/MATα cells. The diploid nature was confirmed by specific PCR on the MAT locus. The pFL39 plasmid is cured from the cells by cultivation (>10 generations) in non selective medium (arabinose as carbon source) and testing for G418 sensitivity. A thus obtained diploid (MATa/MATα) strain is named RN1100 (genotype is PCR verified). The genotype of RN1100 is: *MATa/MATα, his3::loxP /his3::loxP, lys2::loxP/lys2::loxP, loxP-Ptpi-GAL2/*

*loxP-Ptpi-GAL2, delta::araA-araB-araD-LYSI delta::araA-araB-araD-LYS2, delta:: Ptpi1-TAL1-Tadh1+Ptef1- TKL1-Tpgi1+Ptdh3-RPE1-Tpgi1+Ptdh3-RKI1-Tcyc1+HIS3I delta:: Ptpi1-TAL1-Tadh1+Ptef1- TKLI-Tpgi1+Ptdh3- RPE1-Tpgi1 +Ptdh3-RKII-Tcycl +HIS3.*

Both RN1070 and RN1100 were modified to obtain galactose-independent over expression of the endogenous *PGM2* phosphoglucomutase gene. To this end the native *PGM2* promoters are replaced by the *TEF1* promoter via integration of the plasmid pRN914.

For construction of pRN914 an N-terminal fragment (the first 551bp from the total of 1710bp) of the *PGM2* gene is amplified with the primers pgm2f and pgm2r (see

Table 5). The fragment is cut with the restriction enzymes *Xba*I and *Bsp*EI and ligated in pRN835 cut with the same enzymes to create plasmid pRN914 (SEQ ID NO: 48). pRN914 is linearised with restriction enzyme *Nde*I and then used for transformation of strains RN1070 and RN1100. The fragment between the loxP sites including the marker and plasmid sequences are removed after cre-recombinase expression, to create strains RN1135 and RN1159 respectively. Proper integration and marker removal is PCR verified, overexpression is verified with Q-PCR.

Fermentation on both xylose and arabinose is slightly improved in RN1135 and RN1159 as compared to RN1070 and RN1100, respectively, as a result of the PGM2 overexpression. The genotype of the strains RN1135 and RN1159 is:

RN1035: *MATa IMATα, ura3-52I ura3-52, leu2-112Ileu2-112, gre3::loxPI gre3::loxP, loxP-Ptpi::TAL1I loxP-Ptpi:: TAL1, loxP-Ptpi::RKI1I loxP-Ptpi::RKI1, loxP-Ptpi- TKL1I loxP-Ptpi-TKL1, loxP-Ptpi-RPE1I loxP-Ptpi-RPE1, delta:: Padh1XKS1Tcyc1-LEU2I delta::Padh1XKS1Tcycl-LEU2, delta::URA3-Ptpi-xylA-Tcycl delta:: URA3-Ptpi-xylA-Tcycl, loxP-Ptef1::PGM2.*

RN1159: *MATaIMATα, his3::loxP Ihis3::loxP, lys2::loxPIlys2::loxP, loxP-Ptpi-GAL2I loxP-Ptpi-GAL2, delta::araA-araB-araD-LYS2I delta::araA-araB-araD-LYS2, delta:: Ptpi1-TAL1-Tadh1+Ptef1- TKL1-Tpgi1+Ptdh3- RPE1-Tpgi1+Ptdh3-RKI1-Tcyc1+HIS3I delta:: Ptpi1-TAL1-Tadh1+Ptef1-TKL1-Tpgi1+Ptdh3- RPE1-Tpgi1+Ptdh3-RKI1-Tcyc1+HIS3, loxP-Ptefl::PGM2.*

RN1135 and RN1159 are transformed with plasmids using the 'Gietz method' (Gietz et al., 1992, Nucleic Acids Res. 20:1425) as indicated in Table 3. Primary selection of transformants is done on mineral medium (YNB + 2% glucose) using antibiotic selection as indicated in Table 4. Table 2 summarizes the strains thus obtained and the enzymes expressed therein.

Table 2

| Enzyme | phosphoglucomutase | Galactose-1-phosphate uridyl transferase | UDP-glucose-4-epimerase | UDP-glucose pyrophosphorylase | UDP-glucose 6-dehydrogenase | | | UTP-monosaccharide-1-phosphate uridyltransferase | Glucuro-kinase | Galacturo-kinase Y250F | UDP-glucuronate4-epimerase | host | Substrate |
|---|---|---|---|---|---|---|---|---|---|---|---|---|---|
| | *S. cerevisiae* endogenous enzymes | | | | Enzymes heterologous to *S. cerevisiae* | | | | | | | | |
| EC number | 5.4.2.2 | 2.7.7.12 | 5.1.3.2 | 2.7.7.9 | 1.1.1.22 | | | 2.7.7.64 | 2.7.1.43 | 2.7.1.44 | 5.1.3.6 | | |
| strain | PGM2 | GAL7 | GAL10 | UGP1 | PA2022 | | PA3559 | | | | | | |
| RN1070 | wt | wt | wt | wt | - | - | - | - | - | - | - | | Xylose |
| RN1135 | constitutive | wt | wt | wt | - | - | - | - | - | - | - | RN1070 | Xylose |
| RN1136 | constitutive | wt | wt | wt | X anb1 | - | - | X | X | - | - | RN1135 | Glucuronate |
| RN1137 | constitutive | wt | wt | wt | - | - | X anb1 | X | X | - | - | RN1135 | Glucuronate |
| RN1138 | constitutive | wt | wt | wt | - | X adh1 | - | X | X | - | - | RN1135 | Glucuronate |
| RN1139 | constitutive | wt | wt | constitutive | - | X adh1 | - | X | X | - | - | RN1135 | Glucuronate |
| RN1140 | constitutive | wt | wt | constitutive | X anb1 | - | - | X | - | X | X | RN1135 | Galacturonate |
| RN1141 | constitutive | constitutive | wt | constitutive | X anb1 | - | - | X | - | X | - | RN1135 | Galacturonate |
| RN1142 | constitutive | wt | constitutive | wt | X anb1 | - | - | X | - | X | - | RN1135 | Galacturonate |
| RN1143 | constitutive | wt | constitutive | constitutive | X anb1 | - | - | X | - | X | - | RN1135 | Galacturonate |
| RN1100 | wt | wt | wt | wt | - | - | - | - | - | - | - | | Arabinose |
| RN1159 | constitutive | wt | wt | wt | - | - | - | - | - | - | - | RN1100 | Arabinose |
| RN1160 | constitutive | wt | wt | wt | X anb1 | - | - | X | X | - | - | RN1159 | Glucuronate |
| RN1161 | constitutive | wt | wt | wt | - | - | X anb1 | X | X | - | - | RN1159 | Glucuronate |
| RN1162 | constitutive | wt | wt | constitutive | X anb1 | - | - | X | - | X | X | RN1159 | Galacturonate |
| RN1163 | constitutive | wt | constitutive | constitutive | X anb1 | - | - | X | - | X | - | RN1159 | Galacturonate |

Table 3

| | pRN761 | pRN830 | pRN831 | pRN832 | pRN833 | pRN834 | pRN835 | pRN905 | pRN906 | pRN907 | pRN914 | basic fermentation |
|---|---|---|---|---|---|---|---|---|---|---|---|---|
| RN1070 | - | - | - | - | - | - | - | - | - | - | - | Xylose |
| RN1135 | - | - | - | - | - | - | - | - | - | - | X | Xylose |
| R1136 | - | X | - | - | X | - | - | - | - | - | X | Xylose |
| RN1137 | - | - | - | - | X | - | - | - | X | - | X | Xylose |
| RN1138 | - | - | X | - | X | | - | - | - | - | X | Xylose |
| RN1139 | - | - | X | - | | X | - | - | - | - | X | Xylose |
| RN1140 | X | X | - | - | - | - | - | - | - | - | X | Xylose |
| RN1141 | - | X | - | - | - | - | X | - | - | X | X | Xylose |
| RN1142 | - | X | - | X | - | - | - | X | - | - | X | Xylose |
| RN1143 | - | X | - | - | - | - | - | X | - | X | X | Xylose |
| RN1100 | - | - | - | - | - | - | - | - | - | - | - | Arabinose |
| RN1159 | - | - | - | - | - | - | - | - | - | - | X | Arabinose |
| RN1160 | - | X | - | - | - | X | - | - | - | - | X | Arabinose |
| RN1161 | - | - | - | - | X | - | - | - | X | - | X | Arabinose |
| RN1162 | X | X | - | - | - | - | - | X | - | - | X | Arabinose |
| RN1163 | - | X | - | - | - | - | - | X | - | X | X | Arabinose |

Table 4

| | 1.1.1.22 | | 2.7.7.64 | 2.7.1.43 | 2.7.1.44 | 5.1.3.6 | 2.7.7.9 | 2.7.7.12 | 5.1.3.2 | 5.4.2.2 | | |
|---|---|---|---|---|---|---|---|---|---|---|---|---|
| | PA2022 | PA3559 | | | | | UGP1 | GAL7 | GAL10 | PGM2 | | |
| pRN761 | | | | | X | X | | | | | pRN600 | hphMX |
| pRN830 | x (prom anb1) | | X | | | | | | | | pRN599 | kanMX |
| pRN831 | x (prom adh1) | | X | | | | | | | | pRN599 | kanMX |
| pRN832 | | | | | X | | | | | | pRN600 | hphMX |
| pRN833 | | | | X | | | | | | | pRN600 | hphMX |
| pRN834 | | | | X | | | X | | | | pRN600 | hphMX |
| pRN835 | | | | | | | | X | | | tefl GAL7 | natMX |
| pRN905 | | | | | | | | | X | | tefl GAL10 | natMX |
| pRN906 | | X (prom anb1) | X | | | | | | | | pRN599 | kanMX |
| pRN907 | | | | | X | | X | | | | pRN600 | hphMX |
| pRN914 | | | | | | | | | | X | tefl PGM2 | natMX |

Table 5 PCR primers used herein.

| | | |
|---|---|---|
| ugp1f | AACTGCAGAAAATGTCCACTAAGAAGCACACCA | SEQ ID NO.: 49 |
| ugp1r | TTGCGCGCTCAATGTTCCAAGATTTGCAAATTACCAGTAACGAC | SEQ ID NO.: 50 |
| gal7f | AATCTAGAAAATGACTGCTGAAGAATTTGATTTTTCTAGCC | SEQ ID NO.: 51 |
| gal7r | TTTCCGGATTCTAAGCACCAAGCTTGGCCATGTGG | SEQ ID NO.: 52 |
| Pgm2f | AATCTAGAAAAATGTCATTTCAAATTGAAACGGTTCCCACC | SEQ ID NO.: 53 |
| Pgm2r | TTTCCGGAACGAGTAATGGACCGT | SEQ ID NO.: 54 |

2. Fermentations with the constructed of yeast cells of the invention

2.1 Experimental set-up

Precultures of strains were prepared by inoculating a frozen glycerol stock culture of the yeast in an YP (Yeast extract Peptone) medium with addition of the sugar glucose (2.5% w/v) at 32 °C and pH 5.5. After 24 h incubation, cells were harvested by centrifugation and washed with cold distilled water ($dH_2O$). These cells were used to inoculate the fermenting cultures. Yeast inoculation used was 5 gram dry matter yeast per liter of fermentation medium.

Oxic growth experiments (oxygen present) were performed in a defined mineral medium to which either/or glucose, xylose, arabinose, galacturonate or glucuronate were added. Its composition was based on the description by Verduyn et al. (1992, Yeast 8:501-517) but the ammonium in the medium was replaced by 2.3 g/l of urea. To compensate for the reduced sulfate content of this media, 6.6 g of $K_2SO_4$ per liter was added (Luttik et al., 2000, J Bacteriol. 182: 7007-7013). The pH of the medium was set at pH = 7.0 by adding $K_2HPO_4$ and $KH_2PO_4$ resulting in 100 mM phosphate in the medium. Cell growth was assessed in Erlenmeyer flasks (100 ml with 25 ml medium) in the absence or presence of a carbon source. Flasks were incubated in a rotary shaker (200 rpm) at 32 °C. Cell growth was assessed in time by optical density (700nm) readings of the media. From the OD measurements the dry weight was calculated. For this a linear calibration curve between the optical density and dry weight yeast was used. The dry weight of yeast culture samples were determined using 0.45-μm-pore-size nitrocellulose filters (Millipore) and a microwave oven (Postma et al, 1989, Appl Environ Microbiol. 55:3214-3220).

Anoxic fermentations (no oxygen present) were performed by using a fermentation setup which can run six fermentations simultaneously. Anoxic batch fermentations were performed in YP medium to which either/or glucose, xylose, arabinose, galacturonate or glucuronate were added. The pH of the medium was set at pH = 7.0 by adding $K_2HPO_4$ and $KH_2PO_4$ resulting in 100 mM phosphate in the YP medium. The temperature during the fermentations was set at 32 °C. The working volumes of the fermentations used in this study were 200 ml.

Samples for analysis of glucose, xylose, arabinose, galacturonate, glucuronate and ethanol were taken after 72 hours of incubation. Ethanol concentrations were monitored by HPLC analysis. Sugars and uronates were determined by HPAEC (Dionex) analysis.

2.2 Results of both oxic and anoxic fermentations

Table 2 summarizes the strains used in the fermentations and the enzymes expressed therein.

Fermentation by strains RN1070 and RN1135 on either glucose (25 mM) or xylose (25 mM) added to the mineral medium, under the oxic conditions described above, leads to a full consumption of either glucose or xylose by these strains within 72 hours. Similarly, under anoxic conditions in the YP-medium, either glucose or xylose (both added individually at 25 mM) were also consumed to completion within 72 hours. The concentrations of ethanol in the oxic glucose and xylose fermentations were negligible, whereas in the anoxic fermentations after 72 hours ethanol had accumulated at 40 mM (for glucose) and 33 mM for xylose. The 2 strains were also tested for their ability to ferment both under oxic and anoxic conditions either glucuronate (25 mM) or galacturonate (25 mM), but no consumption of these uronic acids was detected.

Fermentation by strain RN1136, RN1137 and RN1138 under either oxic or anoxic conditions as described above for RN1070 and RN1135 on either glucose (25 mM), xylose (25 mM) or galacturonate (25 mM) gave similar results for these 5 strains. But oxic fermentation of glucuronate resulted in cell growth and production of ethanol. The final ethanol concentration after 72 hours of incubation was 12 mM for strains RN1136 and RN1137 and 9 mM for strain RN1138 while all glucuronate had been consumed by all three strains. Under the anoxic conditions described above, no consumption of glucuronate was observed by strains RN1136, RN1137 and RN1138. However, by including 100 mM 2,3-butanediol in the anoxic fermentation medium, the concentration of glucuronate had decreased from 25 to 12 mM (strains RN1136 and RN1137) or to 9 mM (strain RN1138) in 72 hours whereas 19 mM of ethanol had accumulated (19 mM for strains RN1136 and RN1137 and 21 mM for strain RN1138). In control experiments with 2,3-butanediol in the absence of glucuronate, no ethanol production was observed by either of these strains.

Fermentations by strain RN1139 under either oxic or anoxic conditions behaved very similarly to strain RN1138 with respect to the consumption pattern and product formation of glucose, xylose and glucuronate. However, the organism was more active than strain RN1138 in consuming glucuronate either under oxic or anoxic conditions. As a result, for strain RN1139 the oxic fermentation of glucuronate resulted in cell growth and production of ethanol with a final concentration of 5mM after 72 hours of incubation. All glucuronate had then been consumed. Under the anoxic conditions described above, no consumption of glucuronate was observed by strains RN1139, However, by including 100 mM 2,3-butanediol in the anoxic fermentation medium, the concentration of glucuronate had decreased from 25 to 6 mM in 72 hours whereas 23 mM of ethanol had accumulated. In control experiments with 2,3-butanediol in the absence of glucuronate, no ethanol production was observed by strain RN1139.

Fermentations as described above for RN1070, RN1135, RN1140, RN1141, RN1142 and RN1143 on either glucose (25 mM), xylose (25 mM) or glucuronate (25 mM) gave similar results for these 6 strains. Neither of the organisms was able to consume glucuronate at an appreciable rate, but oxic fermentation of galacturonate resulted in cell growth and production of ethanol by strains RN1140, RN1141, RN1142 and RN1143. RN1070 and RN1135 did not consume galacturonate. The final ethanol concentration after 72 hours of incubation was 4 mM for strain RN1140 and 8 mM of galacturonate had been consumed. For strains RN1141, RN1142 and RN1143 the ethanol concentrations after 72 hours of incubation were 1mM, 6 mM and 8 mM, respectively. For strains RN1141, RN1142 and RN1143 the values for galacturonate consumed after 72 hours of incubation were 7mM, 16 mM and 20 mM, respectively. Under the anoxic conditions described above, no consumption of galacturonate was observed by any of the six strains. However, by including 100 mM 2,3-butanediol in the anoxic fermentation medium, the concentration of galacturonate had decreased in the case of strains RN1140, RN1141, RN1142 and RN1143. Respective values for these 4 strains for remaining galacturonate were 21 mM, 18 mM, 16 mM and 14 mM. Respective ethanol concentrations were 5 mM, 8 mM, 14 mM and 16 mM after 72 hours of incubation. In control experiments with 2,3-butanediol in the absence of galacturonate, no ethanol production by strain RN1139 was observed.

Fermentation by strains RN1100 and RN1159 on either glucose (25 mM) or arabinose (25 mM) added to the mineral medium, under the oxic conditions described above, leads to a full consumption of either glucose or arabinose by these strains within 72 hours. Similarly, under anoxic conditions in the YP-medium, either glucose or arabinose (both added individually at 25 mM) were also consumed to completion within 72 hours. The concentrations of ethanol in the oxic glucose and arabinose fermentations were negligible, whereas in the anoxic fermentations after 72 hours ethanol had accumulated at 40 mM (for glucose) and 33 mM for arabinose The 2 strains were also tested for their ability to ferment both under oxic and anoxic conditions either glucuronate (25 mM) or galacturonate (25 mM), but no consumption of these uronic acids was detected.

Fermentation by strain RN1160 and RN1161 under either oxic or anoxic conditions as described above for RN1100 and RN1159 on either glucose (25 mM), arabinose (25 mM) or galacturonate (25 mM) gave similar results for these 4 strains. But oxic fermentation of glucuronate resulted in cell growth and production of ethanol in the case of strains RN1160 and RN1161. The final ethanol concentration after 72 hours of incubation was 12 mM for strain both of these strains while all glucuronate had been consumed. Under the anoxic conditions described above, no consumption of glucuronate was observed by strains RN1160 and RN1161. However, by including 100 mM 2,3-butanediol in the anoxic fermentation medium, the concentration of glucuronate had decreased from 25 to 12 mM (both for strains RN1160 and RN1161) in 72 hours whereas both strains had accumulated 19 mM of ethanol. In control experiments with 2,3-butanediol in the absence of glucuronate, no ethanol production was observed by either of these strains.

Fermentations as described above for RN1100, RN1159, RN1162 and RN1163 on either glucose (25 mM), arabinose (25 mM) or glucuronate (25 mM) gave similar results for these 4 strains. Neither of the organisms was able to consume glucuronate at an appreciable rate, but oxic fermentation of galacturonate resulted in cell growth and production of ethanol by strains RN1162 and RN1163. RN1100 and RN1159 did not consume galacturonate.. The final ethanol concentration after 72 hours of incubation was 4 mM for strain RN1162 and 8 mM of galacturonate had been consumed. For strains RN1163 the ethanol concentrations after 72 hours of incubation was 6 mM and 16 mM of galacturonate had been consumed. Under the anoxic conditions described above, no consumption of galacturonate was observed by any of these four strains tested. However, by including 100 mM 2,3-butanediol in the anoxic fermentation medium, the concentration of galacturonate had decreased in the case of strain RN1162 to 19 mM and to 14 mM for strain RN1163. Respective ethanol concentrations were 8 mM, and 17 mM after these 72 hours of incubation. In control experiments with 2,3-butanediol in the absence of galacturonate, no ethanol production by strains RN1162 or RN1163 was observed.

## Claims

**1.** A yeast cell having the ability of metabolizing a uronic acid, the cell comprising genes coding for:

a) a uronokinase (EC 2.7.1.43 and/or EC 2.7.1.44);
b) a UTP-monosaccharide-1-phosphate uridylyltransferase (EC 2.7.7.64); and,
c) a UDP-glucose 6-dehydrogenase (EC 1.1.1.22).

**2.** A yeast cell according to claim 1, wherein the cell further comprises a gene coding for:

d) a UDP-glucuronate-4-epimerase (EC 5.1.3.6).

**3.** A yeast cell according to claim 1 or 2, wherein the cell further expresses genes coding for at least one of:

i) a galactose-1-phosphate uridyltransferase (EC 2.7.7.12); and
ii) a UDP-glucose-4-epimerase (EC 5.1.3.2), and wherein preferably the galactose-1-phosphate uridyltransferase and the UDP-glucose-4-epimerase are expressed in a galactose-independent manner.

**4.** A yeast cell according to any of the preceding claims, wherein the cell further comprises a genetic modification that increases the specific activity of at least one of a UDP-glucose pyrophosphorylase (EC 2.7.7.9) and a phosphoglucomutase (EC 5.4.2.2), wherein preferably the phosphoglucomutase is expressed in a galactose-independent manner.

**5.** A yeast cell according to any of the preceding claims, wherein the yeast cell further comprises a genetic modification that increases at least one of

i) the specific activity of glycerol dehydrogenase;
ii) the specific activity of dihydroxyacetone kinase; and,
iii) transport of glycerol into the cell.

**6.** A yeast cell according to any of the preceding claims, wherein the yeast cell further comprises a genetic modification that inactivates or reduces the specific activity of one or more enzymes required for NADH-dependent synthesis of glycerol, compared to a corresponding wild-type cell.

**7.** A yeast cell according to any of the preceding claims, where the yeast cell further comprises genes coding for enzymes with at least one of the abilities to:

i) directly isomerise xylose into xylulose (EC 5.3.1.5); and,
ii) convert L-arabinose into D-xylulose 5-phosphate, wherein the enzymes comprise a L-arabinose isomerase (EC 5.3.1.3), a L-ribulokinase (EC 2.7.1.16) and a L-ribulose-5-phosphate 4-epimerase (EC 5.1.3.4).

**8.** A yeast cell according to any of the preceding claims, wherein the cell is a yeast cell selected from the genera *Saccharomyces*, *Kluyveromyces*, *Candida*, *Pichia*, *Schizosaccharomyces*, *Hansenula*, *Kloeckera*, *Schwanniomyces*, *Brettanomyces*, and *Yarrowia,* in particular the cell is a yeast cell selected from the species *S. cerevisiae*, *S. exiguus*, *S. bayanus*, *K. lactis, K. marxianus* and *Schizosaccharomyces pombe.*

**9.** Use of a yeast cell according to any of the preceding claims for the preparation of a fermentation product, wherein preferably the fermentation product selected from the group consisting of ethanol, lactic acid, 3-hydroxy-propionic acid, acrylic acid, 1,3-propane-diol, butanols and isoprenoid-derived products.

**10.** A process for producing a fermentation product, whereby the process comprises the steps of:

a) fermenting a medium with a yeast cell as defined in any one of claim 1-8, whereby the medium contains or is fed with a source of a uronic acid and whereby the yeast cell ferments the uronic acid to the fermentation product; and optionally,
b) recovery of the fermentation product,

wherein preferably the fermentation product selected from the group consisting of ethanol, lactic acid, 3-hydroxy-propionic acid, acrylic acid, 1,3-propane-diol, a butanol and an isoprenoid-derived product.

**11.** A process according to claim 10, wherein the uronic acid is at least one of galacturonic acid and glucuronic acid.

**12.** A process according to claim 10 or 11, wherein the medium further contains or is fed with a carbon source having a more reduced state than glucose.

**13.** A process according to any one of the claims 10 - 12, wherein the medium contains or is fed with a source of at least one of a hexose, a pentose, and acetic acid.

**14.** A process according to any one of the claims 10 - 13, wherein the yeast cell ferments under anaerobic conditions or under oxygen-limited aerobic conditions.

**15.** A process according to any one of the claims 10 - 14, wherein the fermentation product is ethanol.

Fig 1

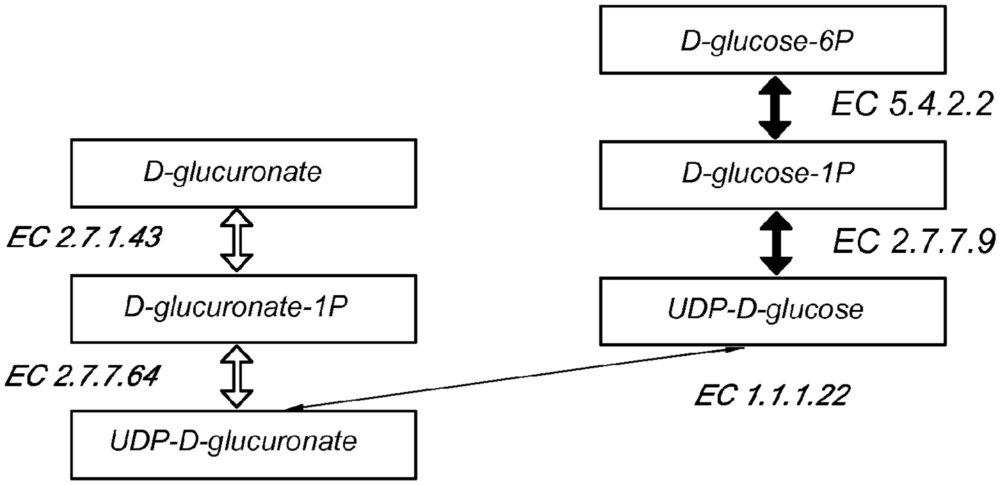
D-glucose-6P
EC 5.4.2.2
D-glucuronate
D-glucose-1P
EC 2.7.1.43
EC 2.7.7.9
D-glucuronate-1P
UDP-D-glucose
EC 2.7.7.64
EC 1.1.1.22
UDP-D-glucuronate

Fig 2

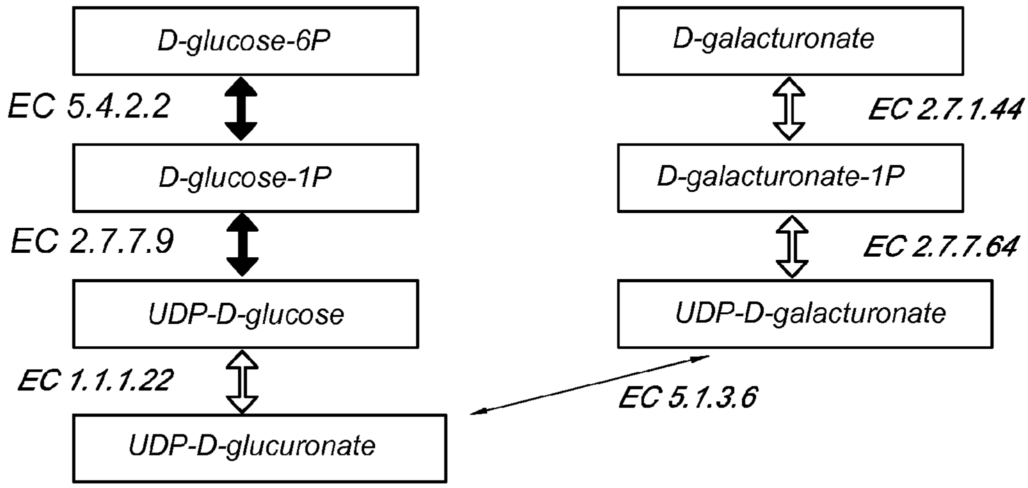
D-glucose-6P
D-galacturonate
EC 5.4.2.2
EC 2.7.1.44
D-glucose-1P
D-galacturonate-1P
EC 2.7.7.9
EC 2.7.7.64
UDP-D-glucose
UDP-D-galacturonate
EC 1.1.1.22
EC 5.1.3.6
UDP-D-glucuronate

Fig 3

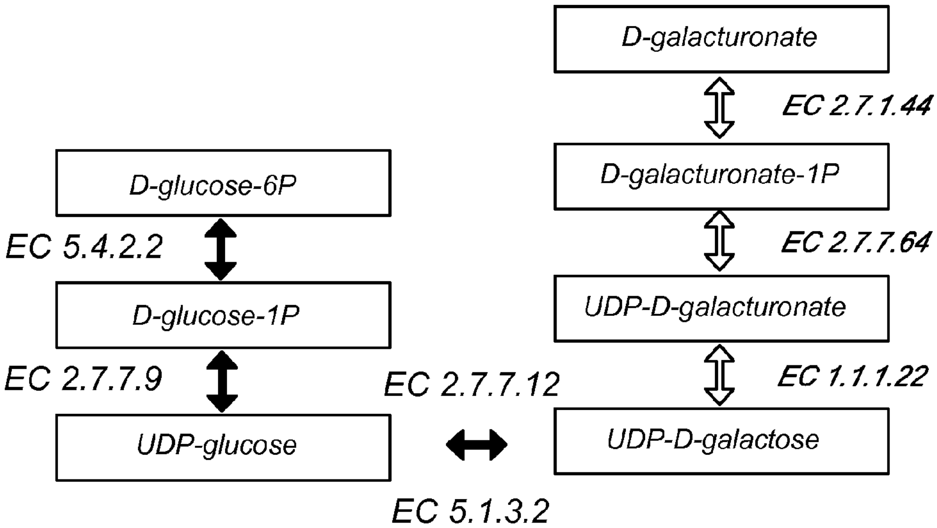
D-galacturonate
EC 2.7.1.44
D-galacturonate-1P
EC 2.7.7.64
UDP-D-galacturonate
EC 1.1.1.22
UDP-D-galactose
D-glucose-6P
EC 5.4.2.2
D-glucose-1P
EC 2.7.7.9
UDP-glucose
EC 2.7.7.12
EC 5.1.3.2

Europäisches Patentamt
European Patent Office
Office européen des brevets

# EUROPEAN SEARCH REPORT

Application Number
EP 11 17 3470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ANTONIUS J A VAN MARIS ET AL: "Alcoholic fermentation of carbon sources in biomass hydrolysates by Saccharomyces cerevisiae: current status", ANTONIE VAN LEEUWENHOEK, KLUWER ACADEMIC PUBLISHERS, DO, vol. 90, no. 4, 11 October 2006 (2006-10-11), pages 391-418, XP019446684, ISSN: 1572-9699, DOI: 10.1007/S10482-006-9085-7 * page 407; figure 5 * ----- | 1-15 | INV. C12N9/02 C12N9/12 C12N9/90 C12P1/02 C12P7/06 |
| Y,D | RICHARD P ET AL: "d-Galacturonic acid catabolism in microorganisms and its biotechnological relevance", APPLIED MICROBIOLOGY AND BIOTECHNOLOGY, SPRINGER VERLAG, BERLIN, DE, vol. 82, no. 4, 1 March 2009 (2009-03-01), pages 597-604, XP002575770, ISSN: 0175-7598, DOI: 10.1007/S00253-009-1870-6 [retrieved on 2009-01-22] * page 603; figures 2-6 * ----- | 1-15 | TECHNICAL FIELDS SEARCHED (IPC) C12N C12P |
| Y | Toshihisa Kotake et al.: "Generation of nucleotide sugars for biomass formation in plants", plant biotechnology, 2010, XP55013839, Retrieved from the Internet: URL:http://www.wdc-jp.biz/pdf_store/jspcmb/pdf/pb27_3/27_231.pdf [retrieved on 2011-12-02] * page 233 * ----- -/-- | 1-15 | |

The present search report has been drawn up for all claims

3

| Place of search | Date of completion of the search | Examiner |
|---|---|---|
| Munich | 7 December 2011 | Espen, Josée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 17 3470

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | A. M. PIESLINGER ET AL: "Cloning of Glucuronokinase from Arabidopsis thaliana, the Last Missing Enzyme of the myo-Inositol Oxygenase Pathway to Nucleotide Sugars", JOURNAL OF BIOLOGICAL CHEMISTRY, vol. 285, no. 5, 29 January 2010 (2010-01-29), pages 2902-2910, XP55014095, ISSN: 0021-9258, DOI: 10.1074/jbc.M109.069369 * the whole document * ----- | 1-15 | |
| A | T. YANG ET AL: "Identification of Galacturonic Acid-1-phosphate Kinase, a New Member of the GHMP Kinase Superfamily in Plants, and Comparison with Galactose-1-phosphate Kinase", JOURNAL OF BIOLOGICAL CHEMISTRY, vol. 284, no. 32, 9 June 2009 (2009-06-09) , pages 21526-21535, XP55014094, ISSN: 0021-9258, DOI: 10.1074/jbc.M109.014761 * the whole document * ----- | 1-15 | TECHNICAL FIELDS SEARCHED (IPC) |
| A | T. KOTAKE: "UDP-sugar Pyrophosphorylase with Broad Substrate Specificity Toward Various Monosaccharide 1-Phosphates from Pea Sprouts", JOURNAL OF BIOLOGICAL CHEMISTRY, vol. 279, no. 44, 1 January 2004 (2004-01-01), pages 45728-45736, XP55014178, ISSN: 0021-9258, DOI: 10.1074/jbc.M408716200 * the whole document * ----- | 1-15 | |

The present search report has been drawn up for all claims

3

| Place of search | Date of completion of the search | Examiner |
|---|---|---|
| Munich | 7 December 2011 | Espen, Josée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 030624430 A
- WO 06009434 A
- WO 10074577 A
- US 20060234364 A
- WO 09109633 A
- WO 10070549 A
- WO 11006136 A
- EP 1499708 A
- WO 2009011591 A
- WO 1101092 A
- WO 9303159 A
- WO 2006009434 A
- WO 2005023998 A
- WO 2005111214 A
- WO 2005091733 A
- WO 2010074577 A


### Non-patent literature cited in the description


- **RICHARD ; HILDITCH.** *Appl Microbiol Biotechnol,* 2009, vol. 82, 597-604
- **HILDITCH.** Thesis. VTT publications, 2010, vol. 739
- **HENIKOFF ; HENIKOFF.** *PNAS,* 1992, vol. 89, 915-919
- **YANG et al.** *J. Biol Chem.,* 2009, vol. 284, 21526-21535
- **PIESLINGER et al.** *J. Biol Chem.,* 2010, vol. 285, 2902-2910
- **KOTAKE et al.** *J. Biol. Chem.,* 2004, vol. 279, 45728-45736
- **LITTERER et al.** *Plant Physiol. Biochem.,* 2006, vol. 44, 171-180
- **KOTAKE et al.** *Biosci. Biotechnol. Biochem.,* 2007, vol. 71, 761-771
- **HUNG et al.** *J. Biol. Chem.,* 2007, vol. 282, 17738-17748
- **CAMPBELL et al.** *Biochemistry,* 2000, vol. 39, 7012-7023
- **MUÑOZ et al.** *Mol. Microbiol.,* 1999, vol. 31, 703-713
- **MØLHØJ et al.** *Plant Physiol.,* 2004, vol. 135, 1221-1230
- **BRAT et al.** *Appl. Environ. Microbiol.,* 2009, vol. 75, 2304-2311
- **WISSELINK et al.** AEM Accepts, published online ahead of print on. *Appl. Environ. Microbiol.,* 01 June 2007
- **OECHSNER et al.** *FEBS Lett.,* 1988, vol. 238, 123-128
- **VOSS et al.** *Yeast,* 1997, vol. 13, 655-672
- **MOLIN et al.** *J. Biol. Chem.,* 2003, vol. 278, 1415-1423
- **NEVES et al.** *FEMS Yeast Res.,* 2004, vol. 5, 51-62
- **REIZER et al.** *CRC Crit. Rev. Biochem. Mol. Biol.,* 1993, vol. 28, 235-257
- **VAN AELST et al.** *EMBO J.,* 1991, vol. 10, 2095-2104
- **LUYTEN et al.** *EMBO J.,* 1995, vol. 14, 1360-1371
- **WAKS ; SILVER.** *Appl. Environ. Microbiol.,* 2009, vol. 75, 1867-1875
- **ERIKSSON et al.** *Mol. Microbiol.,* 1995, vol. 17, 95-107
- **ALBERTYN et al.** *Mol. Cell. Biol.,* 1994, vol. 14, 4135-4144
- **SAMBROOK ; RUSSELL.** Molecular cloning: a laboratory manual. Cold Spring Harbor Laboratory Press, 2001
- Current protocols in molecular biology. Green Publishing and Wiley Interscience, 1987
- **SHERMAN et al.** Methods Yeast Genetics. Cold Spring Harbor Laboratory, 1978
- Guide To Yeast Genetics and Molecular Biology. Academic Press, 1991, vol. 194
- **SHARP ; LI.** *Nucleic Acids Research,* 1987, vol. 15, 1281-1295
- **JANSEN et al.** *Nucleic Acids Res.,* 2003, vol. 31 (8), 2242-51
- **J.A. BARNETT ; R.W. PAYNE ; D. YARROW.** Yeasts: characteristics and identification. Cambridge University Press, 2000
- The yeasts, a taxonomic study. Elsevier Science Publ, 1998
- **DENG ; HO.** *Appl. Biochem. Biotechnol.,* 1990, vol. 24-25, 193-199
- **TRÄFF et al.** *Appl. Environm. Microbiol.,* 2001, vol. 67, 5668-5674
- **SEDLACK ; HO.** *Yeast,* 2004, vol. 21, 671-684

- **KATAHIRA et al.** *Enzyme Microb. Technol.,* 2008, vol. 43, 115-119
- **DIDERICH et al.** *Appl. Environ. Microbiol.,* 2001, vol. 67, 1587-1593
- **MARIS et al.** *Antonie van Leeuwenhoek,* 2006, vol. 90, 391-418
- **KASHYAP et al.** *Bioresour Technol.,* 2001, vol. 77, 215-227
- **HOONDAL et al.** *Appl Microbiol Biotechnol,* 2002, vol. 59, 409-418
- **BACIU ; JÖRDENING.** *Enzyme Microb. Technol.,* 2004, vol. 34, 505-512
- **JAYANI et al.** *Process Biochem,* 2005, vol. 40, 2931-2944
- **TEUNISSEN et al.** *Appl. Environ. Microbiol.,* 2002, vol. 68, 4780-4787
- **GIETZ et al.** *Nucleic Acids Res.,* 1992, vol. 20, 1425
- **VERDUYN et al.** *Yeast,* 1992, vol. 8, 501-517
- **LUTTIK et al.** *J Bacteriol.,* 2000, vol. 182, 7007-7013
- **POSTMA et al.** *Appl Environ Microbiol.,* 1989, vol. 55, 3214-3220